# EUROPEAN PATENT SPECIFICATION

(11) **EP 0 619 804 B1**
(45) Date of publication and mention of the grant of the patent: **22.05.1996**
(21) Application number: 93900687.0
(22) Date of filing: 25.11.1992
(51) Int. Cl.: C07C 233/15, C07C 233/59, C07C 271/28, C07C 233/07, C07C 275/30, C07D 333/36, A01N 37/18, A01N 47/00

(54) **HERBICIDAL ACYLATED AMINO-(PHENYL- OR PYRIDINYL- OR THIENYL-)-PHENYL DERIVATIVES**
HERBIZIDE ACYLIERTE AMINO-(PHENYL-ODER-PYRIDINYL-ODER THIENYL-)PHENYL DERIVATE
DERIVES HERBICIDES A BASE D'AMINO-(PHENYLE- OU PYRIDINYLE- OU THIENYLE-)-PHENYLE ACYLES

(30) Priority: 27.11.1991 US 800771; 16.04.1992 US 869569
(43) Date of publication of application: 19.10.1994
(73) Proprietor: E.I. DU PONT DE NEMOURS AND COMPANY, Wilmington Delaware 19898 (US)
(72) Inventor: DENES, Lucian, Radu, Hockessin, DE 19707 (US)
(74) Representative: Hildyard, Edward Martin
(86) International application number: US9210227
(87) International publication number: WO9311097

(56) References cited:
- EP-A- 0 118 093
- US-A- 3 149 032
- J. ORG. CHEM., 46(8), 1643-6 1981, STASKUN, BENJAMIN 'Empirical quantitative correlation of substituent size with proton nuclear magnetic resonance chemical shift difference between nonequivalent geminal methylene protons of hindered N-substituted acetanilides and difluorooxyboranes'
- ACTA CHEM. SCAND., SER. B, B33(5), 352-8 1979, CHRISTENSEN, LEIF ET AL. 'Electrolytically generated nucleophiles. V. Reductive acetylation of some aliphatic and aromatic nitro and nitroso compounds'
- J. ORG. CHEM., 31(11), 3719-25 1966, SIDDALL, THOMAS H., III 'Proton nonequivalence in N-substituted amides and related compounds'
- CHEMICAL ABSTRACTS, vol. 95 Columbus, Ohio, US; abstract no. 61643, KOST, A. N. ET AL. 'New synthesis of 2-aminobiphenyls'

## Description

This invention relates to certain substituted phenylamine compounds which are useful as herbicides and their agriculturally suitable compositions as well as methods for their use as general or selective preemergent or postemergent herbicides or as plant growth regulants.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. Examples of areas in which complete control of all vegetation is desired are areas around railroad tracks, storage tanks and industrial storage areas. There are many products commercially available for these purposes, but the search continues for products which are more effective, less costly and environmentally safe.

J. Org. Chem., 46, 1643-1646 (1981); Acta Chemica Scandinavia B, 33, 352-358 (1979); J. Org. Chem., 31, 3719-3725 (1966), and Chem. Abstr. 95:61644v disclose compounds wherein the phenyl groups are unsubstituted. There is no mention of herbicidal utility. There is no disclosure of compounds of the present invention.

EP(A)118,093 discloses certain biphenyl compounds wherein the phenyl rings are unsubstituted. Herbicidal utility is not disclosed. There is disclosure of agricultural utility as a fungicide. There is no disclosure of compounds of the present invention.

J. Org. Chem. 28, 1759-62 (1963) and J. Amer. Chem. Soc., 61, 3487-89 (1939) disclose biphenyl compounds which only may have the substituents F or Br substituted on one phenyl ring. There is no disclosure of compounds of the present invention.

### SUMMARY OF THE INVENTION

The invention comprises a method for controlling the growth of undesired vegetation by the preemergent and/or postemergent application to the locus to be protected of an effective amount of compounds of Formula I: 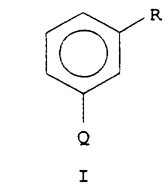 wherein
- Q: is 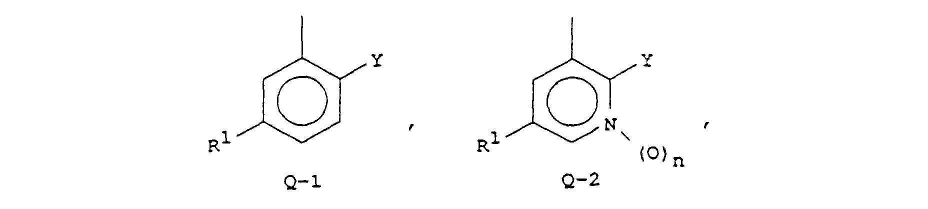 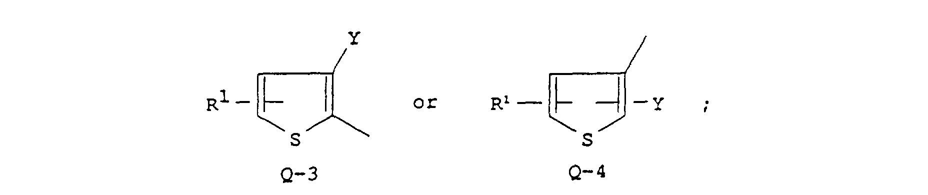
- R: is H, C₁-C₂ alkyl, C₁-C₂ haloalkyl, C₁-C₂ alkoxy, C₁-C₂ haloalkoxy, C₁-C₂ haloalkylthio, halogen, CN or NO₂;
- Y: is NR⁷C(O)XR³;
- X: is a single bond, O, S or NR⁴;
- R¹: is H, C₁-C₃ alkyl, C₁-C₃ alkoxy, C₁-C₃ haloalkoxy, C₁-C₃ alkylthio, C₂-C₃ alkynyl, C₂-C₃ alkoxyalkyl, C₂-C₃ alkylthioalkyl, halogen, NO₂, CN, NHR⁵ or NR⁵R⁶;
- R³: is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's. or 1-2 Cl's; C₂-C₄ alkenyl; C₂-C₄ haloalkenyl; C₃-C₄ alkynyl; C(O) (C₁-C₂ alkyl); benzyl; or a 5- or 6-membered heterocyclic ring containing one heteroatom selected from O, N and S, each ring optionally substituted with halogen or CH₃;
- R⁴: is H, C₁-C₂ alkyl or OCH₃;
- R⁵ and R⁶: are independently C₁-C₂ alkyl;
- R⁷: is H, C₁-C₂ alkyl or C(O)R⁸;
- R⁸: is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O)(C₁-C₂ alkyl); benzyl; or a 5- or 6-membered heterocyclic ring containing one heteroatom selected from O, N and S, each ring optionally substituted with halogen or CH₃; and
- n: is 0 or 1;
provided that when R³ is C₂ alkenyl, C₂ haloalkenyl or a heterocyclic ring then X is a single bond.

In the above definitions, the term "alkyl", used either alone or in compound words such as "alkylthio" or "haloalkyl", includes straight chain or branched alkyl, e.g., methyl, ethyl, n-propyl, isopropyl or the different butyl isomers. Alkoxy includes methoxy, ethoxy, n-propyloxy, isopropyloxy and the different butoxy isomers. Alkenyl includes straight chain or branched alkenes, e.g., 1-propenyl, 2-propenyl, 3-propenyl and the different butenyl isomers. Cycloalkyl includes cyclopropyl, cyclobutyl, cyclopentyl and cyclohexyl. The term "halogen", either alone or in compound words such as "haloalkyl", means fluorine, chlorine, bromine or iodine. Further, when used in compound words such as "haloalkyl" said alkyl may be partially or fully substituted with halogen atoms, which may be the same or different. Examples of haloalkyl include CH₂CH₂F, CF₂CF₃ and CH₂CHFCl. Representative exemplification of heterocyclic rings includes but is not limited to pyrrole, furan, thiophene and pyridine.

The preferred methods of use of the invention for reasons including ease of synthesis and/or greater herbicidal efficacy involve:
1. Compounds of Formula I wherein R is C₁-C₂ haloalkyl, C₁-C₂ haloalkoxy, C₁-C₂ haloalkylthio, halogen or CN;
2. Compounds of Preferred 1 wherein R¹ is C₁-C₂ alkyl, C₁-C₂ alkoxy, C₁-C₂ alkylthio, C₂-C₃ alkoxyalkyl, C₂-C₃ alkylthioalkyl, Cl, Br, CN, NHR⁵ or NR⁵R⁶; and R⁷ is H;
3. Compounds of Preferred 2 wherein
   Q is Q-1 or Q-2; and
   R³ is C₁-C₄ alkyl optionally substituted with C₁-C₂ alkoxy; CH₂ (C₃-C₄ cycloalkyl); C₃-C₄ cycloalkyl optionally substituted with 1-3 CH₃'s; or C₂-C₄ alkenyl;
4. Compounds of Preferred 3 wherein R is C₁-C₂ fluoroalkyl, C₁-C₂ fluoroalkoxy, C₁-C₂ fluoroalkylthio, Cl, Br or CN;

Specifically preferred methods for reasons of greatest ease of synthesis and/or greatest herbicidal efficacy are the method of Preferred 4 involving 1-methylethyl [5-methyl-3'-(trifluoromethyl) [1,1'-biphenyl]-2-yl]carbamate and 3-methyl-N-[5-methyl-3'-(trifluoromethyl) [1,1'-biphenyl]-2-yl]butanamide.

The invention also comprises novel compounds of Formula I and agriculturally suitable compositions containing them. 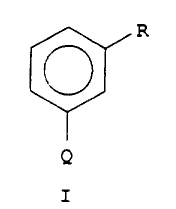 wherein
- Q: is 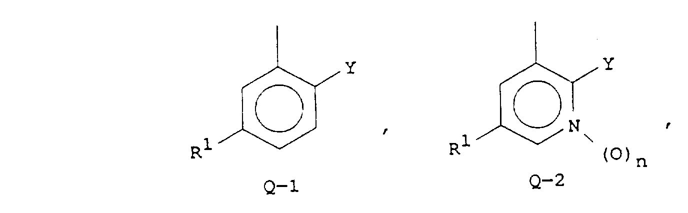 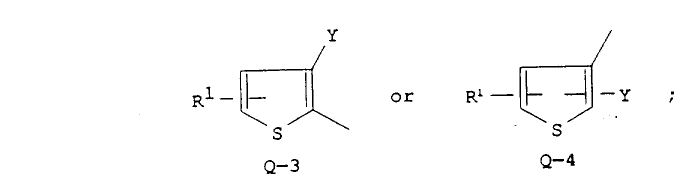
- R: is C₁-C₂ alkyl, C₁-C₂ haloalkyl, C₁-C₂ alkoxy, C₁-C₂ haloalkoxy, C₁-C₂ haloalkylthio, halogen, CN or NO₂;
- Y: is NR⁷C(O)XR³;
- X: is a single bond, O, S or NR⁴;
- R¹: is H, C₁-C₃ alkyl, C₁-C₃ alkoxy, C₁-C₃ haloalkoxy, C₁-C₃ alkylthio, C₂-C₃ alkoxyalkyl, C₂-C₃ alkylthioalkyl, halogen, NO₂, CN, NHR⁵ or NR⁵R⁶;
- R³: is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O) (C₁-C₂ alkyl); benzyl; or a 5- or 6-membered heterocyclic ring containing one heteroatom selected from O, N and S, each ring optionally substituted with halogen or CH₃;
- R⁴: is H, C₁-C₂ alkyl or OCH₃;
- R⁵ and R⁶: are independently C₁-C₂ alkyl; and
- R⁷: is H, C₁-C₂ alkyl or C(O)R⁸;
- R⁸: is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O) (C₁-C₂ alkyl); benzyl; or a 5- or 6-membered heterocyclic ring containing one heteroatom selected from O, N and S, each ring optionally substituted with halogen or CH₃;
- n: is 0 or 1;
provided that
(a) when R³ is C₂ alkenyl, C₂ haloalkenyl or a heterocyclic ring then X is a single bond and
(b) when Q is Q-1, R¹ is H and Y is NHC(O)CH₃ then R is other than Br or F.

The preferred compounds and compositions of the invention are the compounds and compositions involving the compounds set forth above in the preferred methods of use.

### DETAILED DESCRIPTION OF THE INVENTION

Compounds of General Formula I can be readily prepared by one skilled in the art by using the reactions and techniques described in Schemes 1-10 of this section as well as by following the specific procedures given in Examples 1-22.

Scheme 1 illustrates the preparation of compounds of General Formula I (where R, R¹ and Y are defined as above) whereby aromatic compounds of General Formula IV where X¹ is bromine, iodine or trifluoromethylsulfonyloxy (OTf) can be coupled with substituted aryl compounds of Formula III₁₋₅ where X is a trialkyltin (e.g., Me₃Sn), trialkylsilyl (e.g., Me₃Si), or boronic acid (e.g., B(OH)₂) moiety. The coupling is carried out by methods known in the art: for example, see Tsuji, J., *Organic Synthesis with Palladium Compounds,* Springer-Verlag, Berlin **1980**; Negishi, E., Acc. *Chem. Res.* **1982**, 15, 340; Stille, J. K., *Angew. Chem.* **1986**, 98, 504; Yamamoto, A. and Yamagi, A., *Chem. Pharm. Bull.* **1982**, 30, 1731 and 2003; Dondoni et al., *Synthesis* **1987**, 185; Dondoni et al., *Synthesis* **1987**, 693; Hoshino et al., *Bull. Chem. Soc. Jpn.* **1988**, 61, 3008; Sato, M. et al., *Chem. Lett.* **1989**, 1405; Miyaura et al., *Synthetic Commun.* **1981**, 11, 513; Siddiqui and Sniekus, *Tetrahedron Lett.* **1988**, 29, 5463; *Sharp* at al., *Tetrahedron Lett.* **1987**, 28, 5093; Hatanaka et al., **Chem. Lett. 1989**, 1711; Bailey, T. R., *Tetrahedron Lett.* **1986**, 27, 4407 and Echavarren, A. M. and Stille, J. K., *J. Am. Chem. Soc.* **1987**, 109, 5478. The coupling of IV ad III₁₋₅ is carried out by heating in the presence of a transition metal catalyst such as tetrakis(triphenylphosphine) palladium(0) or bis(triphenylphosphine) palladium(II) dichloride in a solvent such as toluene, acetonitrile, glyme, or tetrahydrofuran. As shown in Scheme 1, compounds of General Formula I can also be prepared by coupling aromatic compounds of General Formula IV where X¹ is a trialkyltin (e.g., Me₃Sn), trialkylsilyl (e.g., Me₃Si) or boronic acid (e.g., B(OH)₂) moiety with substituted aryl compounds of Formula III₁₋₅ where X is bromine, iodine, or OTf using the same conditions as described above.

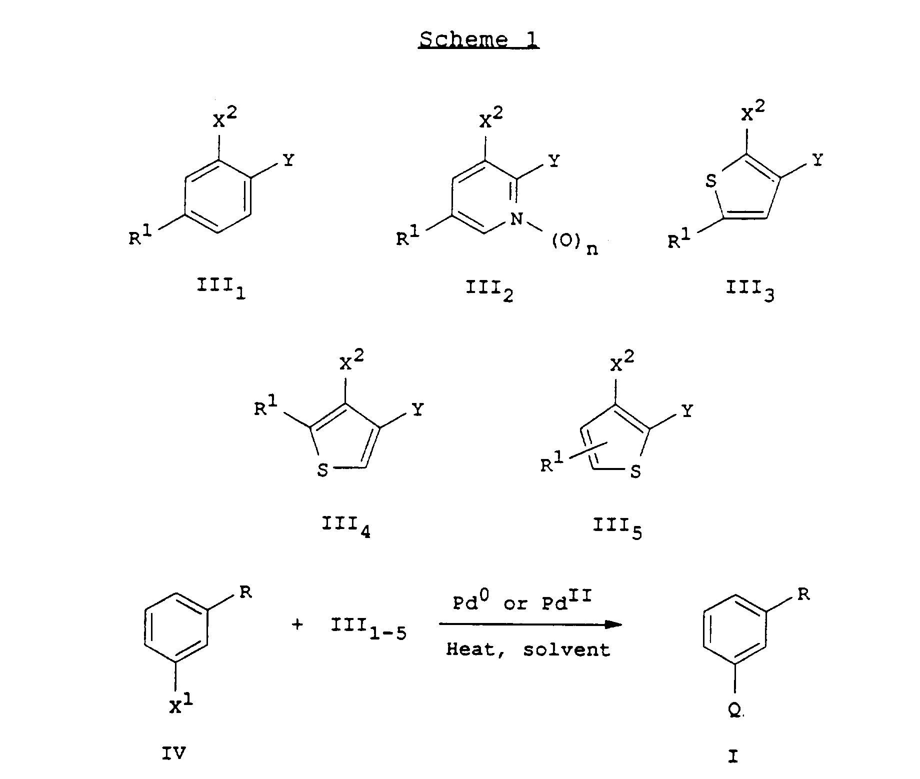

Substituted aryl compounds of General Formula IV and III₁₋₅ where X¹ and X are bromine, iodine or OTf are either known or readily prepared by procedures and techniques well known in the art, for example: Houben-Weil, *Methoden der Organische Chemie,* IV Edition, Eugen Muller, ed., Georg Thieme Verlag. By methods also reported in the above cited literature, treatment of compounds of General Formula IV and III₁₋₅ where X¹ is bromine or iodine with a base such as n-butyllithium followed by quench with a trialkyltin halide, trialkylsilyl halide, boron trichloride, or trialkyl borate gives compounds of General Formula IV and III₁₋₅ where X¹ and X respectively are a trialkyltin (e.g., Me₃sn), trialkylsilyl (e.g., Me₃Si), or boronic acid (e.g., B(OH)₂) moiety. Compounds of General Formula III₁₋₅ where X is a trialkyltin (e.g., Me₃Sn), trialkylsilyl (e.g., Me₃Si), or boronic acid (e.g., B(OH)₂) moiety can also be prepared from compounds of General Formula III₁₋₅ where X is hydrogen by treatment with a base such as n-butyllithium followed by quench with a trialkyltin halide, trialkylsilyl halide, or trialkyl borate as reported in the same literature references, providing that Y is an ortho-directing group known in the art (e.g., trimethylacetylamido): for example, Fuhrer, W., J. *Org. Chem.,* **1979**, 44, 1133.

The remaining schemes and examples illustrate the preparation of compounds of General Formula I wherein Q is Q-1, but can also be applied to Q-2 through Q-5.

Compounds of General Formula I can be readily prepared by one skilled in the art from intermediate compounds of General Formula I where Y is NH₂ or NHR⁷ by reaction with appropriate acyl chlorides, acid anhydrides, chloroformates, carbamyl chlorides, or isocyanates under conditions well known in the literature, for example: Sandler, R. S. and Karo, W., *Organic Functional Group Preparations,* 2nd Edition, *Vol. I,* 274; *Vol. II,* 152, 260, Academic Press. Schemes 2 and 3 and Examples 5-7 and 13 of this section illustrate examples of the preparation and reactions of intermediates of Formula II. Such intermediates are readily prepared by coupling of aromatic compounds of Formula IV where X¹ is bromine or iodine with substituted aryl compounds of Formula III₁₋₅ where Y is NHR⁷ and X is a trialkyltin (e.g., Me₃Sn), trialkylsilyl (e.g., Me₃Si), or boronic acid (e.g., B(OH)₂) moiety, or of aromatic compounds of Formula IV where X¹ is a trialkyltin (e.g., Me₃Sn), trialkylsilyl (e.g., Me₃si), or boronic acid (e.g., B(OH)₂) moiety with substituted aryl compounds of Formula III₁₋₅ where Y is NHR⁴ and X is bromine or iodine under the same conditions as described above.

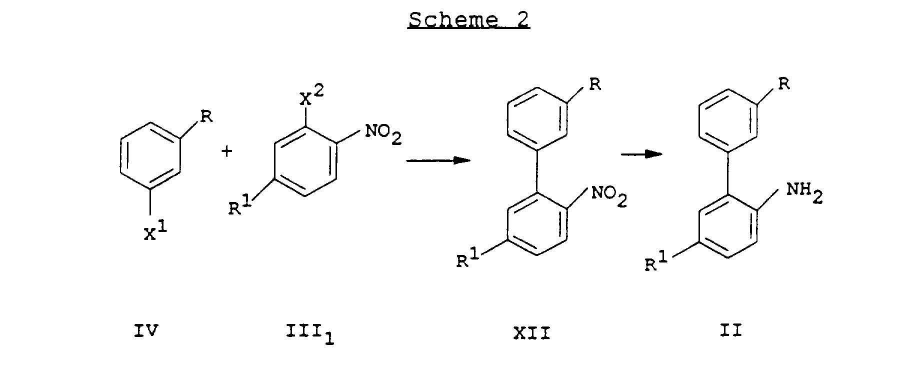

Intermediate compounds of General Formula II can also be prepared, as illustrated in Scheme 2 and Examples 16 and 17 of this section, by palladium catalyzed coupling of aromatic compounds of General Formula IV where X¹ is a trialkyltin (e.g., Me₃Sn), trialkylsilyl (e.g., Me₃Si), or boronic acid (e.g., B(OH)₂) moiety with substituted aryl compounds of Formula III₁₋₅ where X is bromine, iodine or OTf and Y is nitro, using the same conditions as described above, and then catalytically or chemically reducing the nitro group in the intermediate compounds of General Formula XII. Such reduction of nitro to amino groups is well documented in the chemical literature. See for example, Ohme, R. and Zubek, A. R. and Zubek, A. in *Preparative Organic Chemistry,* 557, Hilgetag, G. and Martini, A., Eds., John Wiley & Sons, New York, **1972**.

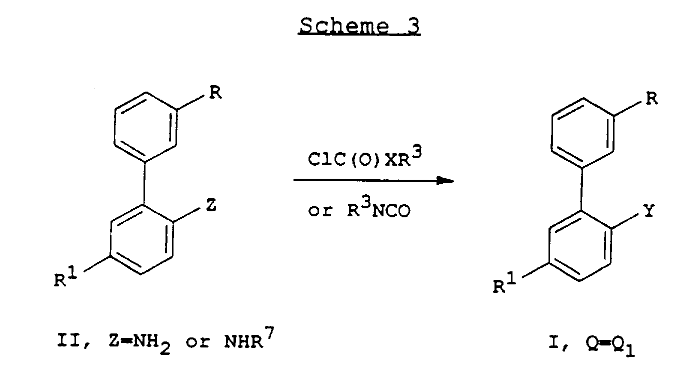

Alternatively, intermediate compounds of General Formula II where Z is NHR⁷ can be prepared from compounds of General Formula V, which contain an appropriately protected primary or secondary amino group, by hydrolysis, hydrogenolysis, or other adequate deprotection procedures, as shown in Scheme 4. Appropriate conditions for such reactions are extensively described in the literature, for example: Theodora W. Green, *Protective Groups in Organic Synthesis,* Chap. 7, John Wiley & Sons, New York, 1981.

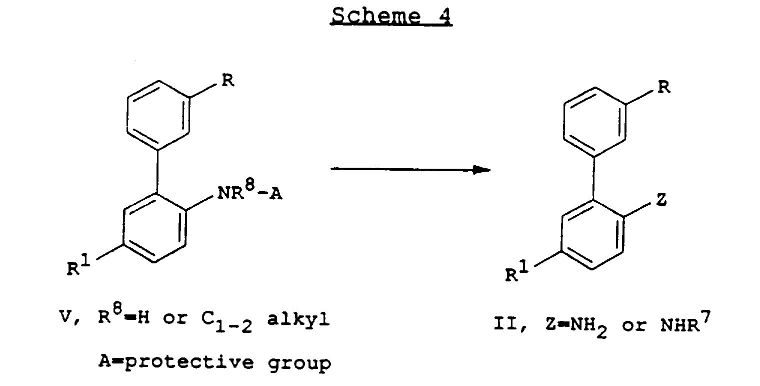

Compounds of General Formula VII containing a urethane or urea moiety can be prepared by one skilled in the art from intermediate compounds of General Formula VI containing an isocyanate moiety by reaction with appropriate alcohols or amines, respectively, as shown ir Scheme 5 and well documented in the literature: for example, Sandler, R. S. and Karo, W., *Organic Functional Group Preparations,* 2nd Edition, Vol. II, 152, 260, Academic Press.

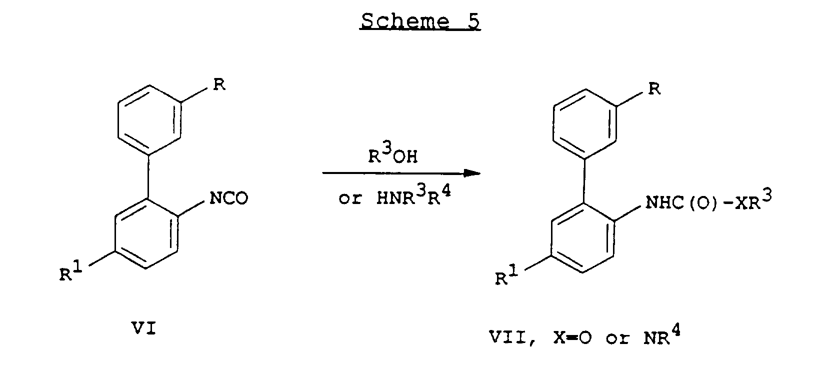

Such isocyano intermediates of Formula VI can be prepared from intermediate compounds of General Formula VIII by reaction with phosgene or a safer phosgene replacement (e.g., diphosgene or triphosgene) as illustrated in Scheme 6 and Example 4 of this section, using procedures and techniques known in the literature: for example, Lehman, G. and Teichman, H. in *Preparative Organic Chemistry,* 472, Hilgetag, G. and Martini, A., Eds., John Wiley & Sons, New York, 1972; Eckert, H. and Forster, B., *Angew. Chem. Int. Ed.,* **1987**, 26, 894; Babad, H. and Zeiler, A. G., *Chem.Rev.,* **1973**, 73, 75.

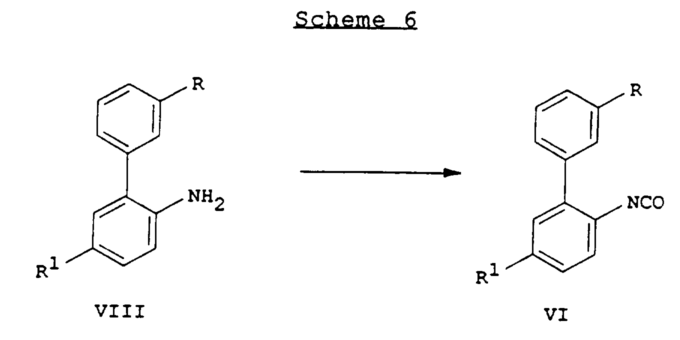

Compounds of General Formula X can be prepared from compounds of General Formula XI by reaction with the appropriate C₁ to C₂ alkyl halide, sulfate, or other adequate equivalent as described and illustrated in Scheme 7 and Example 15, using published procedures and techniques: for example, Lehman, G. and Teichman, H. in *Preparative Organic Chemistry,* 455-6, Hilgetag, G. and Martini, A., Eds., John Wiley & Sons, New York, **1972**; Pachter, I. J. and Kloetzel, M. C., *J. Amer. Chem. Soc.,* 1952, 74, 1321.

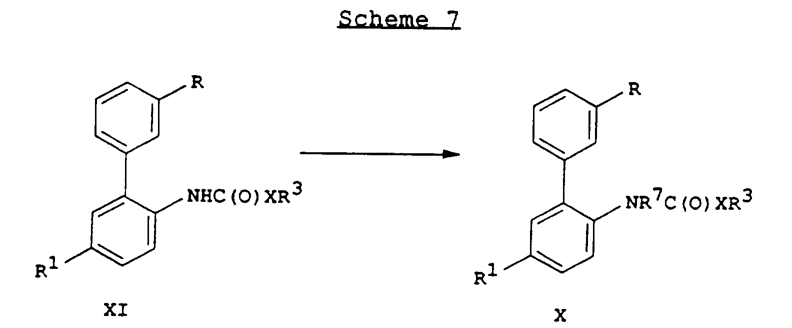

Compounds of General Formula XIII can be prepared from compounds of General Formula XII, where Z is a primary or secondary amine, amide, urethane, or urea by reaction with a halogen, nitric acid or nitric oxide under the appropriate conditions, as illustrated in Scheme 8 and Example 20 and thoroughly described in the chemical literature; for example see, Dorn, H. in *Preparative Organic Chemistry,* Hilgetag, G. and Martini, A., Eds., John Wiley & Sons: New York, **1972**, p. 150.

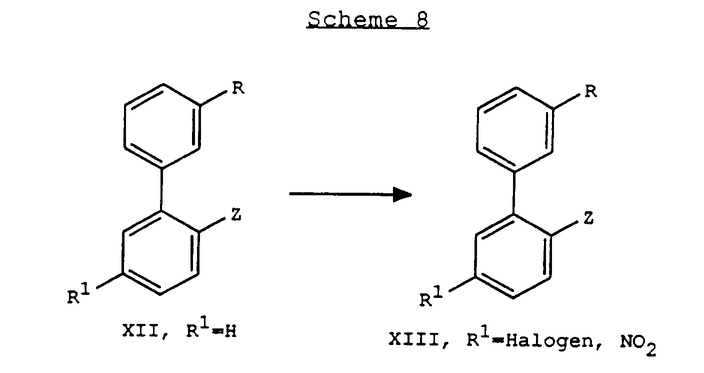

Compounds of General Formula XIII where R¹ is bromine or iodine, can be further elaborated into Compounds of General Formula XIV, as illustrated in Scheme 9 and Examples 20 and 21, under reaction conditions described in the chemical literature; for example see, Gross, H., Bischoff, Ch., Höft, E., and Gründemann, E. in *Preparative Organic Chemistry,* Hilgetag, G. and Martini, A., Eds., John Wiley & Sons: New York, **1972**, p. 923; Takahashi, S., Kuroyama, Y., Sonogashira, K., and Hagihara, N., *Synthesis,* **1980**, 627; De la Rosa, Martha A., Velarde, Esperanza, and Guzman, A.: *Synthetic Communications,* **1990**, *20,* 2059.

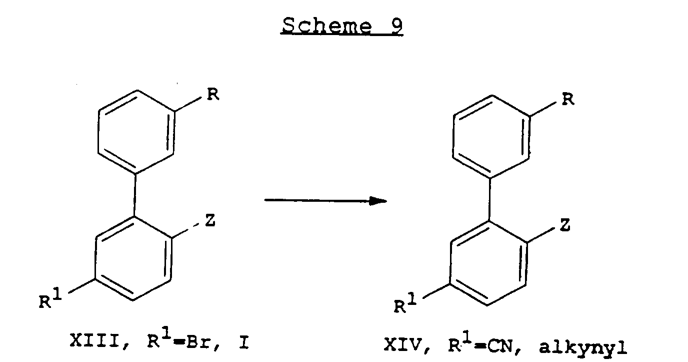

Compounds of General Formula XIII where R¹ is nitro can be also used to prepare Compounds of General Formula XV and Compounds of General Formula XVI, as illustrated in Scheme 10 using procedures and techniques known in the literature, for example, Lehmann, G. and Teichmann, H. in *Preparative Organic Chemistry,* Hilgetag, G. and Martini, A., Eds., John Wiley & Sons: New York, **1972**, p. 419.

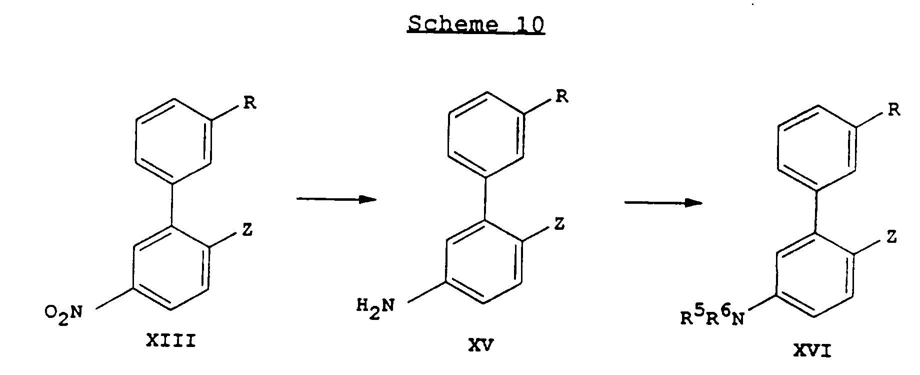

### EXAMPLE 1

### Preparation of 3-(trifluoromethyl)phenylboronic acid

A solution of 50 g (0.222 mol) meta-bromobenzotrifluoride in 175 ml dry ether was cooled at -10°C under nitrogen and 155 ml (0.24 mol) 1.6N n-butyllithium solution in hexanes was added dropwise during 1.5 hrs while maintaining the temperature below 0°C. After complete addition, the lithium salt solution was slowly transferred through a cannula during 30 min, under slight nitrogen pressure, into a solution of 170 g (0.888 mol) triisopropylborate in 150 ml anhydrous tetrahydrofuran at -70 to -60°C. Stirring was continued overnight while allowing the cold bath to melt. The reaction mixture was poured over 200 ml ice-cold 10% hydrochloric acid and stirred for 20 min. Phases were separated, organic phase washed twice with brine, dried and evaporated. The crude product was purified by triturating with hot hexane, to yield 35 g of pure product as white crystals, m.p. 155-6°C.

### EXAMPLE 2

### Preparation of 2,2-dimethyl-N-[5-methyl-3'-(trifluoromethyl)[1,1'-biphenyl]-2-yl]-propanamide

22.8 g (0.1 mol) 2-bromo-4-methyl-N-acetylaniline was dissolved under nitrogen in 30 ml glyme, 0.3 g palladium bis(triphenylphosphine) dichloride was added, and the mixture was stirred at the room temperature for 10 min., after which 20.9 g (0.11 mol) *meta*-trifluoromethylphenylboronic acid was added, followed by a solution of 25.2 g (0.3 mol) sodium bicarbonate in 300 ml water. The mixture was refluxed under nitrogen with good stirring for 2 hrs. After cooling to about 60°C, the reaction mixture was poured over 300 ml 1N sodium hydroxide solution and stirred at the room temperature for 20 min, then extracted with 250 ml ethyl acetate. Organic extracts were washed with brine, dried, and evaporated to dryness. The crude product was purified by chromatography on silica using 20:1 chlorobutane/ethyl acetate mixture as eluent to yield 26.5 g pure product as a white powder, m.p. 110-3°C.

¹H NMR (CDCl₃) : δ 1.98 (s, 3: COCH₃), 2.40 (s, 3: CH₃), 7.02-7.8 (7 aromatic protons, NH).

### EXAMPLE 3

### Preparation of 5-methyl-3'-(trifluoromethyl)[1,1'-biphenyl]-2-yl]-amine

8 g 2,2-Dimethyl-N-[5-methyl-3'-(trifluoromethyl)[1,1'-biphenyl]-2-yl]-propanamide was dissolved in 30 ml glacial acetic acid and the mixture warmed up to about 70°C and then 20 ml 15% hydrochloric acid solution was gradually added. After complete addition the reaction mixture was kept at reflux for 4 hrs. After cooling, volatiles were removed in vacuo, the residue was taken in 50 ml water, ice was added, the mixture was neutralized to pH 10 with 5% sodium hydroxide solution and extracted with ether. Organic extracts were washed with brine, dried, and evaporated to dryness. The crude product was purified by chromatography on silica using chlorobutane as eluent to yield 6.2 g pure product as a tan oil.

¹H NMR (CDCl₃): δ 2.24(s, 3: Me) 3.58(s, 2: NH₂) 6.6-7.8 (7 aromatic protons).

### EXAMPLE 4

### Preparation of 5-methyl-3'-(trifluoromethyl)[1,1'-biphenyl]-2-yl]-isocyanate

To a solution of 0.8 g triphosgene in 25 ml dry chloroform 2 g of 5-methyl-3'-(trifluoromethyl)[1,1'-biphenyl]-2-yl]-amine was added under nitrogen, followed by dropwise addition of 0.7 ml triethylamine. A mildly exothermal reaction occurred. After stirring overnight at the room temperature, aliquots of this reaction mixture were used for further reactions.

### EXAMPLE 5

### Preparation of 3-methyl-N-[5-methyl-3'-(trifluoromethyl)[1,1'-biphenyl]-2-yl]butanamide

To a solution of 2.51 g (0.01 mol) 5-methyl-3'-(trifluoromethyl)[1,1'-biphenyl]-2-yl]-amine in 20 ml dry chloroform 1.2 g (0.012 mol) triethylamine was added under nitrogen, followed by dropwise addition of 1.33 g (0.011 mol) 3-methylbutyryl chloride dissoluted in 5 ml dry chloroform. After stirring for 5 hrs at the room temperature, 5 ml water was added and stirring was continued for 20 min. The reaction mixture was diluted with 50 ml methylene chloride, washed with water, then saturated sodium bicarbonate solution and brine, dried, and evaporated to dryness. The crude product was purified by chromatography on silica using 20:1 chlorobutane/ethyl acetate mixture as eluent to yield 3.1 g pure product as a light tan powder, m.p. 89-91°C.

¹H NMR (CDCl₃): δ 0.95 (d, 6: 2xCH₃), 2.00 (m, 3: CH₂, CH), 2.40 (s, 3: CH₃), 6.8 (s, 1: NH), 7.05-7.65 (7 aromatic protons).

### EXAMPLE 6

### Preparation of 1-methylethyl-N-[5-methyl-3'-(trifluoromethyl)[1,1'-biphenyl]-2-yl]carbamate

To a solution of 2.51 g (0.01 mol) 5-methyl-3'-(trifluoromethyl)[1,1'-biphenyl]-2-yl]-amine in 20 ml dry chloroform 1.2 g (0.012 mol) triethylamine was added under nitrogen, followed by dropwise addition of 1.35 g (0.011 mol) isopropyl chloroformate dissoluted in 5 ml dry chloroform. After stirring for 2 hrs at the room temperature the reaction mixture was refluxed for 5 min, allowed to cool at the room temperature, 5 ml water was added and stirring continued for 20 min. The reaction mixture was diluted with 50 ml methylene chloride, washed with water, then saturated sodium bicarbonate solution and brine, dried, and evaporated to dryness. The crude product was purified by chromatography on silica using 20:1 chlorobutane/ethyl acetate mixture as eluent to yield 3.1 g pure product as white crystals, m.p. 83-85°C.

¹H NMR (CDCl₃): δ 1.22 (d, 6: 2xCH₃), 2.38 (s, 3: CH₃), 4.96 (m, 1: CH), 6.2 (s, 1: NH), 7.02-7.90 (7 aromatic protons).

### EXAMPLE 7

### Preparation of N,N-diethyl-N'-[5-methyl-3'-(trifluoromethyl)[1,1'-biphenyl]-2-yl] urea

To a solution of 2.51 g (0.01 mol) 5-methyl-3'-(trifluoromethyl)[1,1'-biphenyl]-2-yl]-amine in 20 ml dry chloroform 2 g (0.015 mol) diethylcarbamyl chloride was added under nitrogen and the reaction mixture was refluxed for 5 hrs. After cooling to room temperature, 5 ml water was added and stirring continued for 20 min. The reaction mixture was diluted with 50 ml methylene chloride, washed with saturated sodium bicarbonate solution and brine, dried, and evaporated to dryness. The crude product was purified by chromatography on silica using 10:1 chlorobutane/ethyl acetate mixture as eluent to yield 2.1 g pure product as white crystals, m.p. 87-87°C.

¹H NMR (CDCl₃): δ 1.00 (t, 6: 2xCH₃), 2.35 (s, 3: CH₃), 3.15 (q, 4: 2xNCH₂), 6.1 (s, 1: NH), 7.02-8.00 (7 aromatic protons).

### EXAMPLE 8

### Preparation of N-(2-methylpropyl)-N'-[5-methyl-3'-(trifluoromethyl)[1,1'-biphenyl]-2-yl] urea

To 5 ml solution of 5-methyl-3'-(trifluoromethyl)[1,1'-biphenyl]-2-yl]-isocyanate prepared as described under Example 4 was added under nitrogen 0.17 g (1.2 equiv) isobutylamine and the reaction mixture stirred overnight at the room temperature, then diluted with 25 ml methylene chloride, washed with saturated sodium bicarbonate solution and brine, dried, and evaporated to dryness. The crude product was purified by chromatography on silica using 5:1 chlorobutane/ethyl acetate mixture as eluent to yield 0.52 g pure product as white crystals, m.p. 165-7°C.

¹H NMR (CDCl₃): δ 0.91 (d, 6: 2xCH₃), 1.63 (m, 1: CH), 2.40 (s, 3: CH₃), 2.96 (t, 2: NCH₂), 4.6 (t, 1: NH), 5.8 (s, 1: NH), 7.05-7.65 (7 aromatic protons).

### EXAMPLE 9

### Preparation of cyclopentyl-[5-methyl-3'-(trifluoromethyl)[1,1'-biphenyl]-2-yl] carbamate

To 5 ml solution of 5-methyl-3'-(trifluoromethyl)[1,1'-biphenyl]-2-yl]-isocyanate prepared as described under Example 4 was added under nitrogen 0.21 g (1.5 equiv) cyclopentyl alcohol and the reaction mixture stirred overnight at the room temperature, then diluted with 25 ml methylene chloride, washed with saturated sodium bicarbonate solution and brine, dried, and evaporated to dryness. The crude product was purified by chromatography on silica using 5:1 chlorobutane/ethyl acetate mixture as eluent to yield 0.21 g pure product.

### EXAMPLE 10

### Preparation of 4-methyl-2-(trimethyltin)trimethylacetylanilide

19.1 g (0.1 mol) 4-methyl-N-(trimethylacetyl)aniline was dissolved under nitrogen in 250 ml anhydrous tetrahydrofuran and the solution was cooled to -10°C, after which 156 ml (0.25 mol) 1.6N solution of n-butyllithium in hexanes was added dropwise during 1.5 hrs, while maintaining the temperature below 0°C. After complete addition stirring was continued for 0.5 hrs on the cold bath, then overnight at the room temperature. The reaction mixture was cooled to -5°C and a solution of 40 g (0.2 mol) trimethyltin chloride in 100 ml anhydrous tetrahydrofuran was added dropwise during 20 min., while maintaining the temperature below 0°C. After complete addition stirring was continued at 0°C for 4 hrs, then the reaction mixture was poured under nitrogen blanket over 300 ml saturated solution of sodium bicarbonate and stirred for 1 hr. 300 ml Ethyl acetate was added, phases separated, the organic phase washed with brine, dried and evaporated to dryness. The residue was chromatographed on silica using chlorobutane as eluent. 21.3 g of trimethyltin derivative and 6.1 g of starting material were obtained.

¹H NMR (CDCl₃): δ 0.38 (s, 9: SnMe₃), 1.30 (s, 9: C(CH₃)₃), 2.32 (s, 3: CH₃), 7.10-7.7 (3 aromatic protons, NH).

### EXAMPLE 11

### Preparation of 2,2-dimethyl-N-[5-methyl-3'-cyano[1,1'-biphenyl]-2-yl]-propanamide

7.1 g (0.02 mol) 4-methyl-2-(trimethyltin)trimethylacetylanilide was dissolved under nitrogen in 30 ml anhydrous tetrahydrofuran, 0.05 g palladium tetrakis(triphenylphosphine) was added, and the mixture was stirred at the room temperature for 10 min., after which 4 g (0.022 mol) meta-bromobenzonitrile was added. The mixture was refluxed under nitrogen with good stirring for 7 hrs. After cooling, the reaction mixture was poured over 100 ml saturated sodium bicarbonate solution and stirred at the room temperature for 20 min, then extracted with 100 ml ethyl acetate. Organic extracts were washed with brine, dried, and evaporated to dryness. The crude product was purified by chromatography on silica using chlorobutane as eluent to yield 4.55 g pure product as white crystals, m.p. 78-80°C.

¹H NMR (CDCl₃): d 1.16 (s,9: C(CH₃)₃), 2.38 (s,3: CH₃), 7.04-7.9 (7S aromatic protons, NH).

### EXAMPLE 12

### Preparation of 5-methyl-3-[(trifluoromethyl)phenyl]-2-pyridinamine

4.7 g (0.025 mol) 2-amino-3-bromo-5-methylpyridine was dissolved under nitrogen in 50 ml anhydrous tetrahydrofuran, 0.05 g palladium bis(triphenylphosphine) dichloride was added, and the mixture was stirred at the room temperature for 10 min., after which 5.5 g (0.029 mol) *meta*-trifluoromethylphenylboronic acid was added, followed by a solution of 7.2 g (0.075 mol) sodium carbonate in 70 ml water. The mixture was refluxed under nitrogen with good stirring for 2 hrs. After cooling , the reaction mixture was poured over 100 ml 1N sodium hydroxide solution and stirred at the room temperature for 20 min, then extracted with 150 ml ethyl acetate. Organic extracts were washed with brine, dried, and evaporated to dryness. The crude product was purified by chromatography on silica using 20:1 chlorobutane/ethyl acetate mixture as eluent to yield 5.35 g pure product as a light tan solid, m.p. 73-5°C.

¹H NMR (CDCl₃): δ 2.12 (s, 3: CH₃), 4.40 (s, 2: NH₂), 7.10-8.0 (6 aromatic protons).

### EXAMPLE 13

### Preparation of N-[5-methyl-3-[3-(trifluoromethyl) phenyl]-2-pyridinyl cyclopropaneamide

To a solution of 0.50 g (0.002 mol) 5-methyl-3-[(trifluoromethyl)phenyl]-2-pyridinamine in 10 ml dry chloroform 026 g (0.0026 mol) triethylamine was added under nitrogen, followed by dropwise addition of 0.23 g (0.0022 mol) cyclopropancarbonyl chloride dissolved in 2 ml dry chloroform. After stirring for 5 hrs at the room temperature, 5 ml water was added and stirring continued for 20 min. The reaction mixture was diluted with 20 ml methylene chloride, washed with water, then saturated sodium bicarbonate solution and brine, dried, and evaporated to dryness. The crude product was purified by chromatography on silica using 3:1 chlorobutane/ethyl acetate mixture as eluent to yield 0.54 g pure product as a white solid, m.p. 129-31°C.

¹H NMR (CDCl₃): δ 0.65-0.90 (m, 4: 2xCH₂), 1.85 (m, 1: CH), 2.40 (s, 3: CH₃), 7.44-8.50: (6 aromatic protons).

### EXAMPLE 14

### Preparation of N-[5-methyl-3-[3-(trifluoromethyl) phenyl]-2-pyridinyl] cyclopropaneamide N-oxide

0.3 g (1 mmol) N-[5-methyl-3-[3-(trifluoromethyl) phenyl]-2-pyridinyl] cyclopropaneamide was dissolved in 5 ml methylene chloride and 0.42 g (cca. 1.2 mmol) 50-60% meta-chloroperbenzoic acid was added in two portions at 1 hr interval. The mixture was stirred overnight, diluted with 25 ml methylene chloride and washed with 1N sodium thiosulfate, then saturated sodium bicarbonate solution until no *meta*-chlorobenzoic acid was present. The solution was dried over magnesium sulfate and evaporated to dryness. The crude product was purified by chromatography on silica using 1:1 chlorobutane/ethyl acetate mixture as eluent to yield 0.22 g pure product as white crystals, m.p. 125-7°C.

¹H NMR (CDCl₃): δ 0.60-0.85 (m, 4: 2xCH₂), 1.83 (m, 1: CH), 2.40 (s, 3: CH₃), 7.20-8.20: (6 aromatic protons), 9.45 (s, 1: NH).

### EXAMPLE 15

### Preparation of N,2,2-trimethyl-N-[5-methyl-3'-(trifluoromethyl) [1,1'-biphenyl]-2-yl]-propanamide

0.8 g 2,2-dimethyl-N-[5-methyl-3'-(trifluoromethyl)-[1,1'-biphenyl]-2-yl]-propanamide was dissolved in 15 ml acetone, 0.8 g powdered potassium hydroxide added, and the mixture was energetically stirred wile adding 1 g iodomethane after which it was kept at reflux for 5 min. After cooling, the mixture was filtered, the filtrate concentrated *in vacuo,* 30 ml water added and the mixture extracted with ethyl acetate. The organic extracts were washed three times with brine, dried over magnesium sulfate and evaporated to dryness. The crude product was purified by chromatography on silica using 1:1 chlorobutane/ethyl acetate mixture as eluent to yield 0.6 g pure product as a light tan oil.

¹H NMR (CDCl₃): δ 1.06 (s, 9: C(CH₃)₃), 2.40 (s, 3: CH₃), 3.00 (s, 3: NCH₃), 7.05-7.65 (7 aromatic protons).

### EXAMPLE 16

### Preparation of 2-nitro-3'-(trifluoromethyl)-1,1'-biphenyl

A solution of 5 g (0.02 mol) meta-iodonitrobenzene, 6.8 g (0.022 mol) trimethyl [3-(trifluoromethyl)phenyl]stannane, and 0.2 g palladium bis(triphenylphosphine) dichloride in 50 ml dry tetrahydrofuran was refluxed under nitrogen for 6 hrs. After cooling to room temperature, the reaction mixture was poured over 200 ml saturated sodium bicarbonate solution, stirred for 20 min, then extracted with ethyl acetate. The extracts were washed with brine, dried over magnesium sulfate, filtered, and evaporated to dryness. The residue was chromatographed on silica using 1:3 chlorobutane/hexane mixture as eluent to yield 5.6 g pure product as a light tan oil.

### EXAMPLE 17

### Preparation of 3'-(trifluoromethyl)[1,1'-biphenyl]-2-amine

5.4 g (0.02 mol) of 2-nitro-3'-(trifluoromethyl)-1,1'-biphenyl was dissolved in 80 ml ethyl acetate, 5 ml acetic acid and 0.2 g 10% palladium on charcoal were added, and the mixture was hydrogenated at balloon pressure overnight. The catalyst was removed by filtration and the solution evaporated to dryness. The residue was dissolved in hexane, washed with 0.5 M sodium hydroxide solution, then brine, dried over magnesium sulfate, filtered, treated with charcoal, filtered, and evaporated to dryness. The residue was dried in a vacuum oven under nitrogen at 40°C to afford 4.5 g pure product as a light tan oil.

### EXAMPLE 18

### Preparation of N-(2-boronophenyl)-2,2-dimethylpropanamide

A solution of 25.5 (0.1 mol) N-(2-bromophenyl)-2,2-dimethylpropanamide in 300 ml dry ether and 60 ml dry tetrahydrofuran was cooled at -10°C under nitrogen and 137 ml (0.22 mol) 1.6M butyllithium in hexanes was added dropwise during 1 hr while maintaining the temperature below 0°C. After complete addition stirring was continued for 3 hrs on the cold bath after which the reaction mixture was transferred via cannula, under light nitrogen pressure, into a solution of 40 g (0.4 mol) trimethyl borate in 100 ml dry tetrahydrofuran, cooled at -10°C. The rate of addition was adjusted in such a way as to maintain the temperature below 0°C. After complete addition stirring was continued over night, allowing the cold bath to melt. The reaction mixture was evaporated to dryness, 300 ml 10% hydrochloric acid and 100 ml tetrahydrofuran added, and the mixture stirred at the room temperature for 3 hrs. Solvents were removed *in vacuo* and the residue was recrystallized from hot water. After drying in a vacuum oven overnight, 17.7 g (80%) of N-(2-boronophenyl)-2,2-dimethylpropanamide was obtained.

### EXAMPLE 19

### Preparation of N-(3'-cyano[1,1'-biphenyl)-2-yl]-2,2-dimethylpropanamide

A mixture of 9.1 g (0.05 mol) meta-bromobenzonitrile, 0.300 g palladium bis(triphenylphosphine) dichloride, and 15 ml glyme was stirred under nitrogen for 15 min., after which 13.3 g (0.06 mol) N-(2-boronophenyl)-2,2-dimethylpropanamide was added, followed by a solution of 12.7 g (0.12 mol) sodium carbonate in 150 ml water. The reaction mixture was kept at reflux under nitrogen for 6 hrs. After cooling, 150 ml 1N sodium hydroxide solution was added and the mixture was extracted with ethyl acetate. The organic extracts were washed twice with brine, dried over magnezium sulfate, and evaporated to dryness. The crude product was purified by chromatography on silica using chlorobutane/ethyl acetate 40:1 as eluent, to yield 10.6 g (76%) N-(3'-cyano[1,1'-biphenyl]-2-yl)-2,2-dimethylpropanamide, m.p. 178-181°C.

### EXAMPLE 20

### Preparation of N-(5-bromo-3'-cyano[1,1'-biphenyl]-2-yl)-2,2-dimethylpropanamide

A solution of 6.7 g (0.042 mol) bromine in 15 ml glacial acetic acid was added dropwise, during 1 hr, to a solution of 8.4 g (0.3 mol) N-(3'-cyano[1,1'-biphenyl]-2-yl)-2,2-dimethylpropanamide and and 10.6 g (0.1 mol) sodium acetate in 50 ml glacial acetic acid. After complete addition stirring was continued for 6 hrs. The acetic acid was removed *in vacuo,* ice was added and the suspension was made basic with 10% sodium hydroxide and was extracted with ethyl acetate. The organic extracts were washed twice with brine, dried, and evaporated to dryness. The crude product was purified by chromatography on silica using chlorobutane/ethyl acetate 20:1 as eluent, to afford 9.85 g (92%) N-(5-bromo-3'-cyano[1,1'-biphenyl]-2-yl)-2,2-dimethylpropanamide as a viscous oil.

¹H NMR (CDCl₃): δ 1.05 (s,9: C(CH₃)₃), 7.15 (s,1: NH), 7.4-8.1 (7H aromatic).

### EXAMPLE 21

### Preparation of N-(3',5-dicyano[1,1'-biphenyl]-2-yl)-2,2-dimethylpropanamide

A mixture containing 3.6 g (0.01 mol) N-(5-bromo-3'-cyano[1,1'-biphenyl]-2-yl)-2,2-dimethylpropanamide, 1.1 g (0.012 mol) copper cyanide, 0.04 g copper sulfate, and 30 ml dry pyridine was kept at reflux, under nitrogen, for 72 hrs. After cooling, the reaction mixture was treated with 100 ml 10% ammonium hydroxide and extracted with ethyl acetate. The organic extracts were washed twice with 100 ml 10% ammonium hydroxide, then with 10% hydrochloric acid and brine, dried over magnezium sulfate, and evaporated to dryness. The residue was purified by chromatography on silica using chlorobutane/ethyl acetate 20:1 as eluent to afford 1.97 g (656%) N-(3',5-dicyano[1,1'-biphenyl]-2-yl)-2,2-dimethylpropanamide, m.p. 54-59°C.

### EXAMPLE 22

### Preparation of N-[5-ethynyl-3'-(trifluoromethyl)-[1,1'-biphenyl]-2-yl]-2-methylpropanamide

0.385 g (0.001mol) N-[5-bromo-3'-(trifluoromethyl)-[1,1'-biphenyl]-2-yl]-2-methylpropanamide was added under nitrogen to a mixture of 15 ml triethylamine and 5 ml anhydrous acetonitrile, after which 0.2 g trimethylsilylacetylene, 0.4 g triphenylphosphine, 0.2 g 10% palladium on charcoal, and 0.02 g copper iodide were added and the reaction mixture was refluxed for 18 hrs. After cooling, the catalyst was removed by filtration and the filtrates were diluted to 50 ml with ethyl acetate, washed with 50 ml 10% hydrochloric acid, then brine, dried over magnezium sulfate, and evaporated to dryness. The residue was purified by chromatography on silica using chlorobutane/ethyl acetate 40:1 as eluent. 0.350 g (90%) 2-methyl-N-[3'-(trifluoromethyl)-5-[trimethylsilyl)ethynyl][1,1'-biphenyl]-2-yl]-propanamide was obtained, m.p. = 145-149°C.

0.300 g 2-methyl-N-[3'-(trifluoromethyl)-5-[trimethylsilyl)ethynyl][1,1'-biphenyl]-2-yl]-propanamide was disolved in 5 ml absolute methanol, a solution of 0.04 g KOH in 2 ml water was added, and the mixture stirred at the room temperature for 4 hrs. The solvent was removed *in vacuo,* the residue taken in ethyl acetate and washed twice with brine, then dried over magnezium sulfate and evaporated to dryness. The residue was disolved in ether, treated with activated carbon, filtered, and evaporated, to afford 0.285 g (92%) N-[5-ethynyl-3'-(trifluoromethyl)[1,1'-biphenyl]-2-yl]-2-methylpropanamide, m.p. = 108-111°C.

Using the procedures outlined in Schemes 1-10 and Examples 1-22 of this section, the compounds of this invention including those listed in Tables 1-11 can be readily prepared by one skilled in the art.

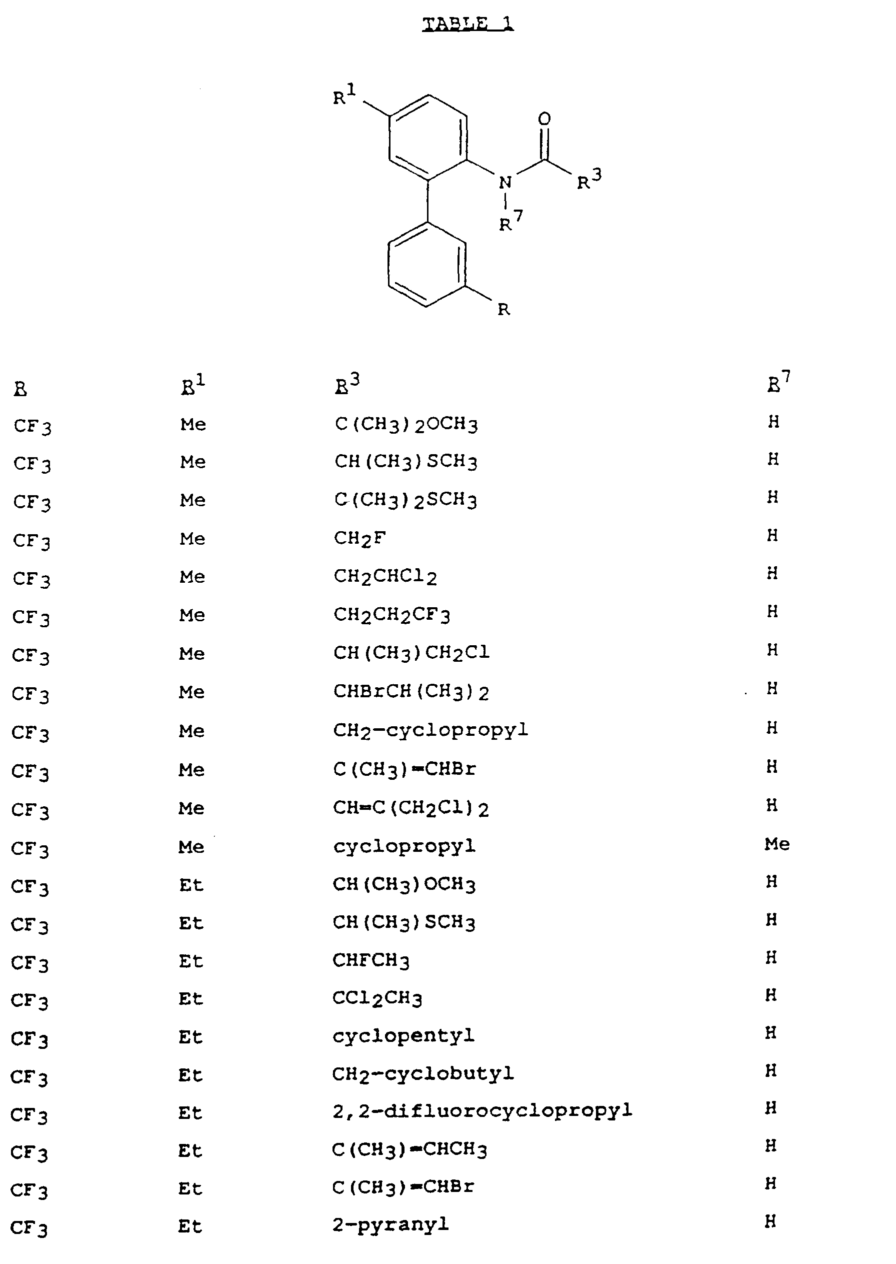

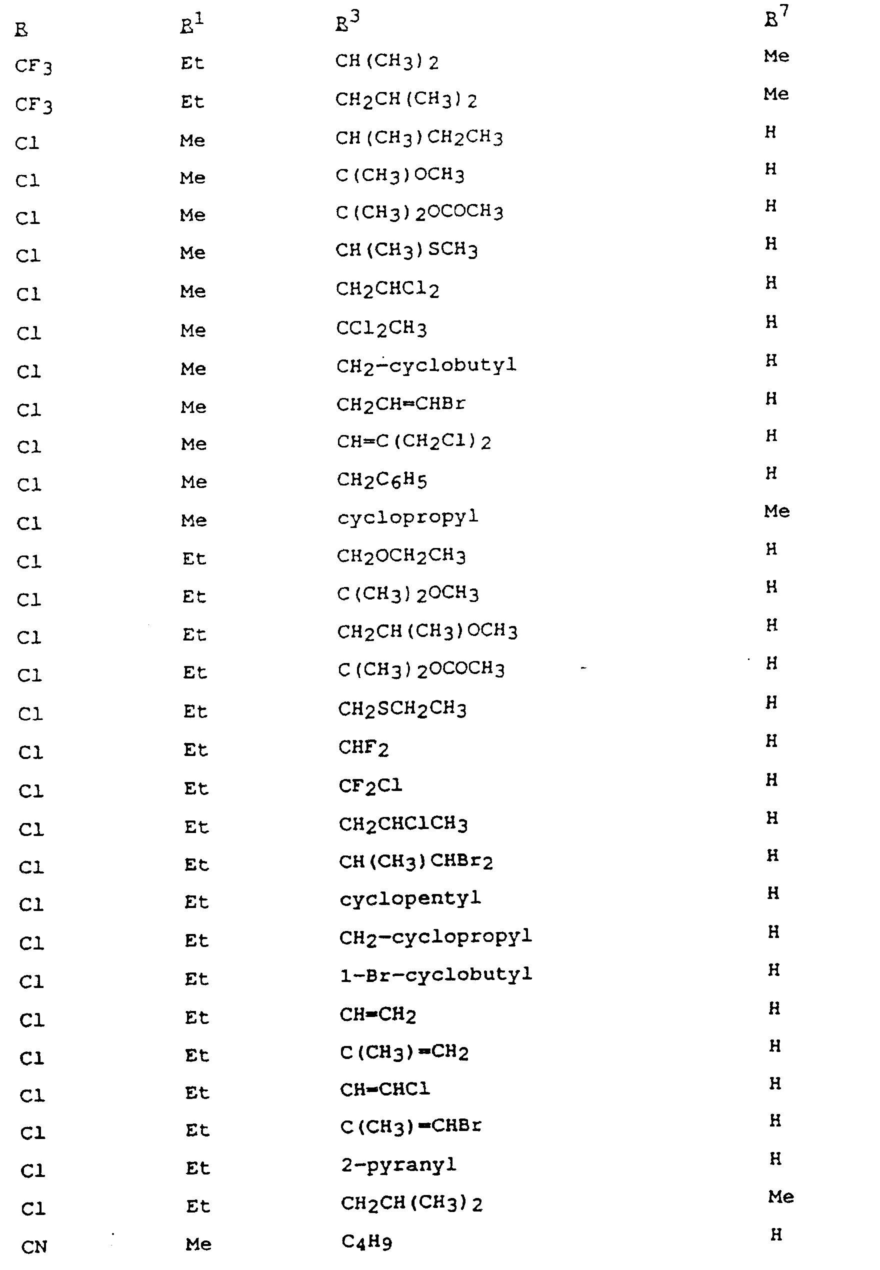

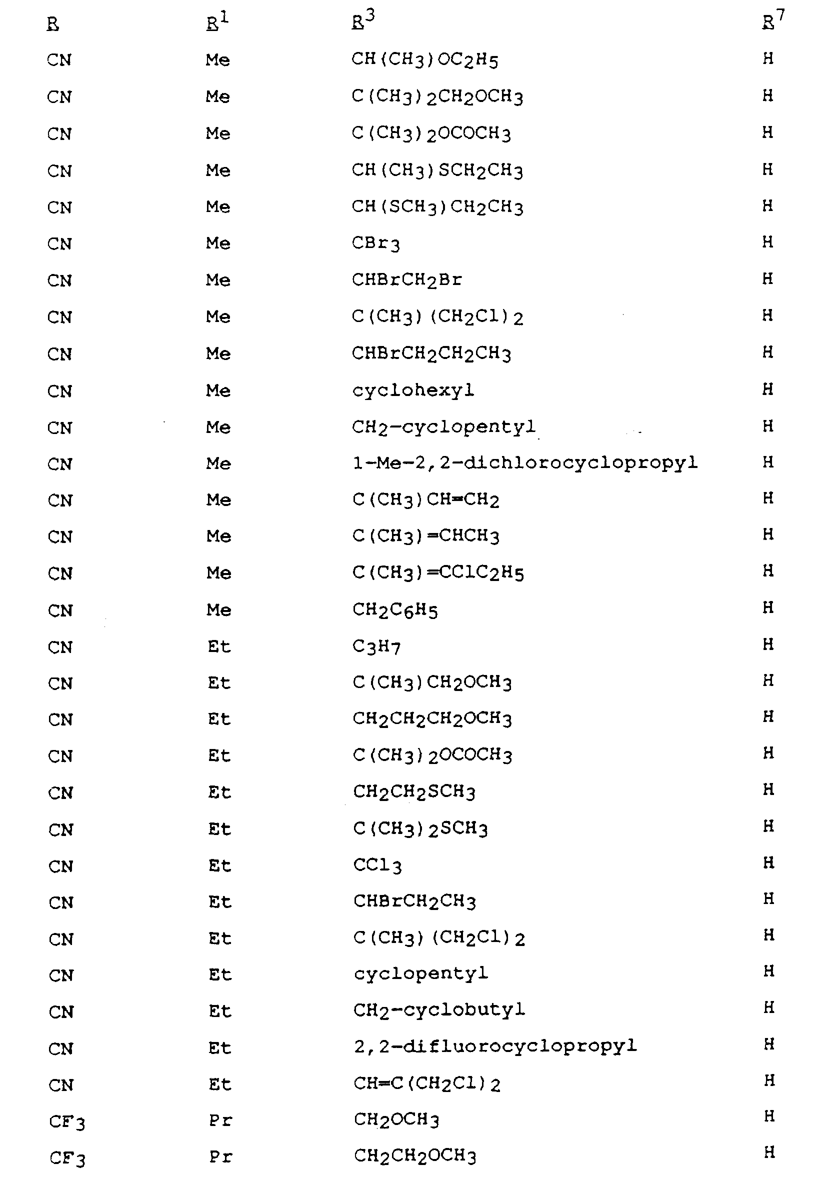

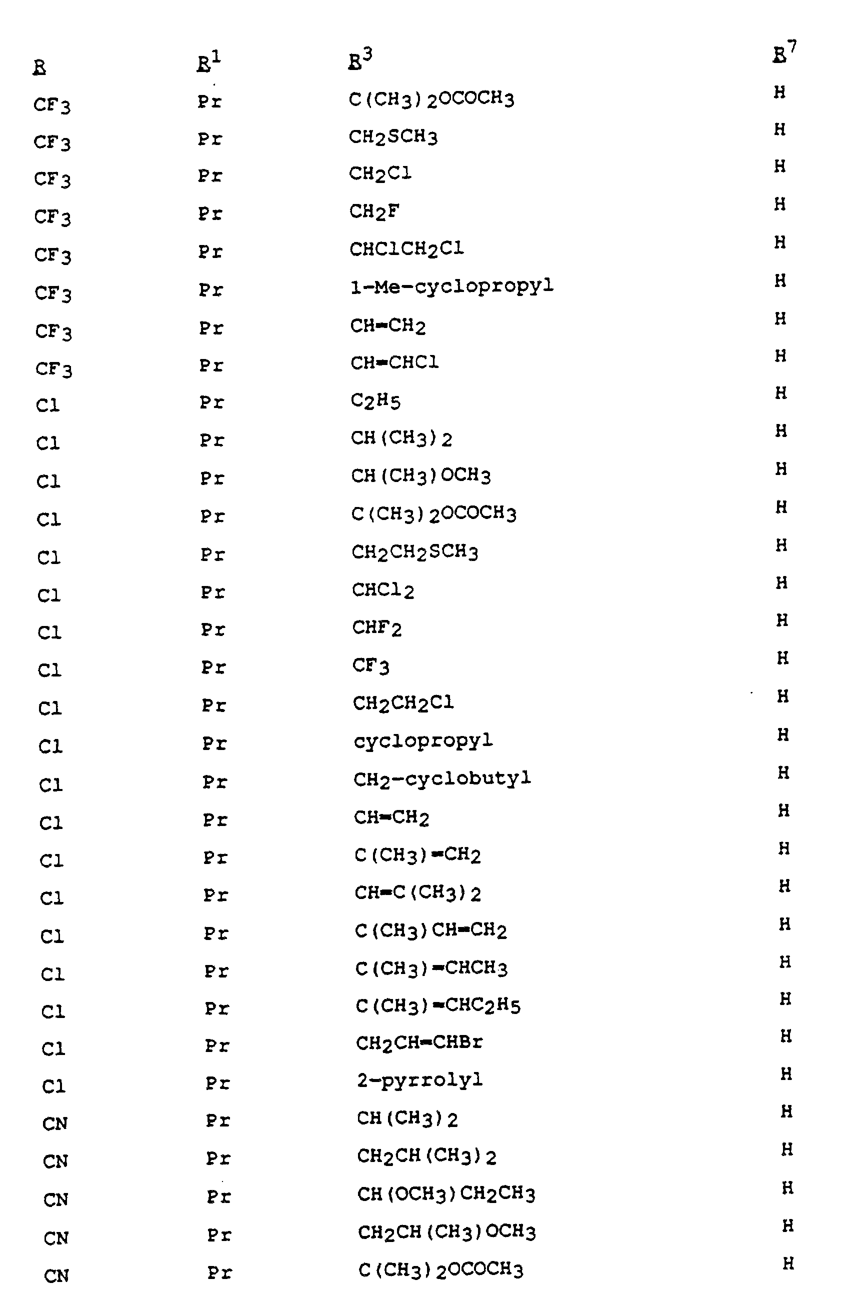

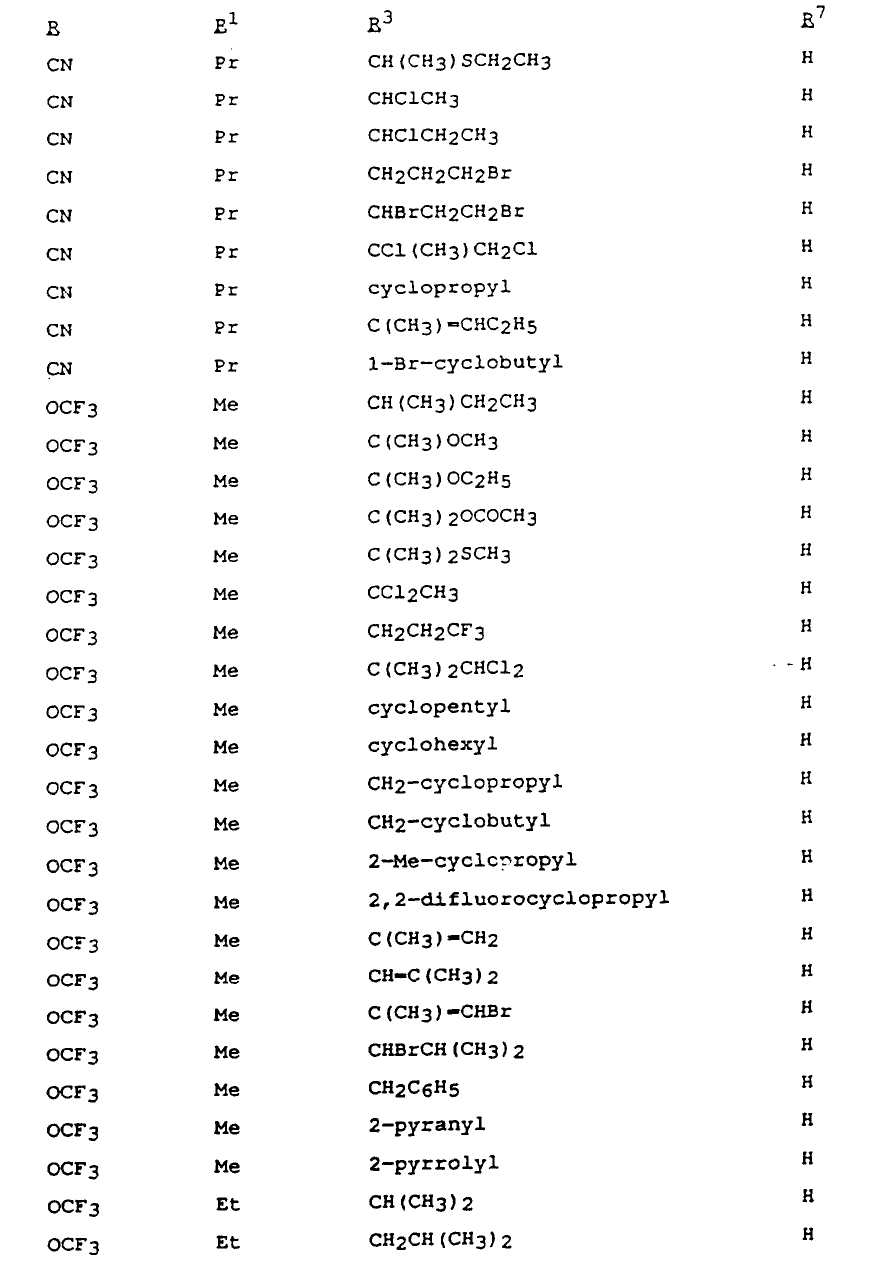

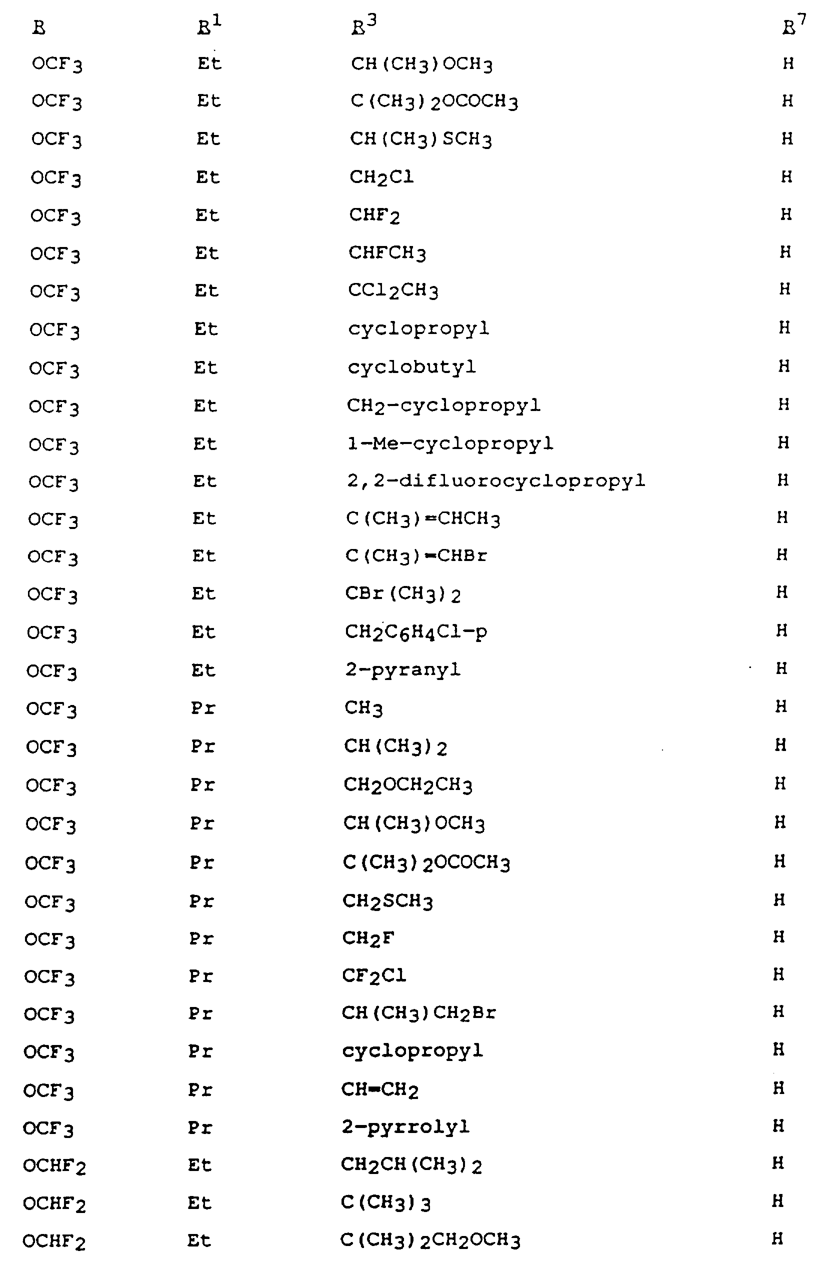

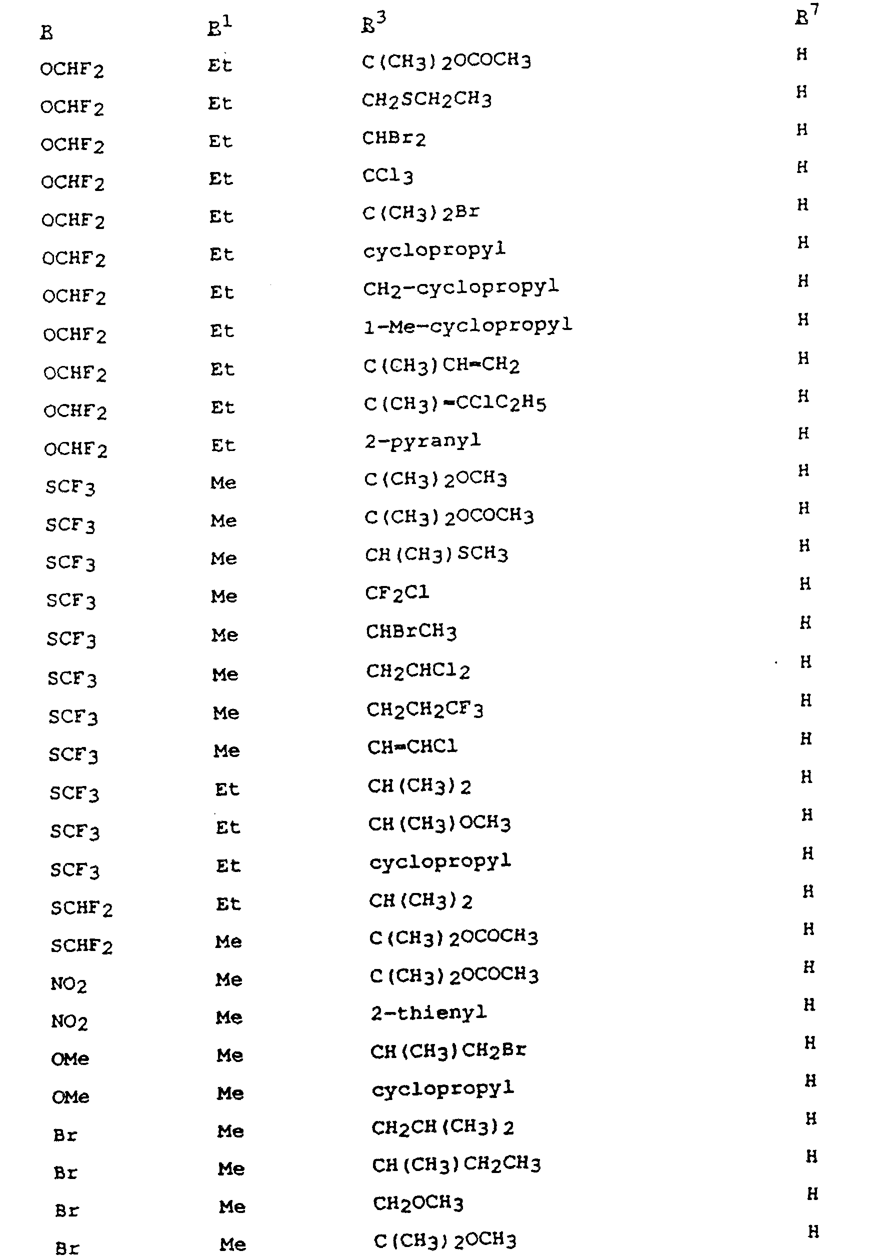

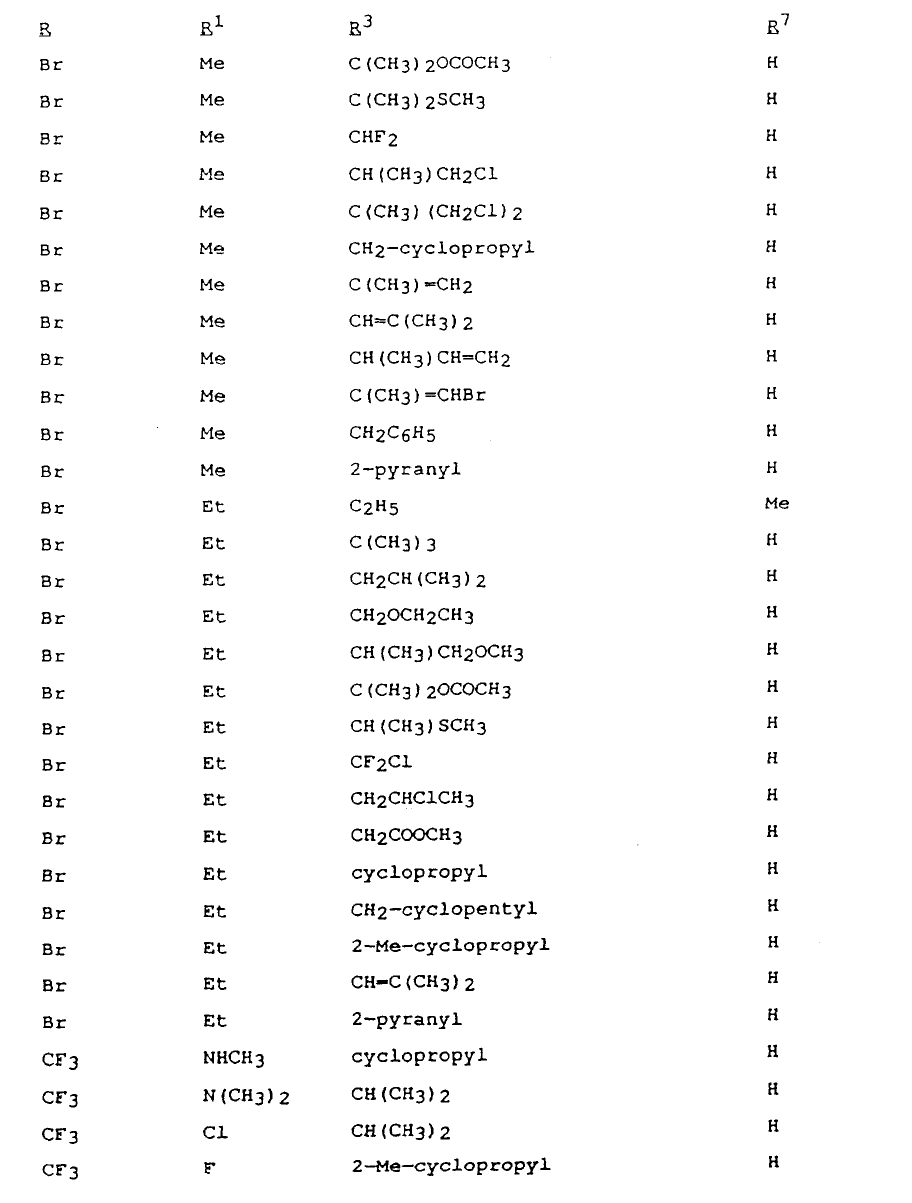

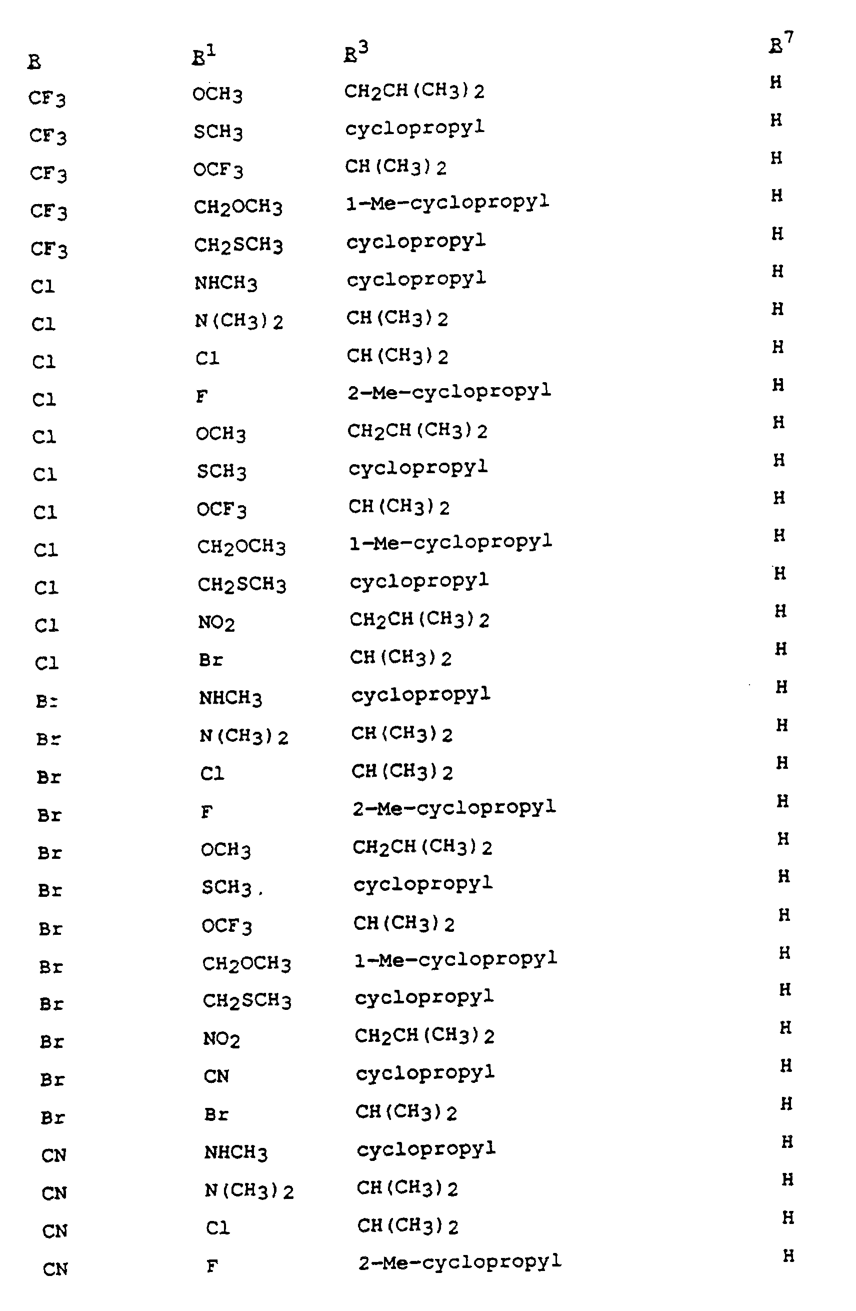

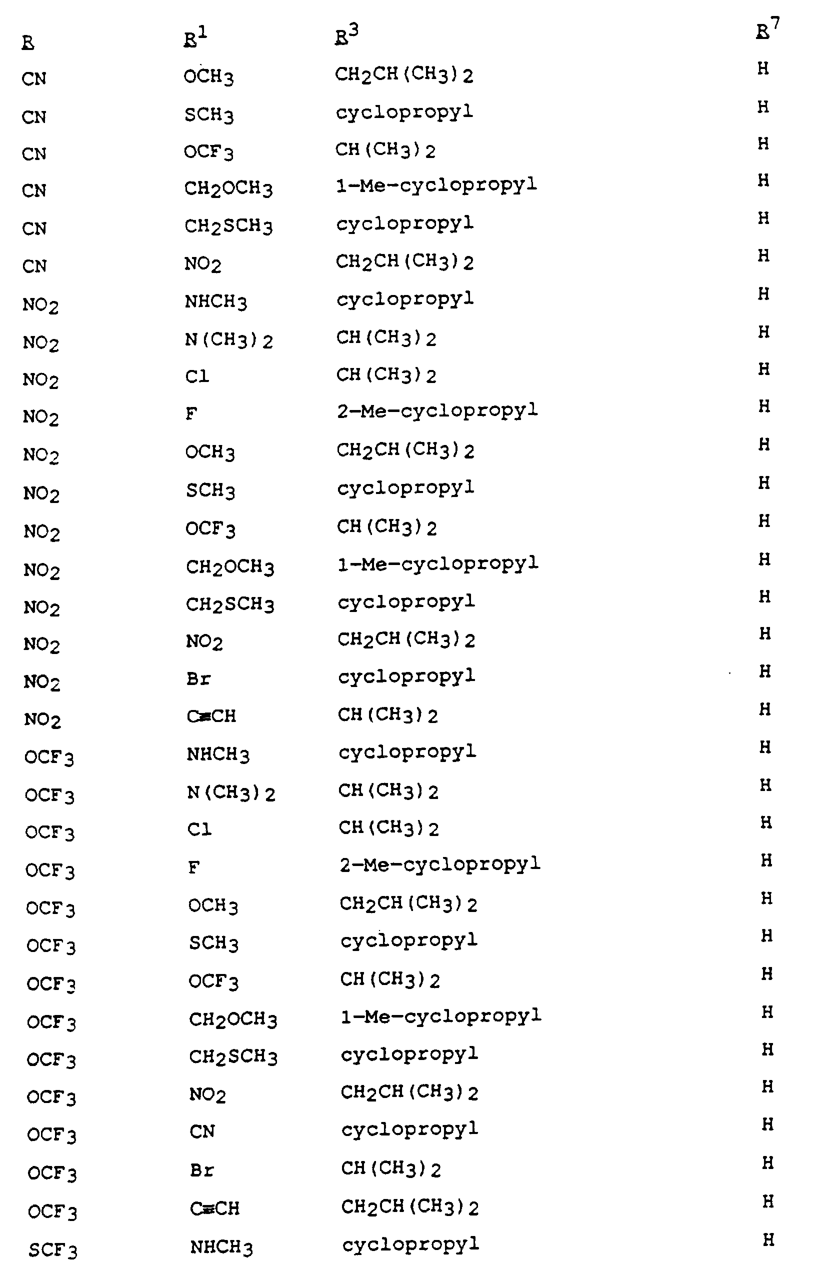

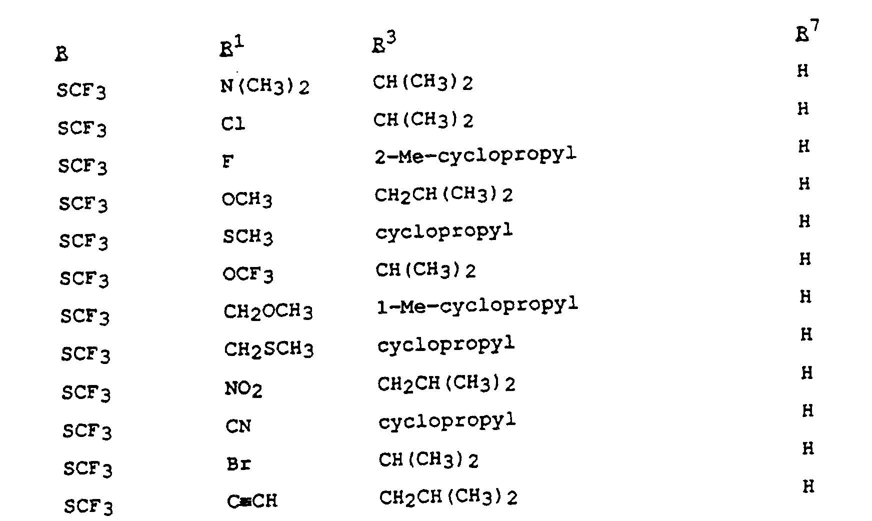

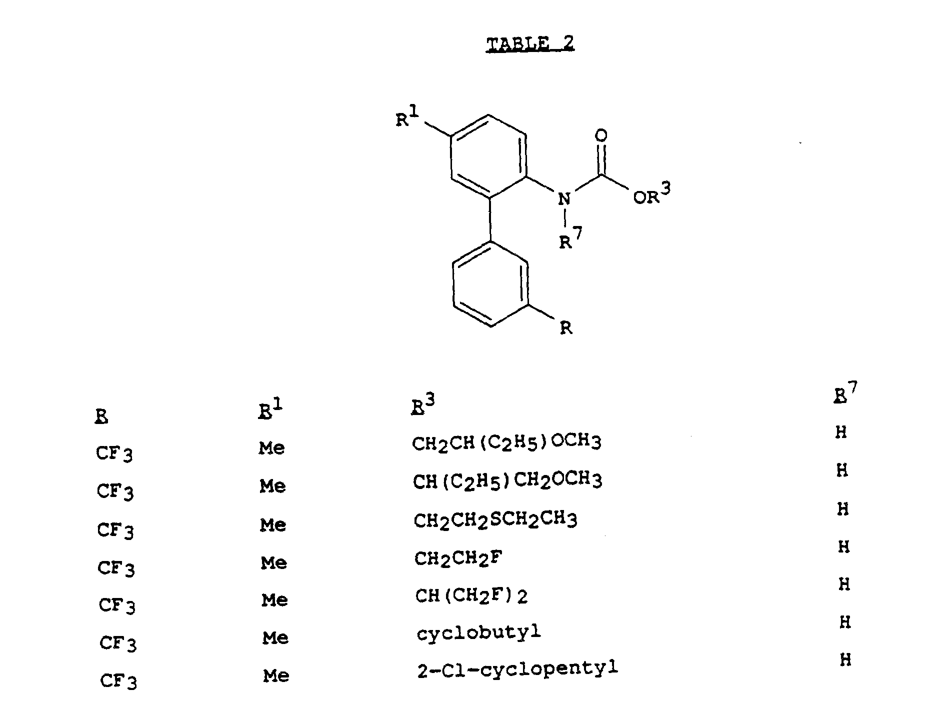

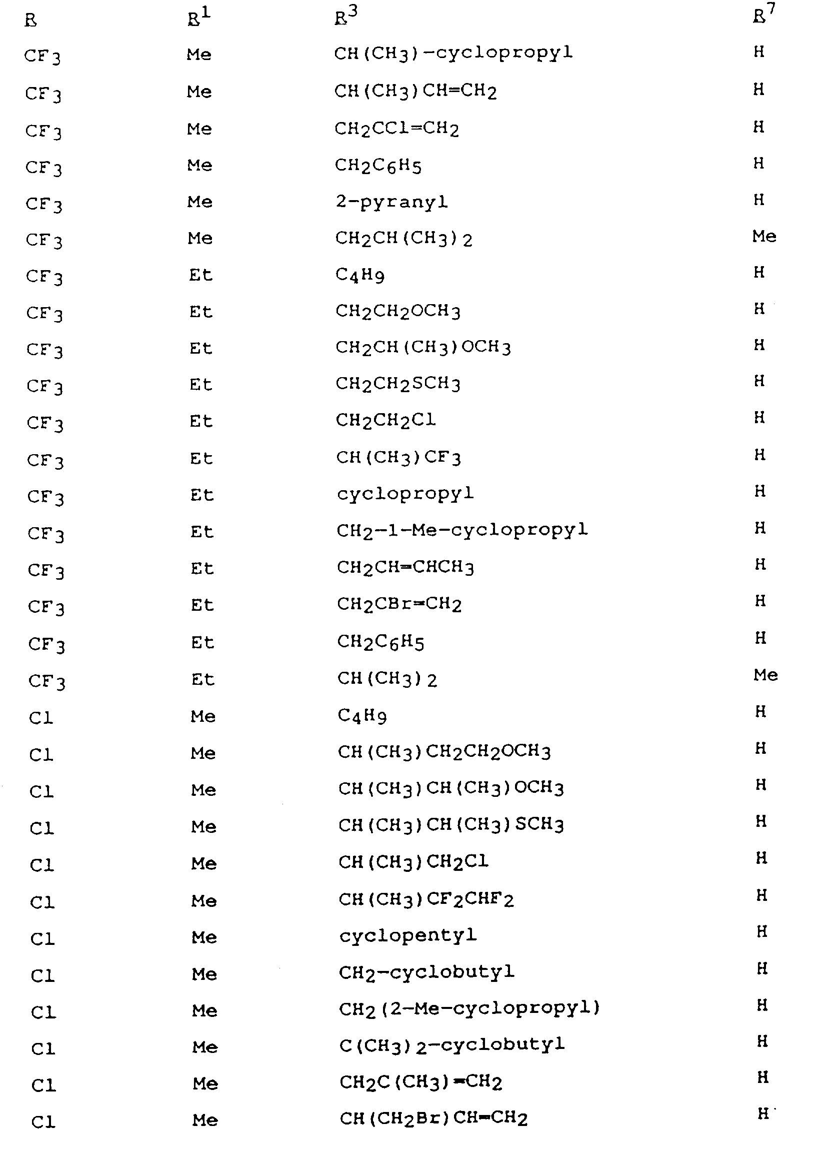

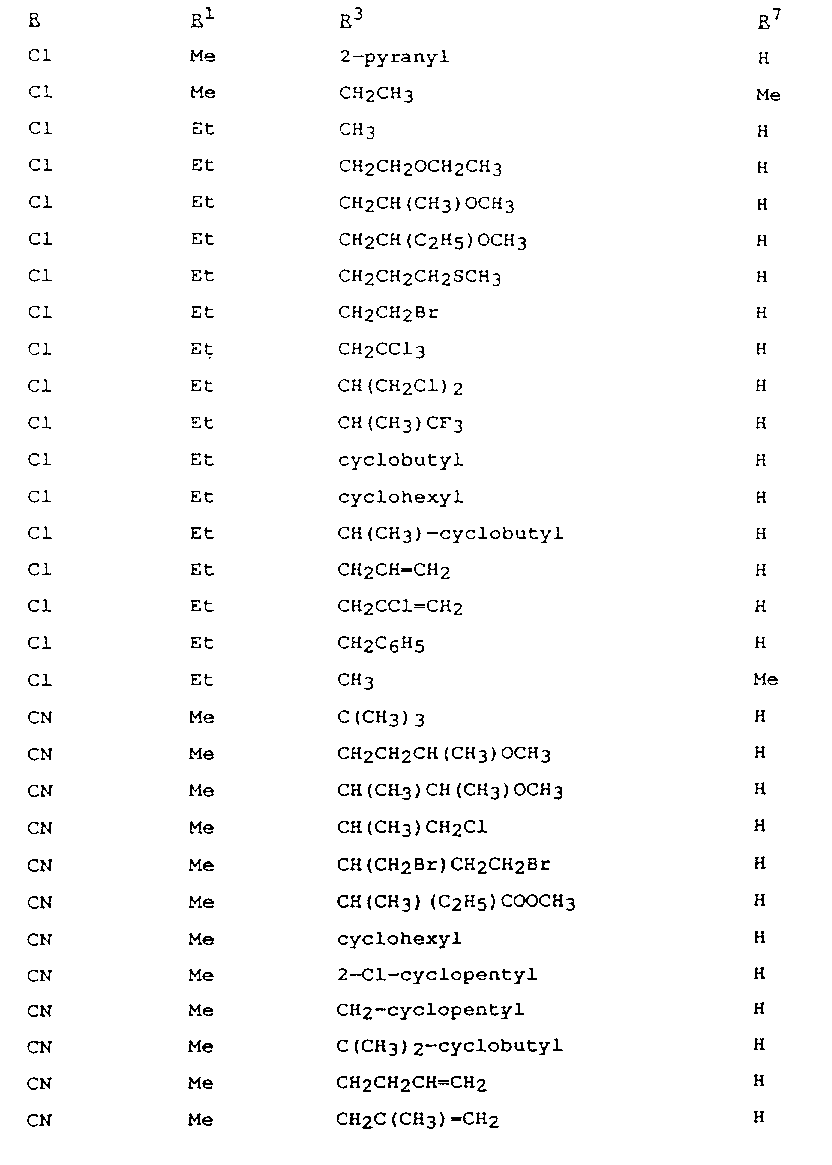

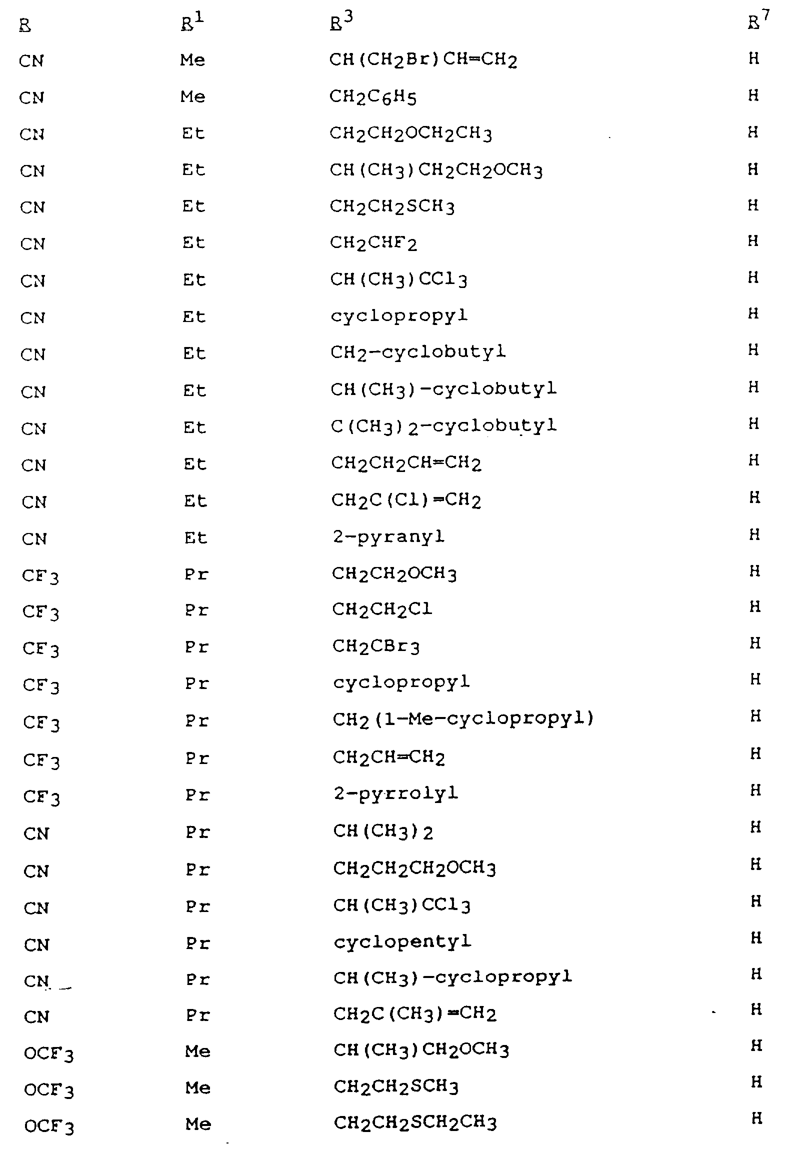

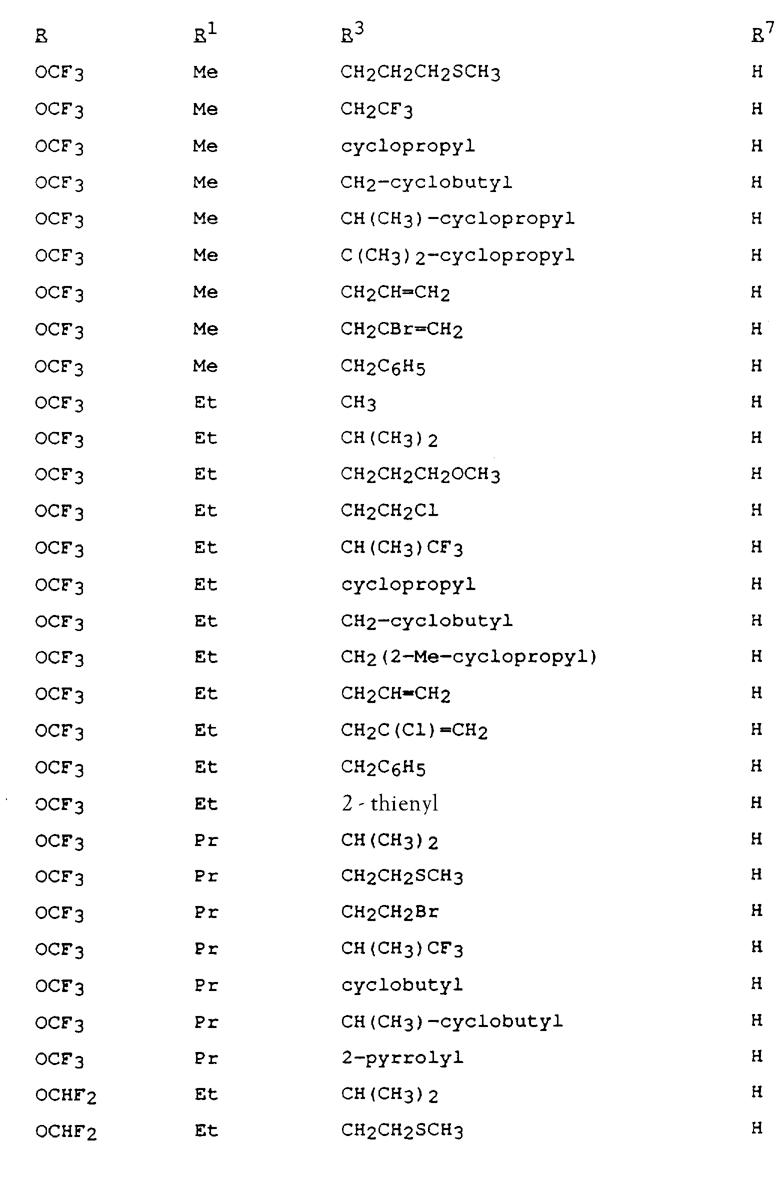

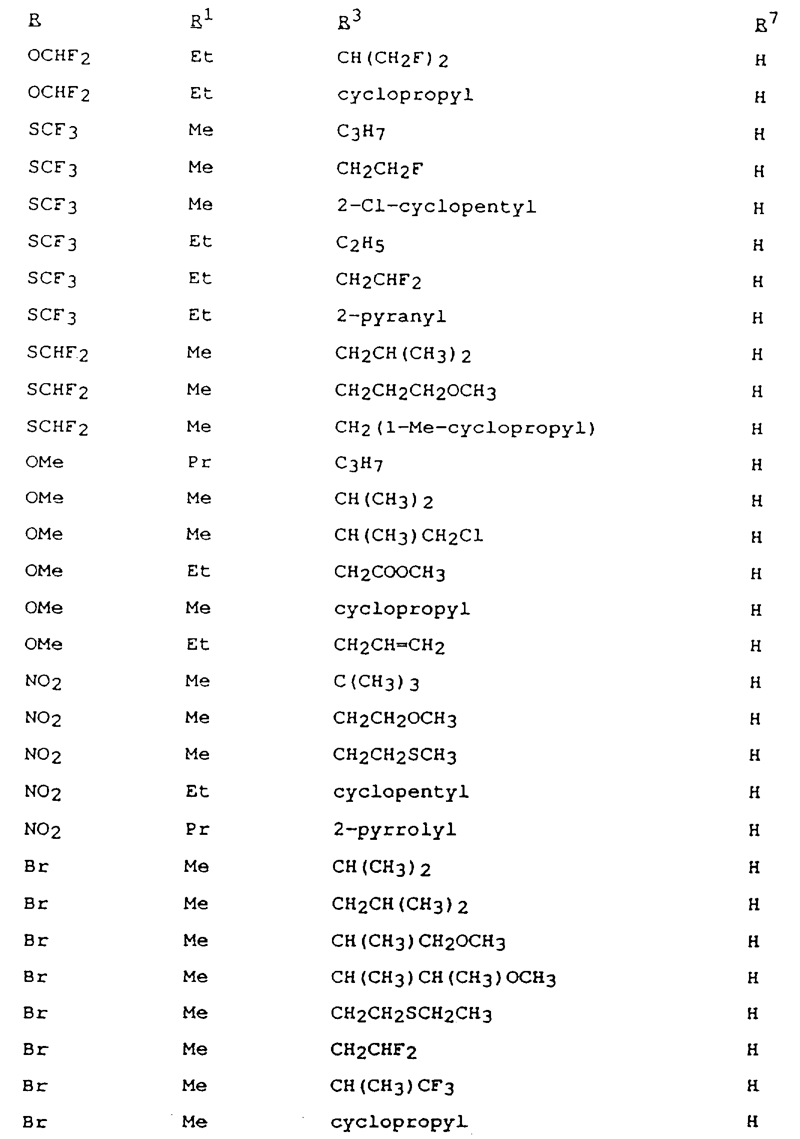

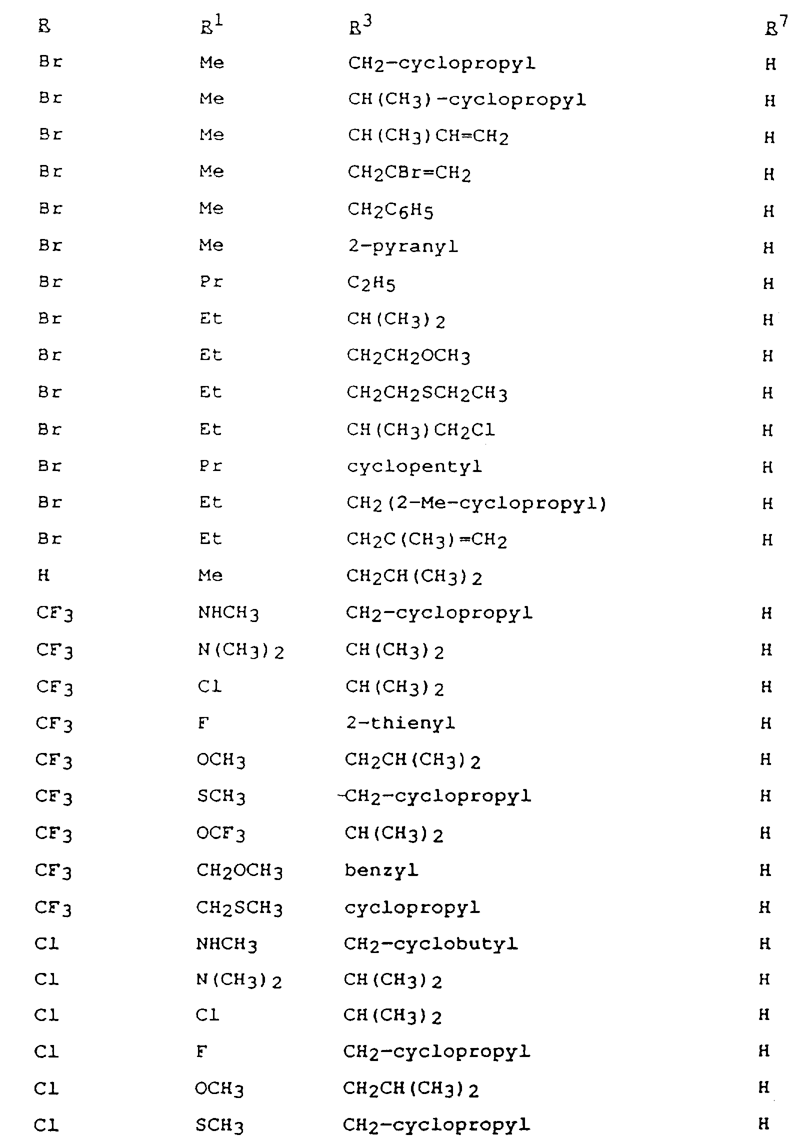

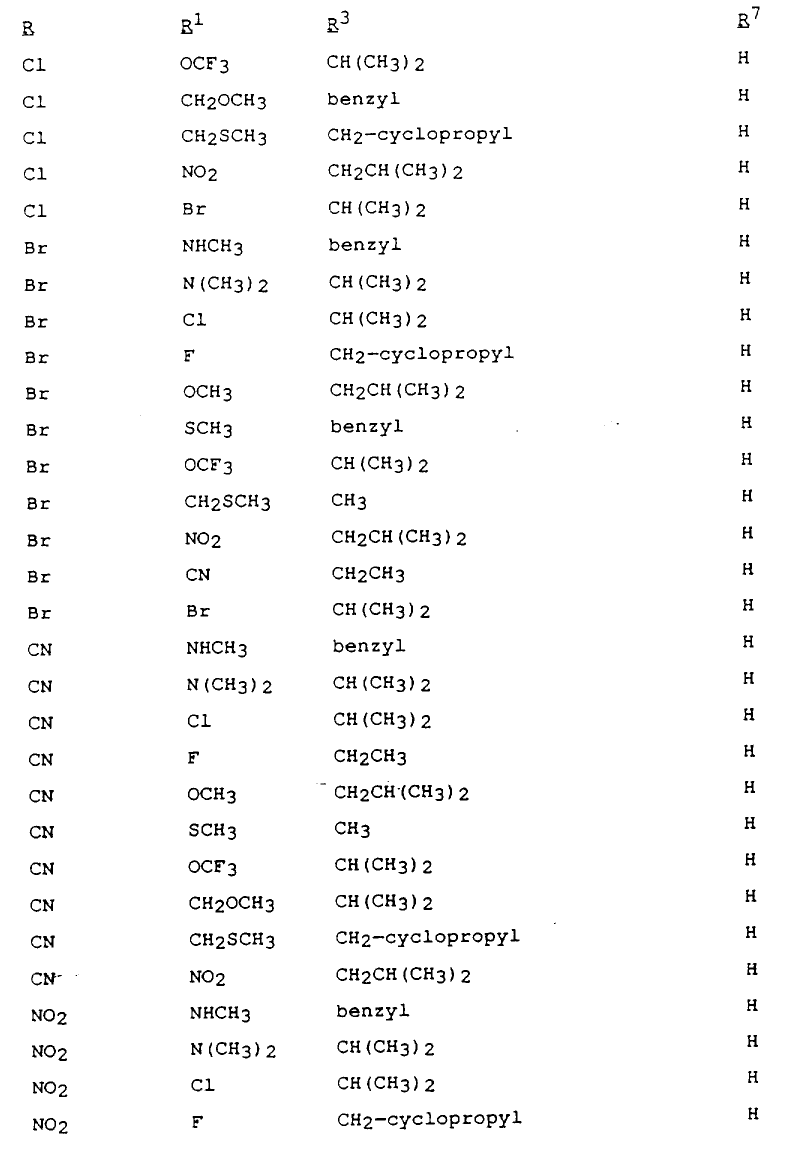

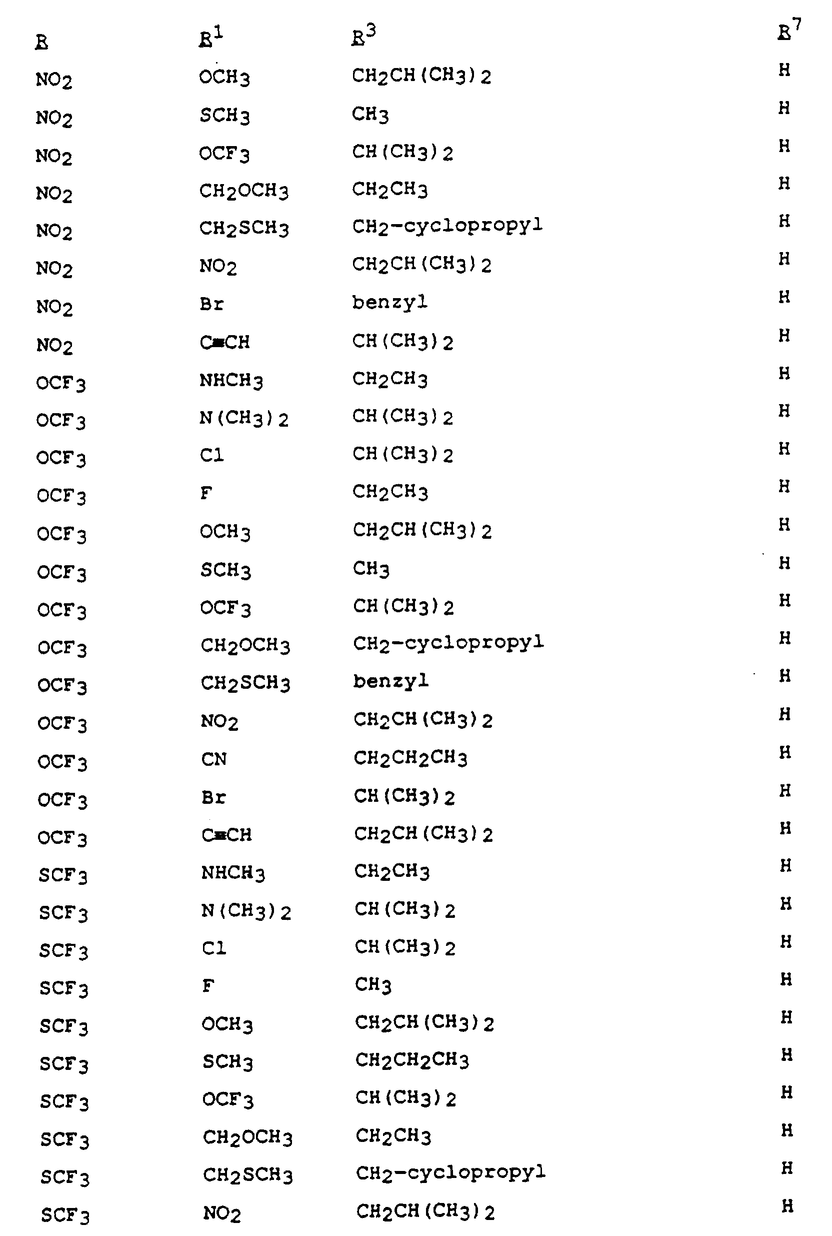

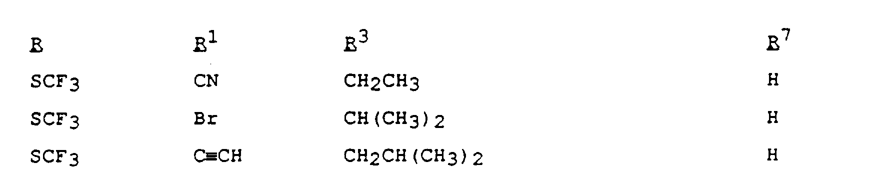

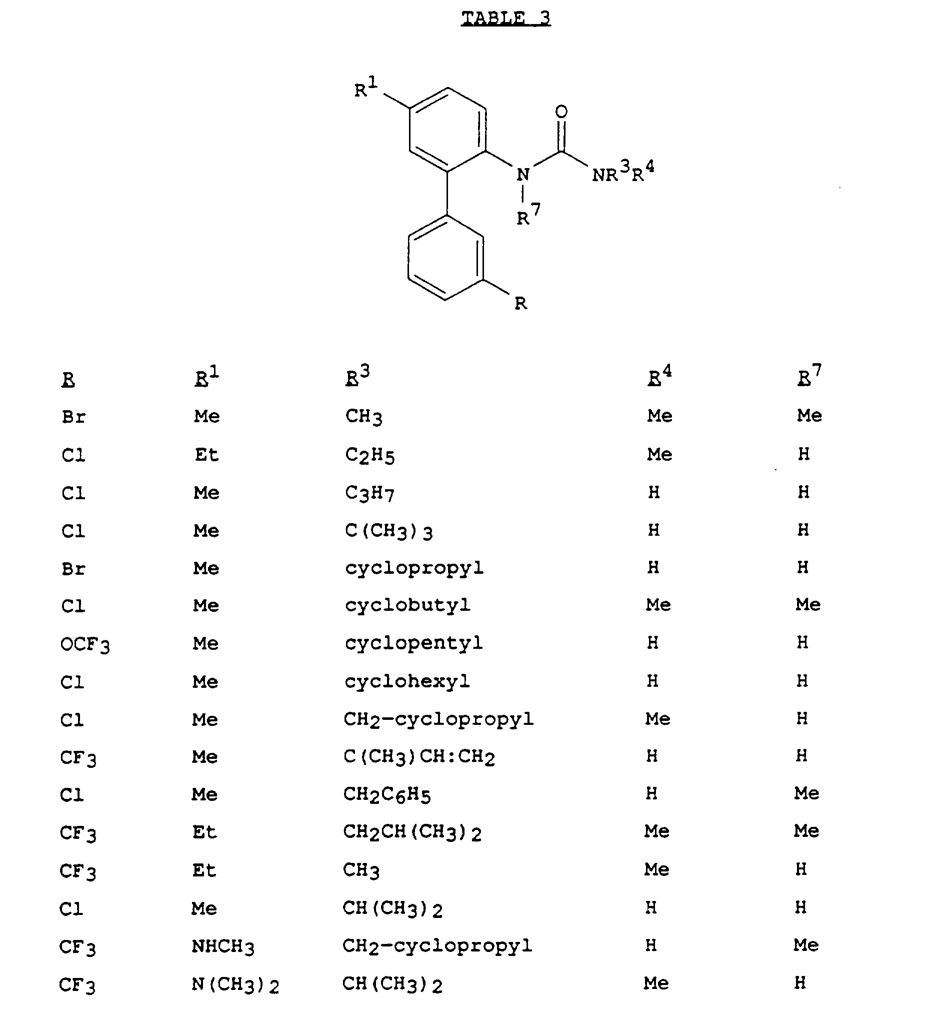

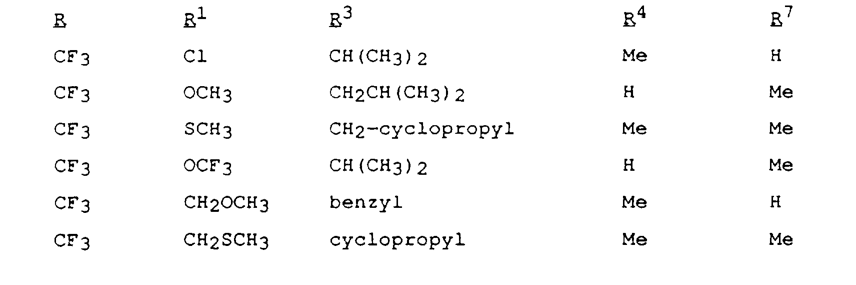

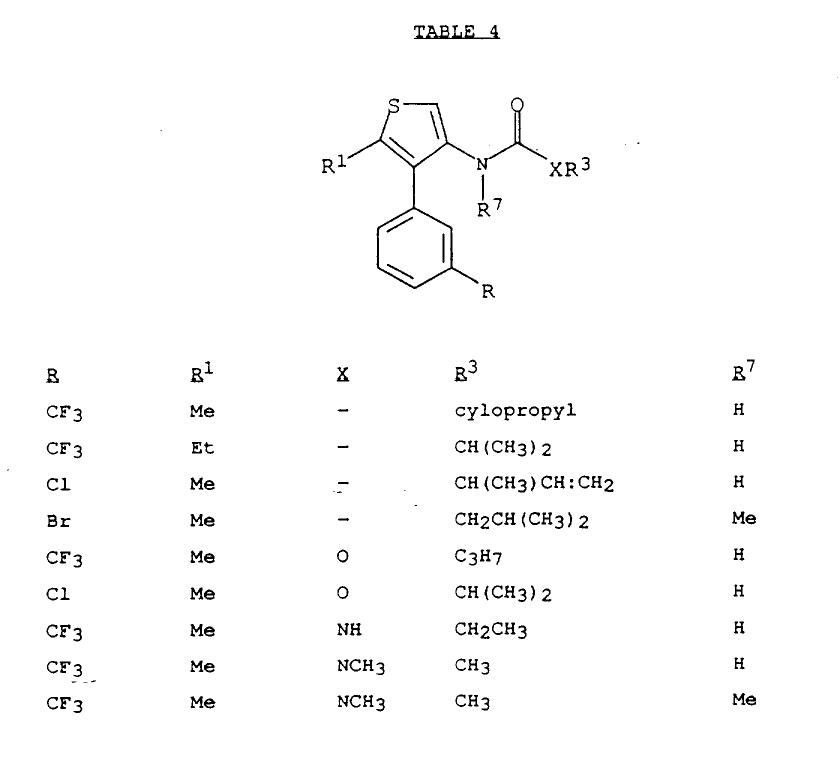

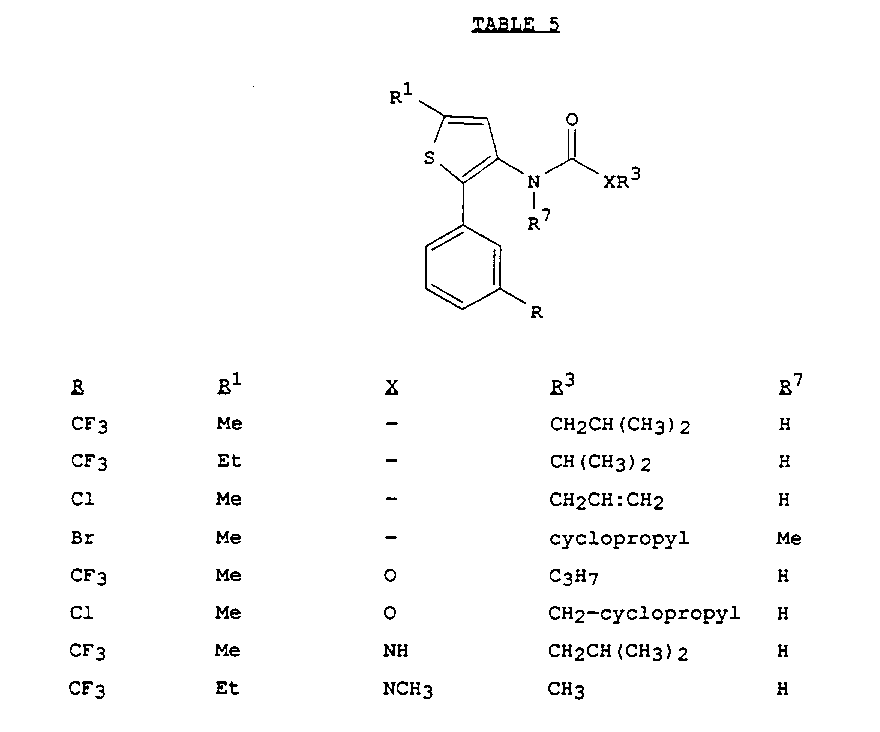

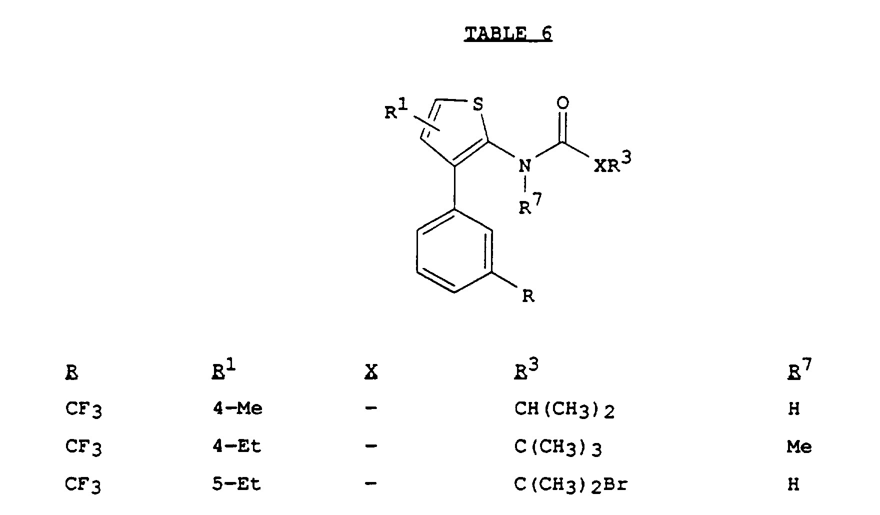

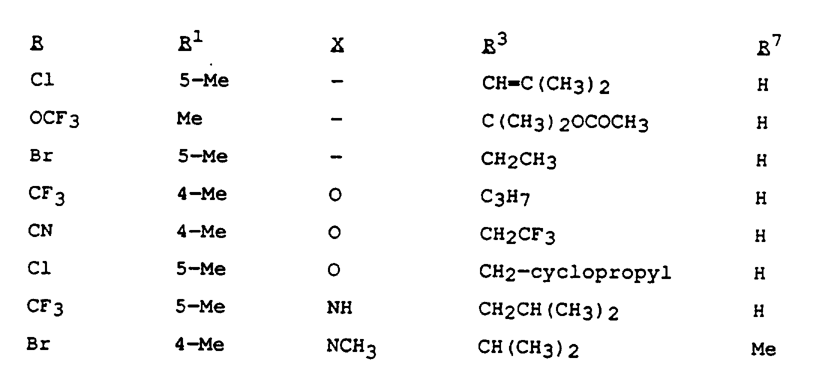

### Formulation

Compounds of this invention will generally be used in formulation with an agriculturally suitable carrier comprising a liquid or solid diluent or an organic solvent. Use formulations include dusts, granules, pellets, solutions, suspensions, emulsions, wettable powders, emulsifiable concentrates, dry flowables and the like, consistent with the physical properties of the active ingredient, mode of application and environmental factors such as soil type, moisture and temperature. Sprayable formulations can be extended in suitable media and used at spray volumes from about one to several hundred liters per hectare. High strength compositions are primarily used as intermediates for further formulation. The formulations will typically contain effective amounts of active ingredient, diluent and surfactant within the following approximate ranges which add up 100 weight percent.

| | Weight Percent | | |
|---|---|---|---|
| | Active Ingredient | Diluent | Surfactant |
| Wettable Powders | 25-90 | 0-74 | 1-10 |
| | | | |
| Oil Suspensions, Emulsions, Solutions, (including Emulsifiable Concentrates) | 5-50 | 40-95 | 0-15 |
| Dusts | 1-25 | 70-99 | 0-5 |
| Granules and Pellets | 0.01-99 | 5-99.99 | 0-15 |
| High Strength Compositions | 90-99 | 0-10 | 0-2 |

Typical solid diluents are described in Watkins, et al., *Handbook of Insecticide Dust Diluents and Carriers,* 2nd Ed., Dorland Books, Caldwell, New Jersey. Typical liquid diluents and solvents are described in Marsden, *Solvents Guide,* 2nd Ed., Interscience, New York, 1950. *McCutcheon's Detergents and Emulsifiers Annual,* Allured Publ. Corp., Ridgewood, New Jersey, as well as Sisely and Wood, *Encyclopedia of Surface Active Agents,* Chemical Publ. Co., Inc., New York, 1964, list surfactants and recommended uses. All formulations can contain minor amounts of additives to reduce foam, caking, corrosion, microbiological growth, etc.

Solutions are prepared by simply mixing the ingredients. Fine solid compositions are made by blending and, usually, grinding as in a hammer mill or fluid energy mill. Water-dispersible granules can be produced be agglomerating a fine powder composition; see for example, Cross et al., *Pesticide Formulations,* Washington, D.C., 1988, pp 251-259. Suspensions are prepared by wet-milling; see, for example, U.S. 3,060,084. Granules and pellets can be made by spraying the active material upon preformed granular carriers or by agglomeration techniques. See Browning, "Agglomeration", *Chemical Engineering,* December 4, 1967, pp 147-48, *Perry's Chemical Engineer's Handbook,* 4th Ed., McGraw-Hill, New York, 1963, pages 8-57 and following, and WO 91/13546. Pellets can be prepared as described in U.S. 4,172,714. Water-dispersible and water-soluble granules can also be prepared as taught in DE 3,246,493.

For further information regarding the art of formulation, see U.S. 3,235,361, Col. 6, line 16 through Col. 7, line 19 and Examples 10-41; U.S. 3,309,192, Col. 5, line 43 through Col. 7, line 62 and Examples 8, 12, 15, 39, 41, 52, 53, 58, 132, 138-140, 162-164, 166, 167 and 169-182; U.S. 2,891,855, Col. 3, line 66 through Col. 5, line 17 and Examples 1-4; Klingman, *Weed Control as a Science,* John Wiley and Sons, Inc., New York, 1961, pp 81-96; and Hance et al., *Weed Control Handbook,* 8th Ed., Blackwell Scientific Publications, Oxford, 1989.

In the following Examples, all percentages are by weight and all formulations are worked up in conventional ways. Compound numbers refer to compounds in Table 7.

### Example A

| High Strength Concentrate | |
|---|---|
| Compound 1 | 98.5% |
| silica aerogel | 0.5% |
| synthetic amorphous fine silica | 1.0% |

### Example B

| Wettable Powder | |
|---|---|
| Compound 1 | 65.0% |
| dodecylphenol polyethylene glycol ether | 2.0% |
| sodium ligninsulfonate | 4.0% |
| sodium silicoaluminate | 6.0% |
| montmorillonite (calcined) | 23.0% |

### Example C

| Granule | |
|---|---|
| Compound 1 | 10.0% |
| attapulgite granules (low volative matter, 0.71/0.30 mm; U.S.S. No. 25-50 sieves) | 90.0% |

### Example D

| Extruded Pellet | |
|---|---|
| Compound 1 | 25.0% |
| anhydrous sodium sulfate | 10.0% |
| crude calcium ligninsulfonate | 5.0% |
| sodium alkylnaphthalenesulfonate | 1.0% |
| calcium/magnesium bentonite | 59.0% |

### UTILITY

Test results indicate compounds of this invention are active postemergence and preemergence herbicides. Many compounds in this invention are useful for the control of selected grass and broadleaf weeds with tolerance to important agronomic crops such as barley (Hordeum vulgare), corn (Zea mays), cotton (Gossypium hirsutum), rice (Oryza sativa), sorghum (Sorghum bicolor), soybean (Glycine max) and wheat (Triticum aestivum). Grass and broadleaf weed species controlled include, but are not limited to, barnyardgrass (Echinochloa crusgralli), bedstraw (Galium aparine), blackgrass (Alopecurus myosuroides), chickweed (Stellaria media), crabgrass (Digitaria spp.), foxtail (Setaria spp.), lambsquarters (Chenopodium spp.), velvetleaf (Abutilon theophrasti), wild buckwheat (Polygonum convolvulus) and wild oats (Avena fatua).

These compounds also have utility for weed control of selected vegetation in specified areas such as around storage tanks, parking lots, highways, and railways; in fallow crop areas; and in citrus and plantation crops such as banana, coffee, oil palm, and rubber. Alternatively, these compounds are useful to modify plant growth.

Effective amounts of the compounds of this invention are determined by a number of factors. These factors include: formulation selected, method of application, amount and type of vegetation present, growing conditions, etc. In general, effective amounts of compounds are applied at rates from 0.01 to 20 kg/ha with a preferred rate range of 0.02 to 4 kg/ha. Although a small number of compounds show slight herbicidal activity at the rates tested, it is anticipated these compounds are herbicidally active at higher application rates. One skilled in the art can easily determine effective application rates necessary for desired level of weed control.

Compounds of this invention can be used alone or in combination with other commercial herbicides, insecticides or fungicides. A mixture of one or more of the following herbicides with a compound of this invention may be particularly useful for weed control. Examples of other herbicides with which compounds of this invention can be formulated are: acetochlor, acifluorfen, acrolein, 2-propenal, alachlor, ametryn, amidosulfuron, ammonium sulfamate, amitrole, anilofos, asulam, atrazine, barban, benefin, bensulfuron methyl, bensulide, bentazon, benzofluor, benzoylprop, bifenox, bromacil, bromoxynil, bromoxynil heptanoate, bromoxynil octanoate, butachlor, buthidazole, butralin, butylate, cacodylic acid, 2-chloro-*N,N*-di-2-propenylacetamide, 2-chloroallyl diethyldithiocarbamate, chloramben, chlorbromuron, chloridazon, chlorimuron ethyl, chlormethoxynil, chlornitrofen, chloroxuron, chlorpropham, chlorsulfuron, chlortoluron, cinmethylin, cinosulfuron, clethodim, clomazone, cloproxydim, clopyralid, calcium salt of methylarsonic acid, cyanazine, cycloate, cycluron, cyperquat, cyprazine, cyprazole, cypromid, dalapon, dazomet, dimethyl 2,3,5,6-tetrachloro-1,4-benzenedicarboxylate, desmedipham, desmetryn, dicamba, dichlobenil, dichlorprop, diclofop, diethatyl, difenzoquat, diflufenican, dimepiperate, dinitramine, dinoseb, diphenamid, dipropetryn, diquat, diuron, 2-methyl-4,6-dinitrophenol, disodium salt of methylarsonic acid, dymron, endothall, S-ethyl dipropylcarbamothioate, esprocarb, ethalfluralin, ethametsulfuron methyl, ethofumesate, fenac, fenoxaprop, fenuron, salt of fenuron and trichloroacetic acid, flamprop, fluazifop, fluazifop-P, fluchloralin, flumesulam, flumipropyn, fluometuron, fluorochloridone, fluorodifen, fluoroglycofen, flupoxam, fluridone, fluroxypyr, fluzasulfuron, fomesafen, fosamine, glyphosate, haloxyfop, hexaflurate, hexazinone, imazamethabenz, imazapyr, imazaquin, imazamethabenz methyl, imazethapyr, imazosulfuron, ioxynil, isopropalin, isoproturon, isouron, isoxaben, karbutilate, lactofen, lenacil, linuron, metobenzuron, metsulfuron methyl, methylarsonic acid, monoammonium salt of methylarsonic acid, (4-chloro-2-methylphenoxy)acetic acid, *S,S'*-dimethyl-2-(difluoromethyl)-4-(2-methylpropyl)-6-(trifluoromethyl)-3,5-pyridinedicarbothioate, mecoprop, mefenacet, mefluidide, methalpropalin, methabenzthiazuron, metham, methazole, methoxuron, metolachlor, metribuzin, 1,2-dihydropyridazine-3,6-dione, molinate, monolinuron, monuron, monuron salt and trichloroacetic acid, monosodium salt of methylarsonic acid, napropamide, naptalam, neburon, nicosulfuron, nitralin, nitrofen, nitrofluorfen, norea, norflurazon, oryzalin, oxadiazon, oxyfluorfen, paraquat, pebulate, pendimethalin, perfluidone, phenmedipham, picloram, 5-[2-chloro-4-(trifluoromethyl)phenoxy]-2-nitroacetophenone oxime-O-acetic acid methyl ester, pretilachlor, primisulfuron, procyazine, profluralin, prometon, prometryn, pronamide, propachlor, propanil, propazine, propham, prosulfalin, prynachlor, pyrazolate, pyrazon, pyrazosulfuron ethyl, quinchlorac, quizalofop ethyl, rimsulfuron, secbumeton, sethoxydim, siduron, simazine, 1-(a,a-dimethylbenzyl)-3-(4-methylphenyl)urea, sulfometuron methyl, trichloroacetic acid, tebuthiuron, terbacil, terbuchlor, terbuthylazine, terbutol, terbutryn, thifensulfuron methyl, thiobencarb, tri-allate, trialkoxydim, triasulfuron, tribenuron methyl, triclopyr, tridiphane, trifluralin, trimeturon, (2,4-dichlorophenoxy)acetic acid, 4-(2,4-dichlorophenoxy)butanoic acid, vernolate, and xylachlor.

Herbicidal properties of the subject compounds were discovered in a number of greenhouse tests. Test procedures and results follow.

Selective herbicidal properties of the subject compounds were discovered in greenhouse tests as described below.

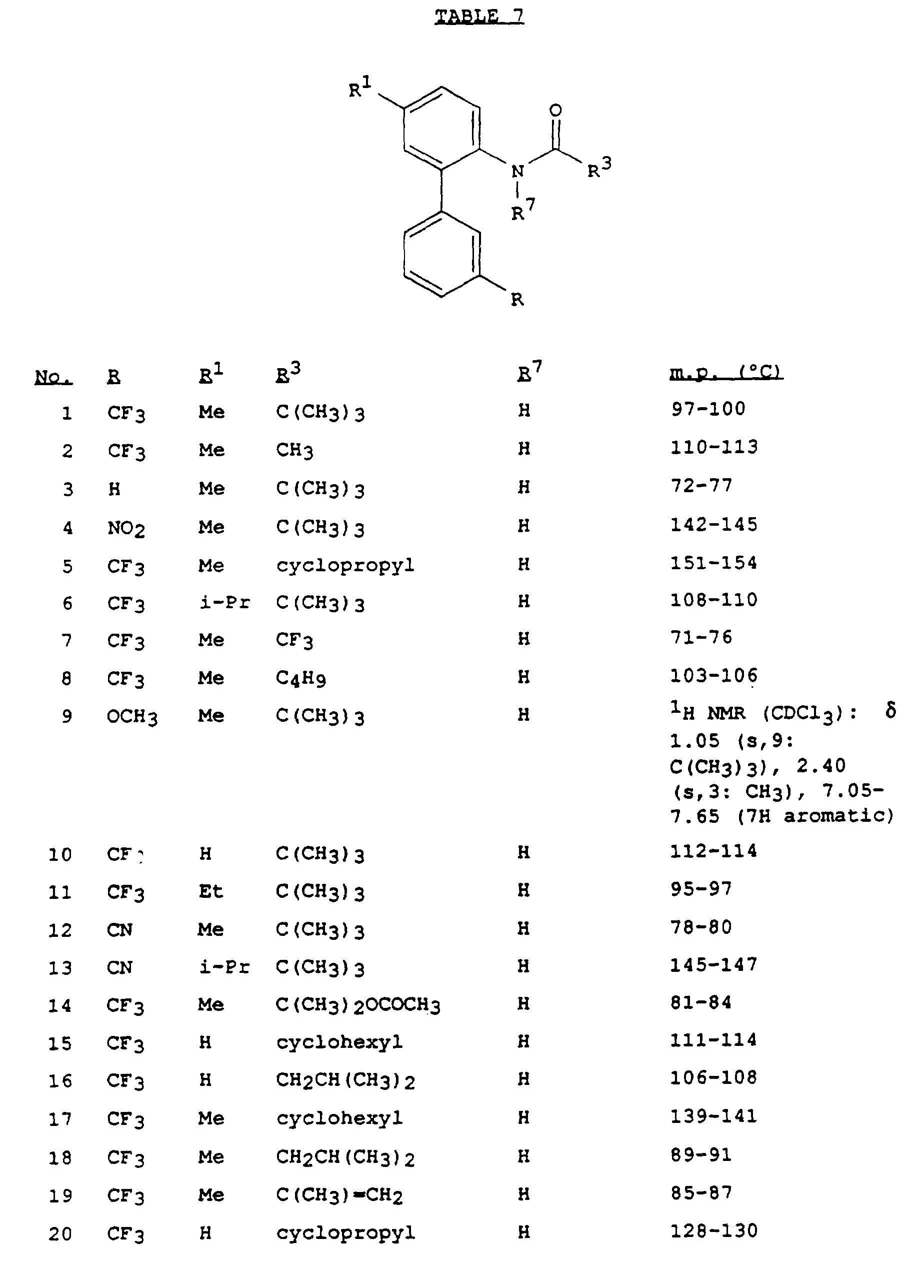

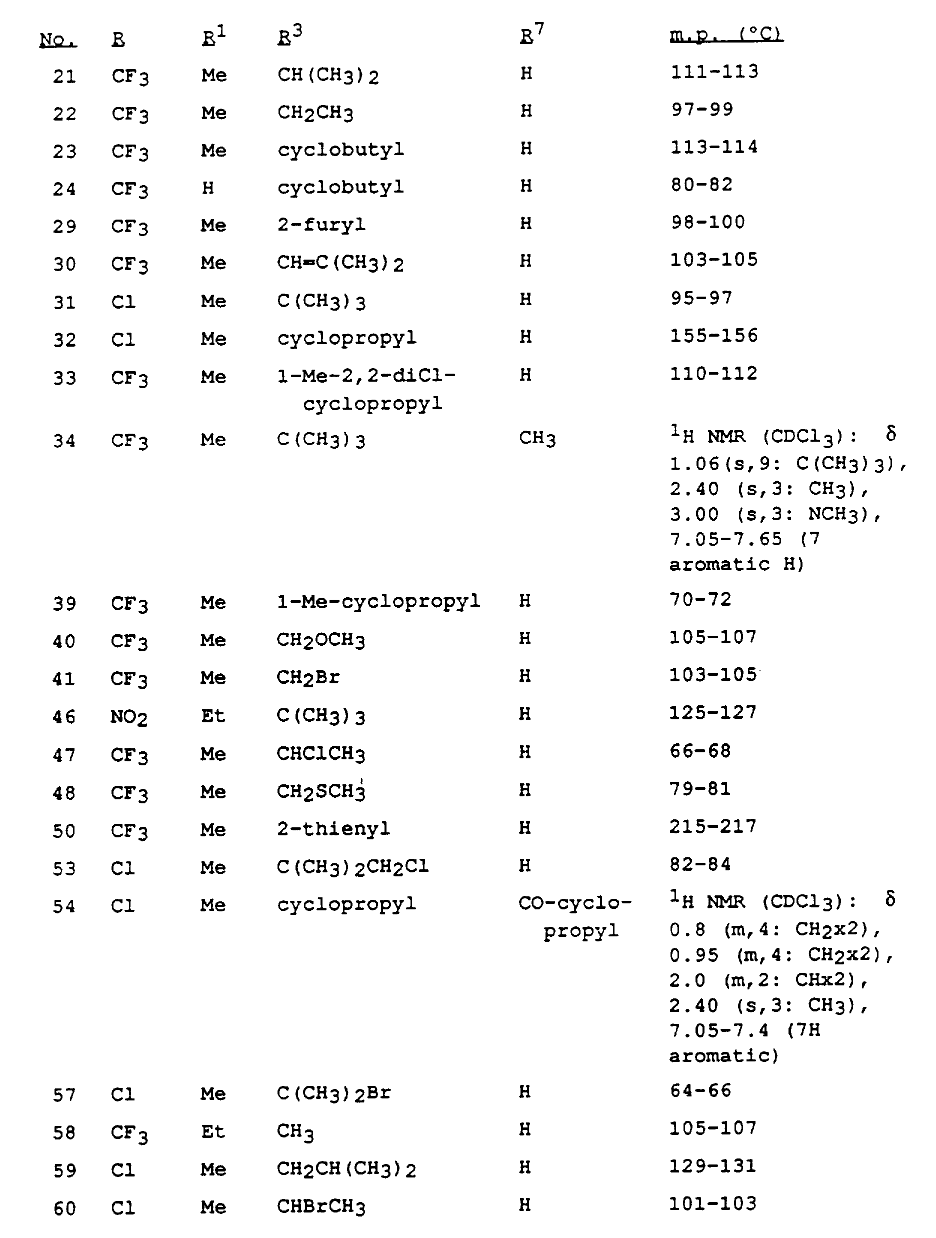

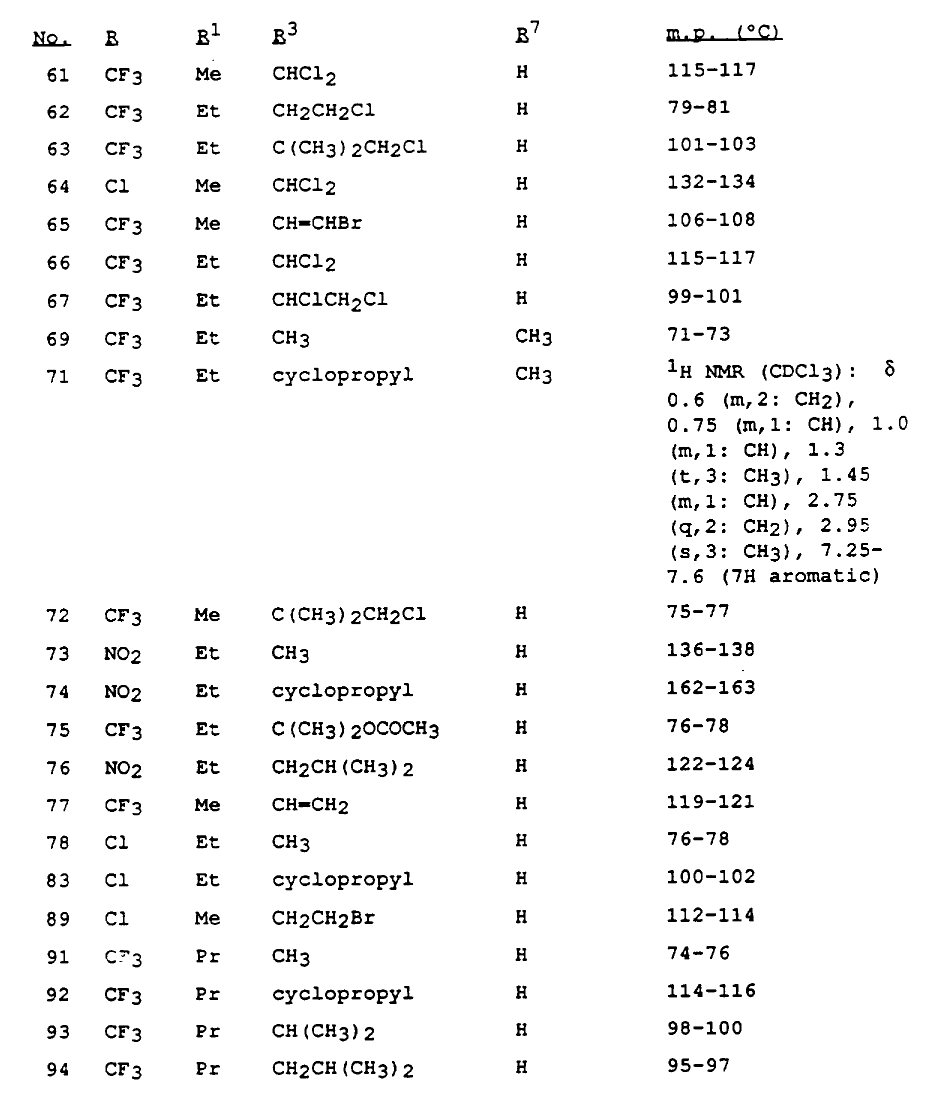

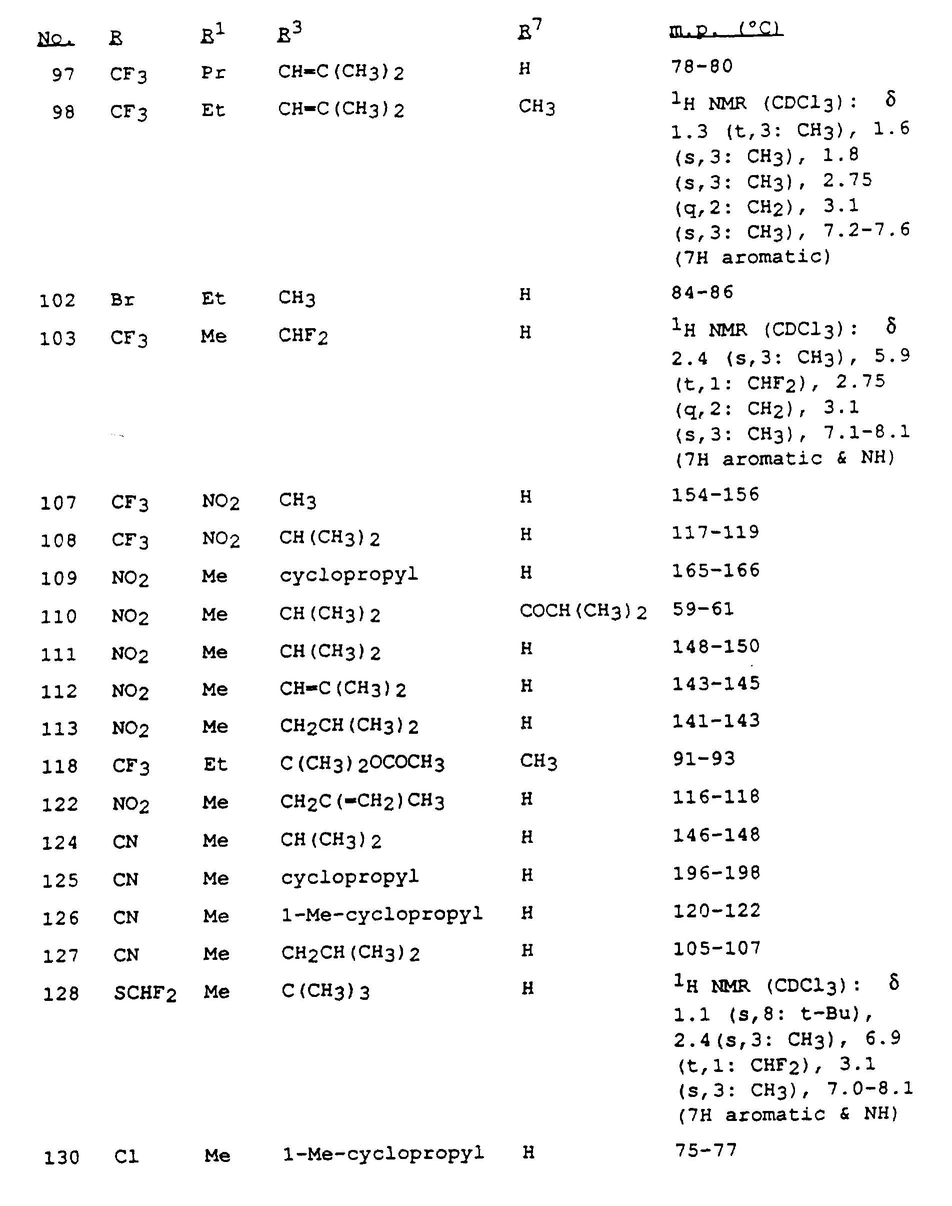

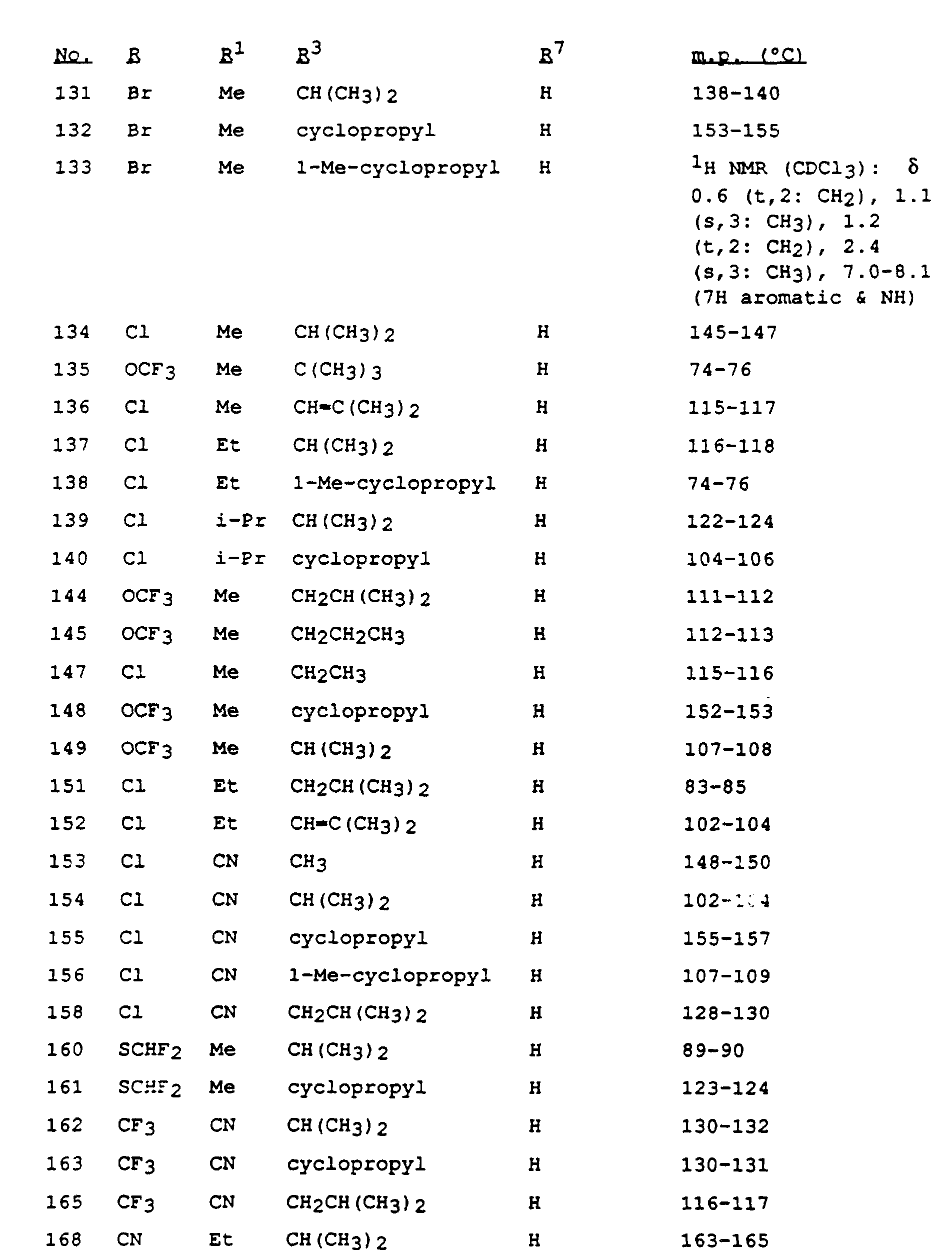

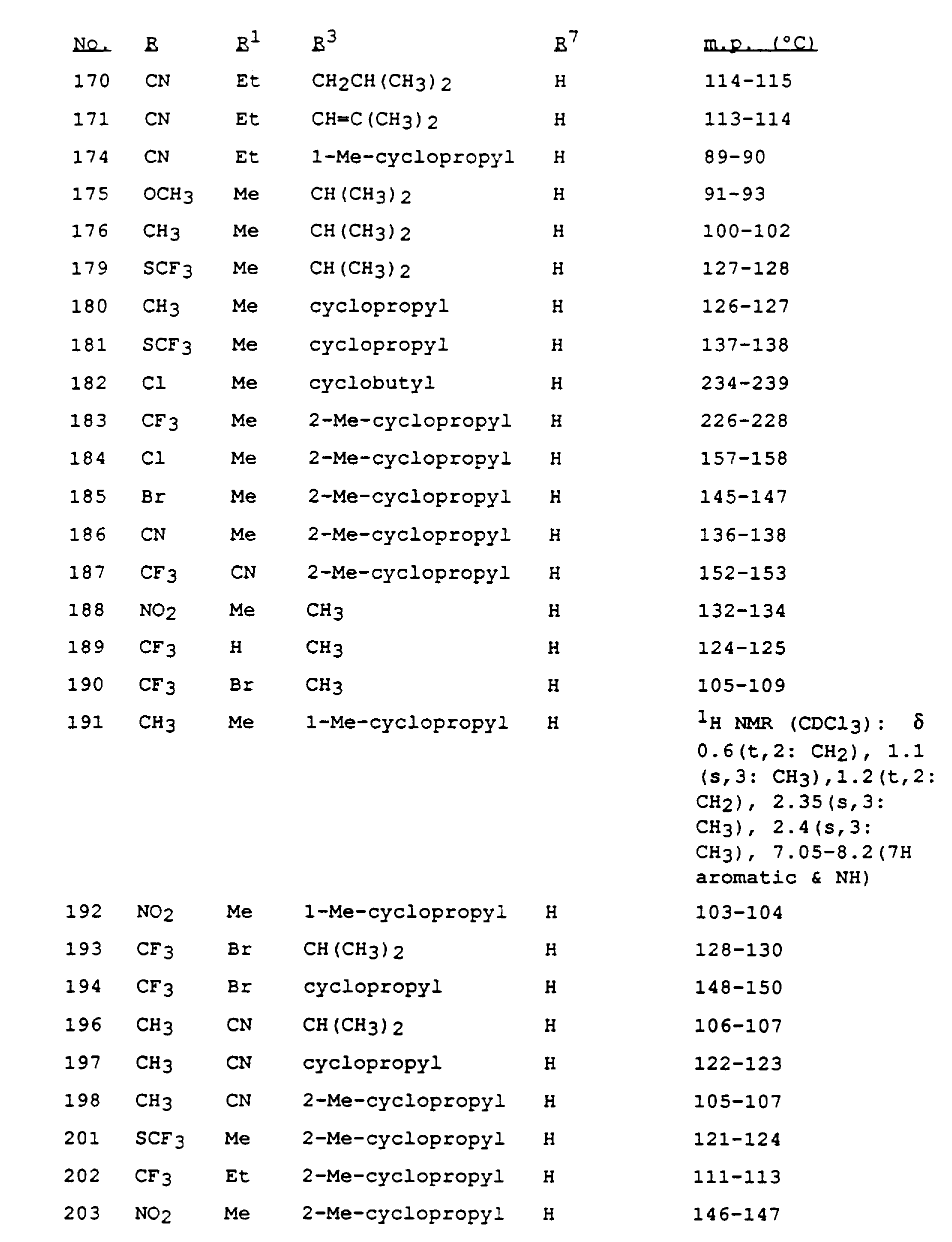

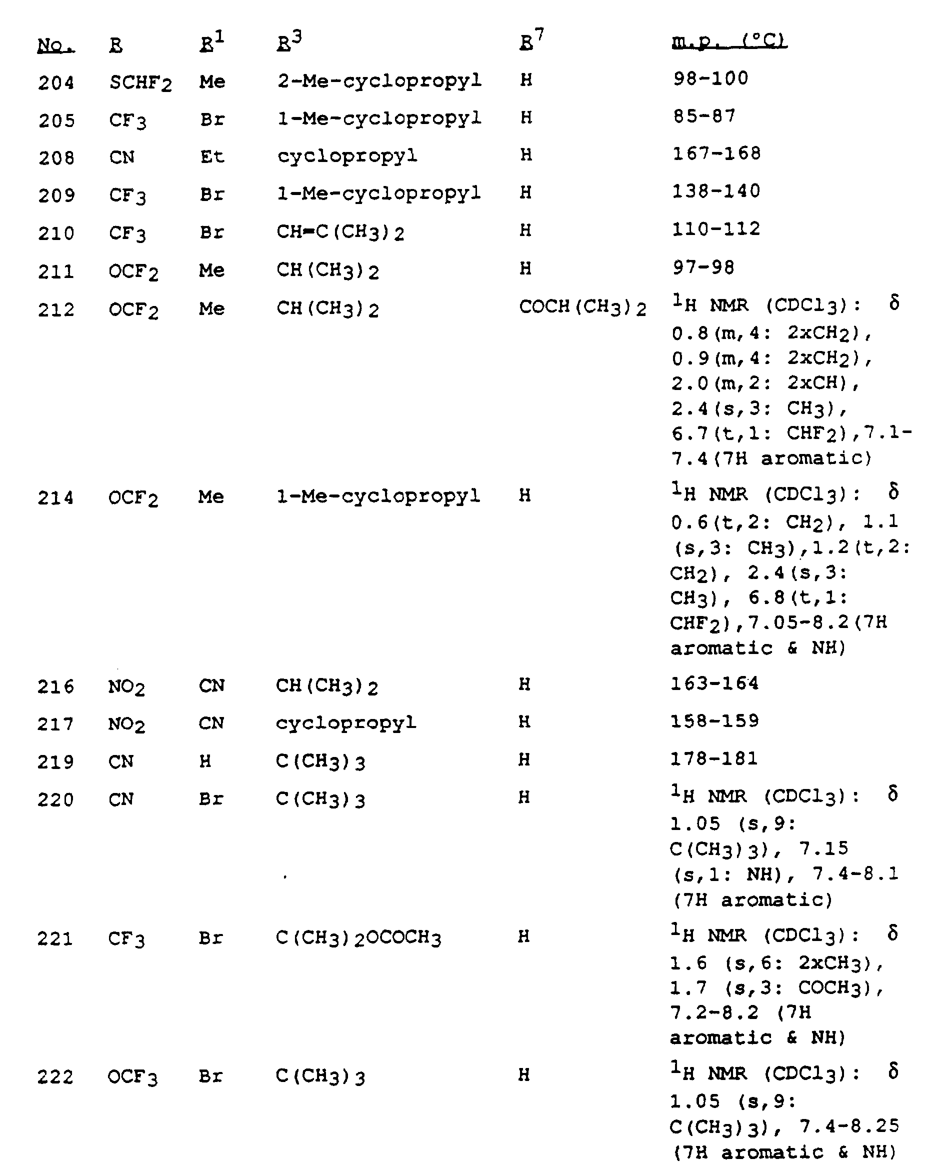

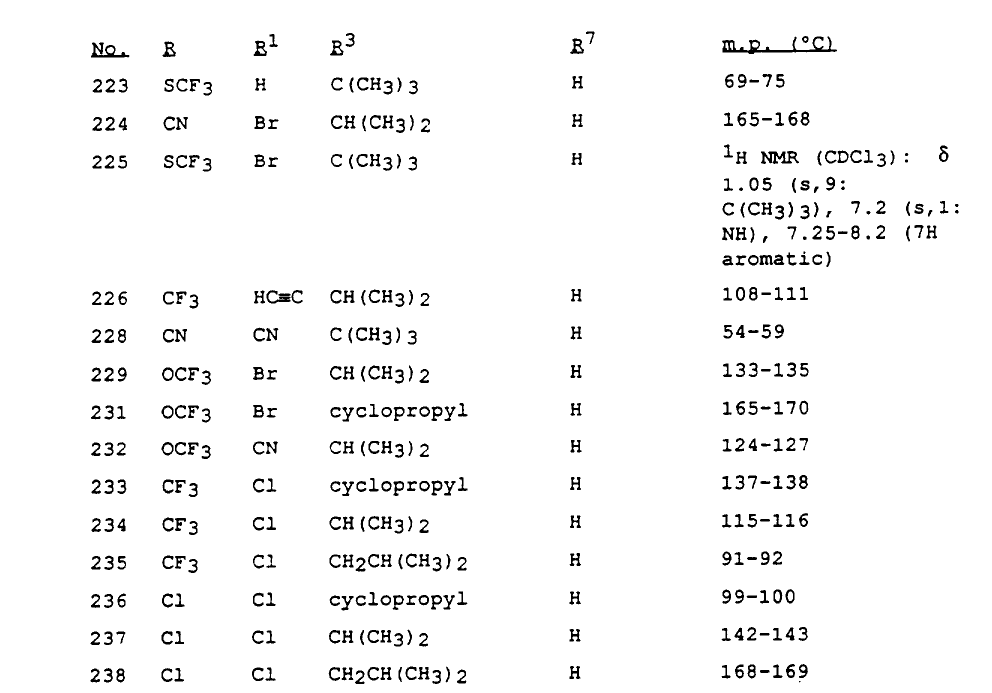

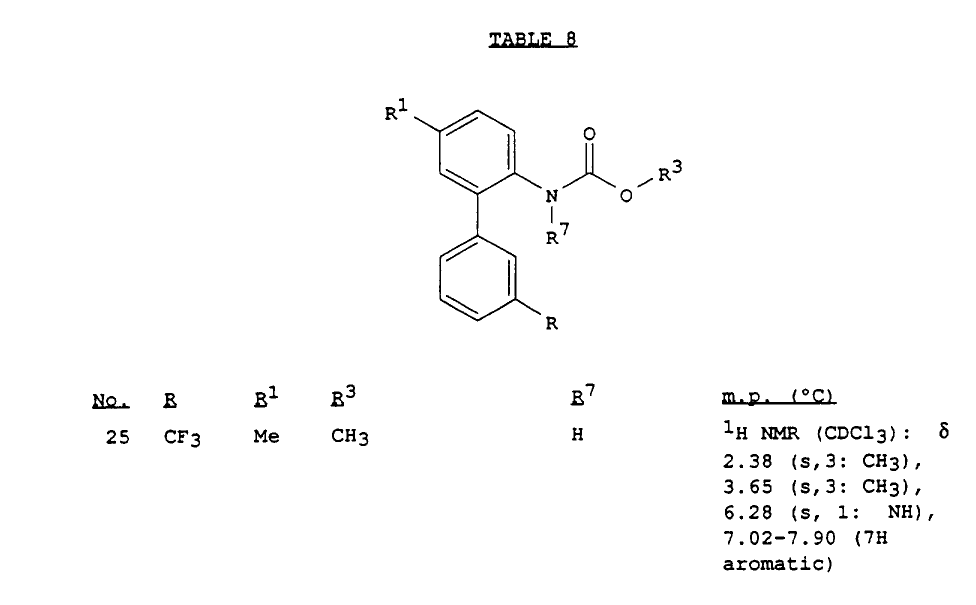

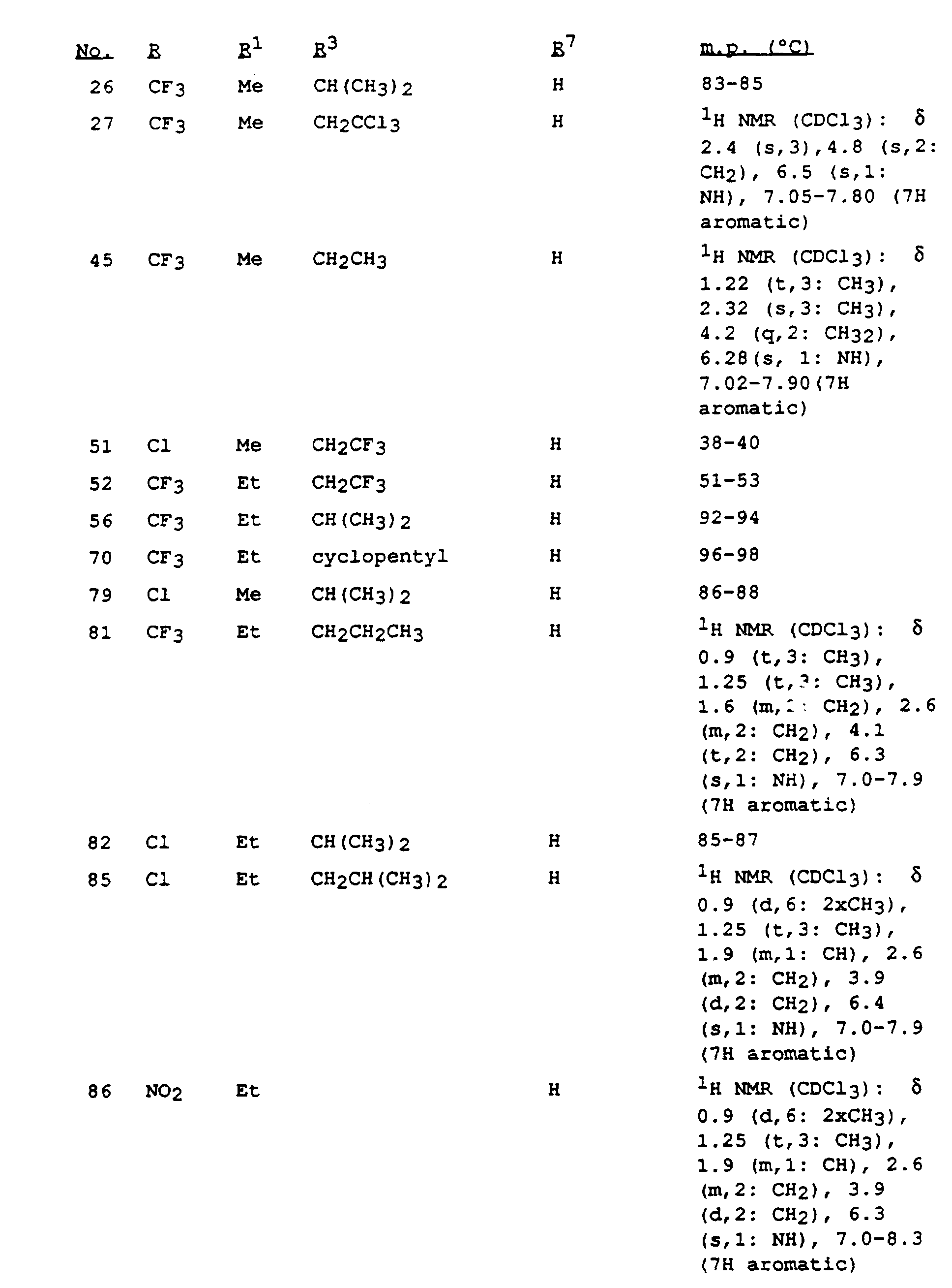

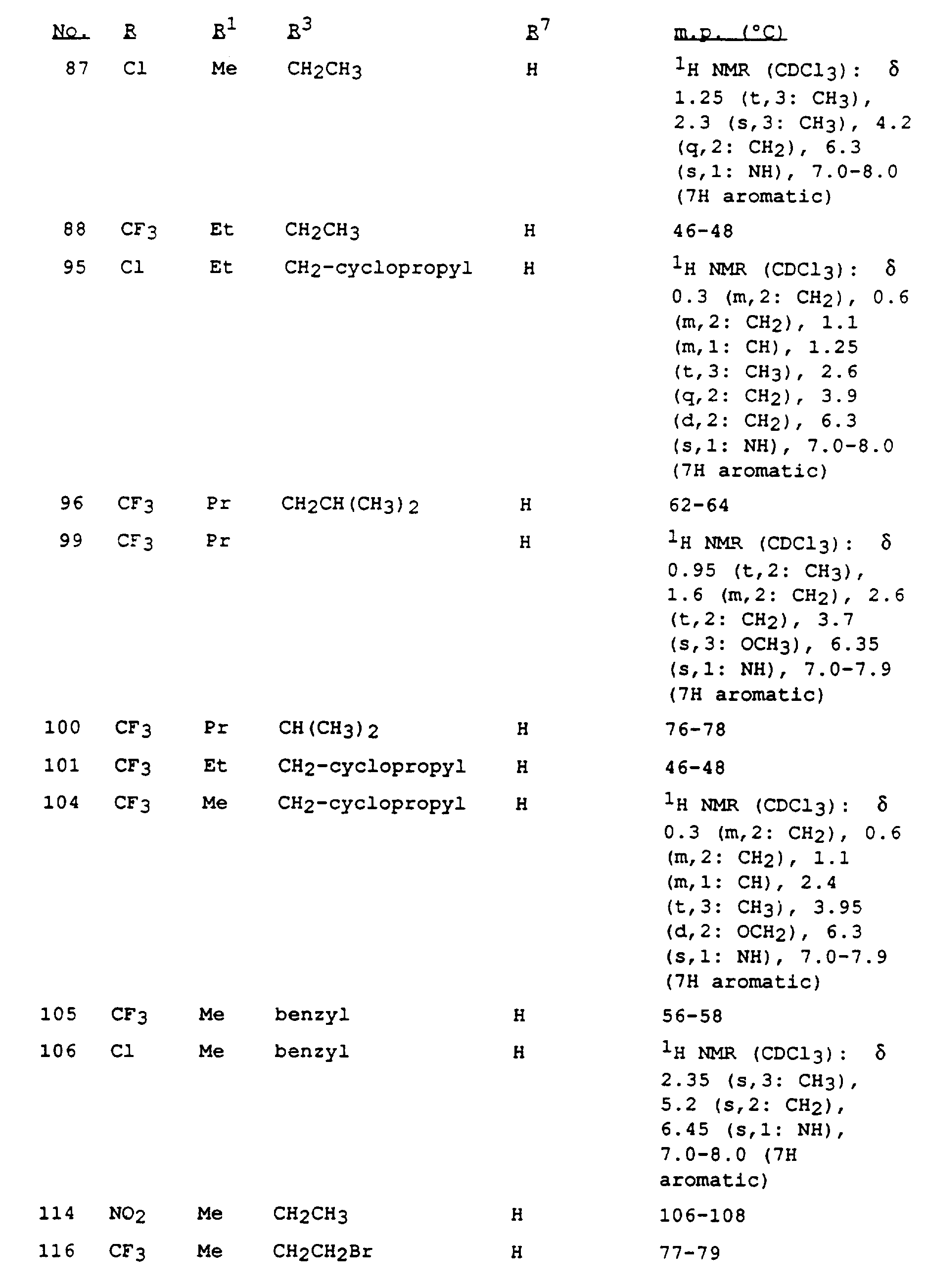

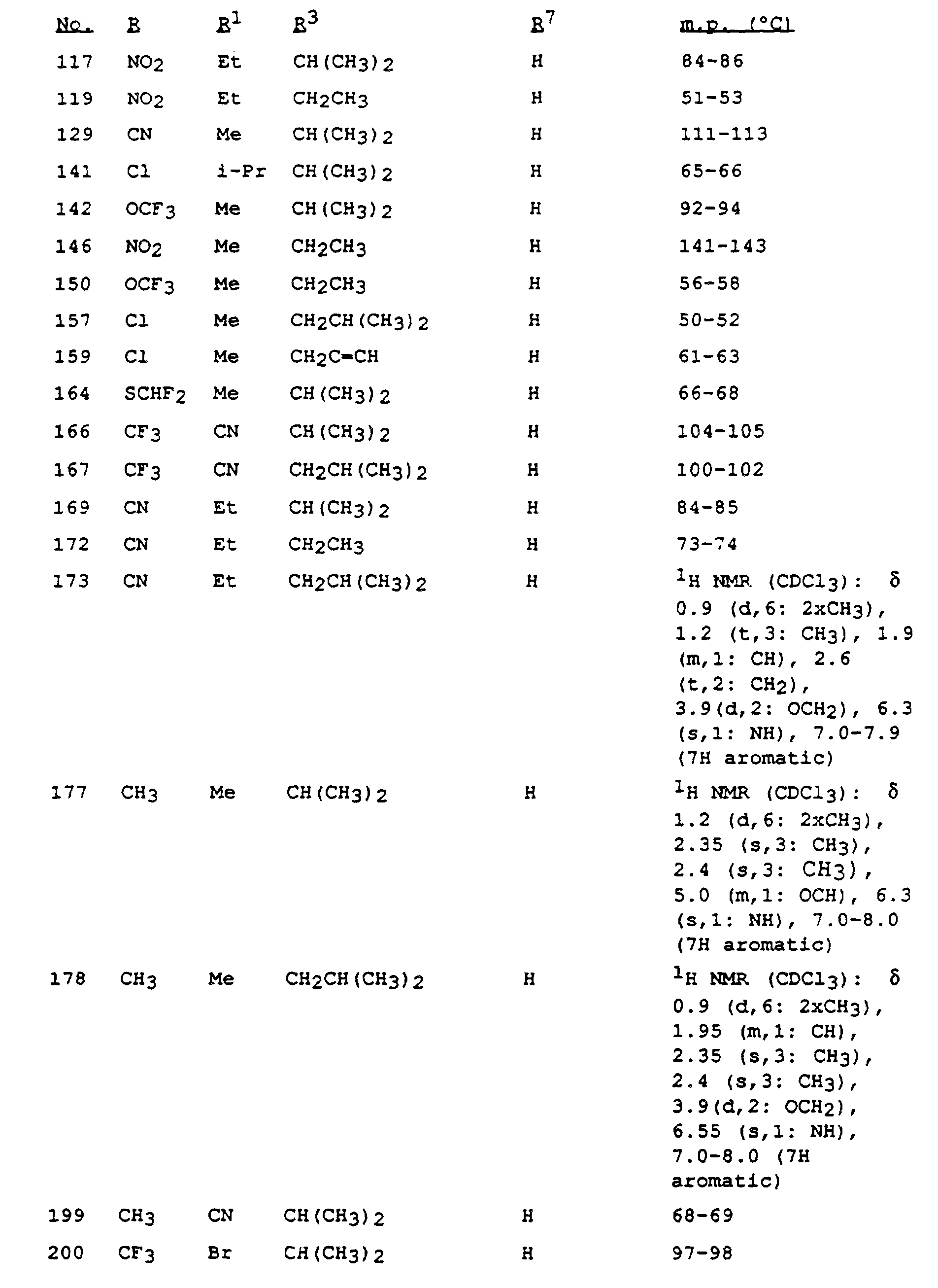

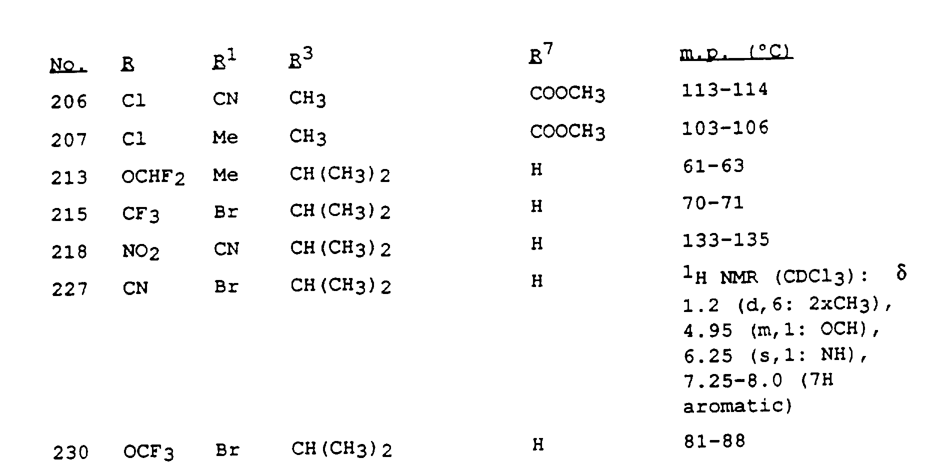

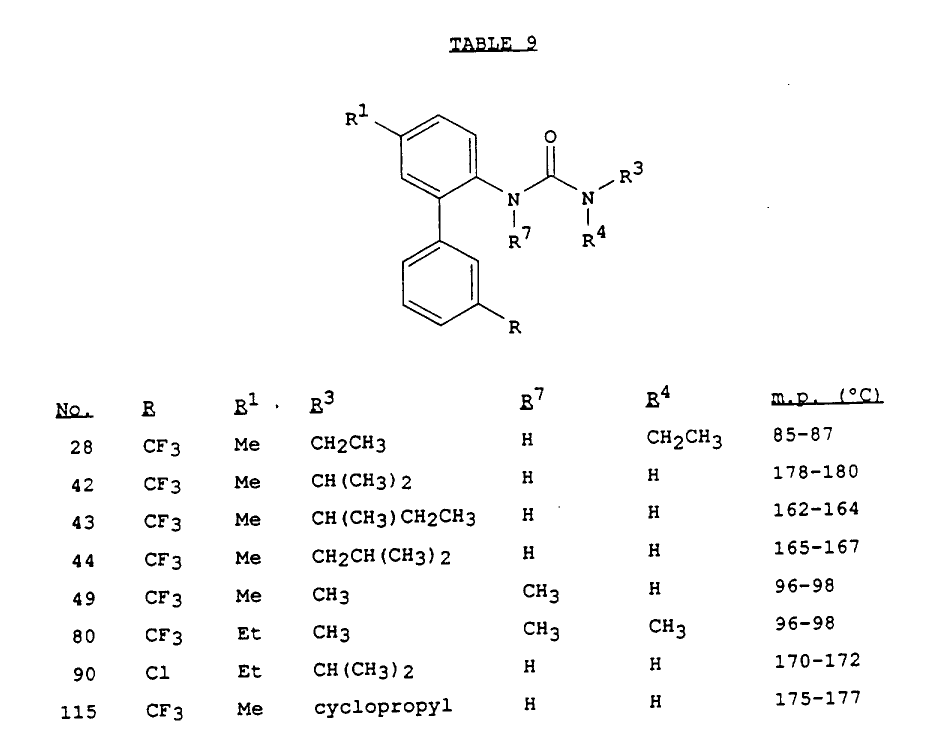

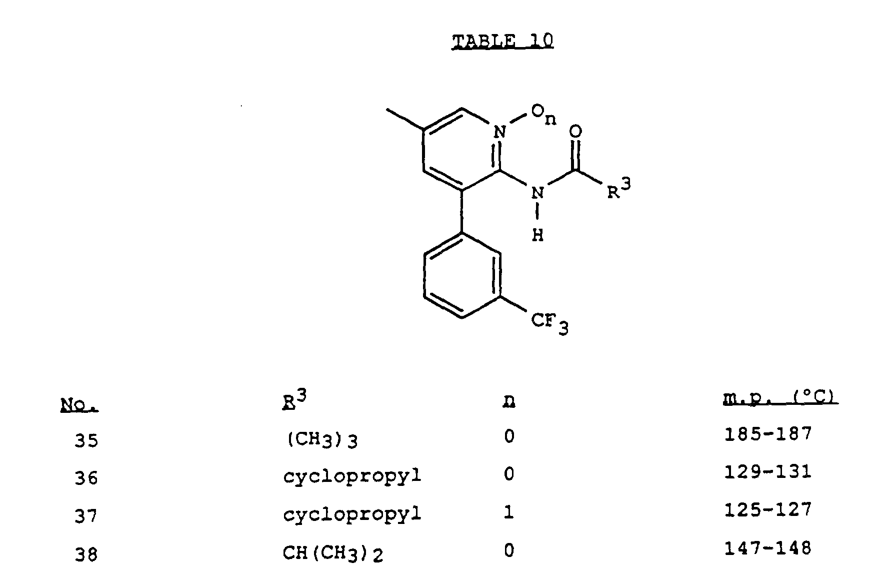

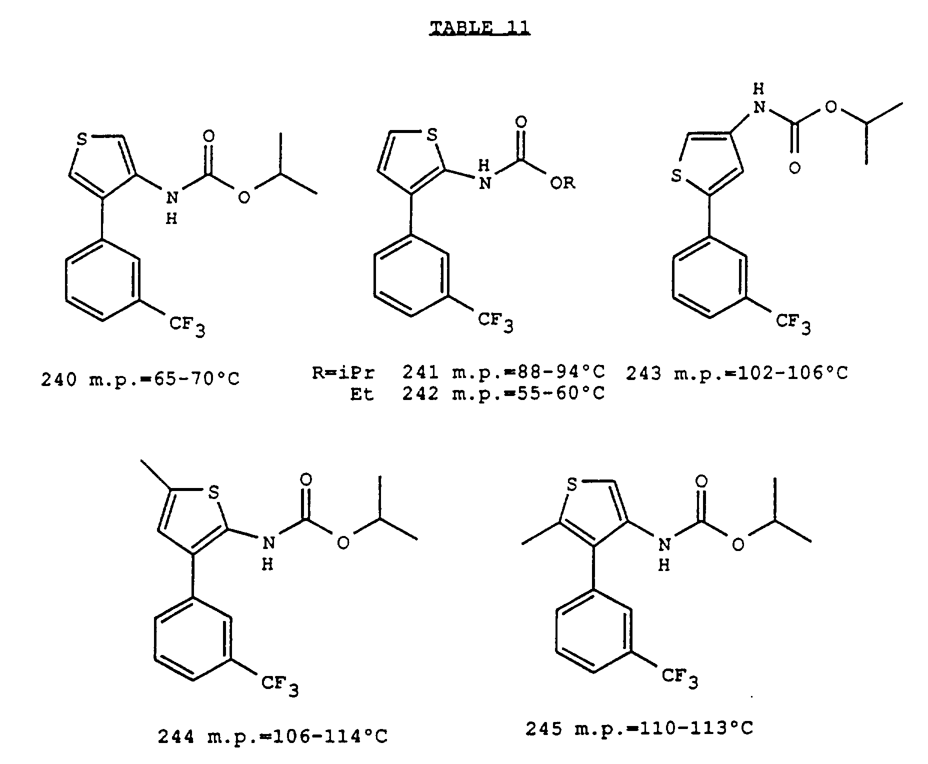

### TEST A

Seeds of barnyardgrass (*Echinochloa crus-galli*), cheatgrass (*Bromus secalinus*)*,* cocklebur (*Xanthium pensylvanicum*), crabgrass (*Digitaria* spp.), giant foxtail (*Setaria faberii*)*,* morningglory (*Ipomoea* spp.), sorghum (*Sorghum bicolor*)*,* velvetleaf (*Abutilon theophrasti*), and wild oat (*Avena fatua*) were planted into a sandy loam soil and treated preemergence with test chemicals dissolved in a non-phytotoxic solvent. At the same time, these crop and weed species were also treated postemergence with test chemicals. Plants ranged in height from two to eighteen cm and were in the two to three leaf stage for the postemergence treatment. Treated plants and untreated controls were maintained in a greenhouse for approximately eleven days, after which all treated plants were compared to untreated controls and visually evaluated for injury. Plant response ratings, summarized in Table A, are based on a 0 to 10 scale where 0 is no effect and 10 is complete control. A dash (-) response means *no* test results.

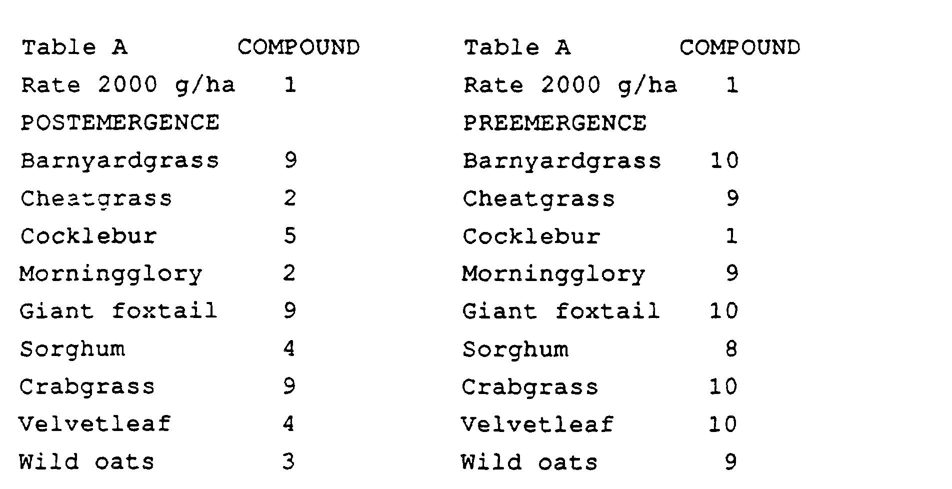

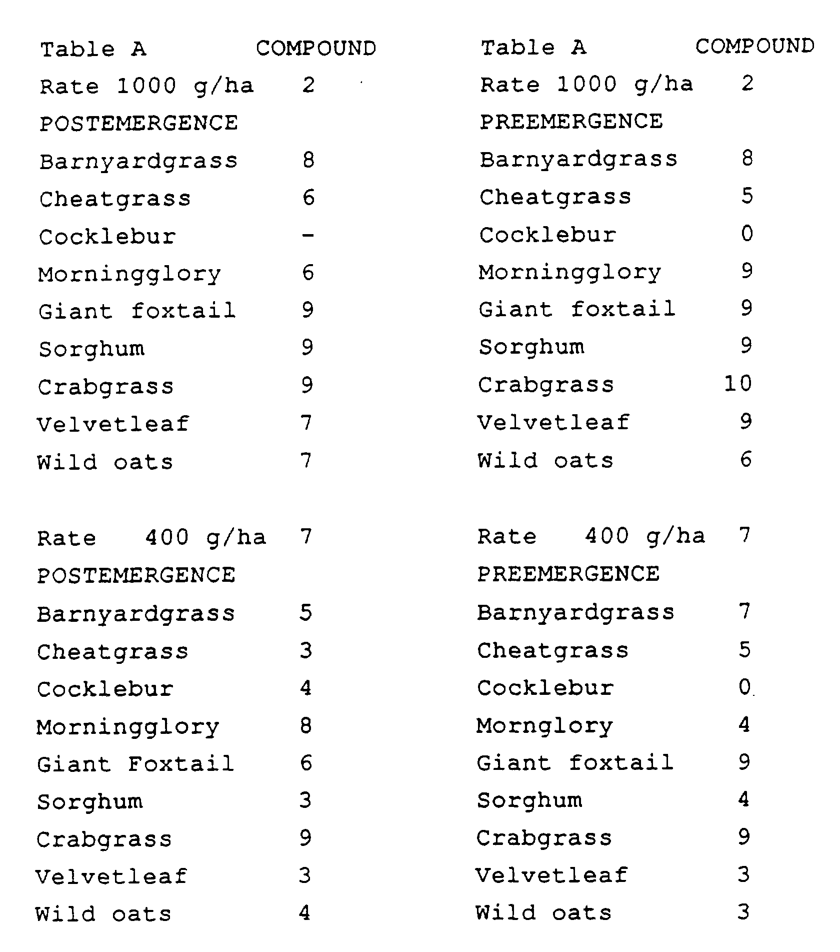

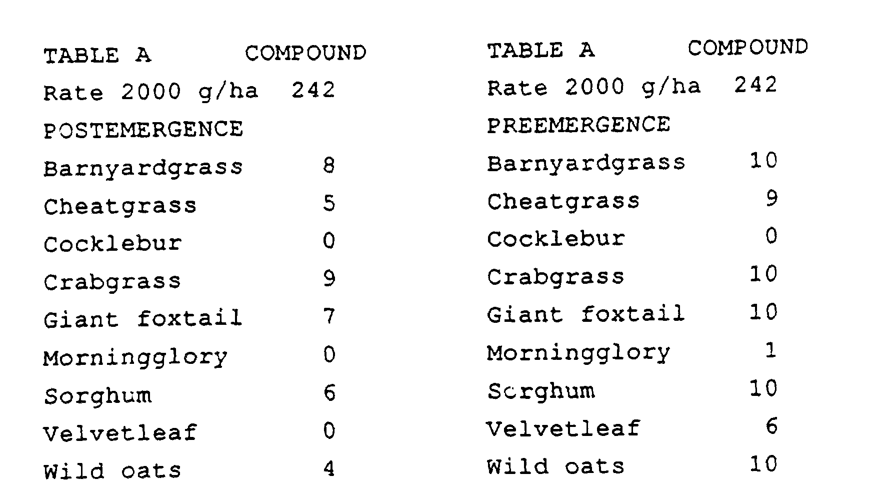

### TEST B

Seeds of barley (*Hordeum vulgare*), barnyardgrass (*Echinochloa crus-galli*), bedstraw (*Galium aparine*), blackgrass (*Alopecurus myosuroides*), cheatgrass (*Bromus secalinus*), chickweed (*Stellaria media*), cocklebur (*Xanthium pensylvanicum*), corn (*Zea mays*), cotton (*Gossypium hirsutum*), crabgrass (*Digitaria* spp.), giant foxtail (*Setaria faberii*)*,* lambsquarters (*Chenopodium album*), morningglory (*Ipomoea hederacea*), rape (*Brassica napus*)*,* rice (*Oryza sativa*), sorghum (*Sorghum bicolor*), soybean (*Glycine max*), sugar beet (*Beta vulgaris*), velvetleaf (*Abutilon theophrasti*), wheat (*Triticum aestivum*), wild buckwheat (*Polygonum convolvulus*), and wild oat (*Avena fatua*) and purple nutsedge (*Cyperus rotundus*) tubers were planted and treated preemergence with test chemicals dissolved in a non-phytotoxic solvent. At the same time, these crop and weed species were also treated with postemergence applications of test chemicals. Plants ranged in height from two to eighteen cm (one to four leaf stage) for postemergence treatments. Treated plants and controls were maintained in a greenhouse for twelve to sixteen days, after which all species were compared to controls and visually evaluated. Plant response ratings, summarized in Table A, are based on a scale of 0 to 10 where 0 is no effect and 10 is complete control. A dash (-) response means no test result.

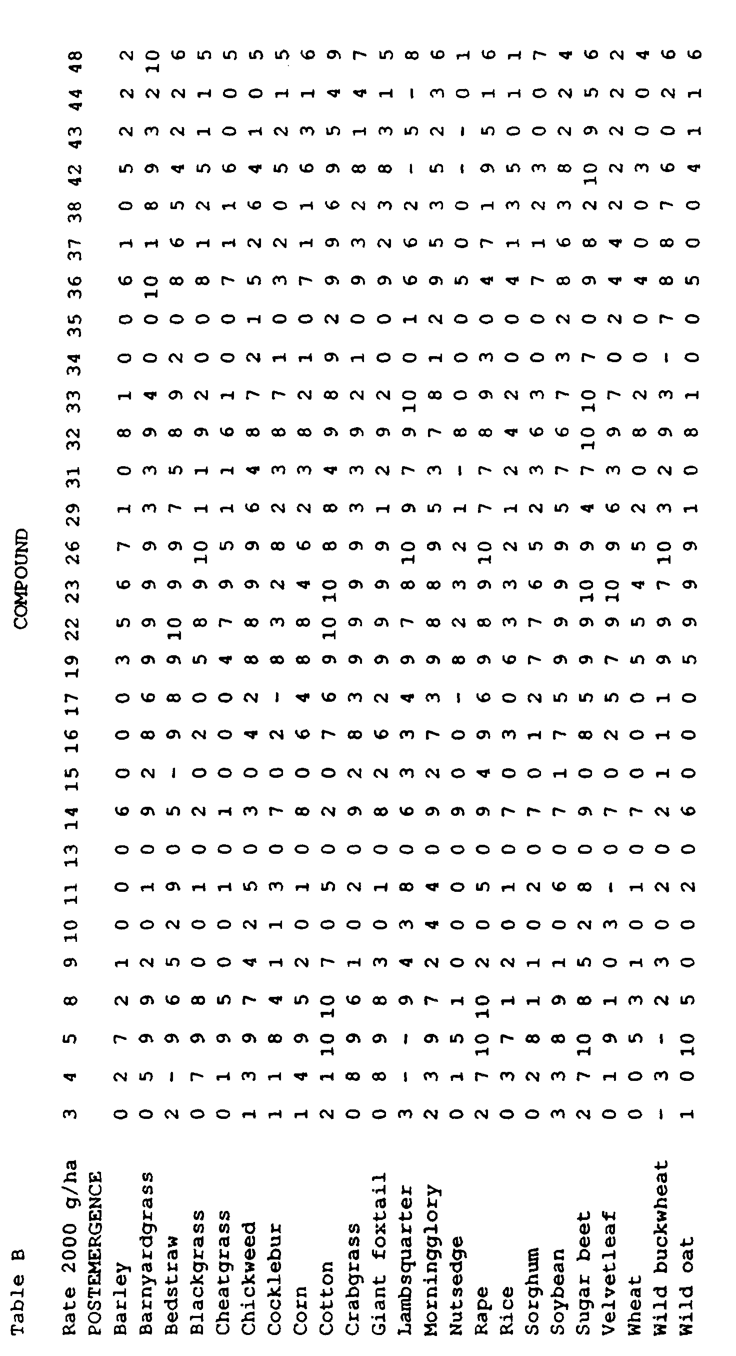

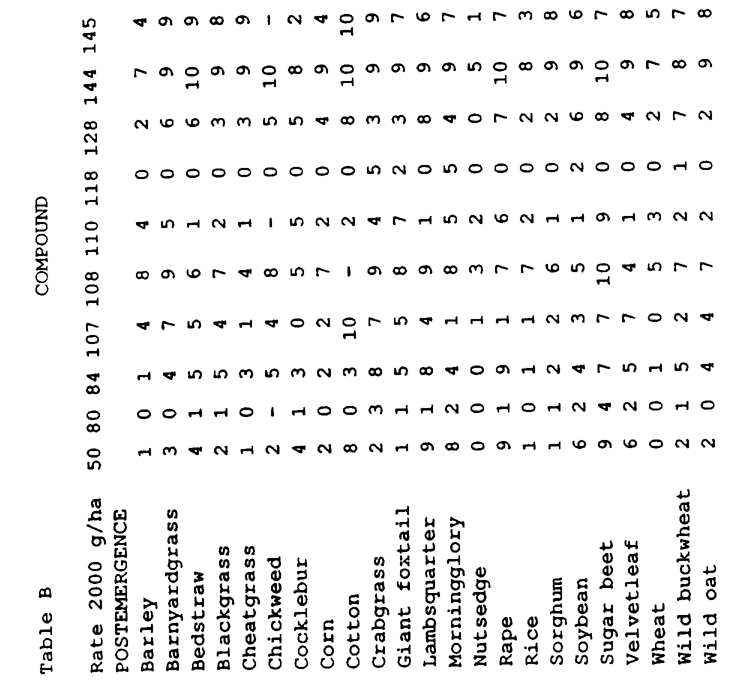

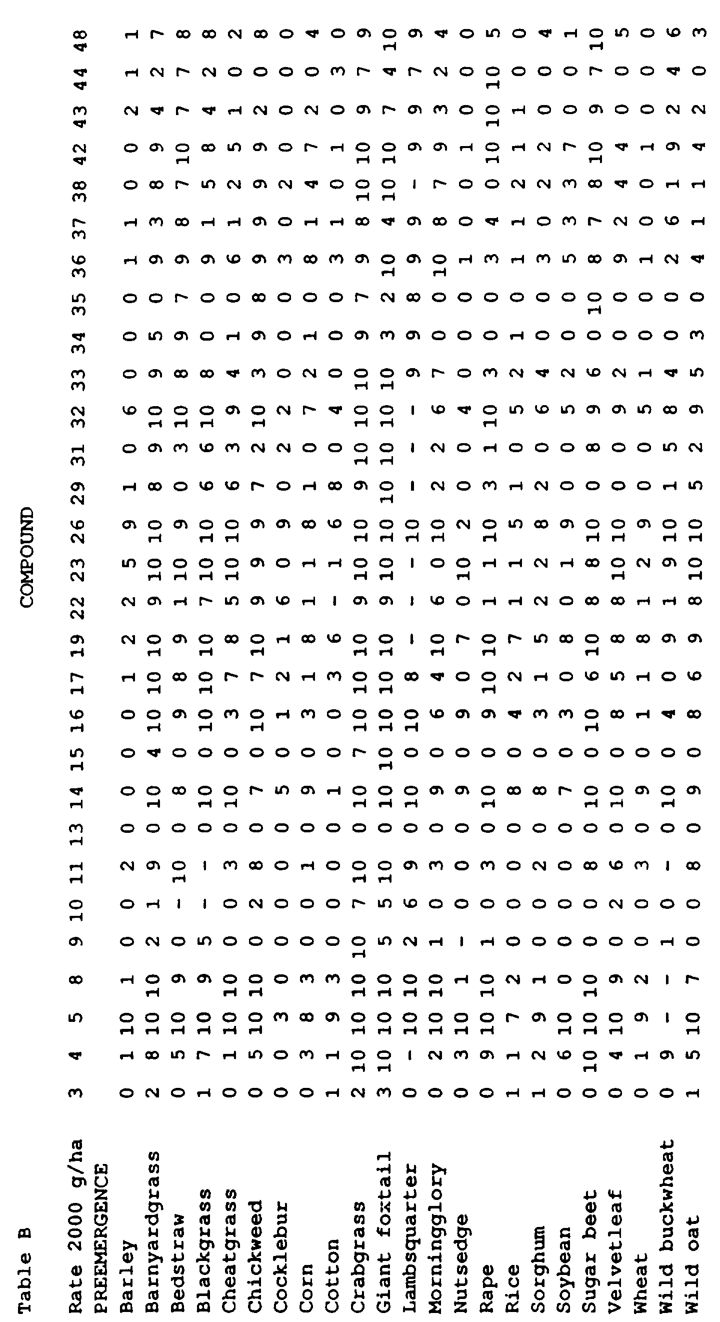

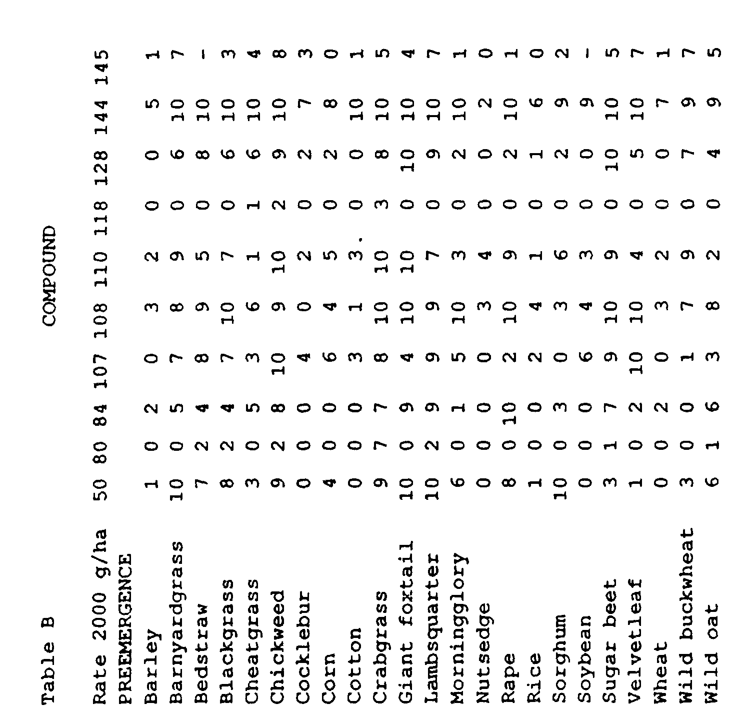

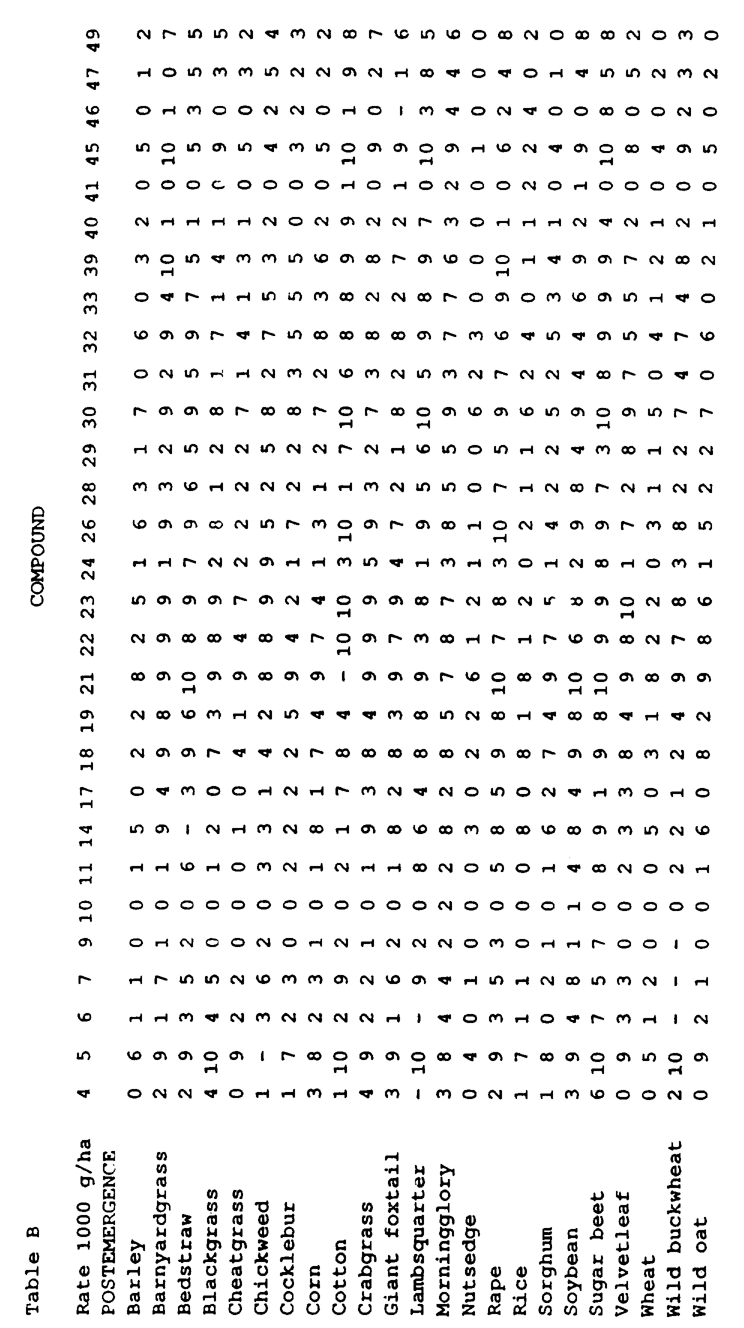

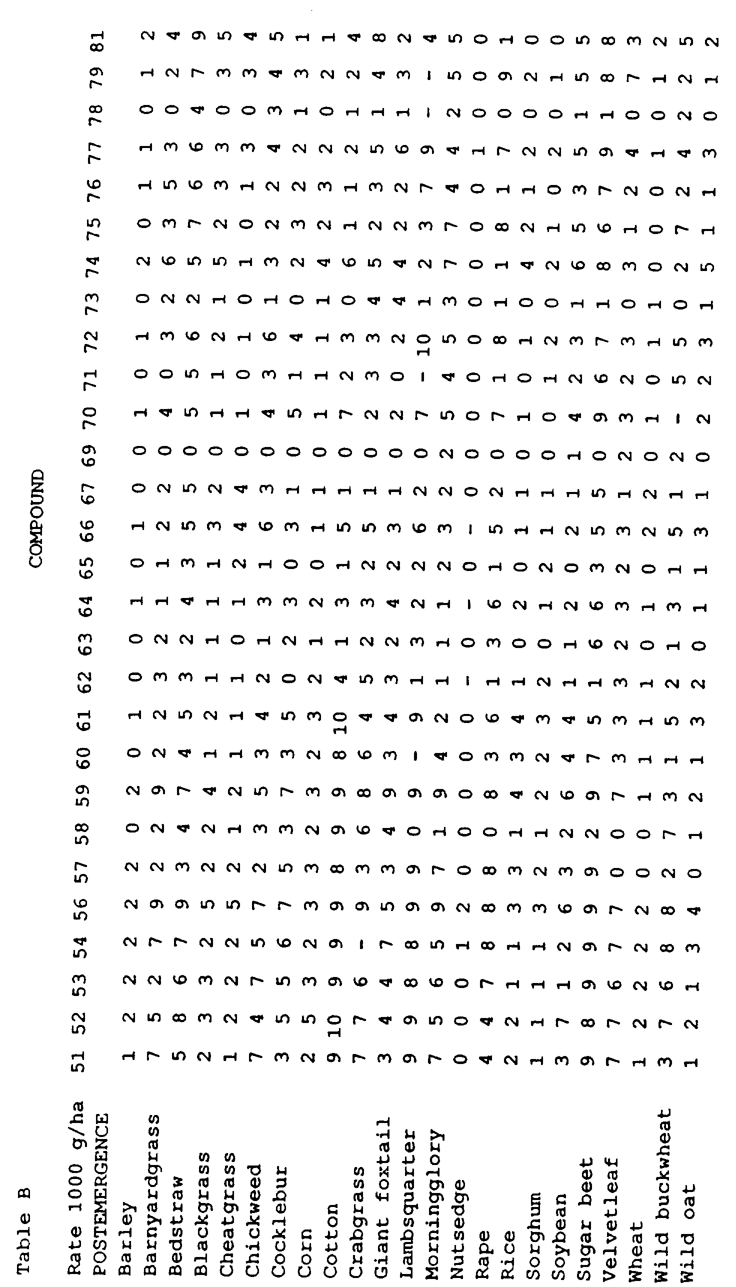

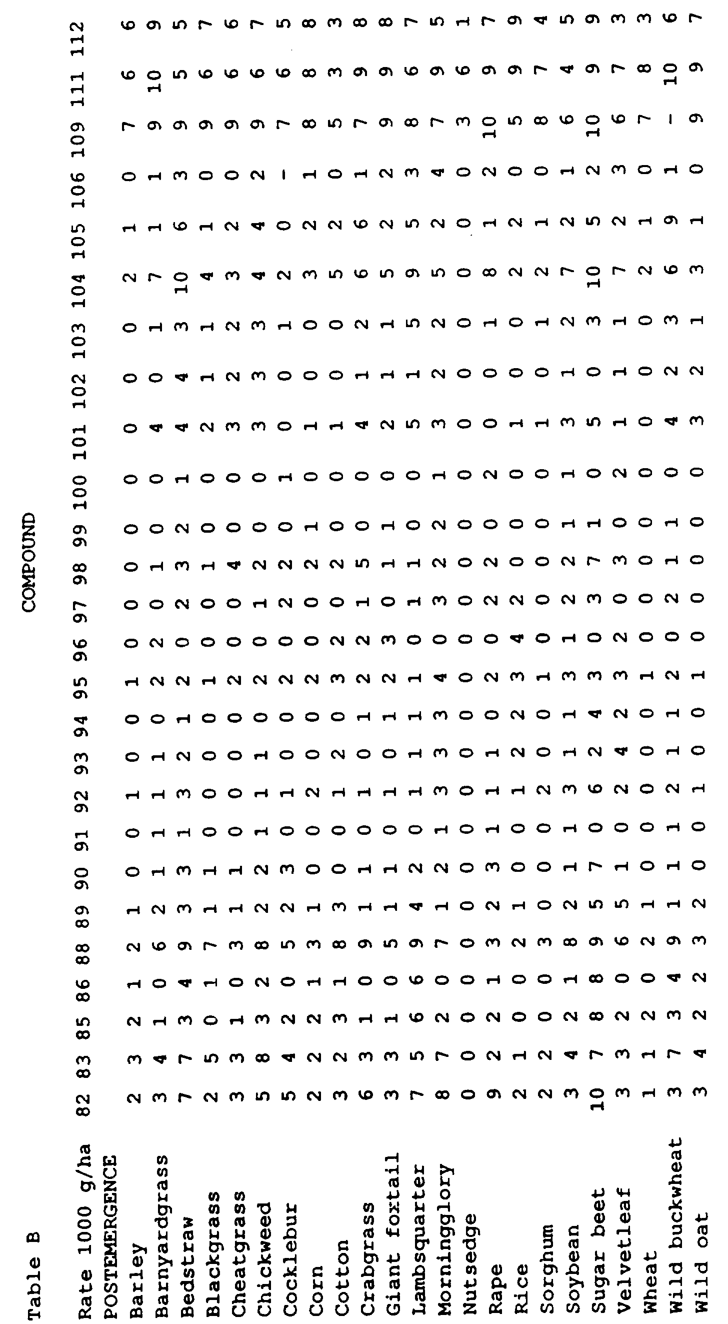

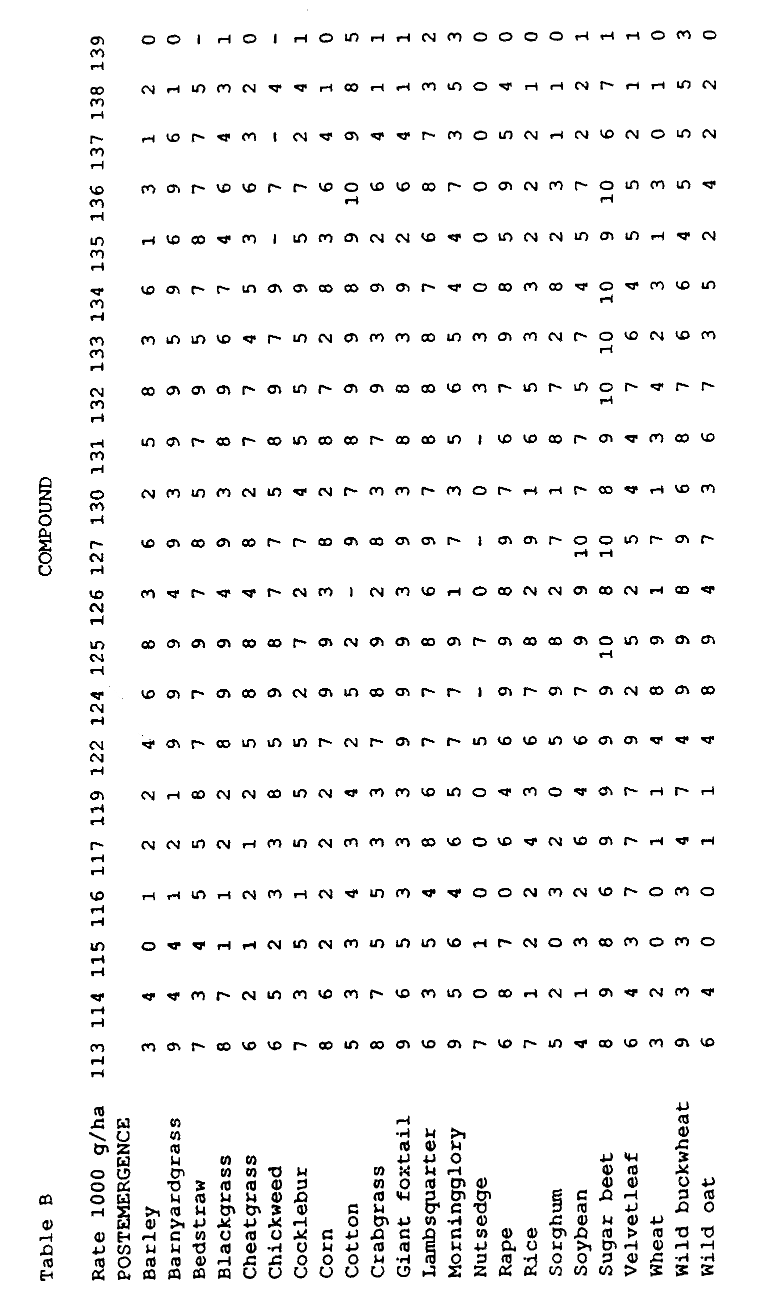

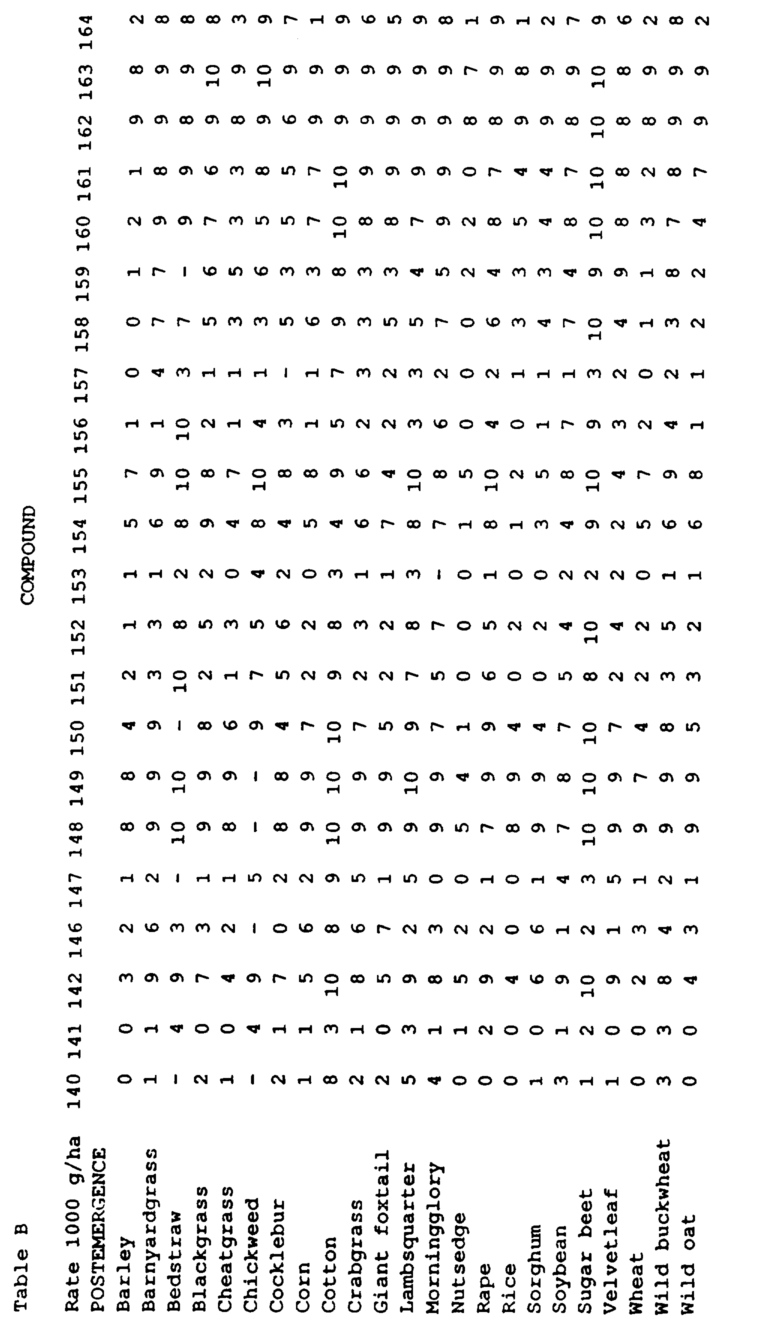

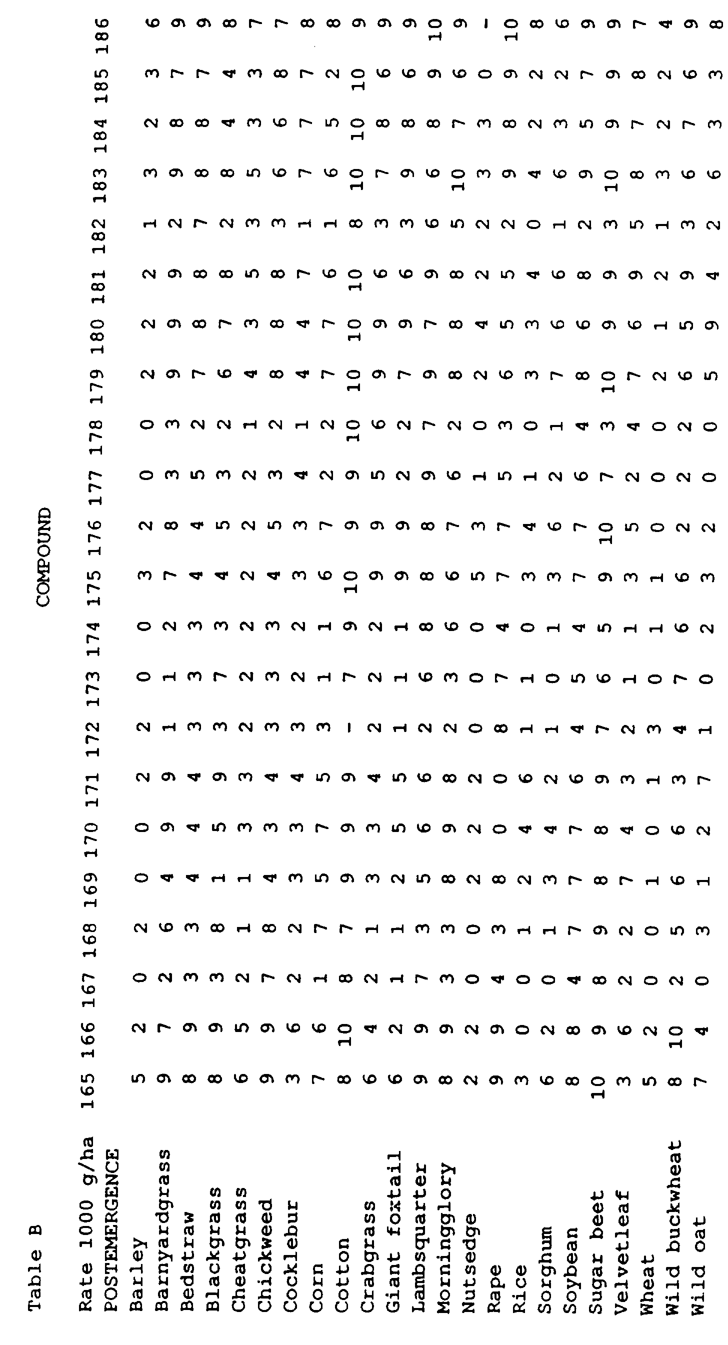

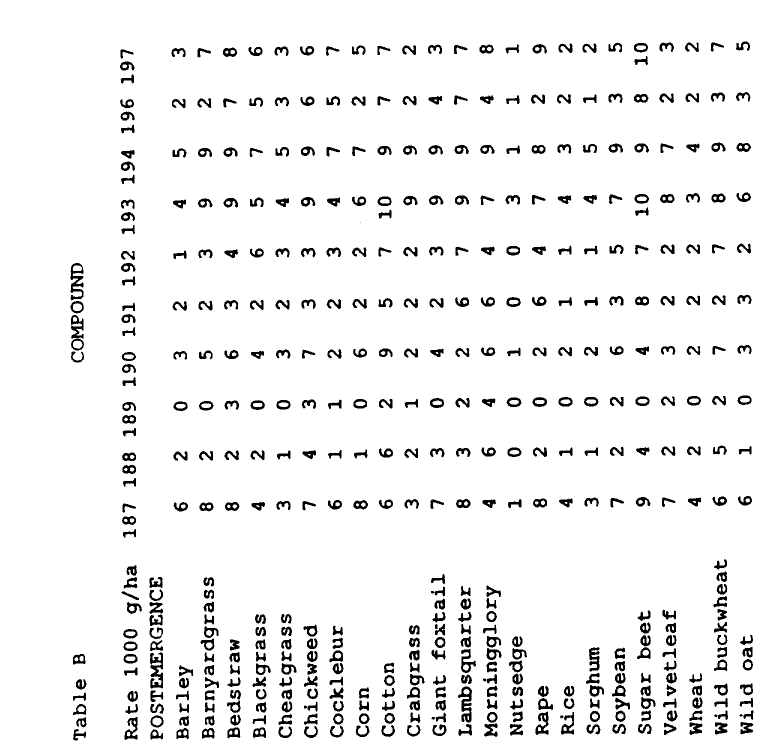

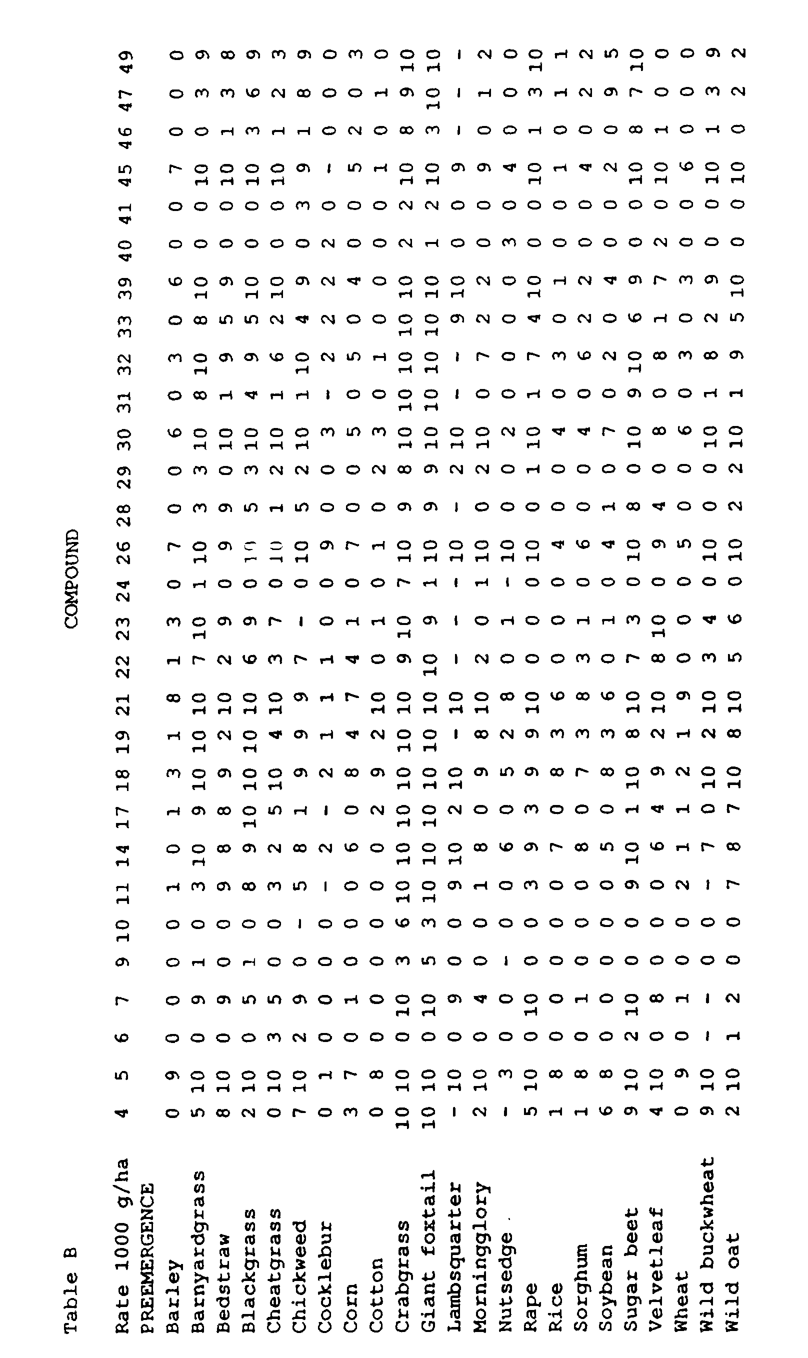

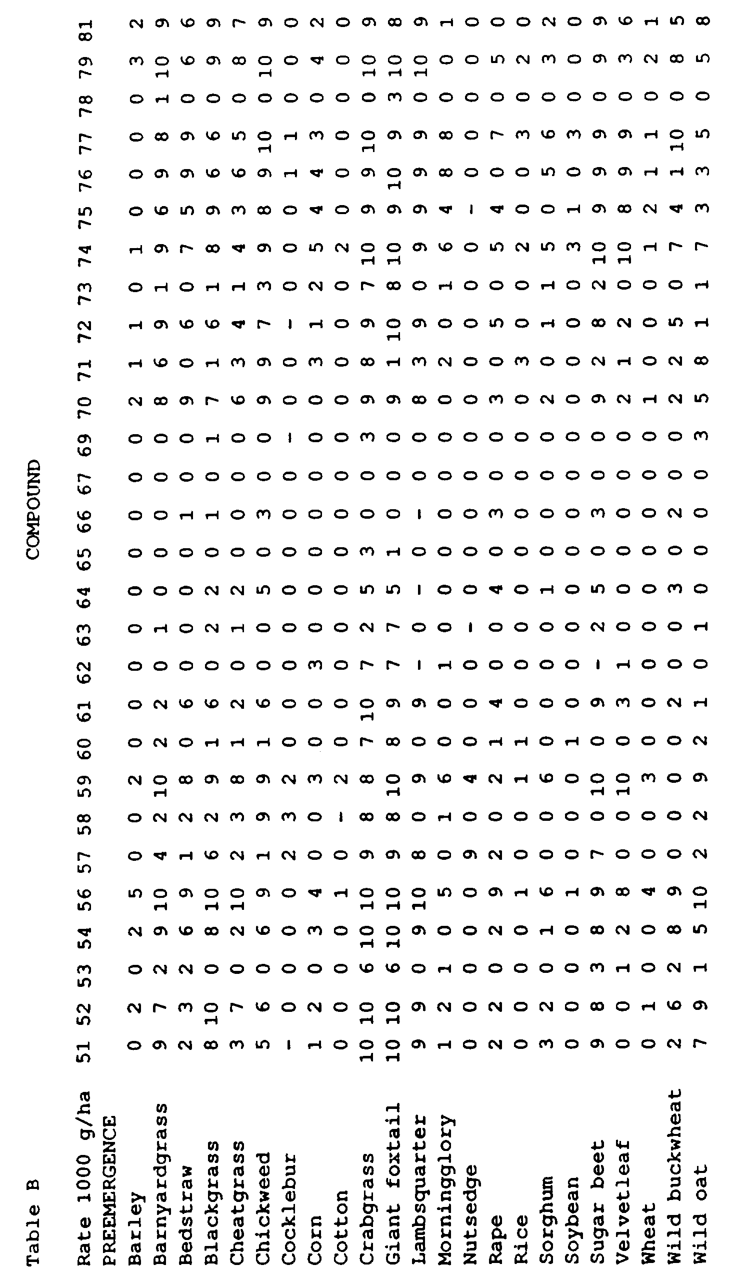

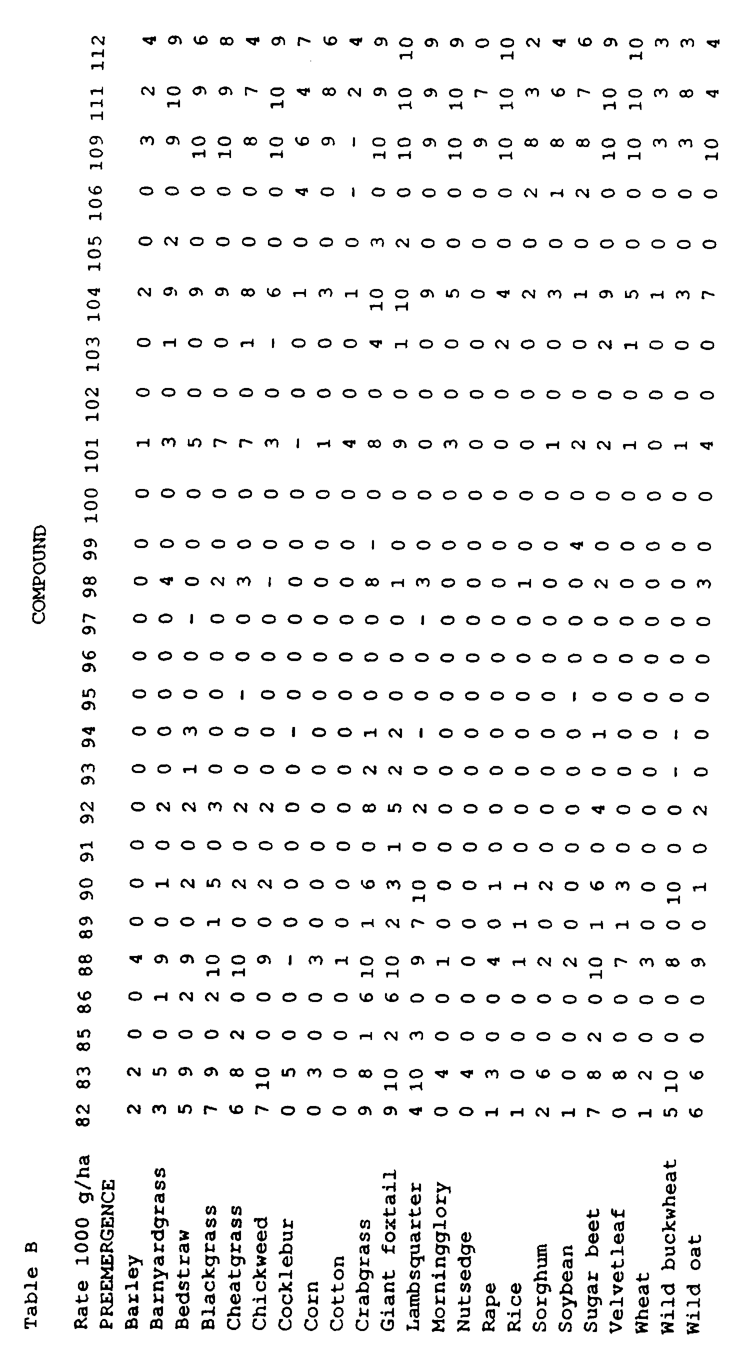

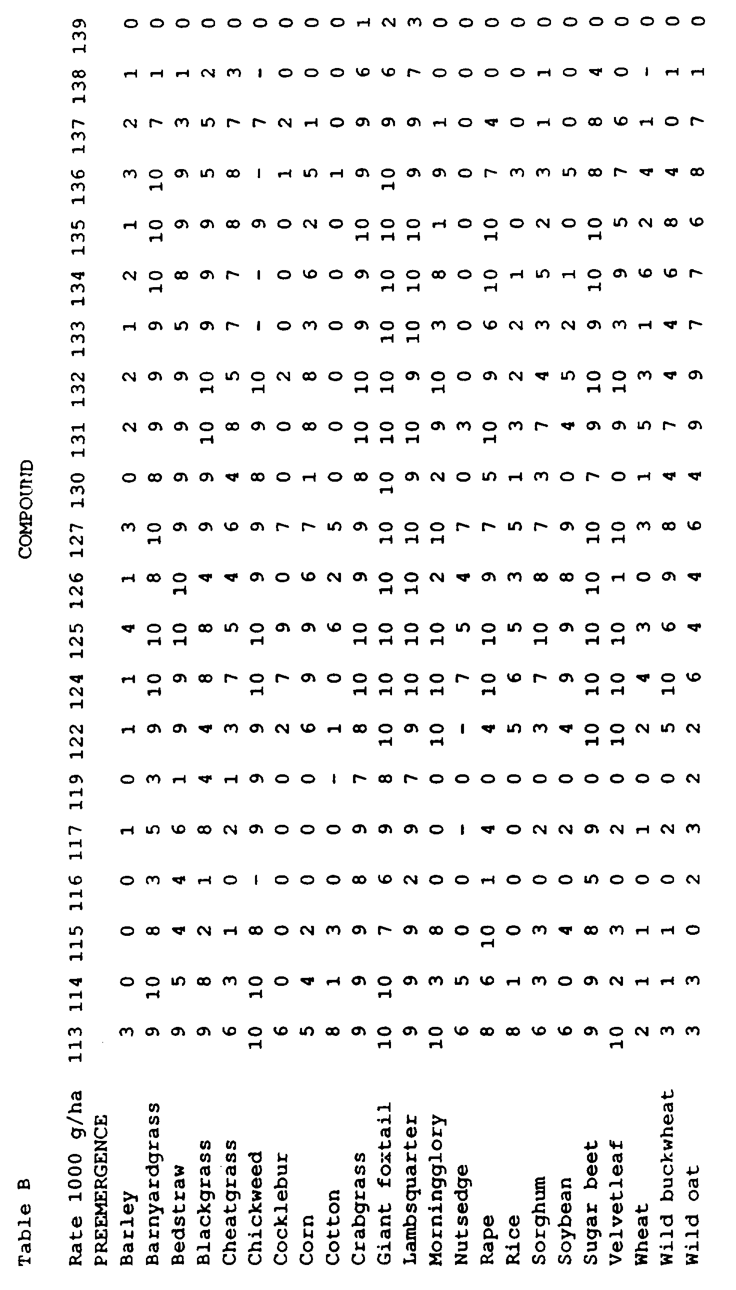

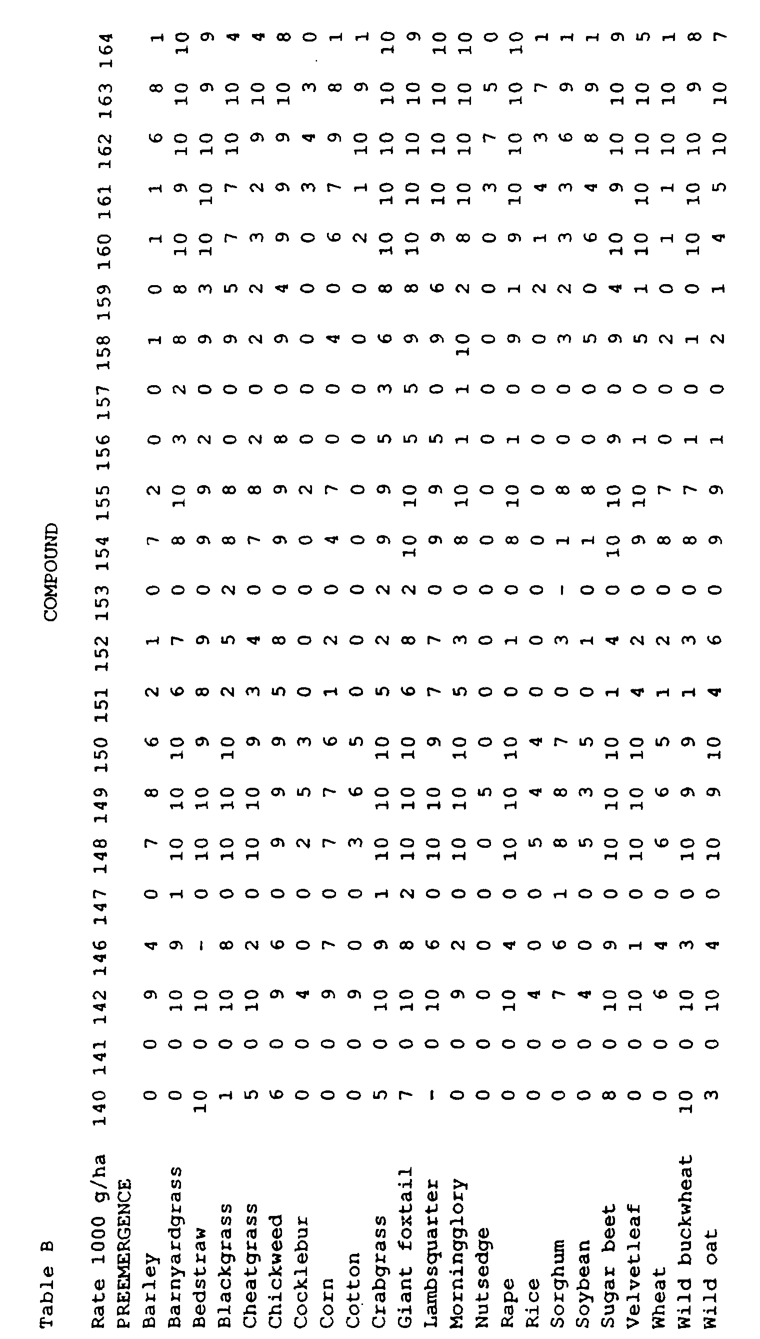

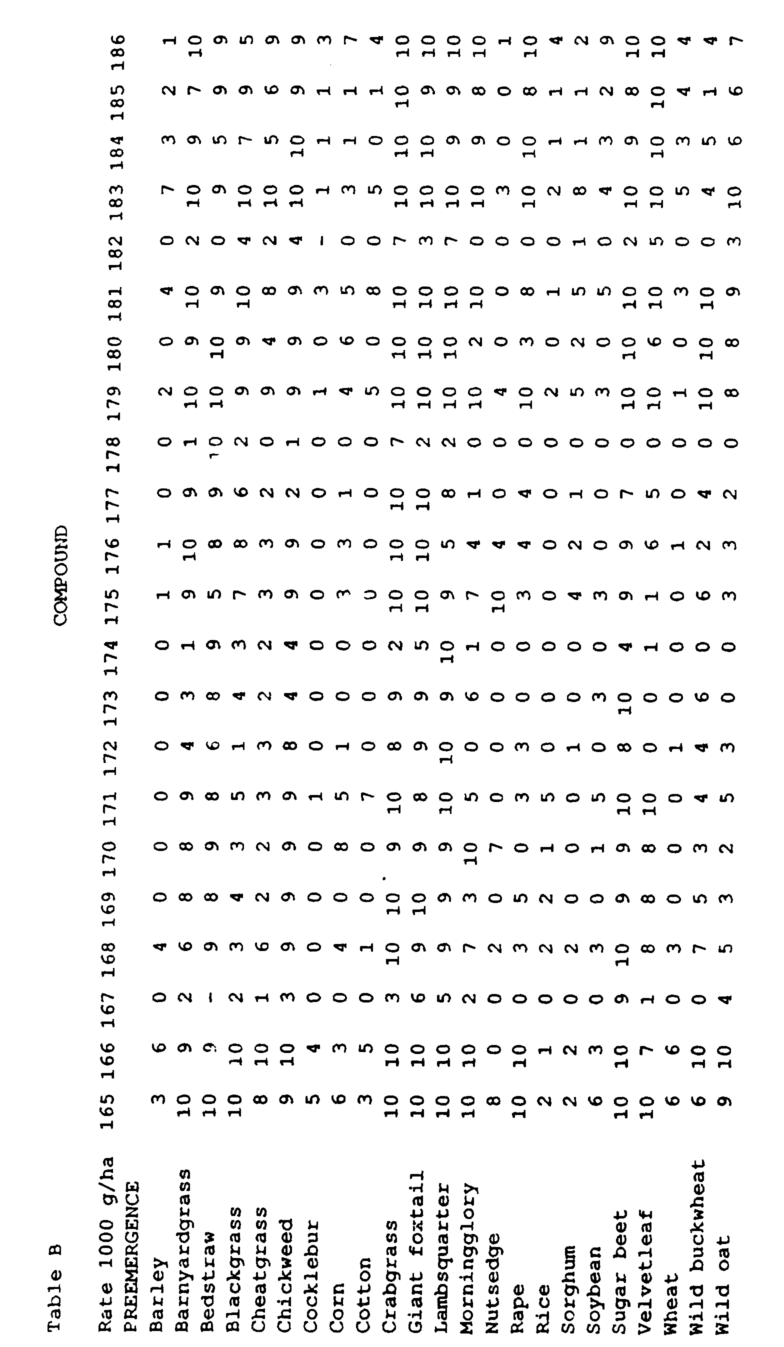

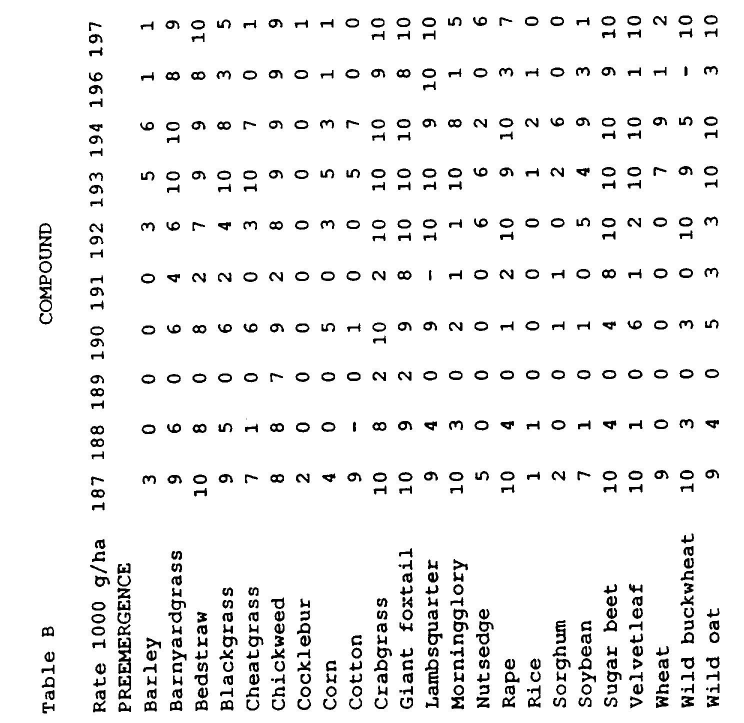

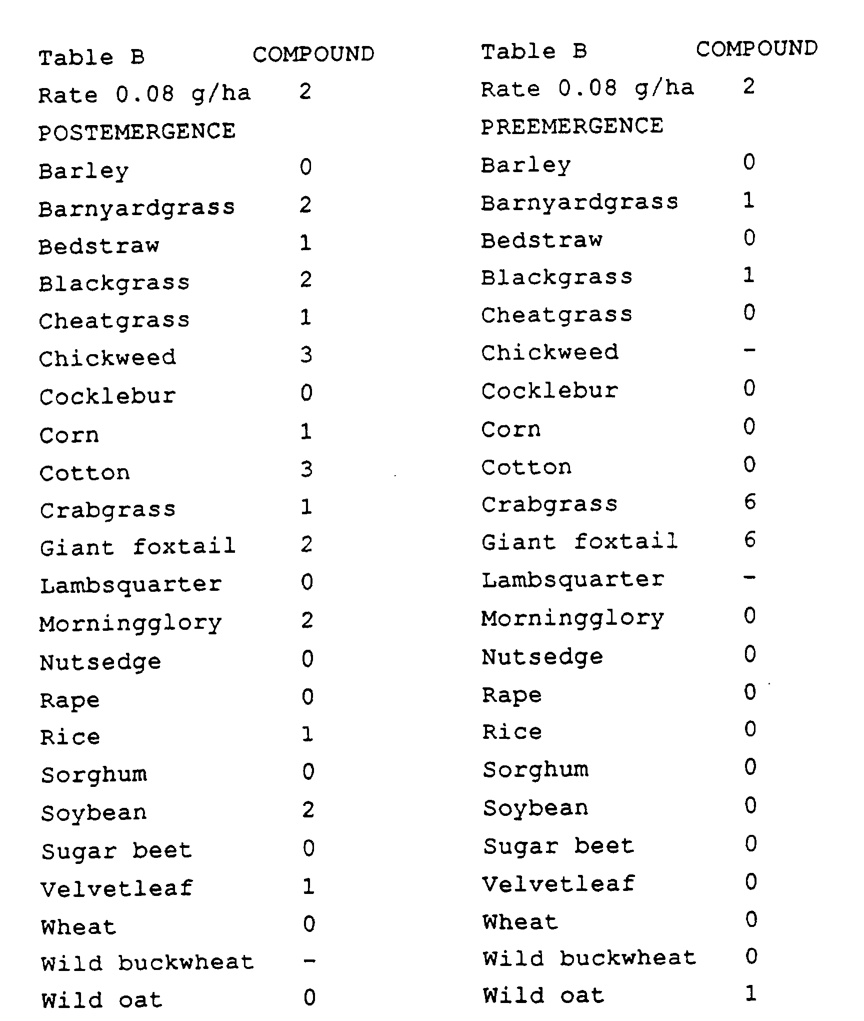

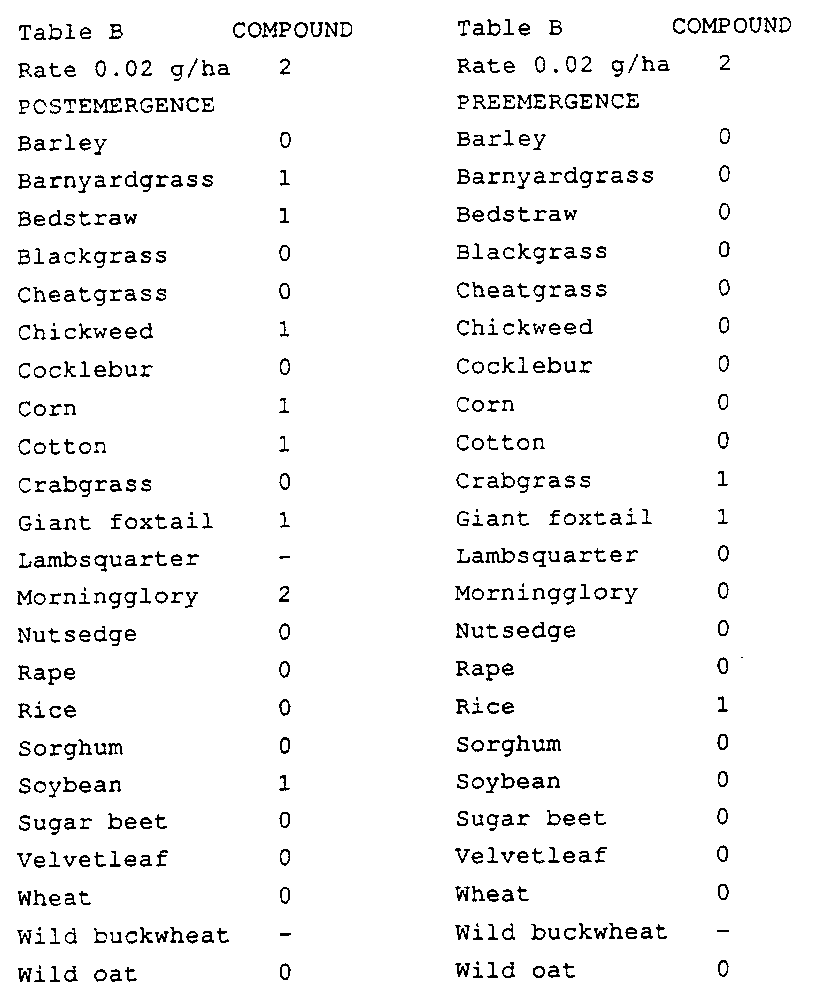

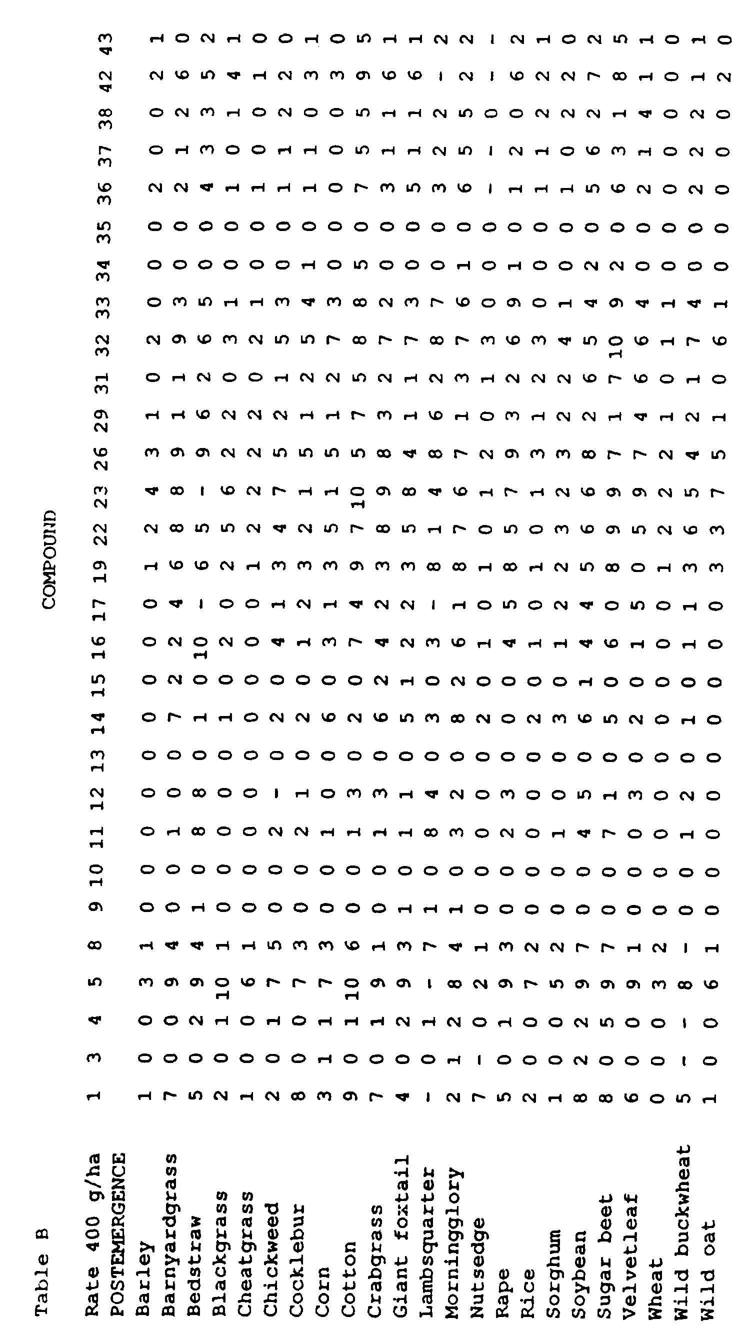

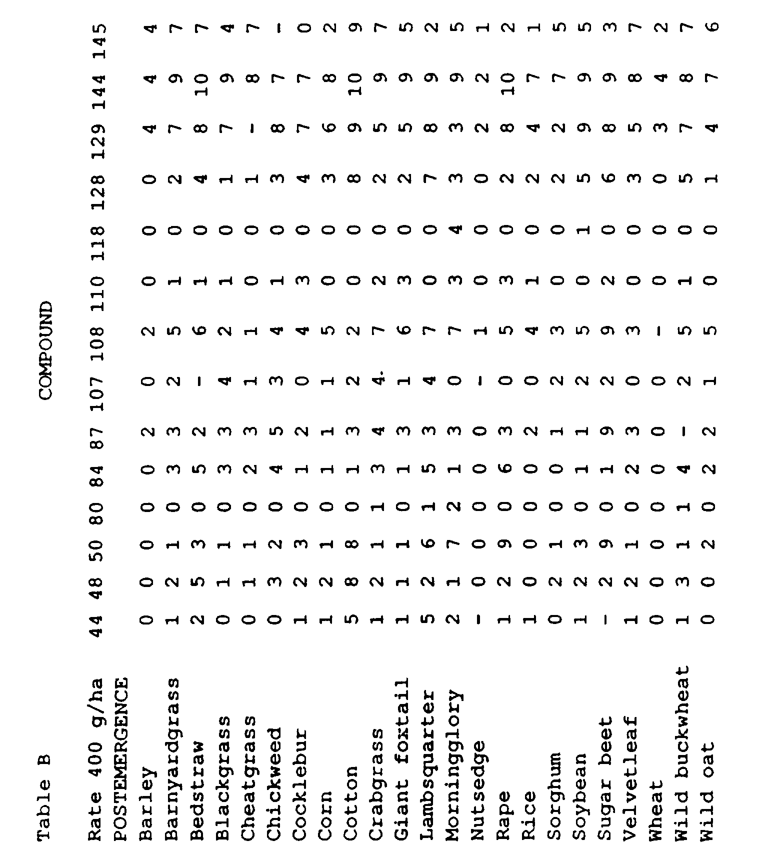

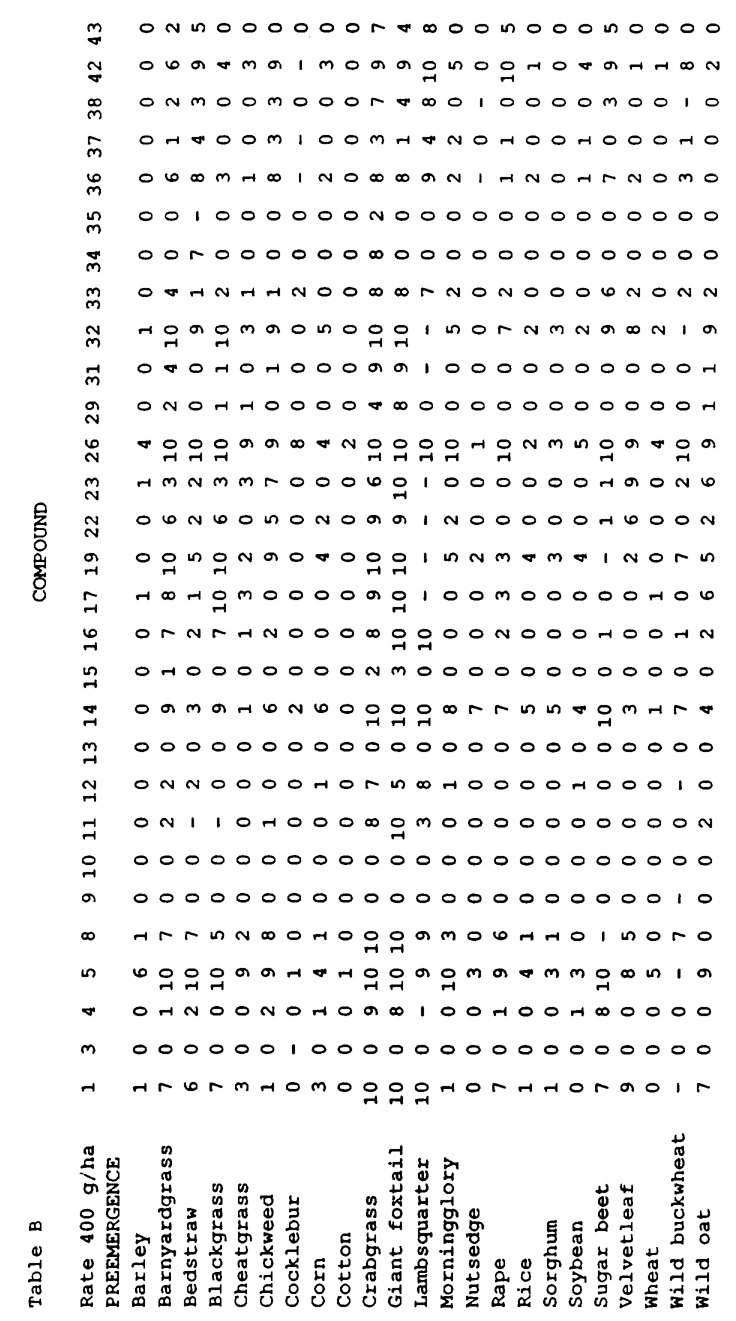

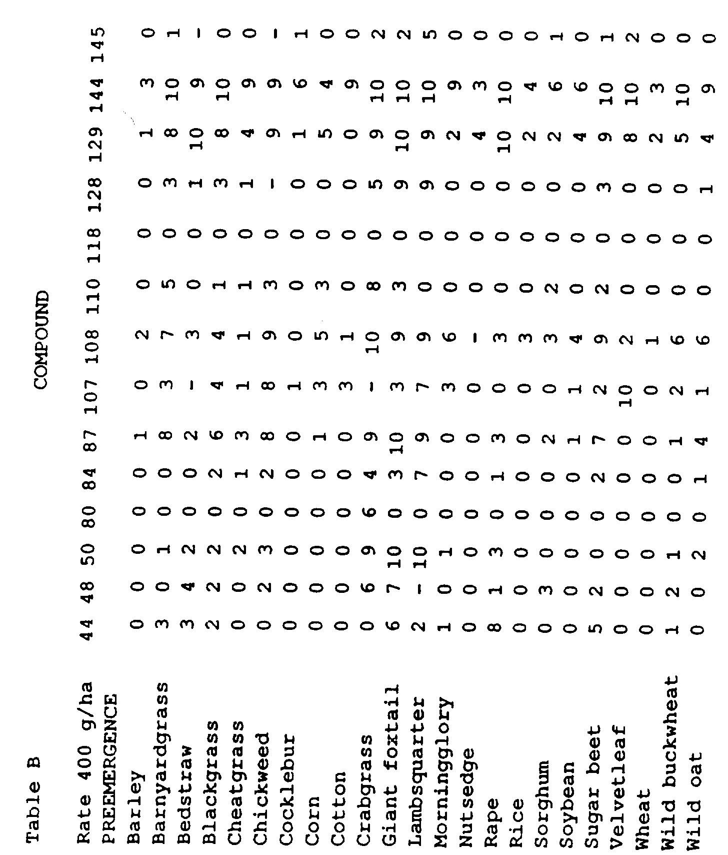

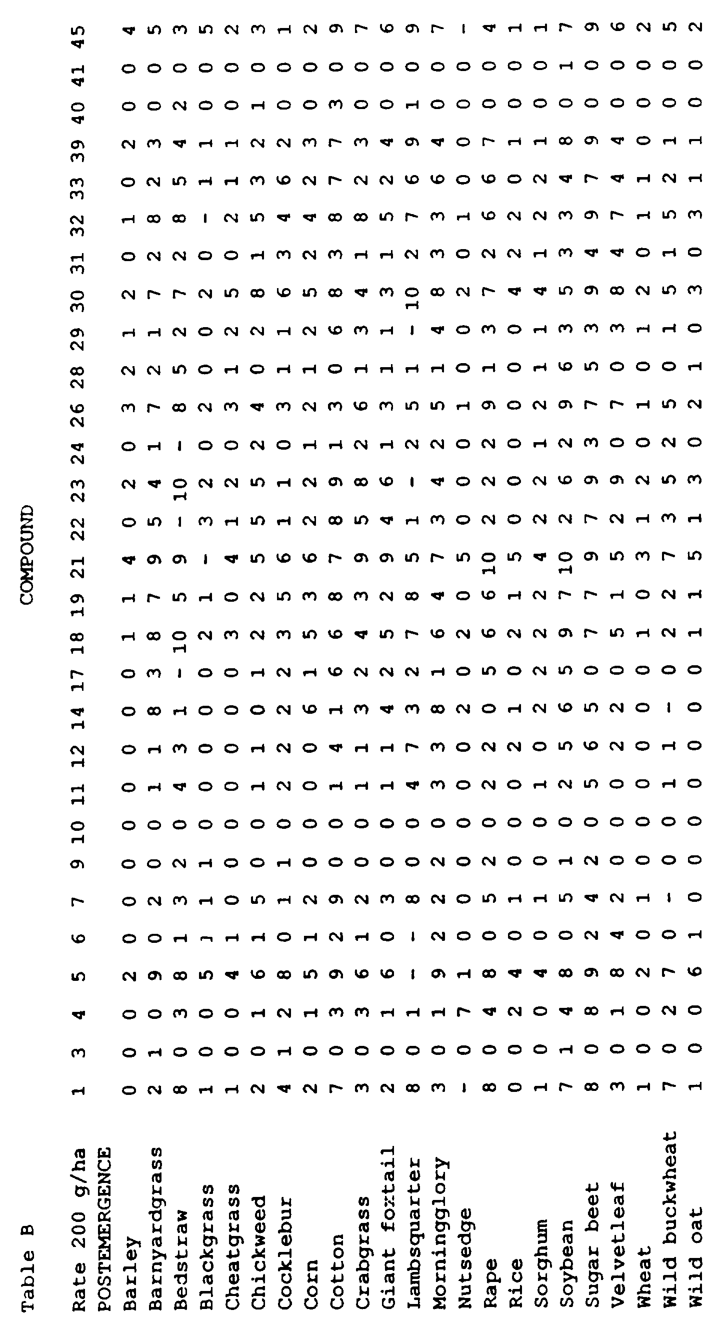

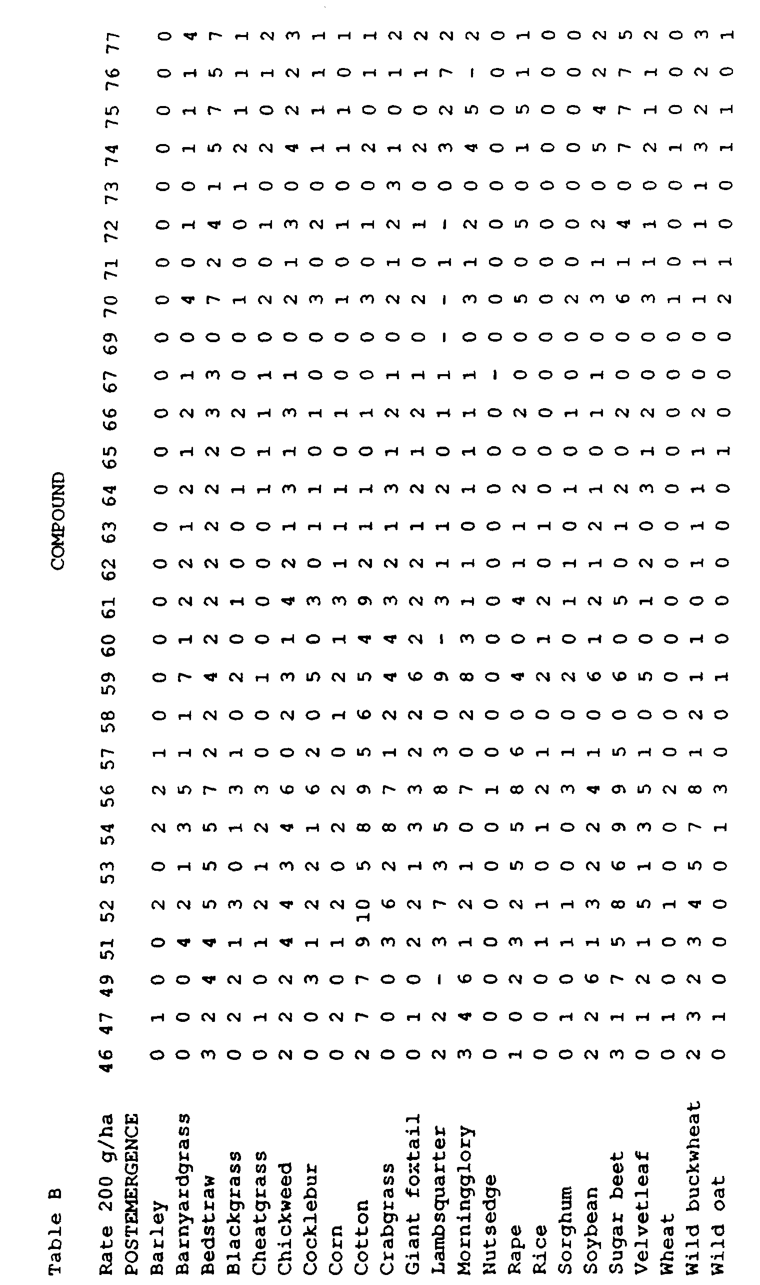

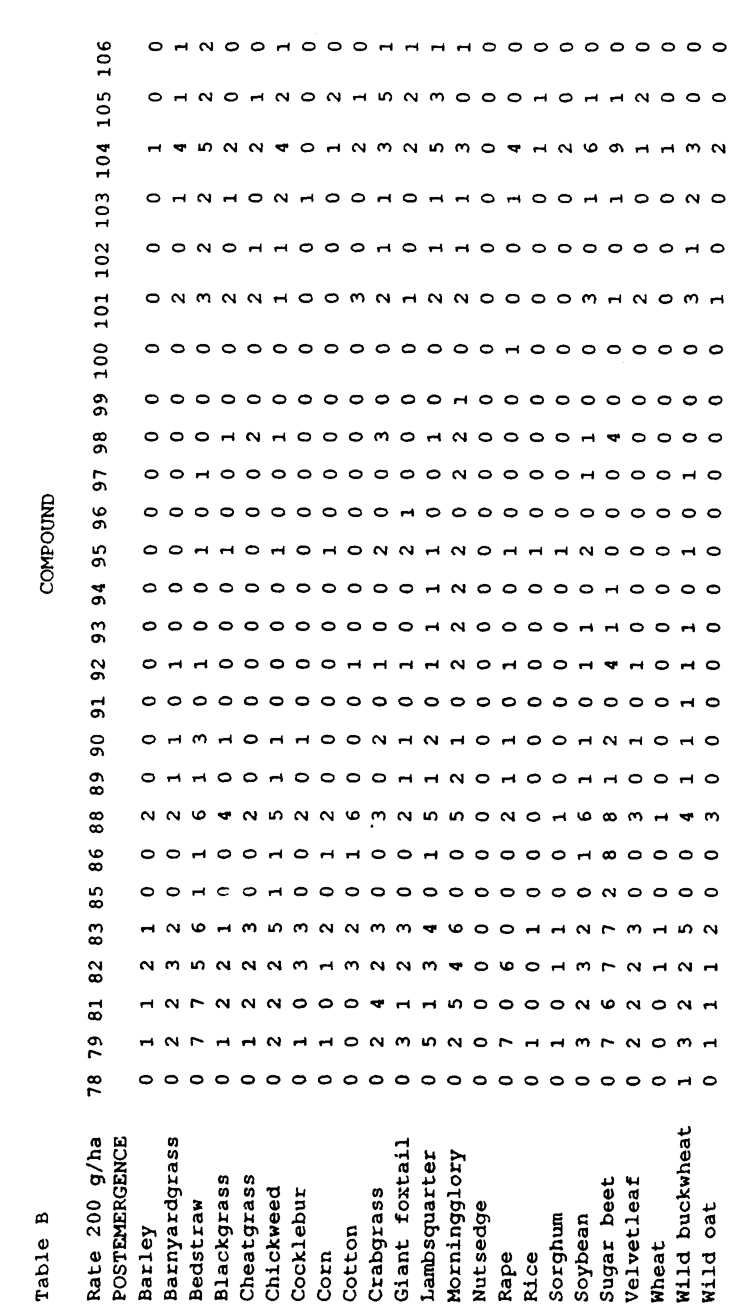

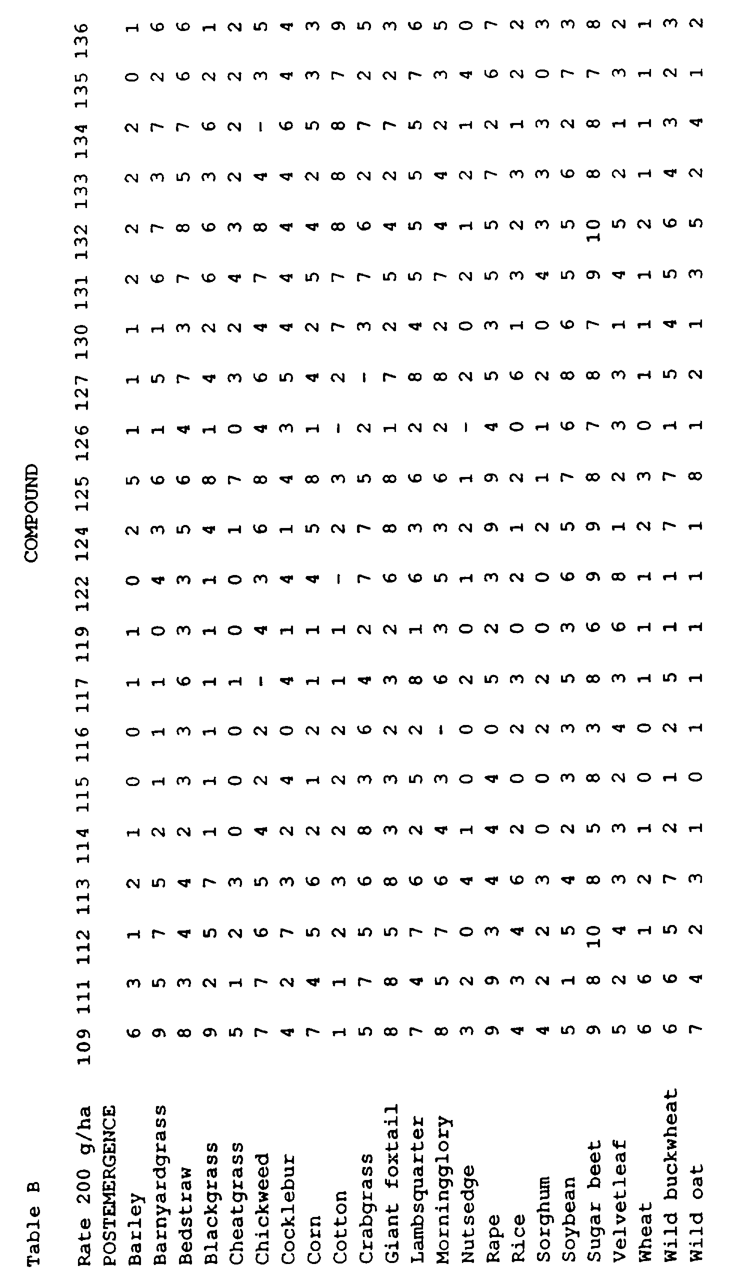

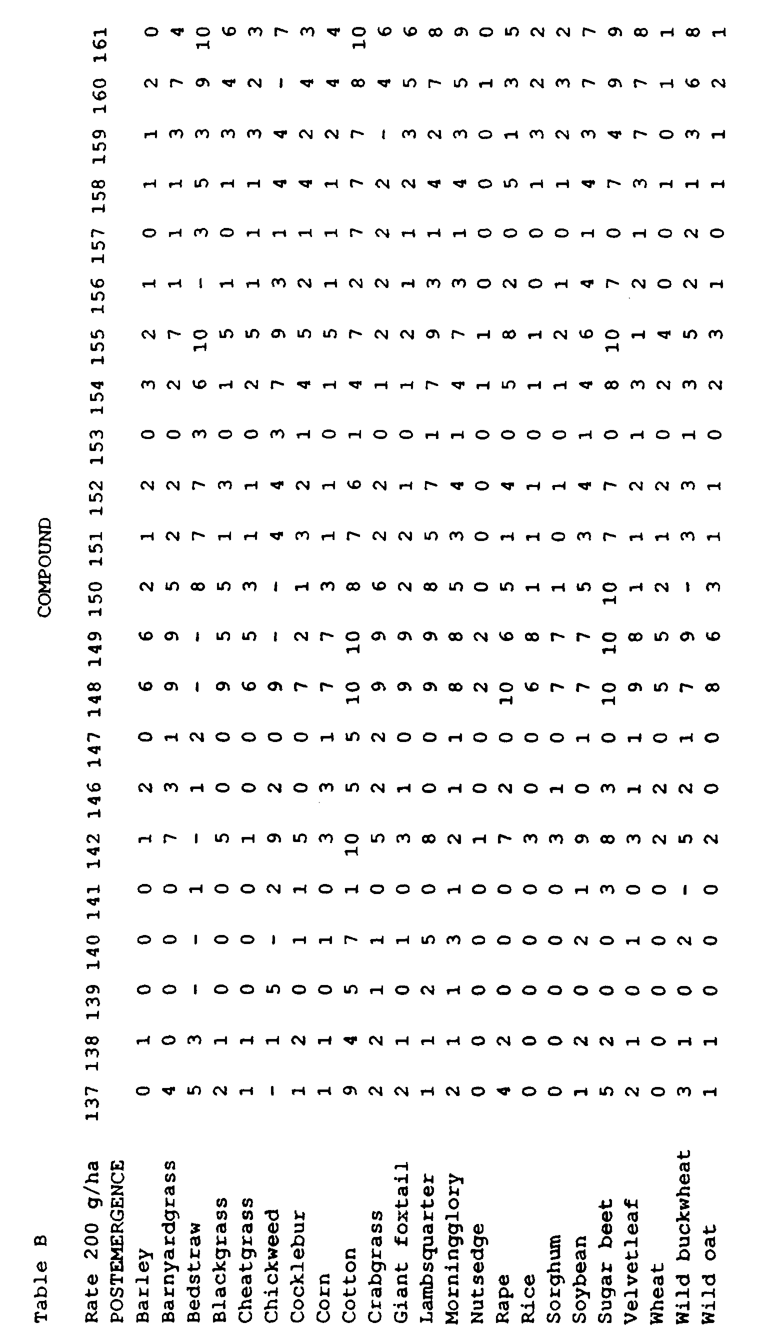

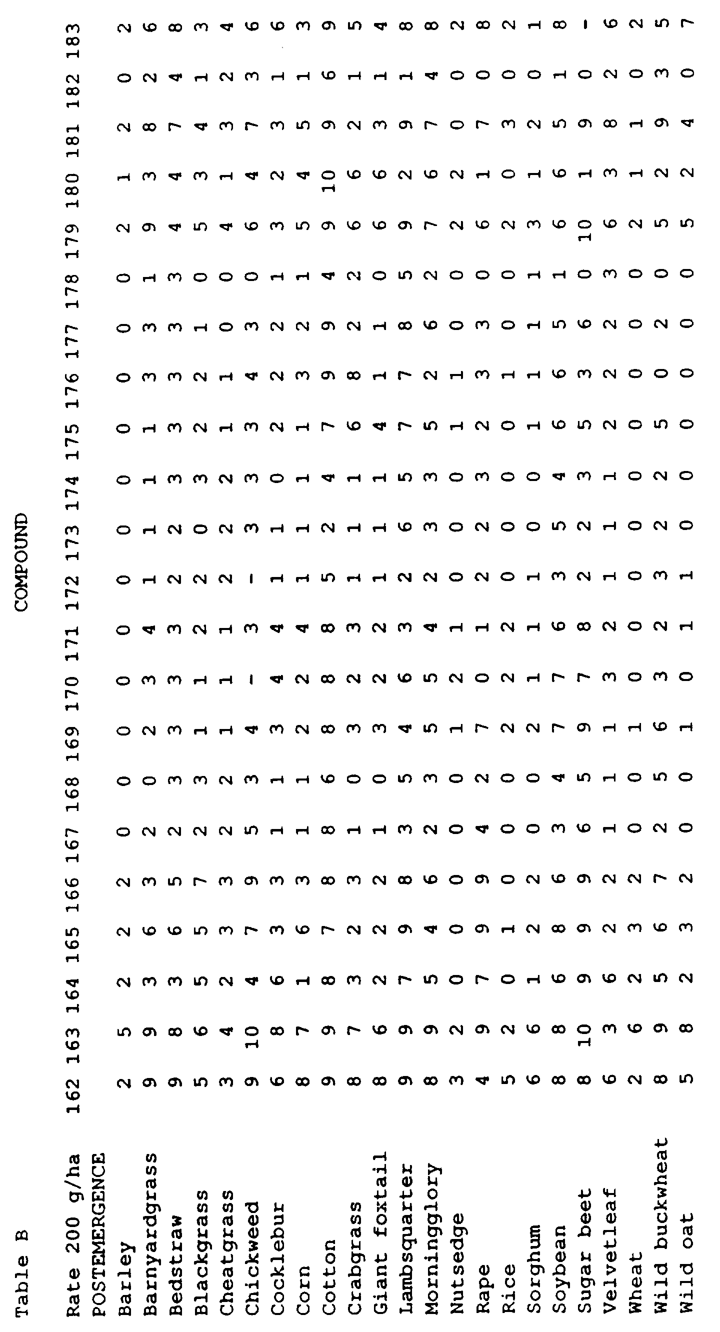

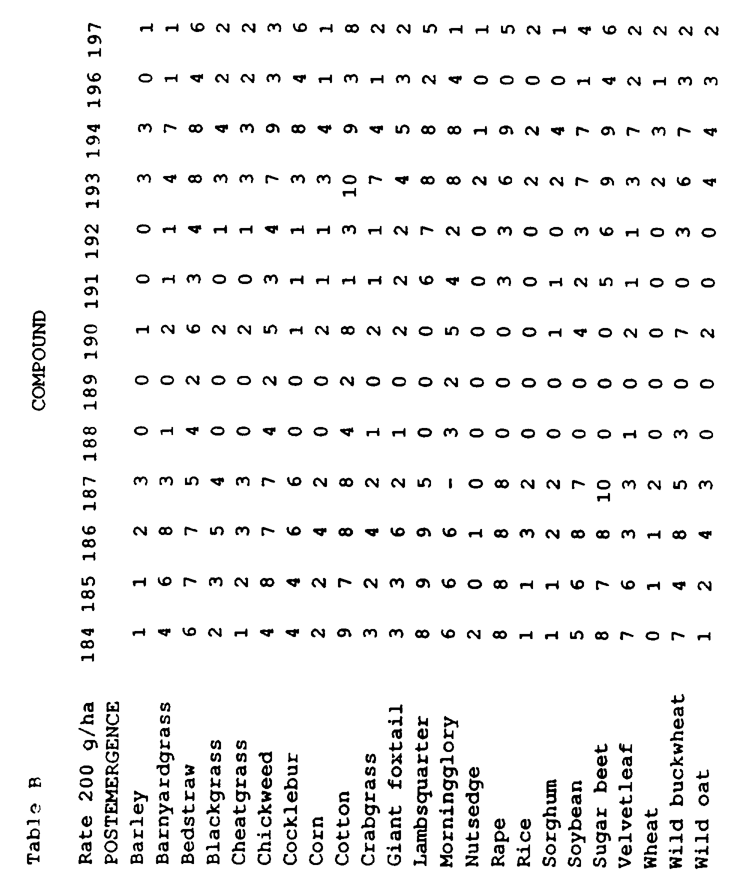

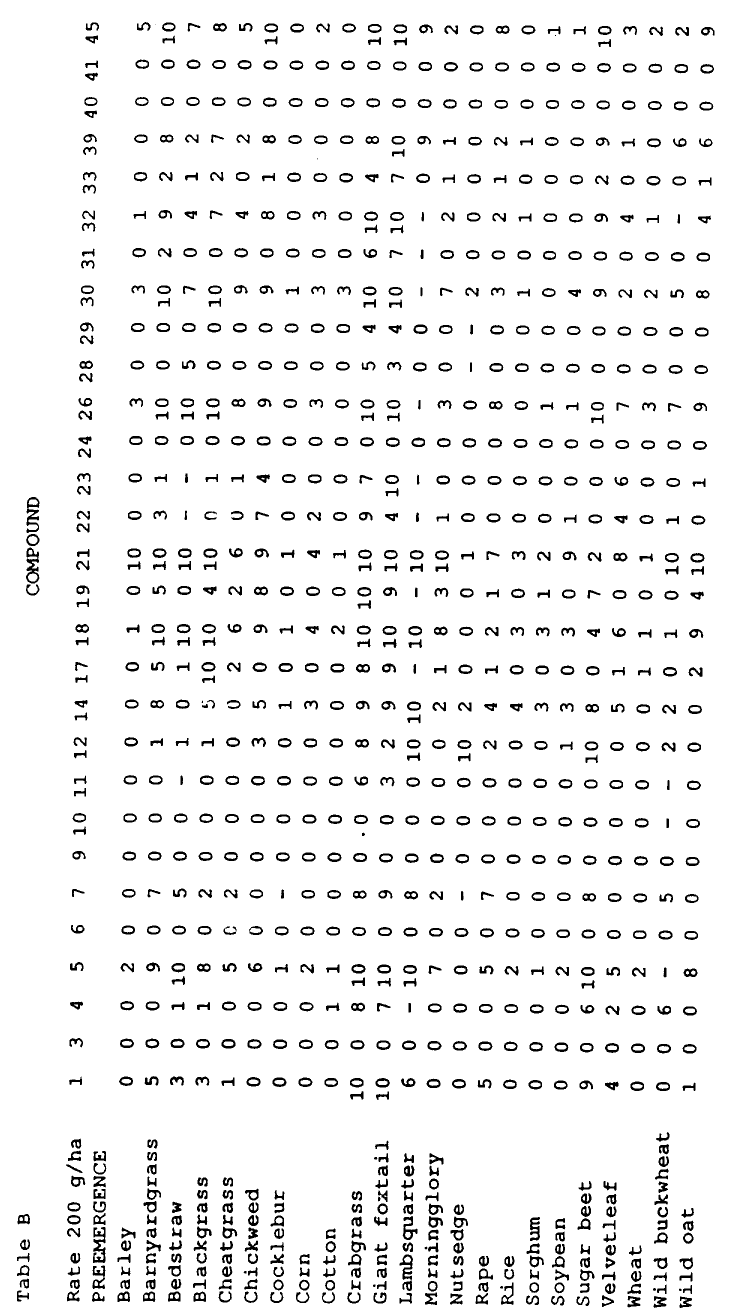

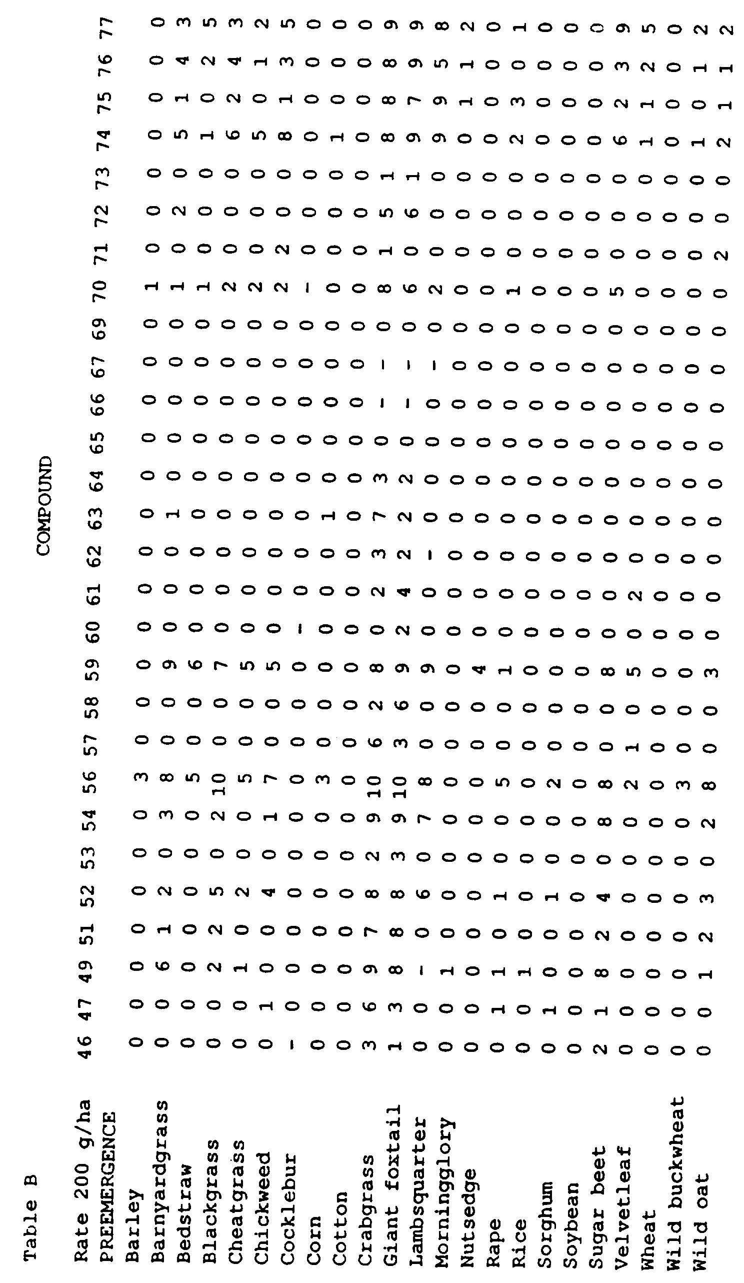

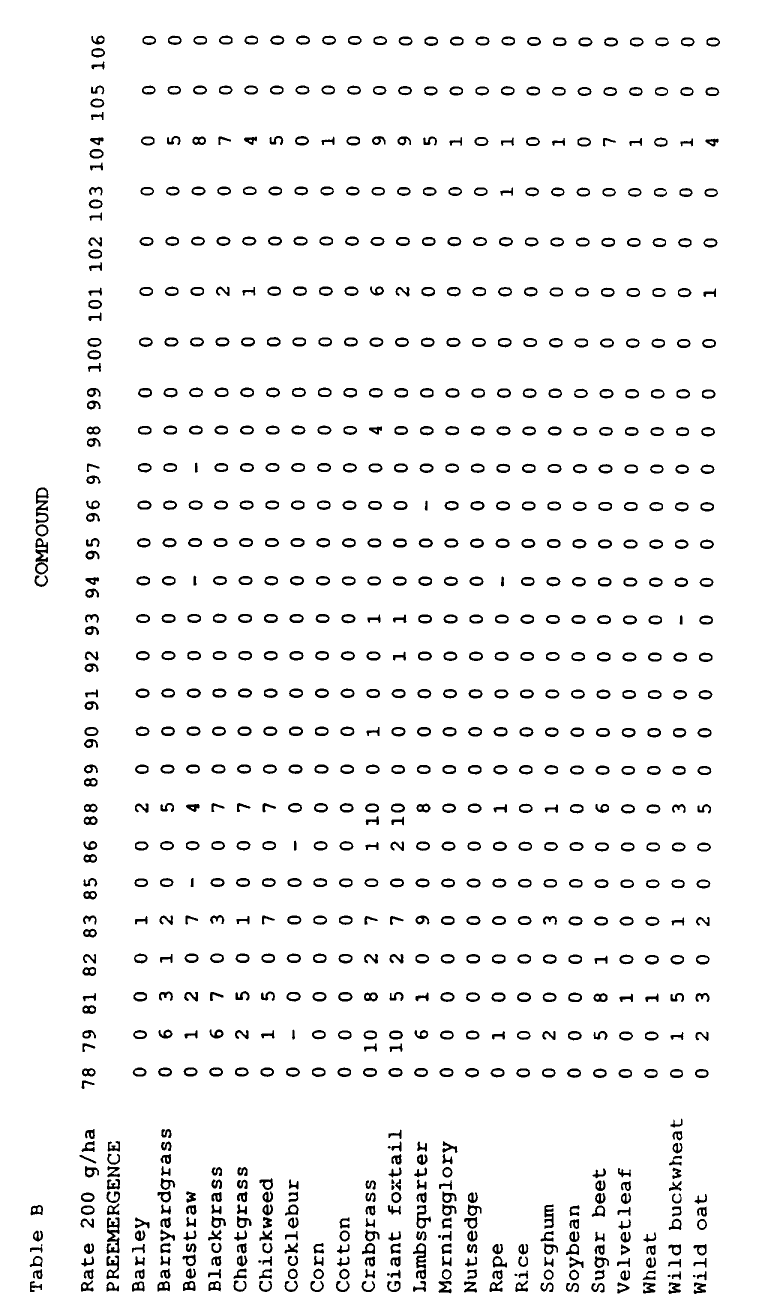

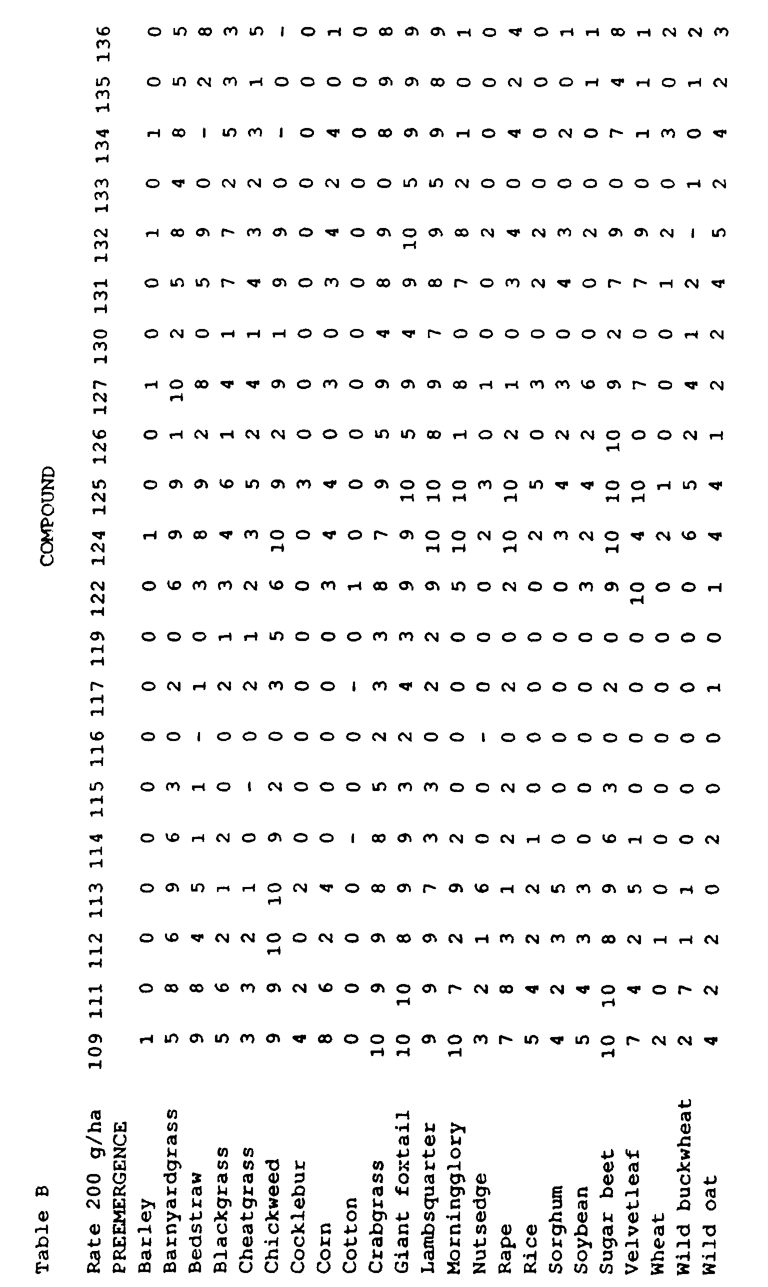

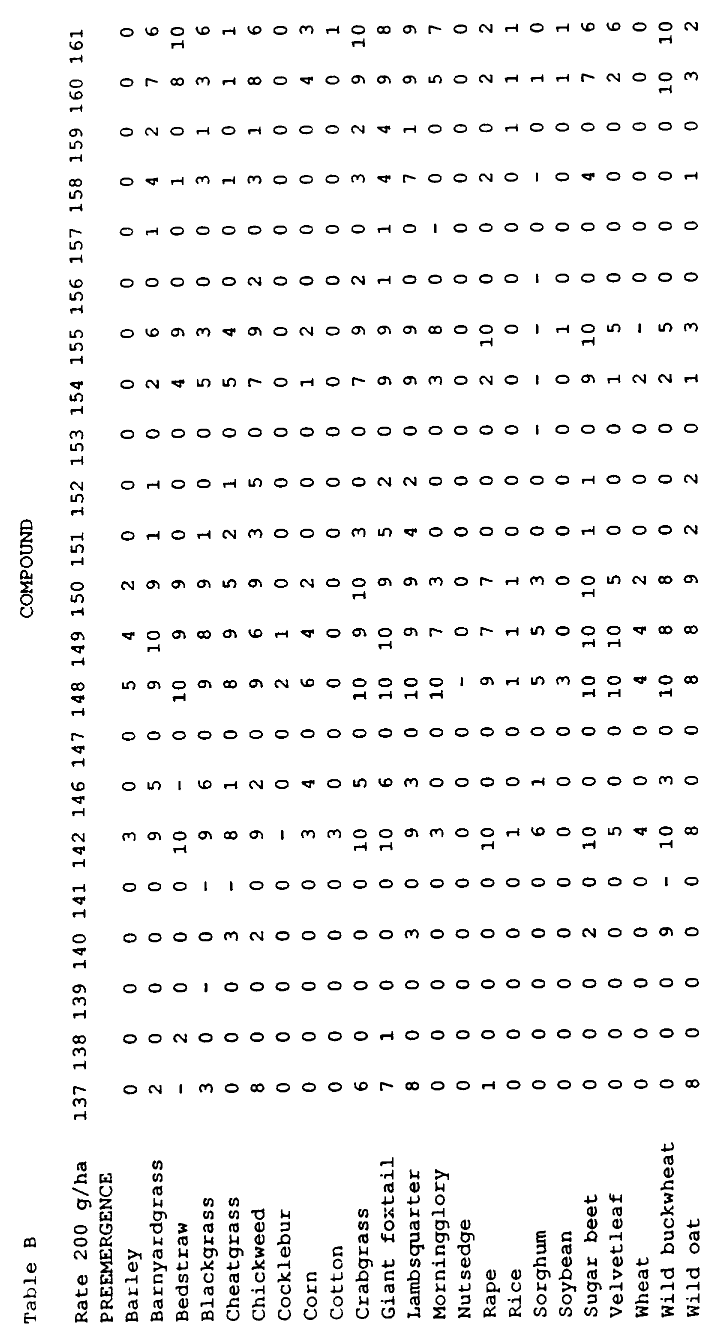

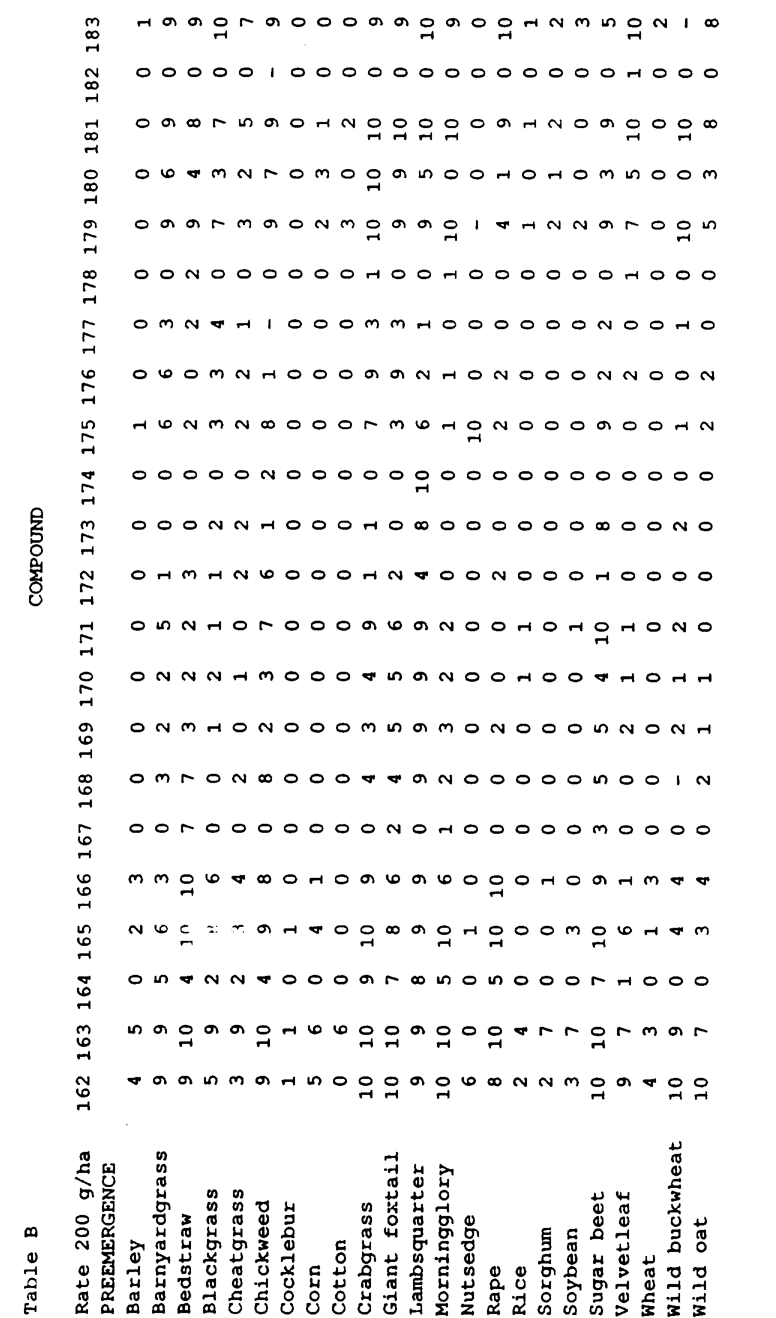

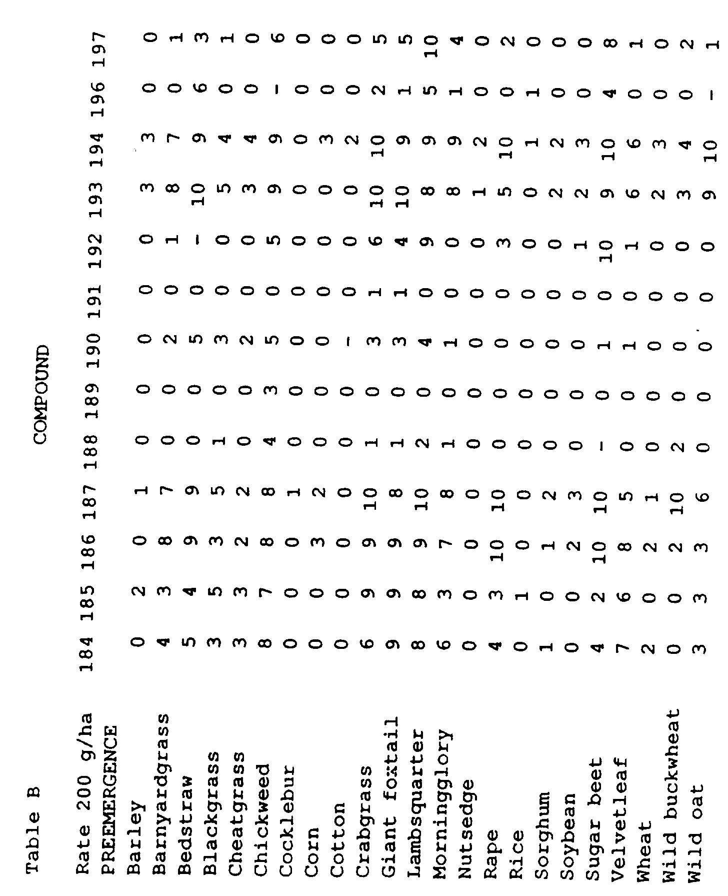

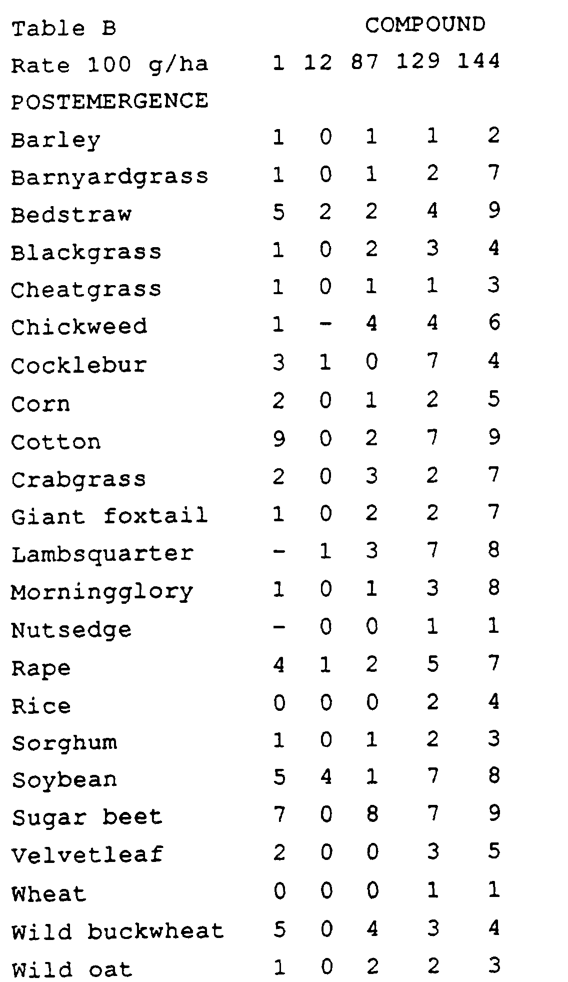

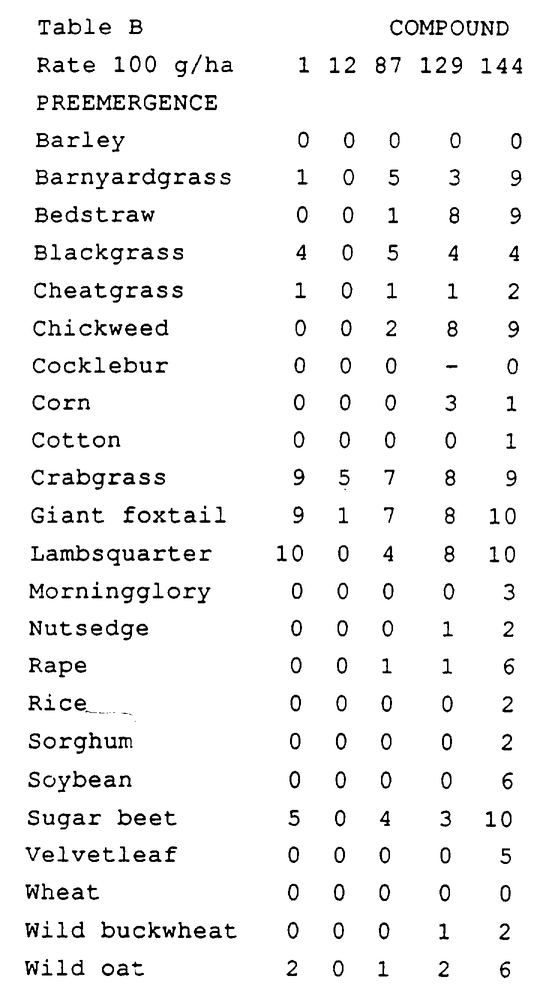

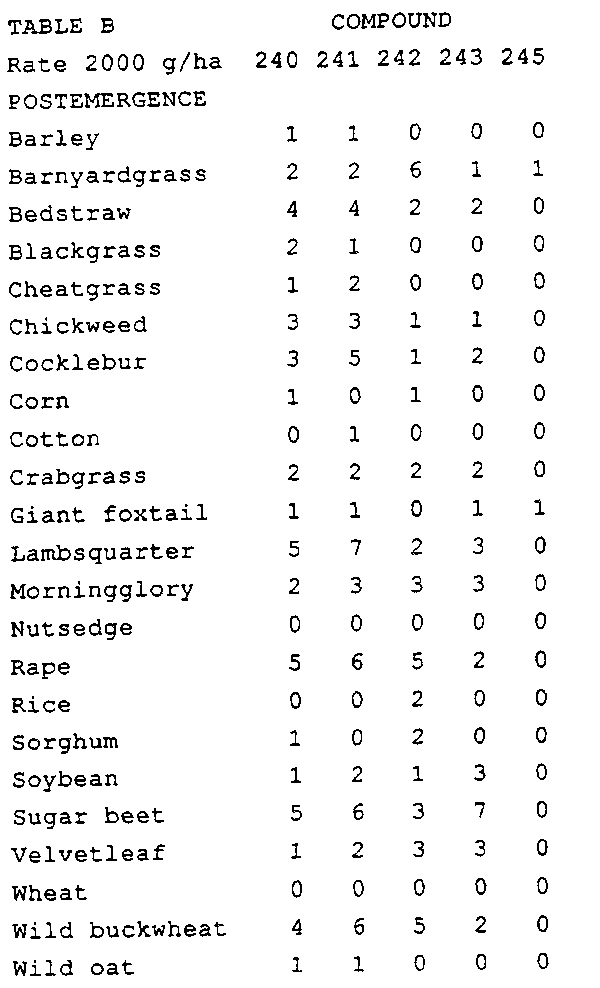

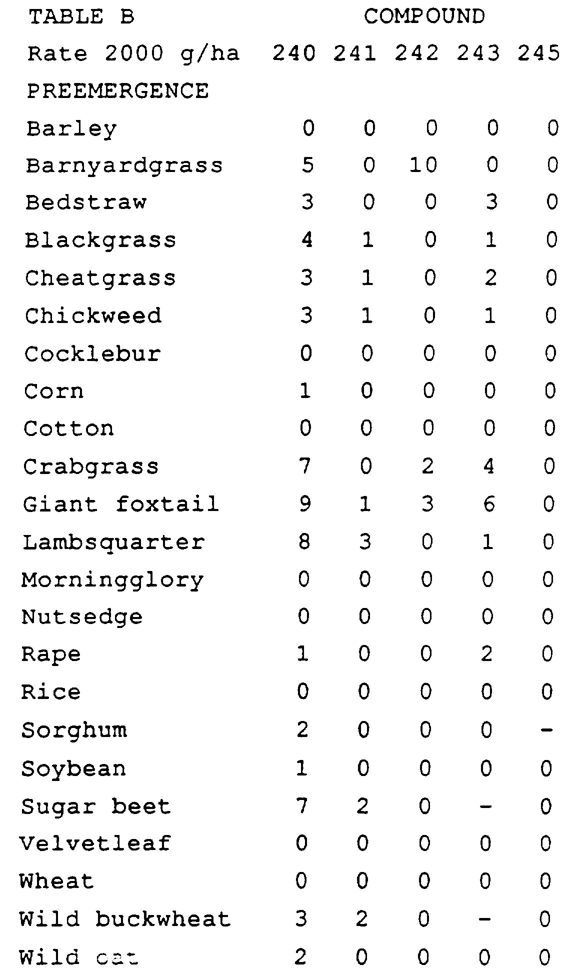

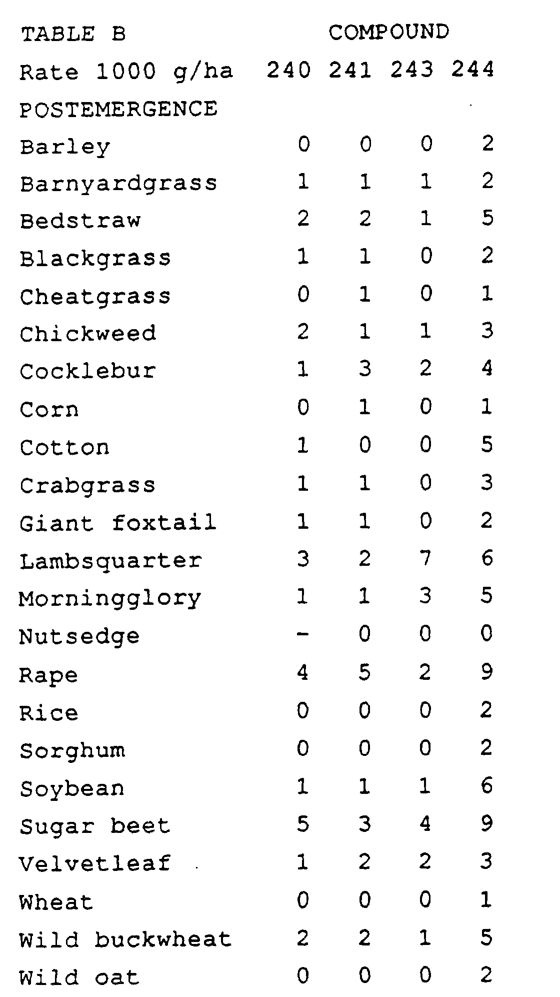

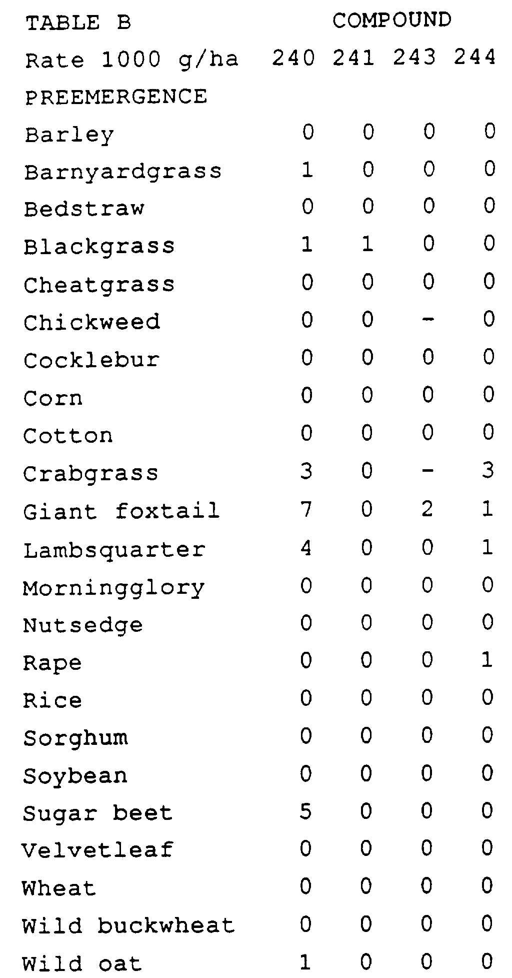

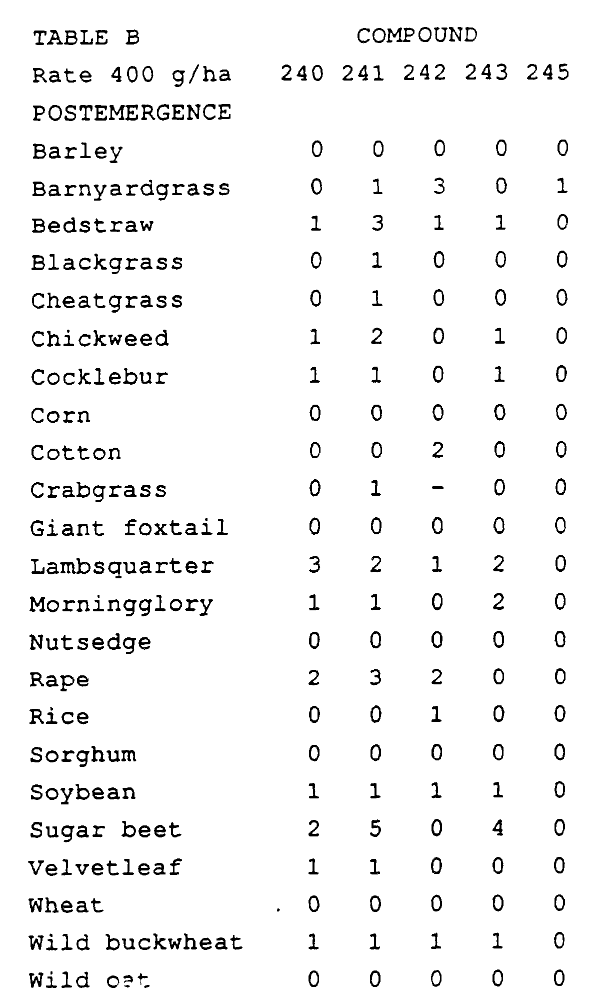

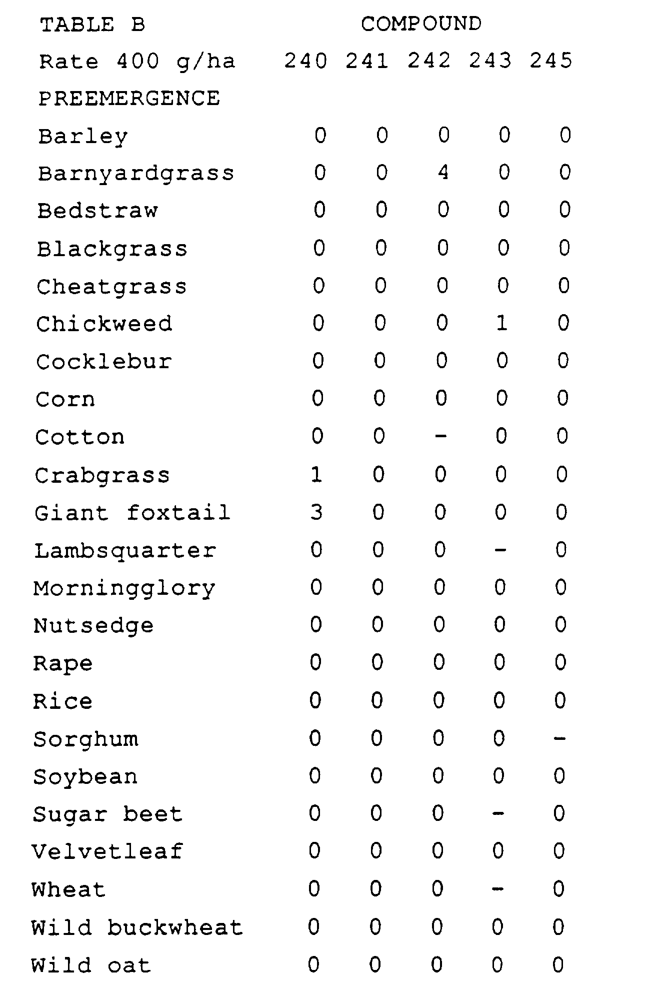

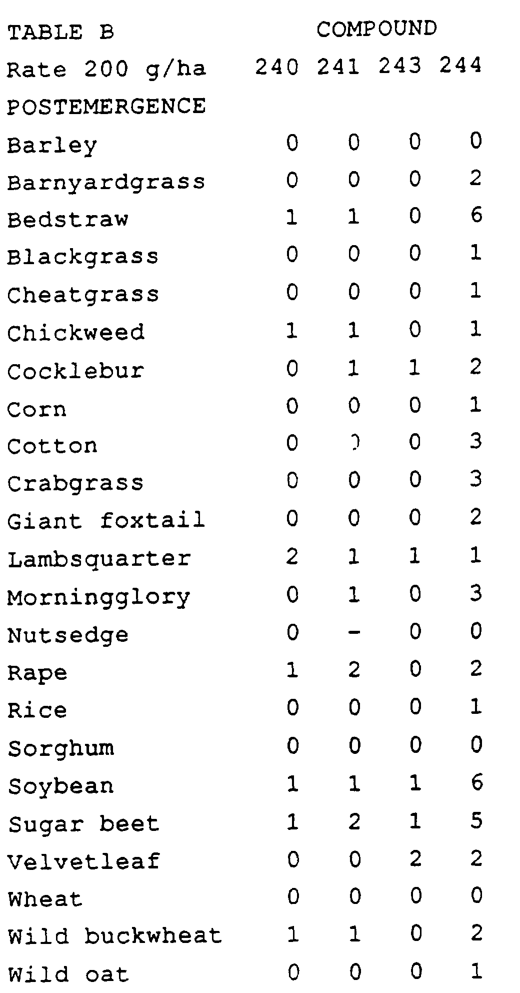

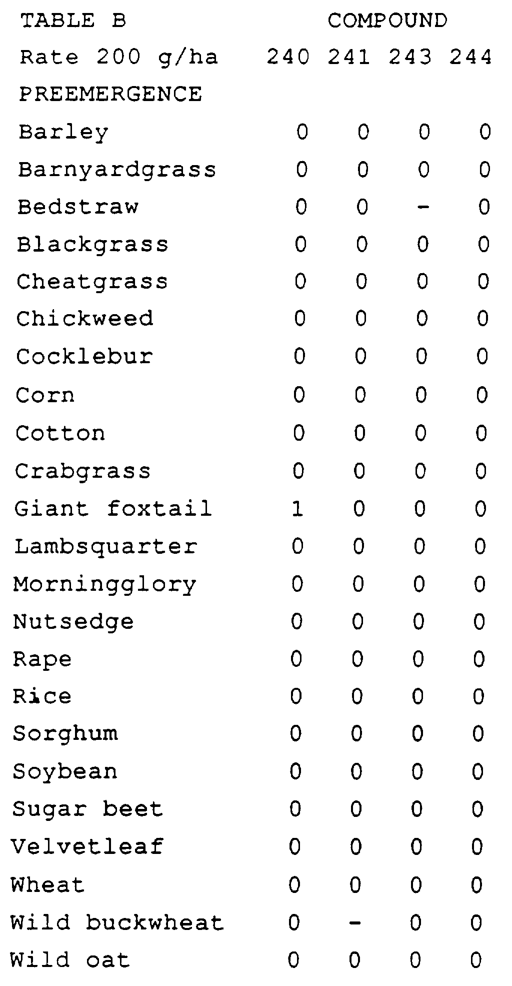

### TEST C

The compounds evaluated in this test were formulated in a non-phytoxic solvent and applied to the soil surface before plant seedlings emerged (preemergence application), to water that covered the soil surface (paddy application), and to plants that were in the one-to-four leaf stage (postemergence application). A sandy loam soil was used for the preemergence and postemergence tests, while a silt loam soil was used in the paddy test. Water depth was approximately 2.5 cm for the paddy test and was maintained at this level for the duration of the test.

Plant species in the preemergence and postemergence tests consisted of barley (*Hordeum vulgare*), bedstraw (*Galium aparine*), blackgrass (*Alopecurus myosuroides*), chickweed (*Stellaria media*), corn (*Zea mays*), cotton (*Gossypium hirsutum*), crabgrass (*Digitaria sanguinalis*), downy brome (*Bromus tectorum*), duck salad (*Heteranthera limosa*), giant foxtail (*Setaria faberii*), lambs.quarters (*Chenopodium album*), morningglory (*Ipomoea hederacea*), pigweed (*Amaranthusretroflexus*)*,* rape (*Brassica napus*), ryegrass (*Lolium multiflorum*), sorghum (*Sorghum bicolor*), soybean (*Glycine max*), speedwell (*Veronica persica*), sugar beet (*Beta vulgaris*), velvetleaf (*Abutilon theophrasti*), wheat (*Triticum aestivum*), wild buckwheat (*Polygonum convolvulus*), and wild oat (*Avena fatua*). All plant species were planted one day before application of the compound for the preemergence portion of this test. Plantings of these species were adjusted to produce plants of appropriate size for the postemergence portion of the test. Plant species in the paddy test consisted of barnyardgrass (*Echinochloa crus-galli*), rice (*Oryza sativa*), and umbrella sedge (*Cyperus difformis*).

All plant species were grown using normal greenhouse practices. Visual evaluations of injury expressed on treated plants, when compared to untreated controls, were recorded approximately fourteen to twenty-one days after application of the test compound. Plant response ratings, summarized in Table C, were recorded on a 0 to 10 scale where 0 is no injury and 10 is complete control. A dash (-) response means no test result.

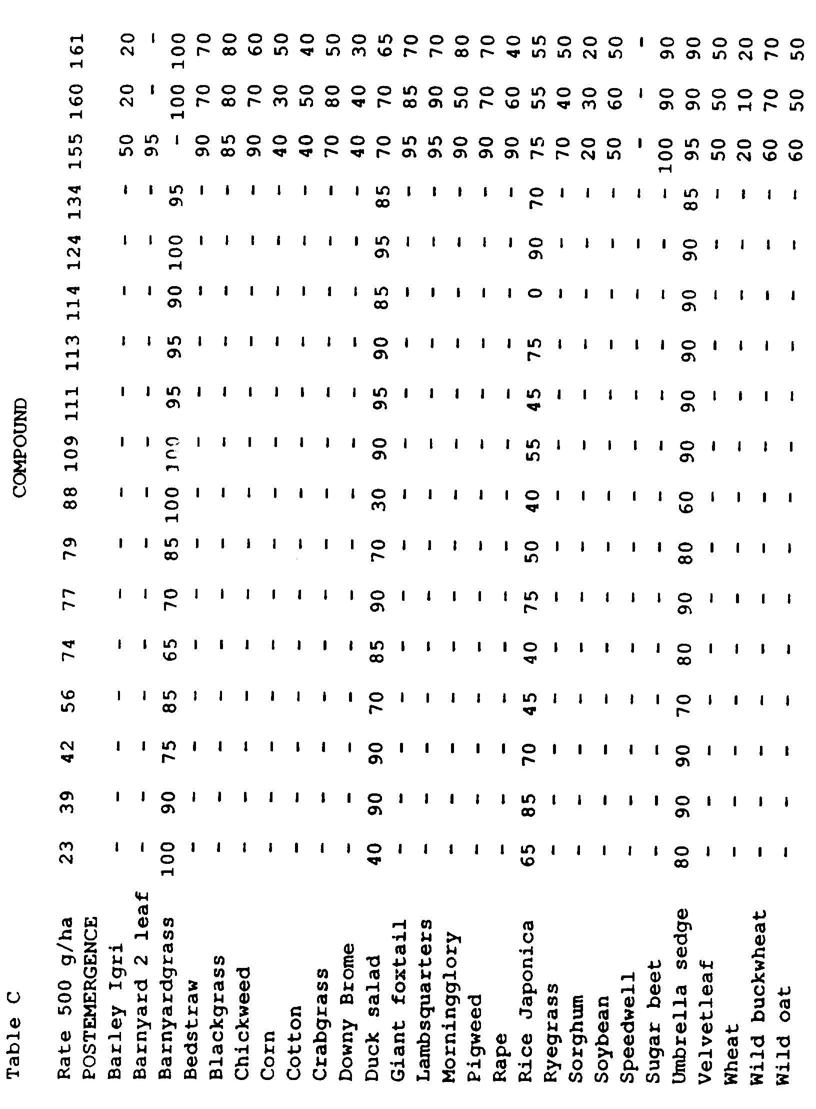

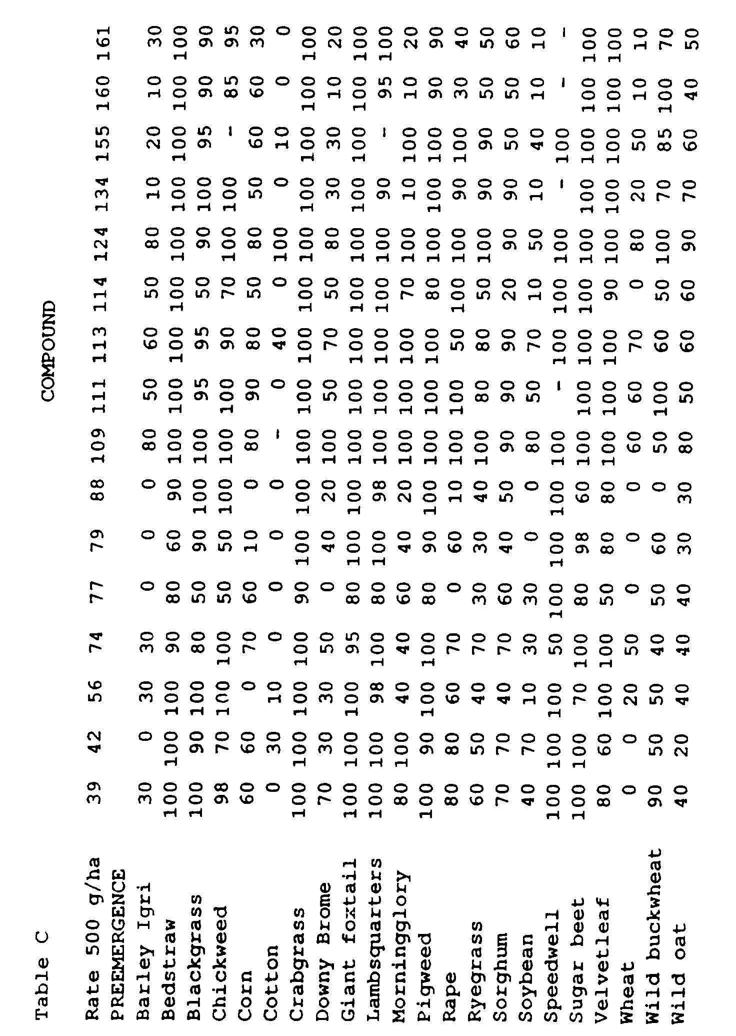

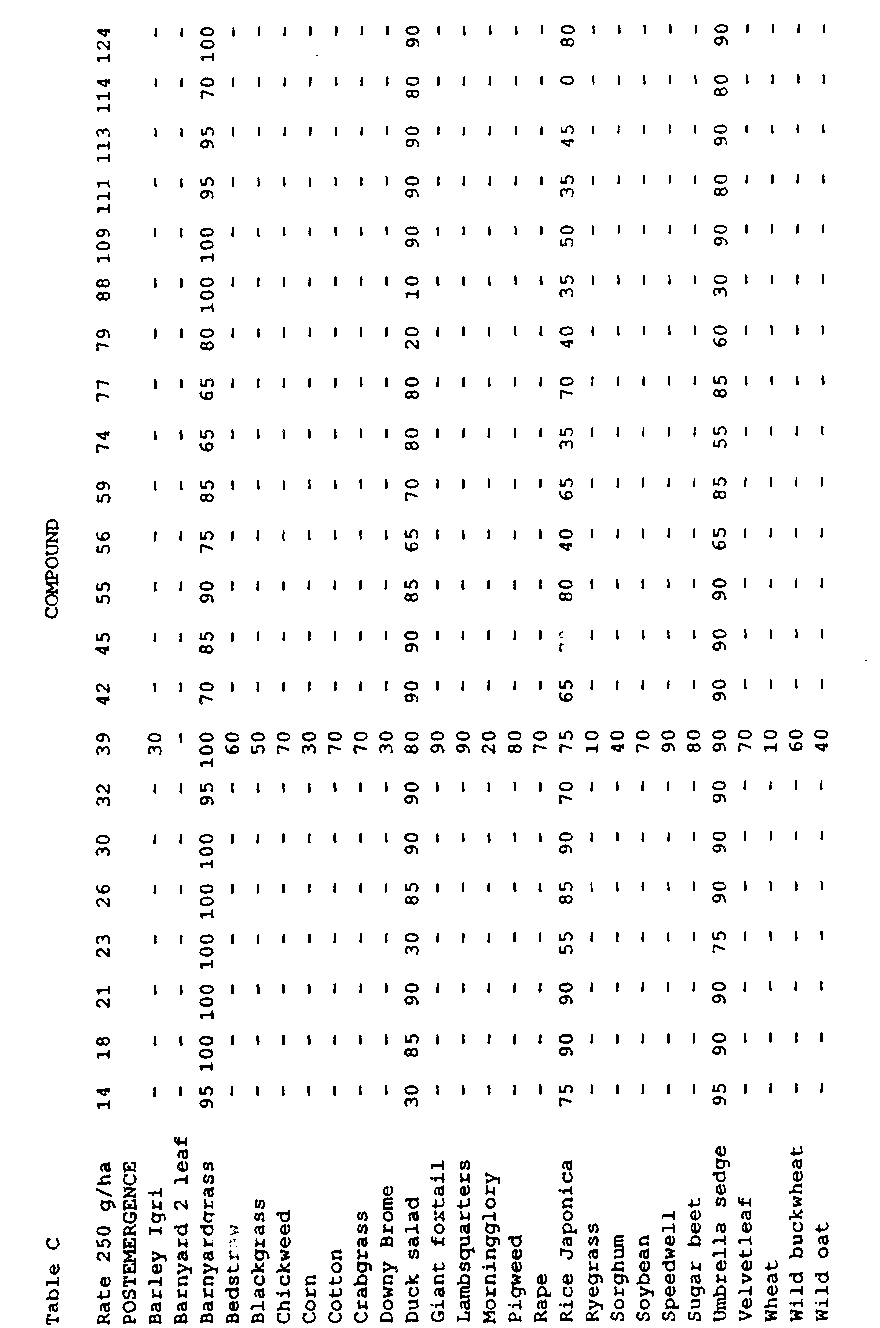

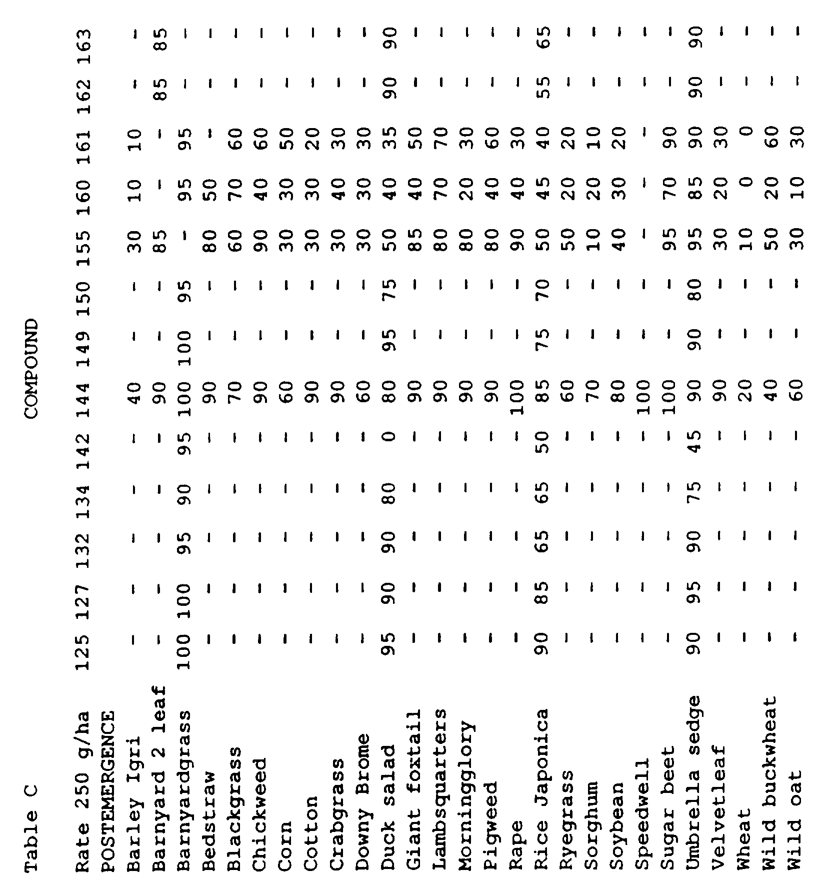

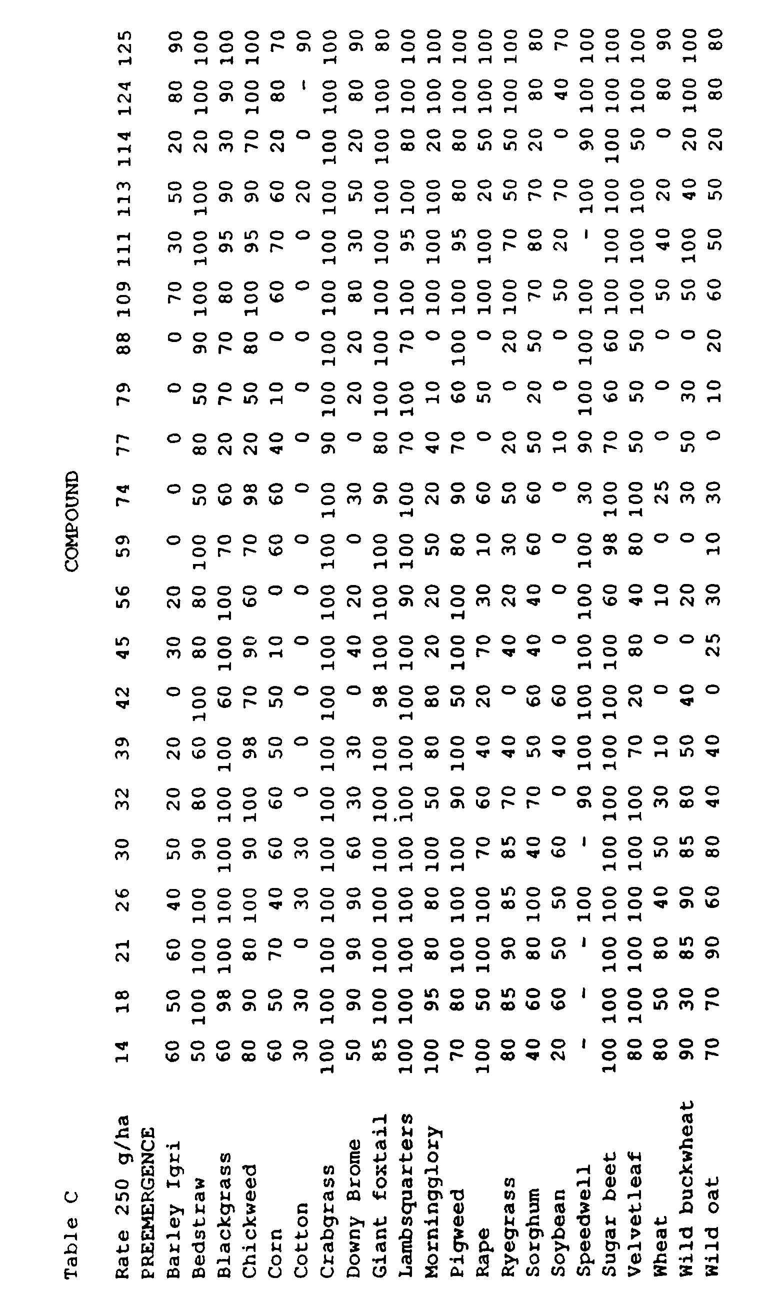

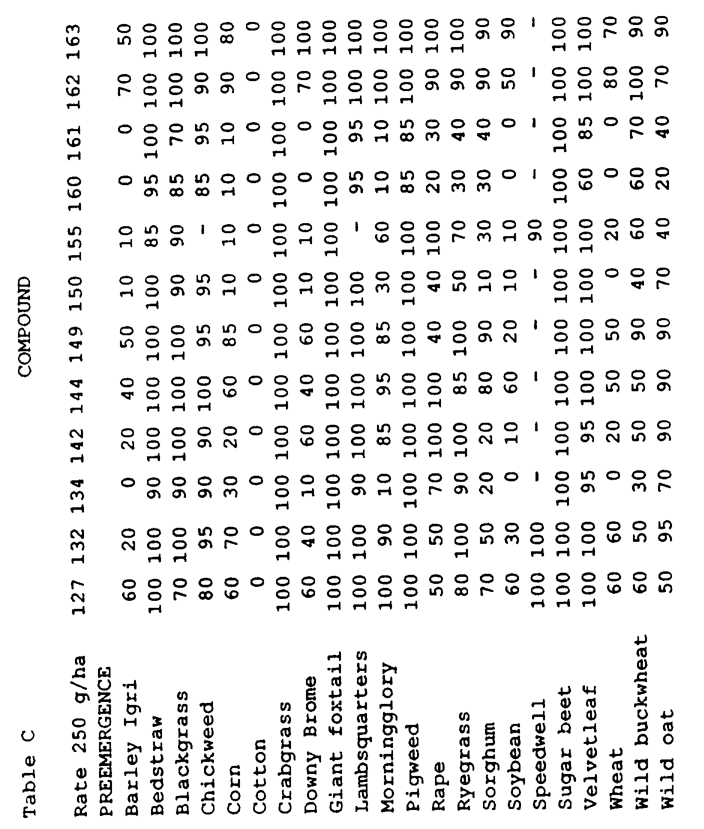

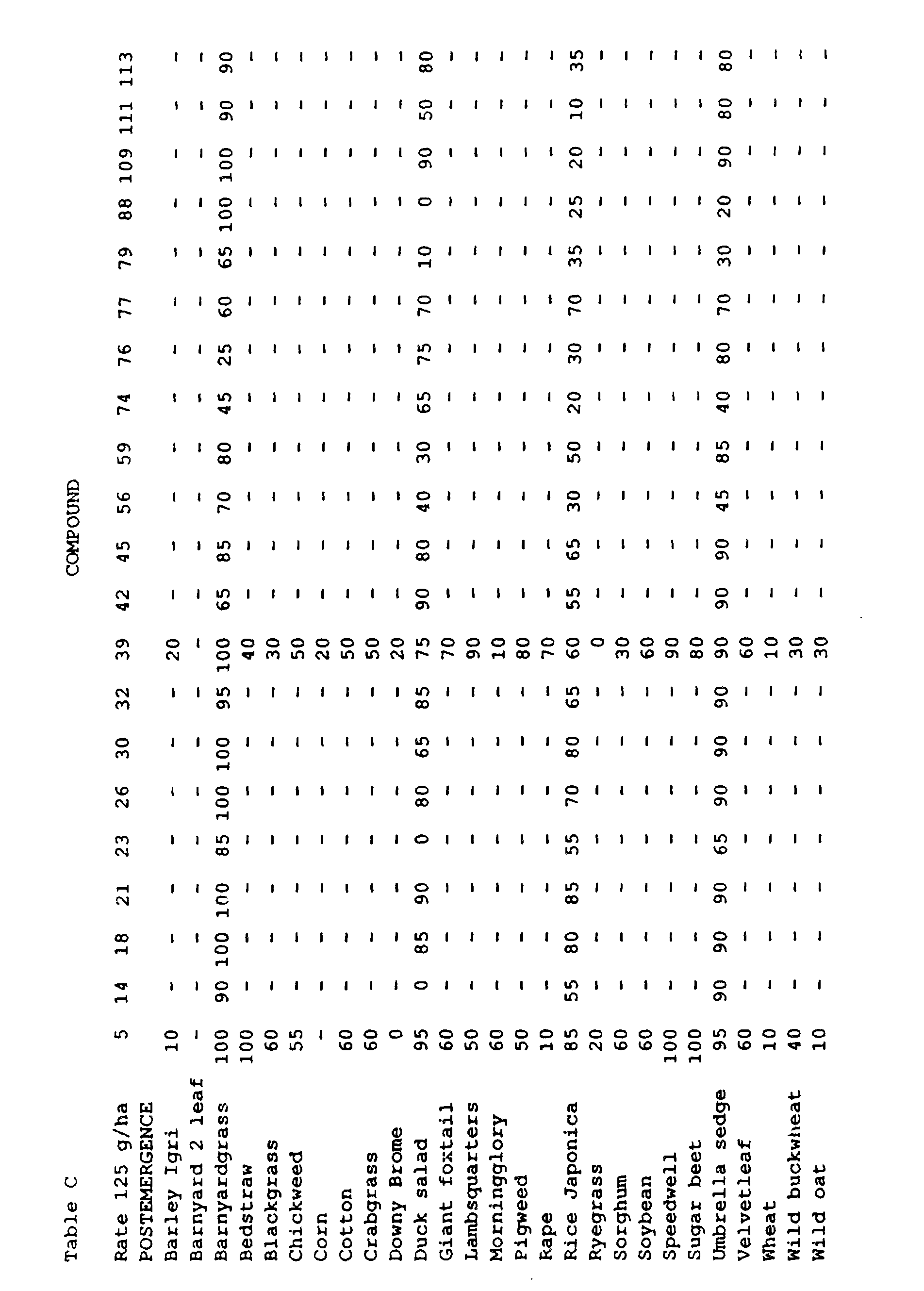

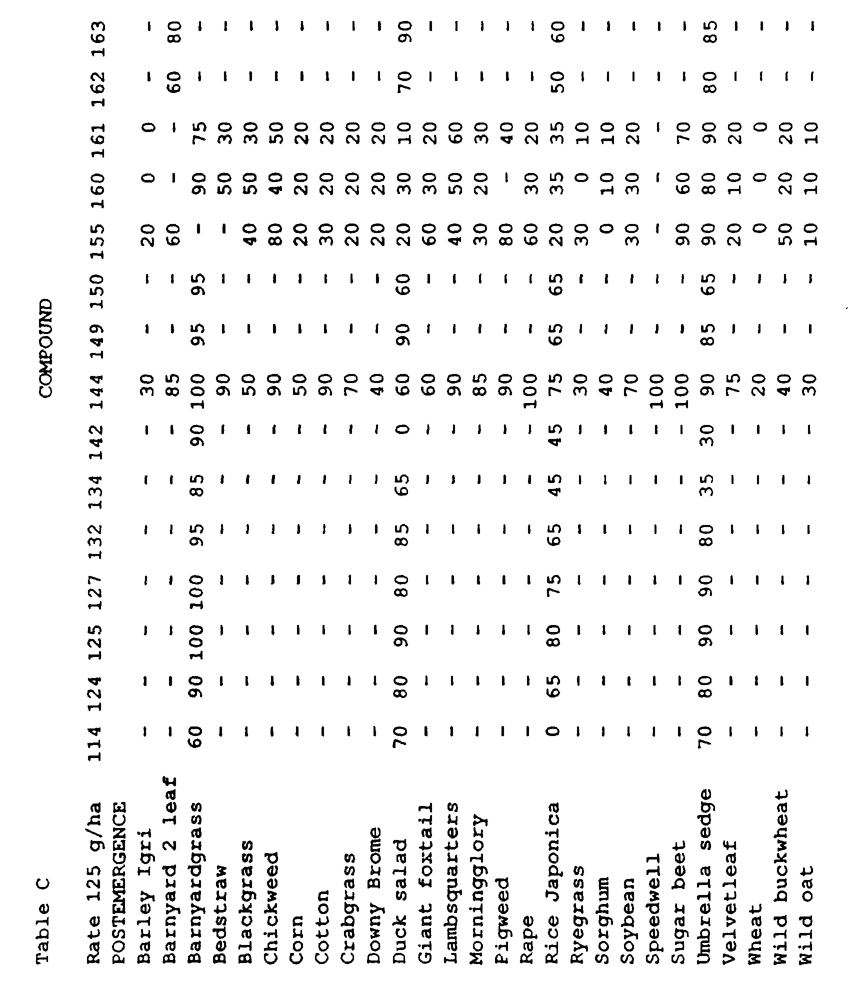

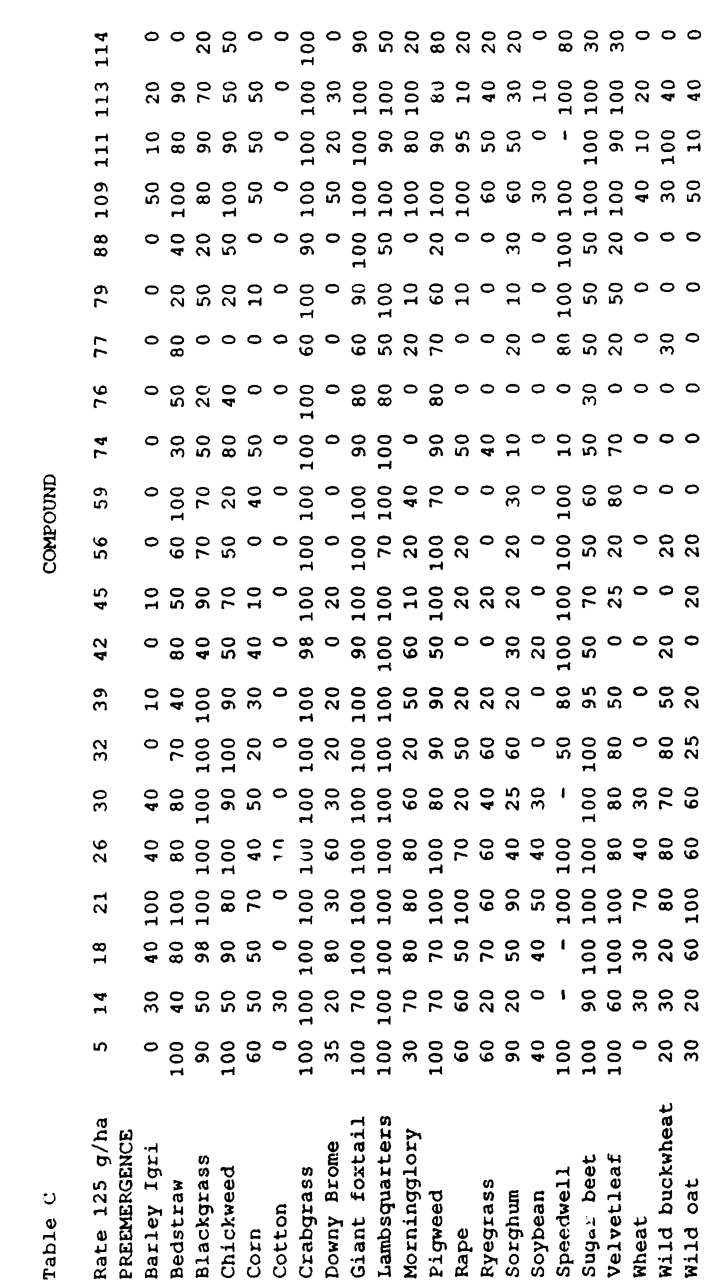

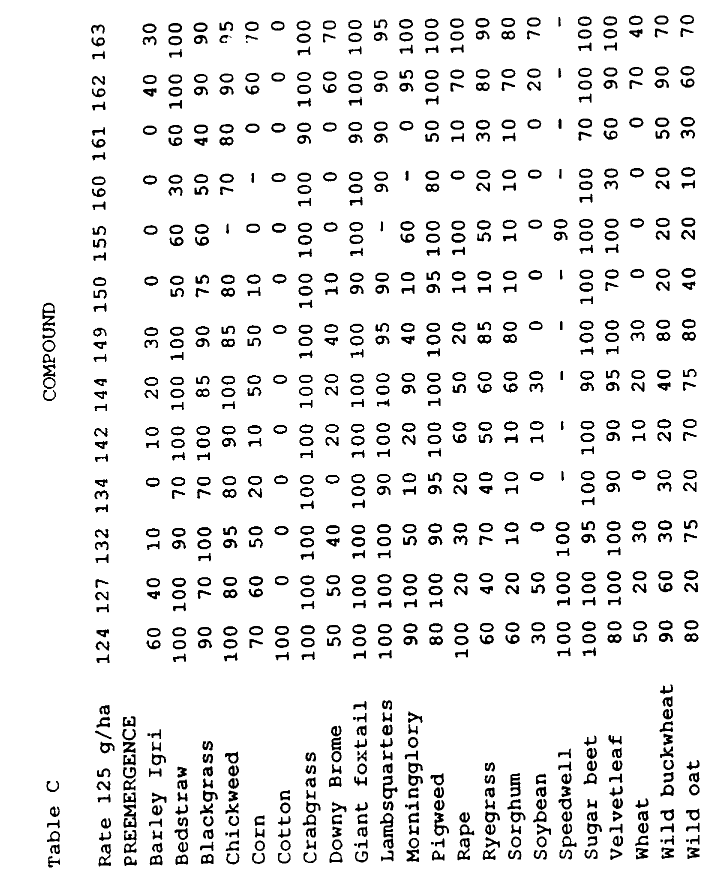

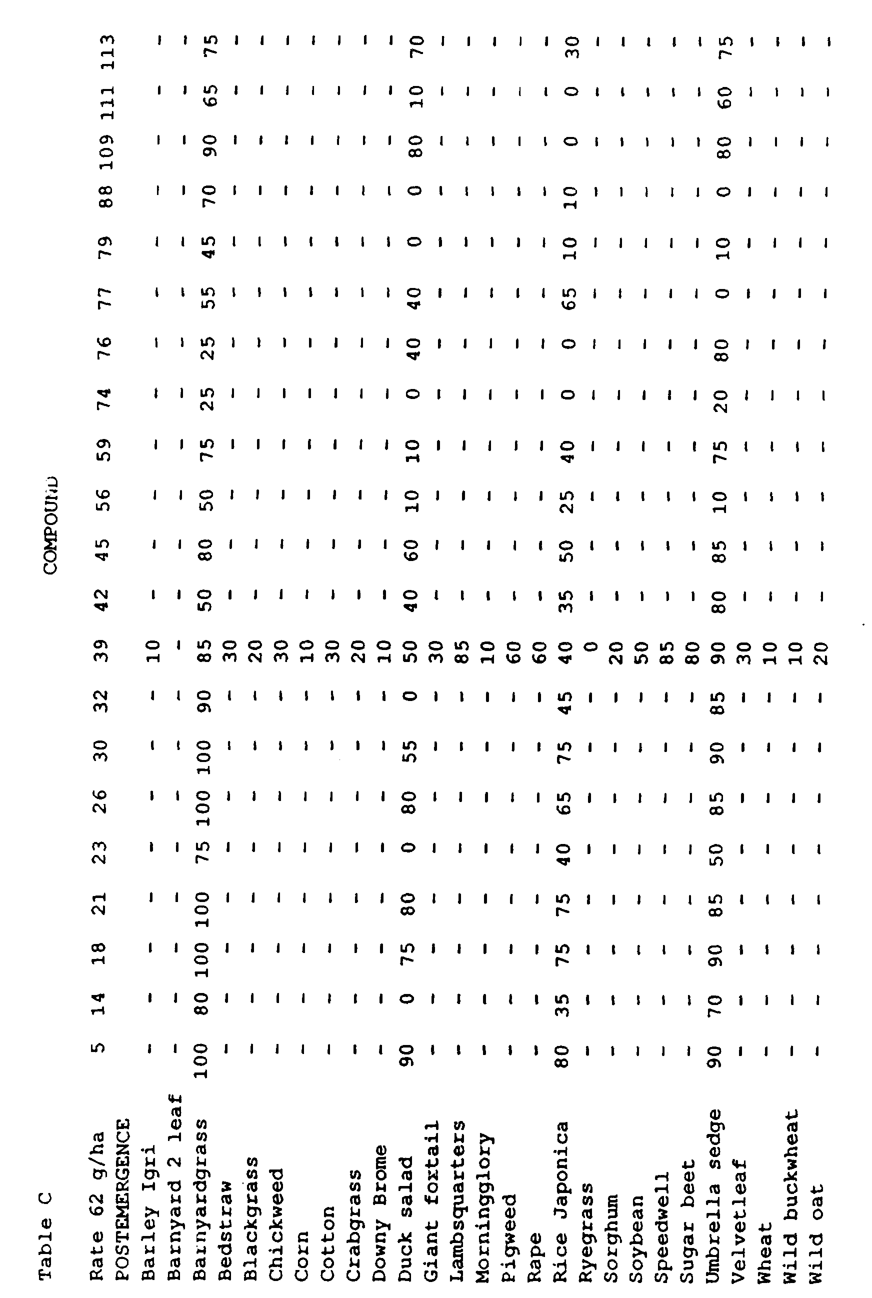

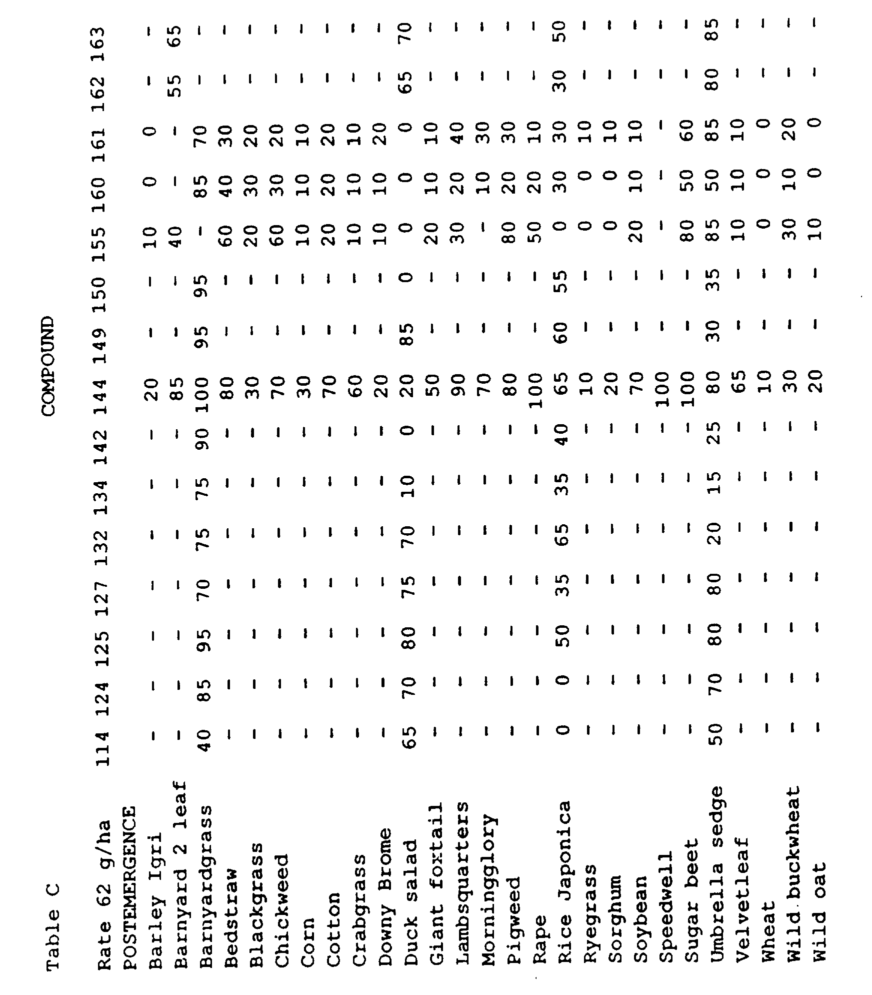

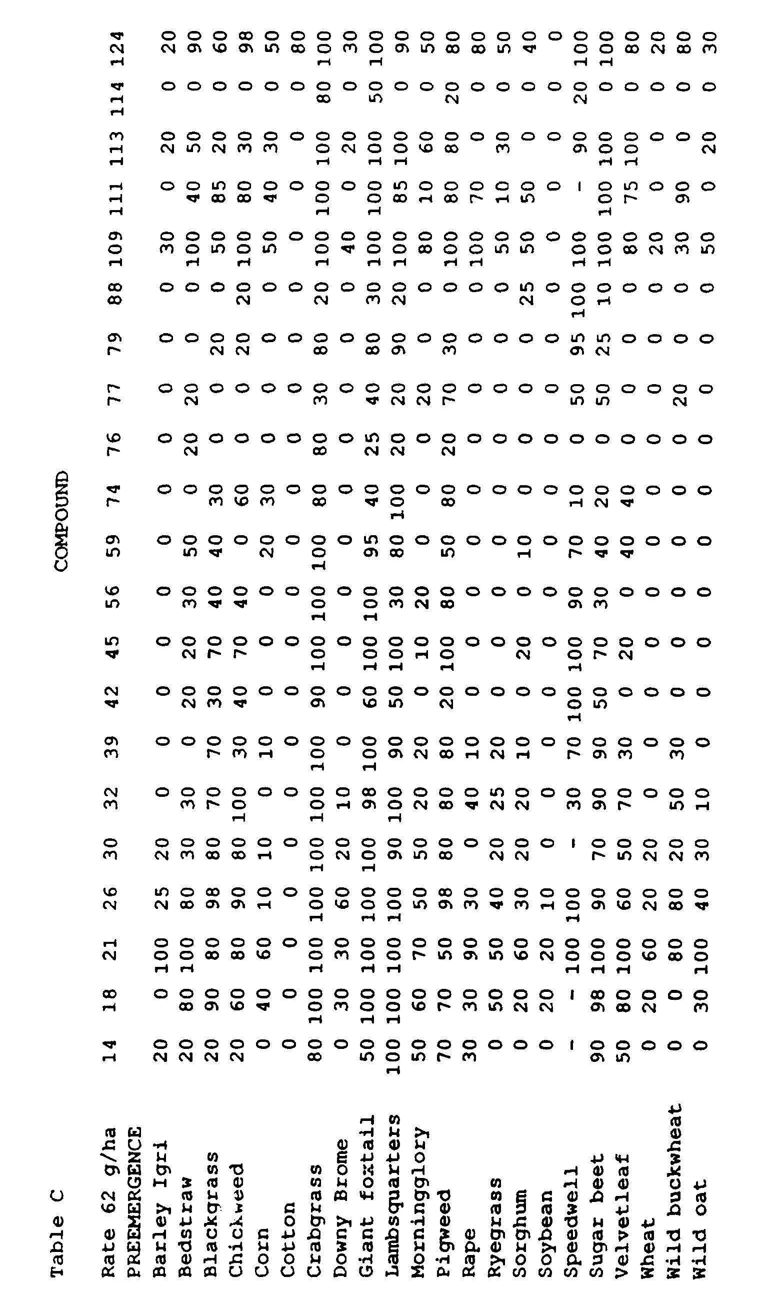

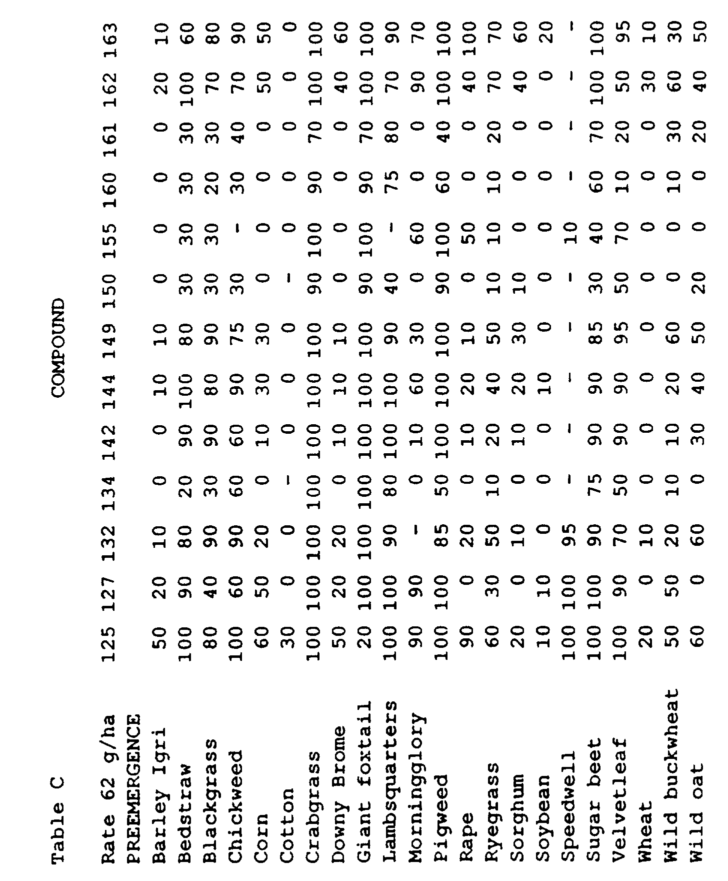

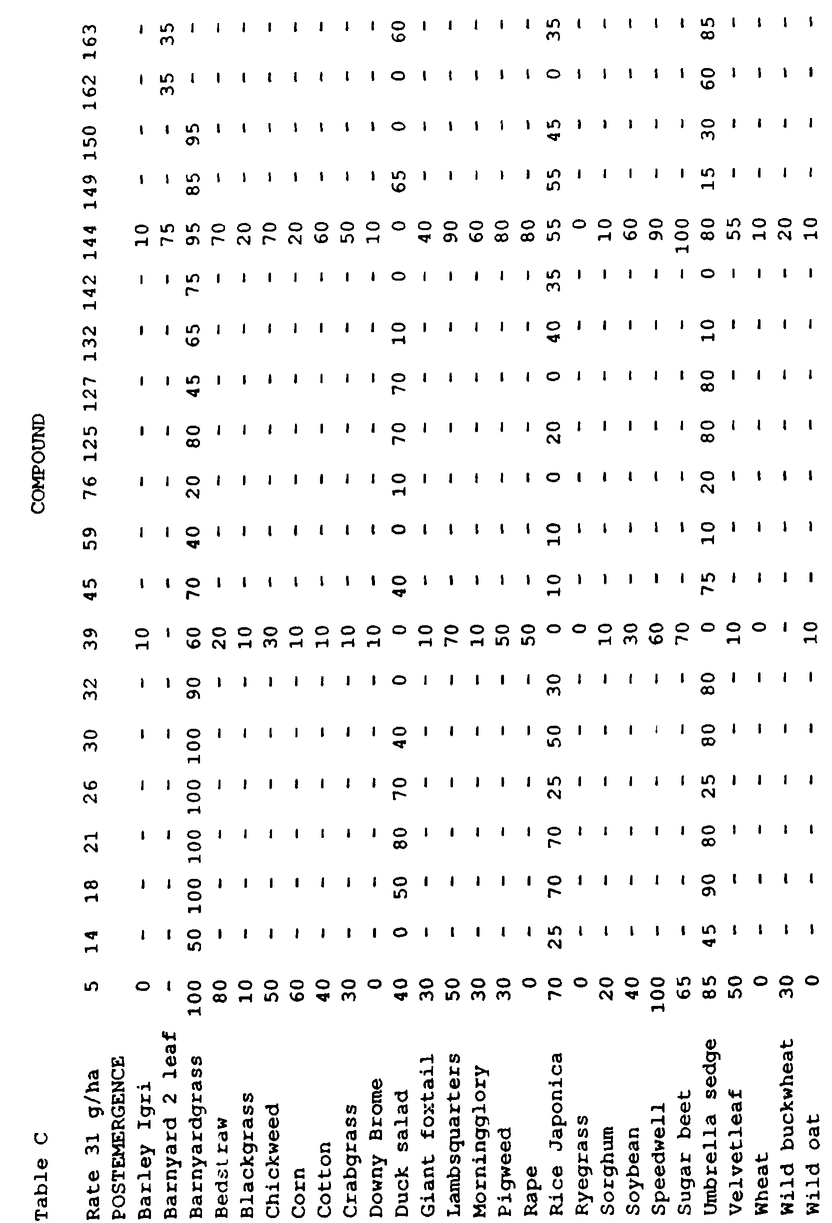

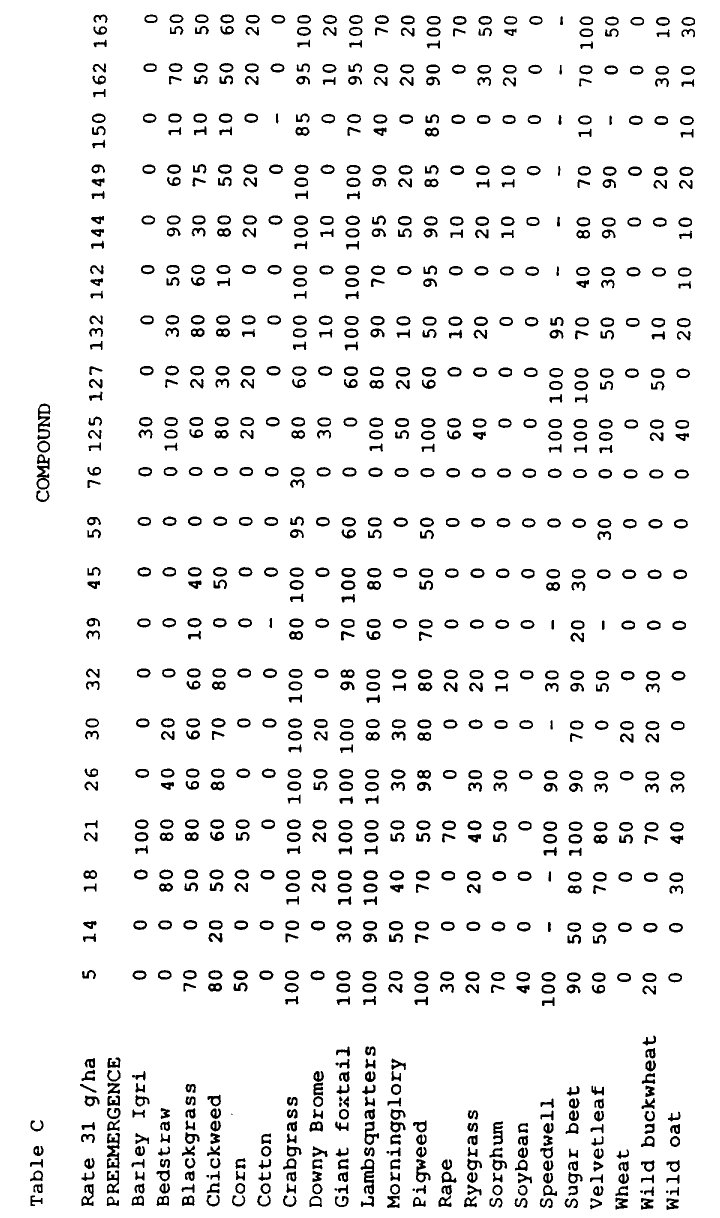

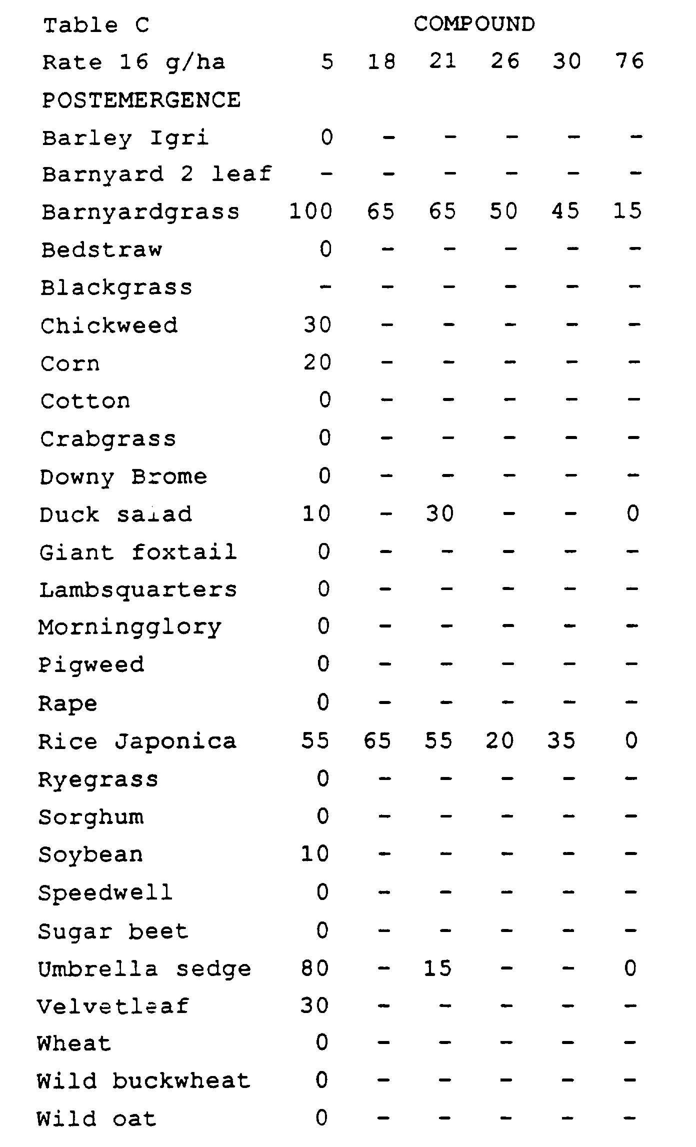

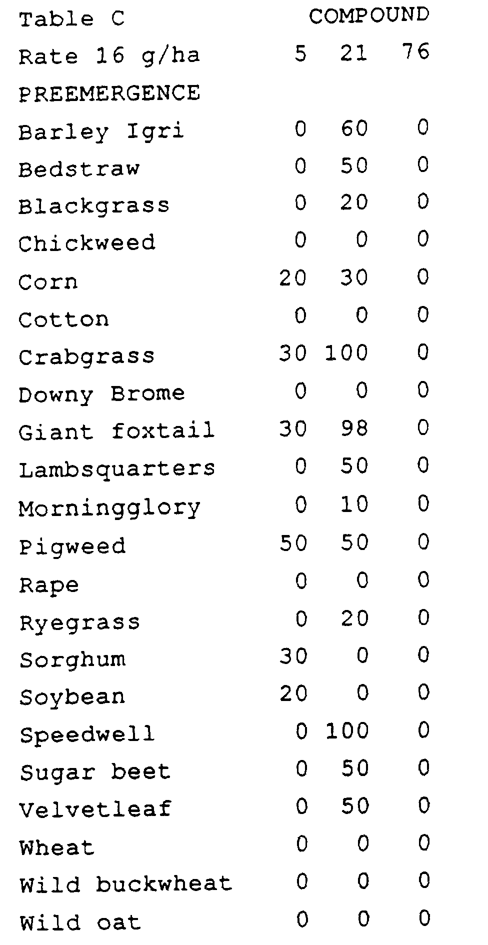

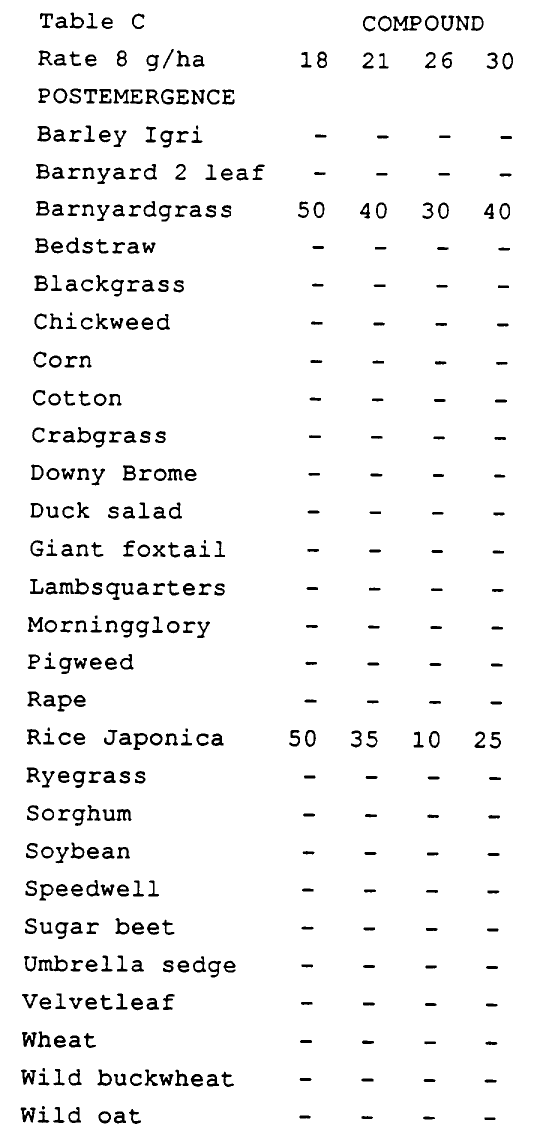

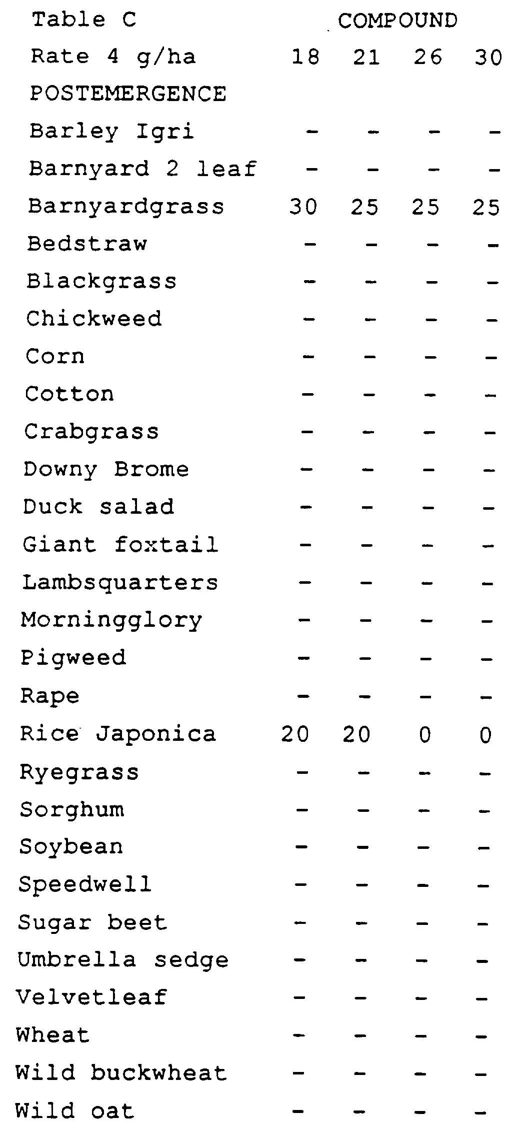

### TEST D

Seeds of barnyardgrass (*Echinochloa crus-galli*), cassia (*Cassia obtusifolia*), cocklebur (*Xanthium pensylvanicum*)*,* common ragweed (*Ambrosia elatior*), corn (*Zea mays*), cotton (*Gossypium hirsutam*), crabgrass (*Digitaria* spp.), fall panicum (*Panicum dicholomiflorum*), giant foxtail (*Setaria faberii*), green foxtail (*Setaria vividis*), jimson weed (*Datura stramonium*), johnson grass (*Sorghum halepense*), morningglory (*Ipomoea* spp.), prickly sida (*Sida spinose*), signalgrass (*Brachiaria platyphylla*), soybean (*Glycine max*), velvetleaf (*Abutilon theophrasti*) and wild proso (*Pancium miliaceum*) were planted into a silt loam soil. Test chemicals, dissolved in a non-phytotoxic solvent, were then applied to the soil surface within one day after the seeds were planted. Pots receiving these preemergence treatments were placed in the greenhouse and maintained according to routine greenhouse procedures.

Treated plants and untreated controls were maintained in the greenhouse approximately 21 days after application of the test compound. Visual evaluations of plant injury responses were then recorded. Plant response ratings, summarized in Table D, are reported on a 0 to 100 scale where 0 is no effect and 100 is complete control. A dash (-) response means no test.

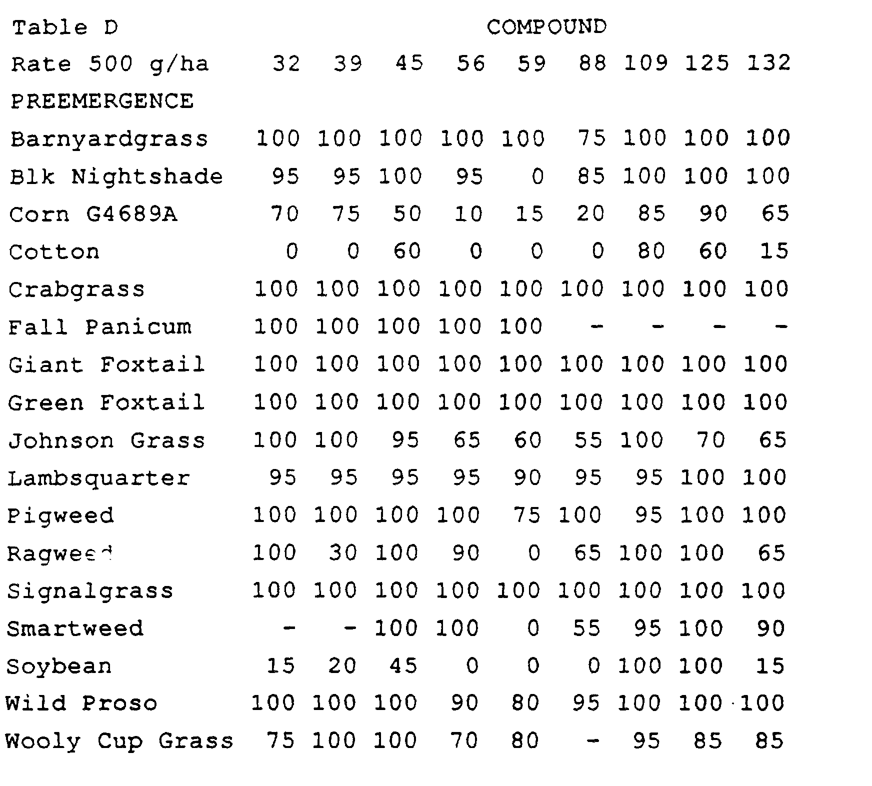

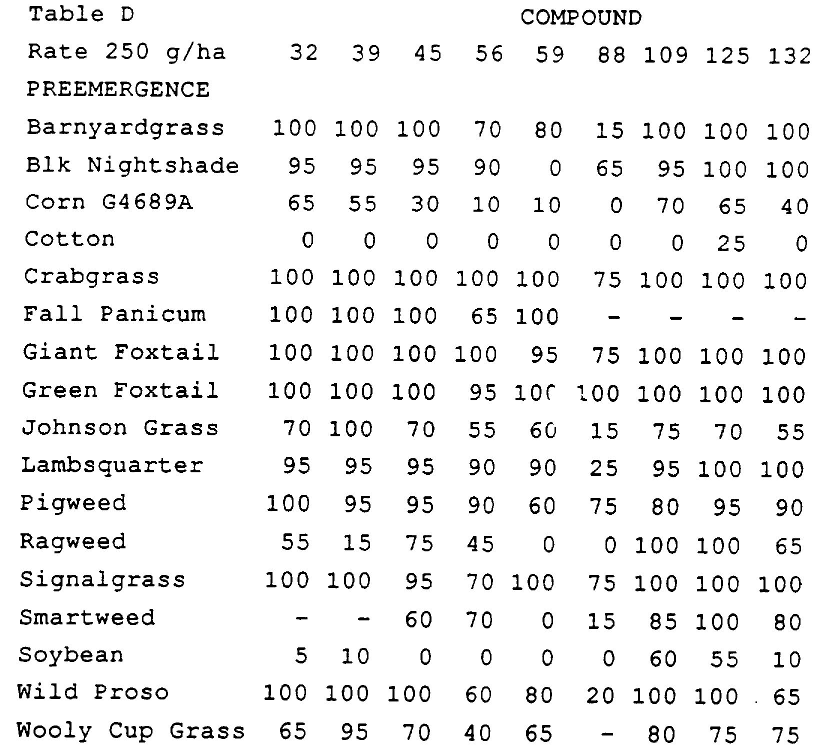

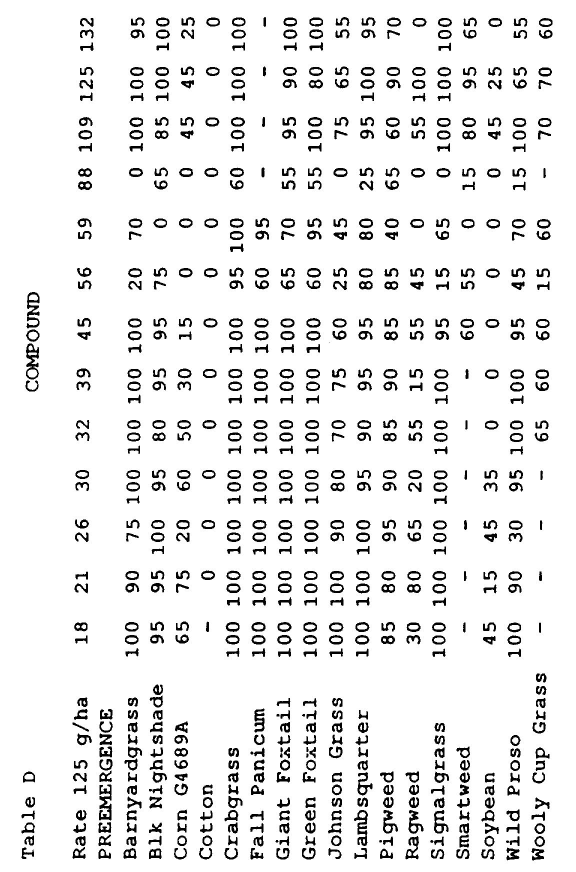

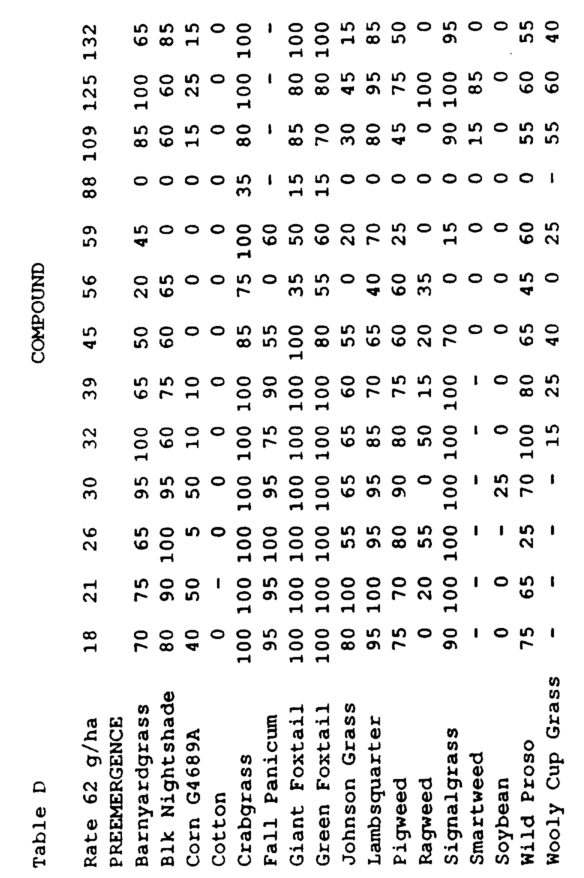

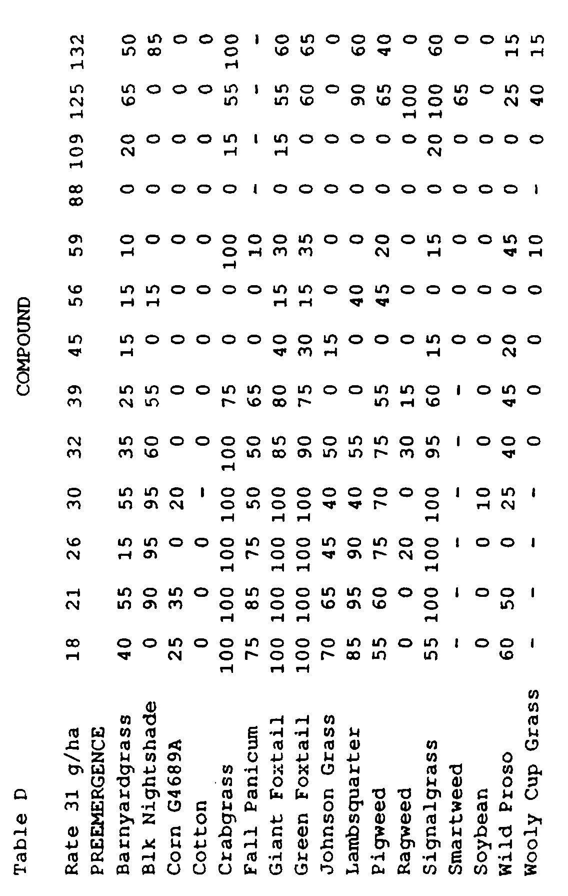

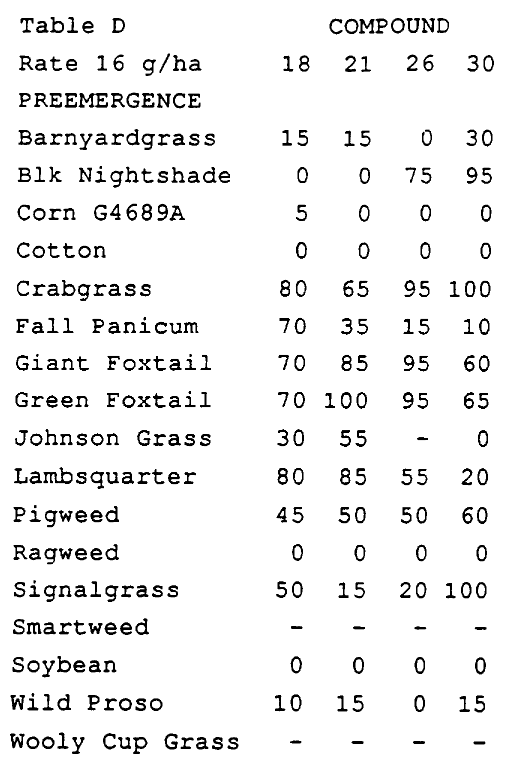

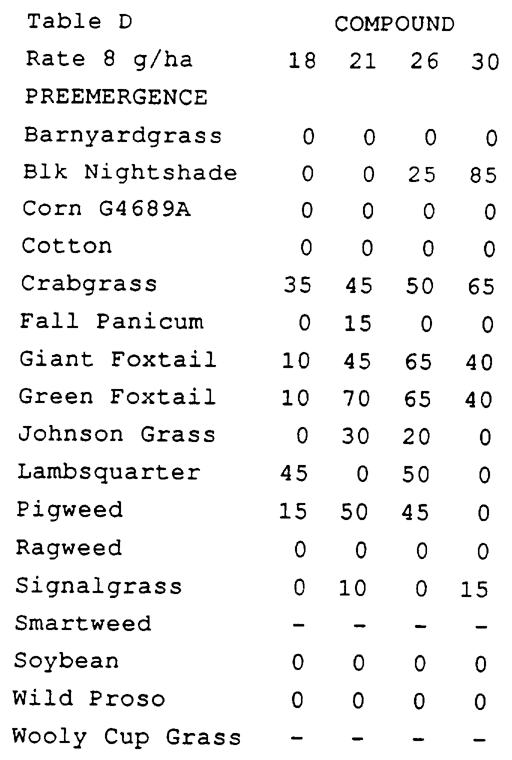

### TEST E

Plastic pots were partially filled with silt loam soil. The soil was then saturated with water. Indica and Japonica rice (*Oryza sativa*) seedlings at the 2.0 to 2.5 leaf stage, seeds selected from barnyardgrass (*Echinochloa crus-galli*), bulrush (*Scirpus mucronatus*), duck salad (*Heteranthera limosa*), umbrella sedge (*Cyperus difformis*), and tubers selected from arrowhead (*Sagittaria* spp.), waterchestnut (*Eleocharis* spp.), were planted into this soil. After planting, water levels were raised to 3 cm above the soil surface and maintained at this level throughout the test. Chemical treatments were formulated in a non-phytotoxic solvent and applied directly to the paddy water. Treated plants and controls were maintained in a greenhouse for approximately 21 days, after which all species were compared to controls and visually evaluated. Plant response ratings, summarized in Table E, are reported on a 0 to 100 scale where 0 is no effect and 100 is complete control. A dash (-) response means no test result.

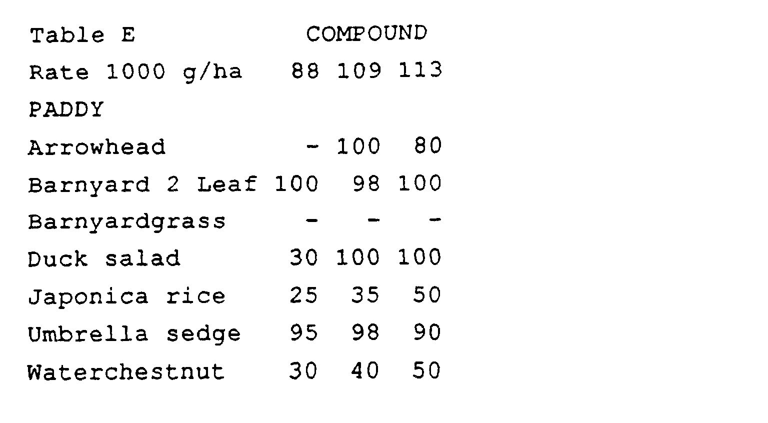

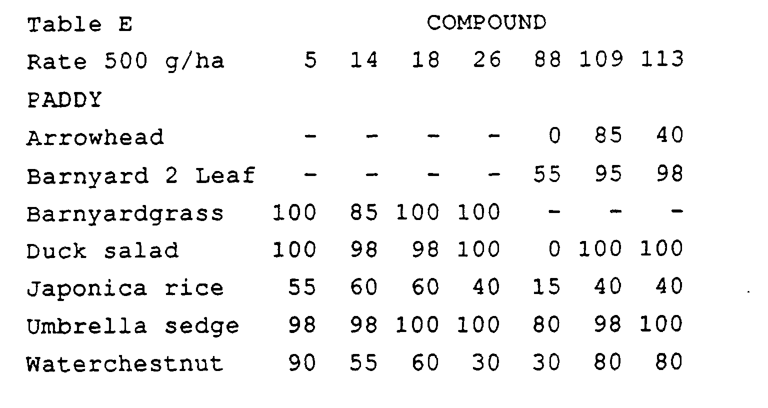

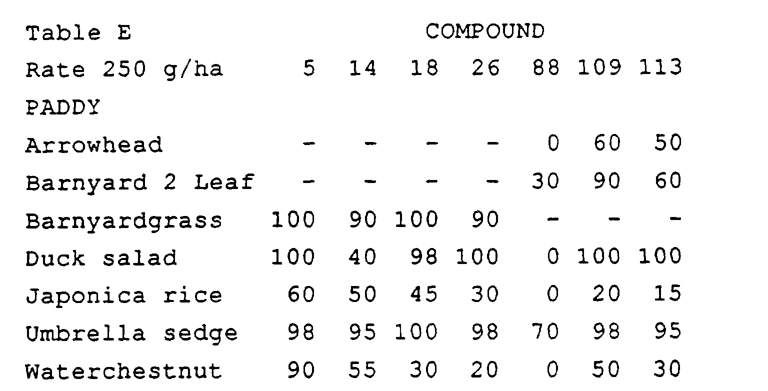

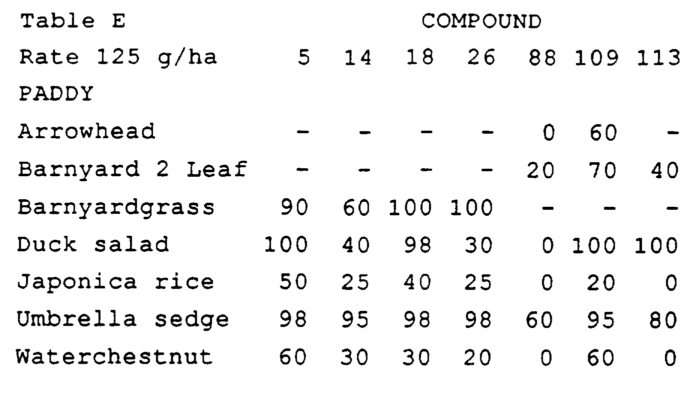

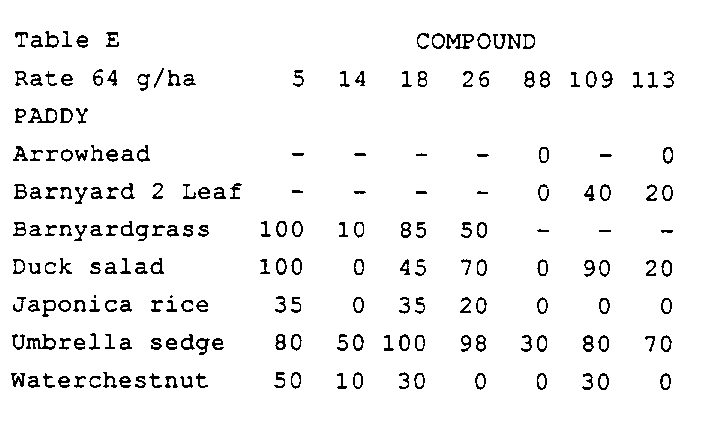

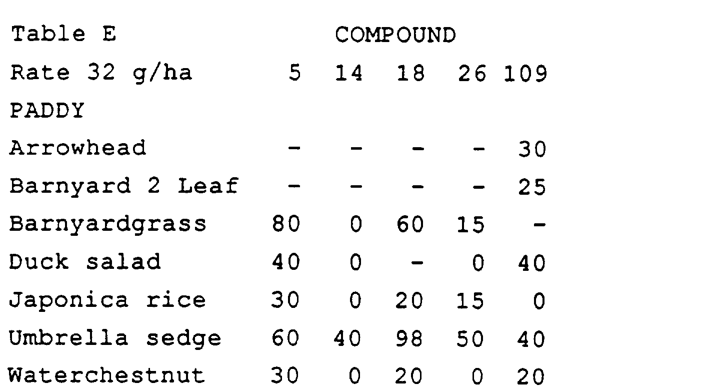

### TEST F

Plastic pots were partially filled with silt loam soil. The soil was then flooded with water, Japonica rice (*Oryza sativa*) sprouted seeds and 1.5 leaf transplants were planted in the soil. Seeds of barnyardgrass (*Echinochloa crus-galli*) were planted in saturated soil and plants grown to the 1 leaf, 2 leaf and 3 leaf stages for testing. At testing, the water level for all plantings was raised to 2 cm above the soil surface. Chemical treatments were formulated in a non-phytotoxic solvent and applied directly to the paddy water. Treated plants and controls were maintained in a greenhouse for approximately 21 days, after which all species were compared to controls and visually evaluated. Plant response ratings, summarized in Table F are reported on a 0 to 100 scale where 0 is no effect and 100 is complete control. A dash (-) response means no test result.

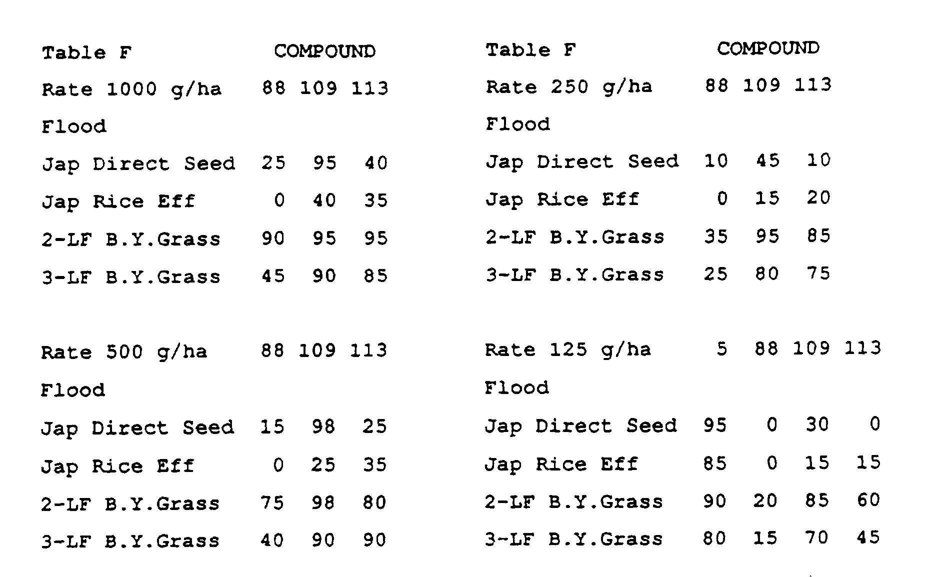

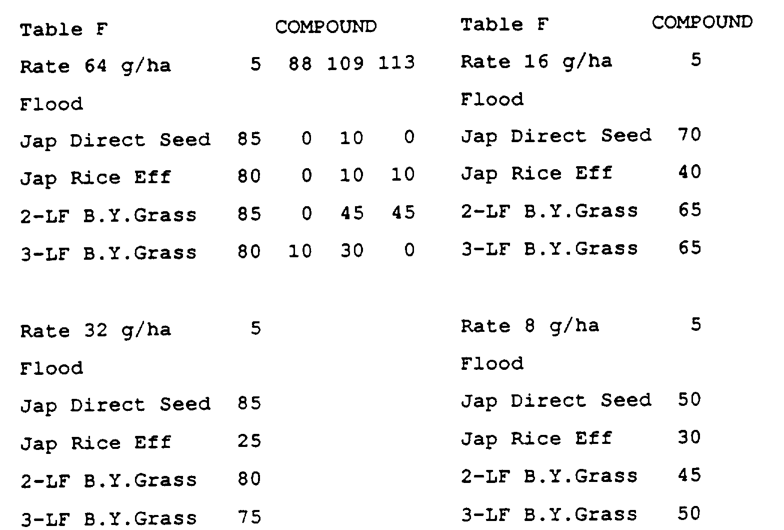

### TEST G

Compounds evaluated in this test were formulated in a non-phytoxic solvent and applied to the soil surface before plant seedlings emerged (preemergence application) and to plants that were in the one-to-four leaf stage (postemergence application). A sandy loam soil was used for the preemergence test while a mixture of sandy loam soil and greenhouse potting mix in a 60:40 ratio was used for the postemergence test. Test compounds were applied within approximately one day after planting seeds for the preemergence test. Plantings of these crops and weed species were adjusted to produce plants of appropriate size for the postemergence test. All plant species were grown using normal greenhouse practices. Crop and weed species include winter barley (*Hordeum vulgare cv.* 'Igri'), bedstraw (*Galium aparine*), blackgrass (*Alopecurus myosuroides*), chickweed (*Stellaria media*), downy brome (*Bromus tectorum*), field violet (*Viola arvensis*), green foxtail (*Setaria viridis*), kochia (*Kochia scoparia*), lambsquarters (*Chenopodium album*), Persian speedwell (*Veronica persica*), rape (*Brassica napus* cv. 'Jet Neuf'), ryegrass (*Lolium multiflorum*), sugar beet (*Beta vulgaris cv.* 'US1'), sunflower (*Helianthus annuus* cv. 'Russian Giant'), spring wheat (*Triticum aestivum* cv. 'ERA'), winter wheat (*Triticum aestivum* cv. 'Talent'), wild buckwheat (*Polygonum convolvulus*), wild mustard (*Sinapis arvensis*), wild oat (*Avena fatua*), and wild radish (*Raphanus raphanistrum*). Blackgrass, Galium and wild oat were treated postemergence at two growth stages. The first stage (1) was when the plants had two to three leaves. The second stage (2) was when the plants had approximately four leaves or in the initial stages of tillering. Treated plants and untreated controls were maintained in a greenhouse for approximately 21 to 28 days, after which all treated plants were compared to untreated controls and visually evaluated. Plant response ratings, summarized in Table G, are based upon a 0 to 100 scale where 0 is no effect and 100 is complete control. A dash response (-) means no test result.

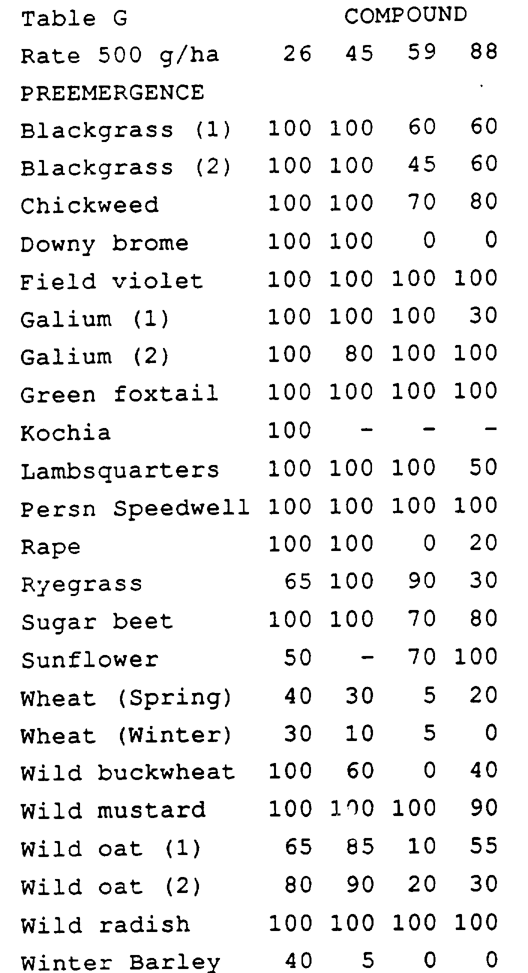

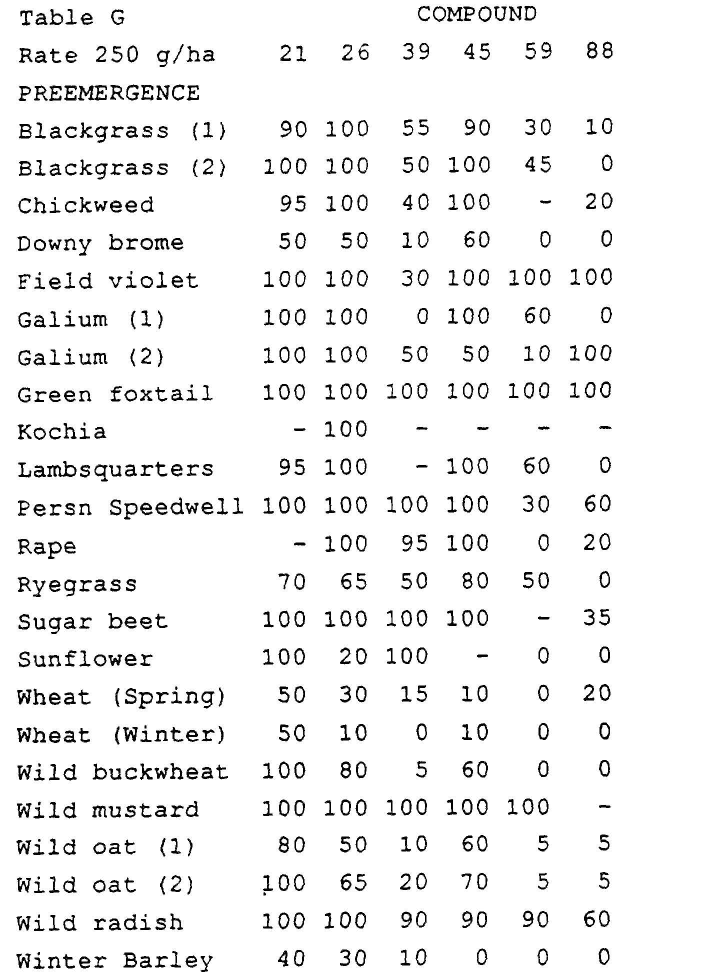

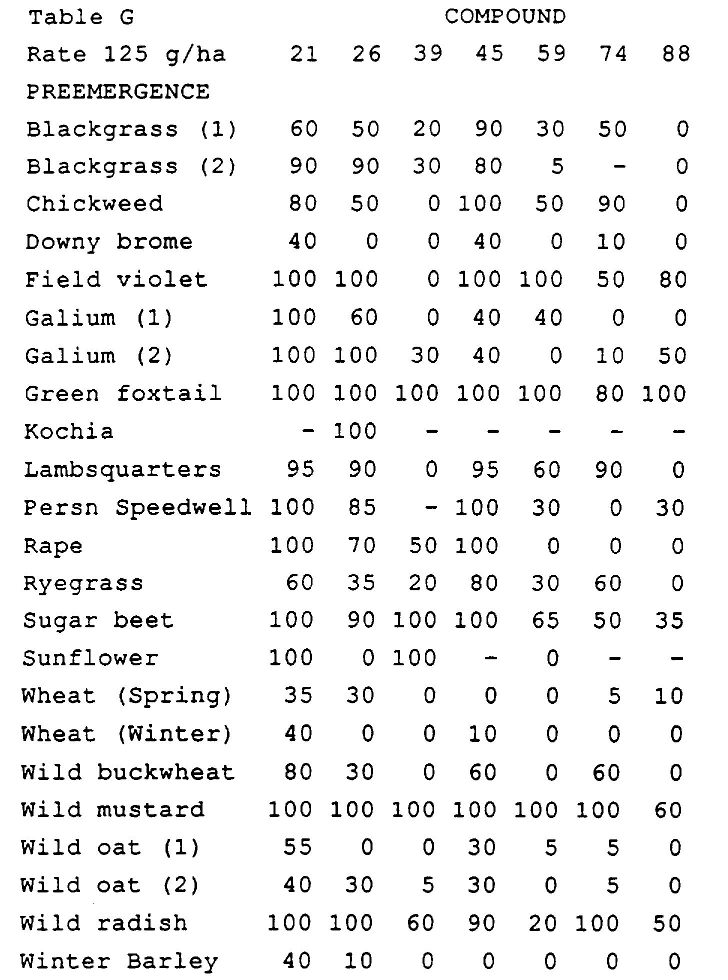

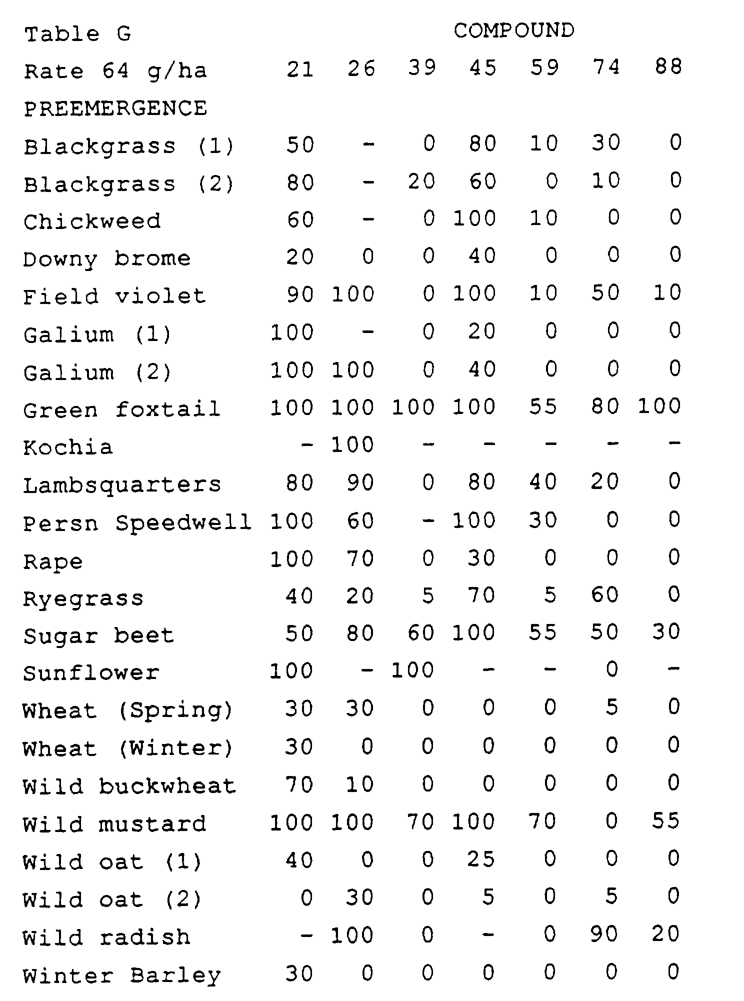

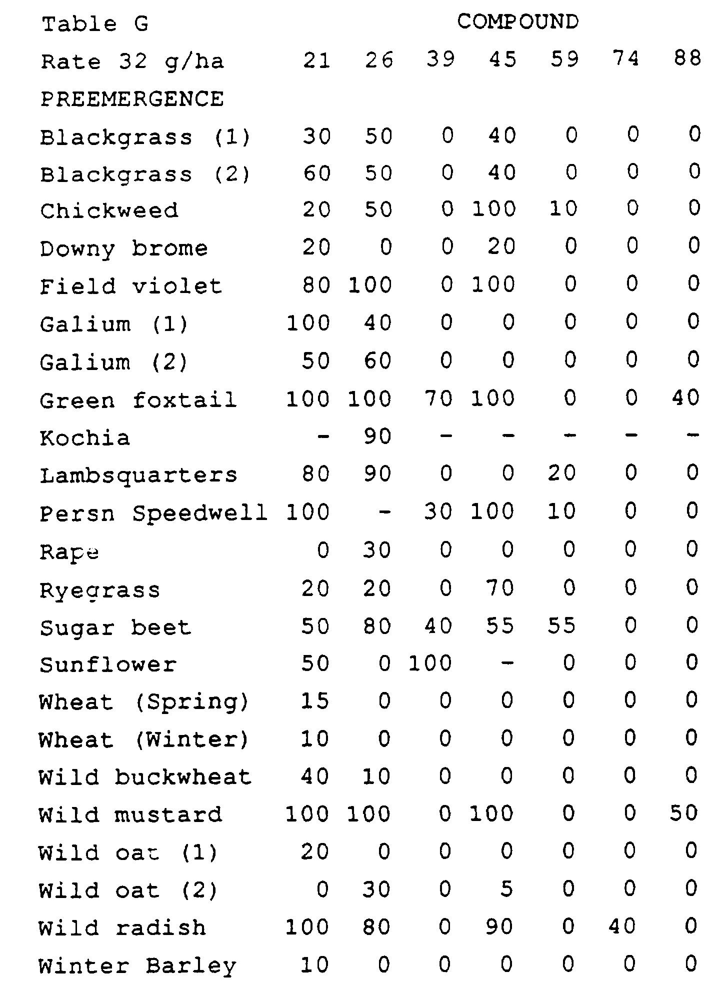

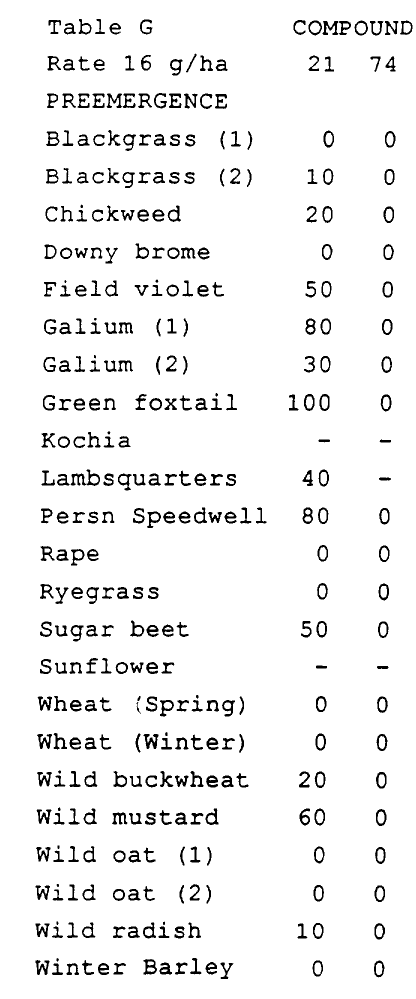

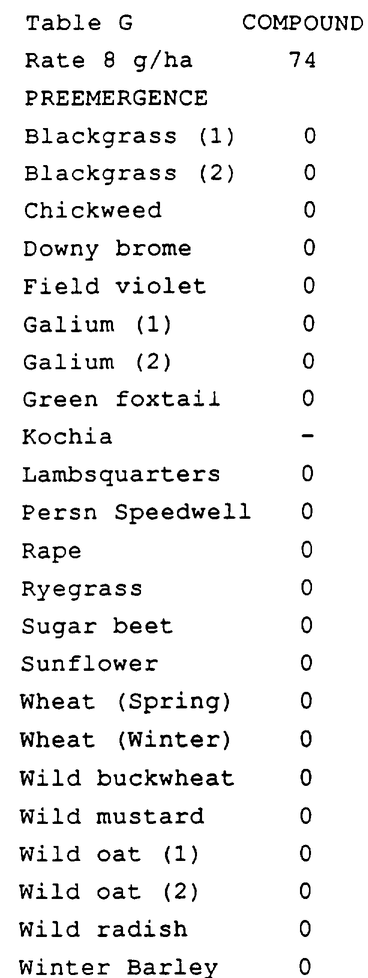

## Claims

1. A method for controlling the growth of undesired vegetation by applying to the locus to be protected an effective amount of a compound of Formula I: 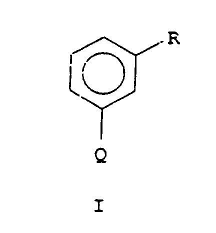 wherein
Q is 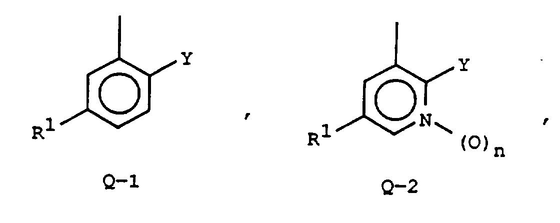 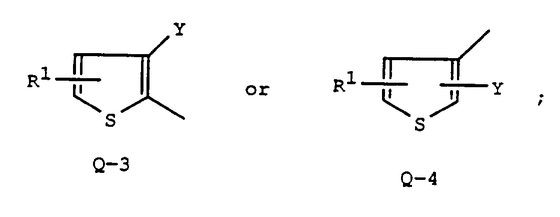
R is H, C₁-C₂ alkyl, C₁-C₂ haloalkyl, C₁-C₂ alkoxy, C₁-C₂ haloalkoxy, C₁-C₂ haloalkylthio, halogen, CN or NO₂;
Y is NR⁷C(O)XR³;
X is a single bond, O, S or NR⁴;
R¹ is H, C₁-C₃ alkyl, C₁-C₃ alkoxy, C₁-C₃ haloalkoxy, C₁-C₃ alkylthio, C₂-C₃ alkynyl, C₂-C₃ alkoxyalkyl, C₂-C₃ alkylthioalkyl, halogen, NO₂, CN, NHR⁵ or NR⁵R⁶;
R³ is C₁-C₅ alkyl optionally substituted with C₁-C₂ alkoxy, OH, 1-3 halogens, OC(O) (C₁-C₂ alkyl) or C₁-C₂ alkylthio; CH₂(C₃-C₄ cycloalkyl); C₃-C₄cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O) (C₁-C₂ alkyl); benzyl; or a 5- or 6-membered heterocyclic ring containing one heteroatom selected from O, N and S, each ring optionally substituted with halogen or CH₃;
R⁴ is H, C₁-C₂ alkyl or OCH₃;
R⁵ and R⁶ are independently C₁-C₂ alkyl;
R⁷ is H, C₁-C₂ alkyl or C(O)R⁸;
R⁸ is C₁-C₅ alkyl optionally substituted with C₁-C₂ alkoxy, OH, 1-3 halogens, OC(O) (C₁-C₂ alkyl) or C₁-C₂ alkylthio; CH₂(C₃-C₄ cycloalkyl); C₃-C₄ cycloalkyl optionally subtituted with 1-3 CH₃'s, 1-2 F's or 1-2 Cl's, C₂-C₄ alkenyl; C₂-C₄ haloalkenyl; C₃-C₄ alkynyl; C(O) (C₁-C₂ alkyl); benzyl; or a 5- or 6-membered heterocyclic ring containing one hetaroatom selected from O, N and S, each ring optionally substituted with halogen or CH₃; and
n is 0 or 1;
provided that when R³ is C₂ alkenyl, C₂ haloalkenyl or a heterocyclic ring then X is a single bond.

2. The method of Claim 1 wherein R is C₁-C₂ haloalkyl, C₁-C₂ haloalkoxy, C₁-C₂ haloalkylthio, halogen or CN.

3. The method of Claim 2 wherein R¹ is C₁-C₂ alkyl, C₁-C₂ alkoxy, C₁-C₂ alkylthio, C₂-C₃ alkoxyalkyl, C₂-C₃ alkylthioalkyl, Cl, Br, CN, NHR⁵ or NR⁵R⁶; and R⁷ is H.

4. The method of Claim 3 wherein
Q is Q-1 or Q-2; and
R³ is C₁-C₄ alkyl optionally substituted with C₁-C₂ alkoxy; CH₂ (C₃-C₄ cycloalkyl); C₃-C₄ cycloalkyl optionally substituted with 1-3 CH₃'S; or C₂-C₄ alkenyl.

5. The method of Claim 4 wherein R is C₁-C₂ fluoroalkyl, C₁-C₂ fluoroalkoxy, C₁-C₂ fluoroalkylthio, Cl, Br or CN.

6. The method of Claim 4 wherein Formula I is 1-methylethyl [5-methyl-3'-(trifluoromethyl) [1,1'-biphenyl]-2-yl]carbamate.

7. The method of Claim 4 wherein Formula I is 3-methyl-N-[5-methyl-3'-(trifluoromethyl)[1,1'-biphenyl]-2-yl]butanamide.

8. An agriculturally suitable composition for controlling the growth of undesired vegetation comprising an effective amount of a compound of any of Claims 1-7 with the further proviso that when Q is Q-1, R¹ is H and Y is NHC(O)CH₃, then R is other than Br or F, and at least one of the following: surfactant, solid or liquid diluents.

9. A compound of Formula I 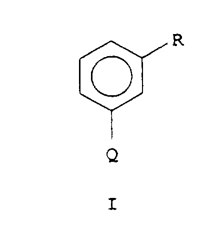 wherein
Q is 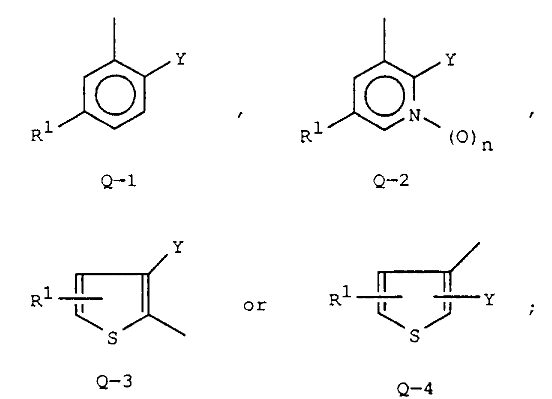
R is C₁-C₂ alkyl, C₁-C₂ haloalkyl, C₁-C₂ alkoxy, C₁-C₂ haloalkoxy, C₁-C₂ haloalkylthio, halogen, CN or NO₂;
Y is NR⁷C(O)XR³;
X is a single bond, O, S or NR⁴;
R¹ is H, C₁-C₃ alkyl, C₁-C₃ alkoxy, C₁-C₃ haloalkoxy, C₁-C₃ alkylthio, C₂-C₃ alkynyl, C₂-C₃ alkoxyalkyl, C₂-C₃ alkylthioalkyl, halogen, NO₂, CN, NHR⁵ or NR⁵R⁶;
R³ is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O) (C₁-C₂ alkyl); benzyl; or a 5- or 6-membered heterocyclic ring containing one heteroatom selected from O, N and S, each ring optionally substituted with halogen or CH₃;
R⁴ is H, C₁-C₂ alkyl or OCH₃;
R⁵ and R⁶ are independently C₁-C₂ alkyl;
R⁷ is H, C₁-C₂ alkyl or C(O)R⁸;
R⁸ is C₁-C₅ alkyl optionally substituted with C₁-C₂ alkoxy, OH, 1-3 halogens, OC(O)(C₁-C₂ alkyl) or C₁-C₂ alkylthio; CH₂(C₃-C₄ cycloalkyl); C₃-C₄ cycloalkyl optionally substituted with 1-3 CH₃'s, 1-2 F's or 1-2 Cl's; C₂-C₄ alkenyl; C₂-C₄ haloalkenyl; C₃-C₄ alkynyl; C(O)(C₁-C₂ alkyl); benzyl; or a 5- or 6-membered heterocyclic ring containing one heteroatom selected from O, N and S, each ring optionally substituted with halogen or CH₃; and
n is 0 or 1;
provided that when R³ is C₂ alkenyl, C₂ haloalkenyl or a heterocyclic ring then X is a single bond, and when Q is Q-1, R¹ is H, and Y is NHC(O)CH₃, then R is other than Br or F.

## Patentansprüche

1. Verfahren zur Kontrolle des Wachstums von unerwünschter Vegetation durch Ausbringen einer wirksamen Menge von einer Verbindung der Formel I: 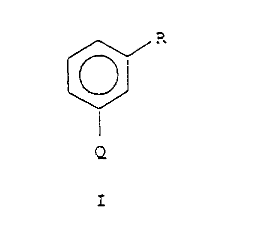 an den zu schützenden Ort,
worin
Q 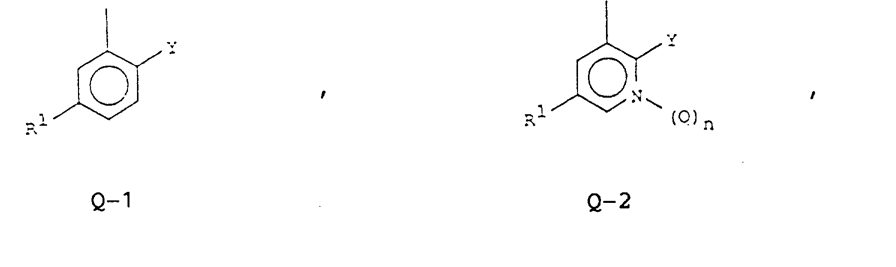 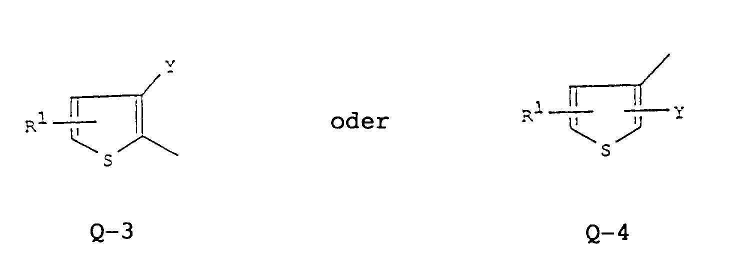 ist;
R H, C₁-C₂-Alkyl, C₁-C₂-Halogenalkyl, C₁-C₂-Alkoxy, C₁-C₂-Halogenalkoxy, C₁-C₂-Halogenalkylthio, Halogen, CN oder NO₂ ist;
Y NR⁷C(O)XR³ ist;
X eine Einfachbindung, O, S oder NR⁴ ist;
R¹ H, C₁-C₃-Alkyl, C₁-C₃-Alkoxy, C₁-C₃-Halogenalkoxy, C₁-C₃-Alkylthio, C₂-C₃-Alkinyl, C₂-C₃-Alkoxyalkyl, C₂-C₃-Alkylthioalkyl, Halogen, NO₂, CN, NHR⁵ oder NR⁵R⁶ ist;
R³ C₁-C₅-Alkyl, gegebenenfalls mit C₁-C₂-Alkoxy, OH, 1-3 Halogenen, OC(O)(C₁-C₂-Alkyl) oder C₁-C₂-Alkylthio substituiert; CH₂(C₃-C₄-Cycloalkyl); C₃-C₄-Cycloalkyl, gegebenenfalls mit 1-3 CH₃-Gruppen, 1-2 F-Atomen oder 1-2 Cl-Atomen substituiert; C₂-C₄-Alkenyl; C₂-C₄-Halogenalkenyl; C₃-C₄-Alkinyl; C(O)(C₁-C₂-Alkyl); Benzyl oder ein 5- oder 6-gliedriger heterocyclischer Ring, der ein Heteroatom, ausgewählt aus O, N und S enthält, wobei jeder Ring gegebenenfalls mit Halogen oder CH₃ substituiert ist, ist;
R⁴ H, C₁-C₂-Alkyl oder OCH₃ ist;
R⁵ und R⁶ unabhängig voneinander C₁-C₂-Alkyl sind;
R⁷ H, C₁-C₂-Alkyl oder C(O)R⁸ ist;
R⁸ C₁-C₅-Alkyl, gegebenenfalls mit C₁-C₂-Alkoxy, OH, 1-3 Halogenen, OC(O)(C₁-C₂-Alkyl) oder C₁-C₂-Alkylthio substituiert; CH₂(C₃-C₄-Cycloalkyl); C₃-C₄-Cycloalkyl, gegebenenfalls mit 1-3 CH₃-Gruppen, 1-2 F-Atomen oder 1-2 Cl-Atomen substituiert; C₂-C₄-Alkenyl; C₂-C₄-Halogenalkenyl; C₃-C₄-Alkinyl; C(O)(C₁-C₂-Alkyl); Benzyl oder ein 5- oder 6-gliedriger heterocyclischer Ring, der ein Heteroatom, ausgewählt aus O, N und S enthält, wobei jeder Ring gegebenenfalls mit Halogen oder CH₃ substituiert ist, ist; und
n 0 oder 1 ist;
vorausgesetzt, daß X eine Einfachbindung ist, wenn R³ C₂-Alkenyl, C₂-Halogenalkenyl oder ein heterocyclischer Ring ist.

2. Verfahren nach Anspruch 1, worin R C₁-C₂-Halogenalkyl, C₁-C₂-Halogenalkoxy, C₁-C₂-Halogenalkylthio, Halogen oder CN ist.

3. Verfahren nach Anspruch 2, worin R¹ C₁-C₂-Alkyl, C₁-C₂-Alkoxy, C₁-C₂-Alkylthio, C₂-C₃-Alkoxyalkyl, C₂-C₃-Alkylthioalkyl, Cl, Br, CN, NHR⁵ oder NR⁵R⁶ ist, und R⁷ H ist.

4. Verfahren nach Anspruch 3, worin
Q Q-1 oder Q-2 ist; und
R³ C₁-C₄-Alkyl, gegebenenfalls mit C₁-C₂-Alkoxy substituiert; CH₂(C₃-C₄-Cycloalkyl); C₃-C₄-Cycloalkyl, gegebenenfalls mit 1-3 CH₃-Gruppen substituiert; oder C₂-C₄-Alkenyl ist.

5. Verfahren nach Anspruch 4, worin R C₁-C₂-Fluoralkyl, C₁-C₂-Fluoralkoxy, C₁-C₂-Fluoralkylthio, Cl, Br oder CN ist.

6. Verfahren nach Anspruch 4, worin Formel I 1-Methylethyl-[5-methyl-3'-(trifluormethyl)[1,1'-biphenyl]-2-yl]carbamat ist.

7. Verfahren nach Anspruch 4, worin Formel I 3-Methyl-N-[5-methyl-3'-(trifluormethyl)[1,1'-biphenyl]-2-yl]butanamid ist.

8. Landwirtschaftlich geeignete Zusammensetzung zur Kontrolle des Wachstums von unerwünschter Vegetation, umfassend eine wirksame Menge von einer Verbindung nach irgendeinem der Ansprüche 1-7, mit der weiteren Maßgabe, daß R anders als Br oder F ist, wenn Q Q-1 ist, R¹ H ist, und Y NHC(O)CH₃ ist, und wenigstens einen der nachfolgenden Stoffe: Tensid, festes oder flüssiges Verdünnungsmittel.

9. Verbindung der Formel I 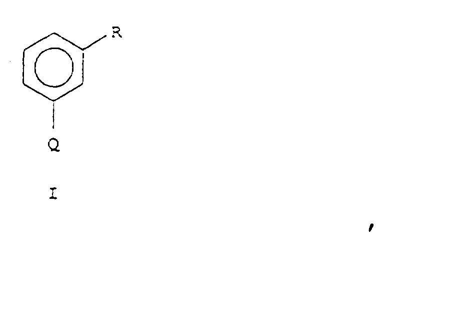 worin
Q 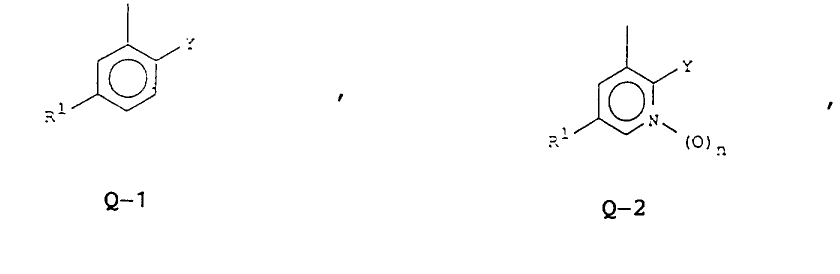 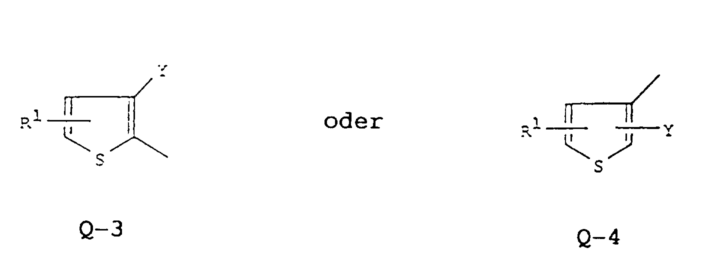 ist;
R C₁-C₂-Alkyl, C₁-C₂-Halogenalkyl, C₁-C₂-Alkoxy, C₁-C₂-Halogenalkoxy, C₁-C₂-Halogenalkylthio, Halogen, CN oder NO₂ ist;
Y NR⁷C(O)XR³ ist;
X eine Einfachbindung, O, S oder NR⁴ ist;
R¹ H, C₁-C₃-Alkyl, C₁-C₃-Alkoxy, C₁-C₃-Halogenalkoxy, C₁-C₃-Alkylthio, C₂-C₃-Alkinyl, C₂-C₃-Alkoxyalkyl, C₂-C₃-Alkylthioalkyl, Halogen, NO₂, CN, NHR⁵ oder NR⁵R⁶ ist;
R³ C₁-C₅-Alkyl, gegebenenfalls mit C₁-C₂-Alkoxy, OH, 1-3 Halogenen, OC(O)(C₁-C₂-Alkyl) oder C₁-C₂-Alkylthio substituiert; CH₂(C₃-C₄-Cycloalkyl); C₃-C₄-Cycloalkyl, gegebenenfalls mit 1-3 CH₃-Gruppen, 1-2 F-Atomen oder 1-2 Cl-Atomen substituiert; C₂-C₄-Alkenyl; C₂-C₄-Halogenalkenyl; C₃-C₄-Alkinyl; C(O)(C₁-C₂-Alkyl); Benzyl oder ein 5- oder 6-gliedriger heterocyclischer Ring, der ein Heteroatom, ausgewählt aus O, N und S enthält, wobei jeder Ring gegebenenfalls mit Halogen oder CH₃ substituiert ist, ist;
R⁴ H, C₁-C₂-Alkyl oder OCH₃ ist;
R⁵ und R⁶ unabhängig voneinander C₁-C₂-Alkyl sind;
R⁷ H, C₁-C₂-Alkyl oder C(O)R⁸ ist;
R⁸ C₁-C₅-Alkyl, gegebenenfalls mit C₁-C₂-Alkoxy, OH, 1-3 Halogenen, OC(O)(C₁-C₂-Alkyl) oder C₁-C₂-Alkylthio substituiert; CH₂(C₃-C₄-Cycloalkyl); C₃-C₄-Cycloalkyl, gegebenenfalls mit 1-3 CH₃-Gruppen, 1-2 F-Atomen oder 1-2 Cl-Atomen substituiert; C₂-C₄-Alkenyl; C₂-C₄-Halogenalkenyl; C₃-C₄-Alkinyl; C(O)(C₁-C₂-Alkyl); Benzyl oder ein 5- oder 6-gliedriger heterocyclischer Ring, der ein Heteroatom, ausgewählt aus O, N und S enthält, wobei jeder Ring gegebenenfalls mit Halogen oder CH₃ substituiert ist, ist; und
n 0 oder 1 ist;
vorausgesetzt, daß X eine Einfachbindung ist, wenn R³ C₂-Alkenyl, C₂-Halogenalkenyl oder ein heterocyclischer Ring ist, und R anders als Br oder F ist, wenn Q Q-1 ist, R¹ H ist, und Y NHC(O)CH₃ ist.

## Revendications

1. Un procédé pour combattre la croissance de végétation indésirable par application au site à protéger d'une quantité efficace d'un composé de Formule I : 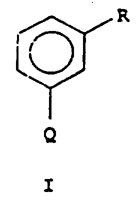 où
Q est 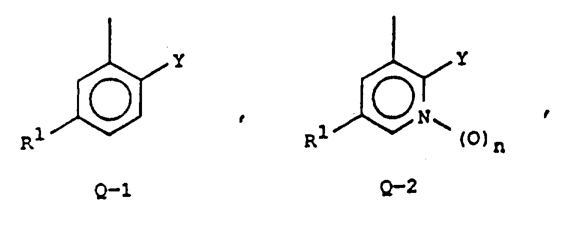 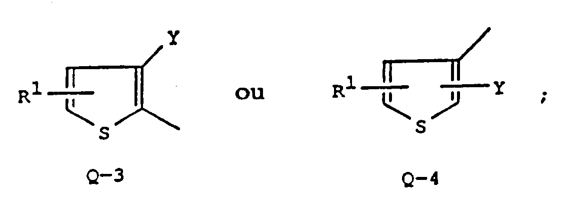
R est H, un groupe alkyle en C₁-C₂, halogénoalkyle en C₁-C₂, alcoxy en C₁-C₂, halogénoalcoxy en C₁-C₂, halogénoalkylthio en C₁-C₂, halogéno, CN ou NO₂ ;
Y est NR⁷C(O)XR³;
X est une liaison simple, O, S ou NR⁴ ;
R¹ est H, un groupe alkyle en C₁-C₃, alcoxy en C₁-C₃, halogénoalcoxy en C₁-C₃, alkylthio en C₁-C₃, alcynyle en C₂-C₃, alcoxyalkyle en C₂-C₃, alkylthioalkyle en C₂-C₃, halogéno, NO₂, CN, NHR⁵ ou NR⁵R⁶ ;
R³ est un groupe alkyle en C₁-C₅ facultativement substitué par alcoxy en C₁-C₂, OH, 1 à 3 atomes d'halogène, OC(O)(alkyle en C₁-C₂) ou alkylthio en C₁-C₂ ; CH₂(cycloalkyle en C₃-C₄) ; cycloalkyle en C₃-C₄ facultativement substitué par 1 à 3 CH₃, 1 à 2 F ou 1 à 2 Cl ; alcényle en C₂-C₄ ; halogénoalcényle en C₂-C₄ ; alcynyle en C₃-C₄ ; C(O)(alkyle en C₁- C₂) ; benzyle ; ou un hétérocycle penta- ou hexagonal contenant un seul hétéroatome choisi parmi O, N et S, chaque cycle étant facultativement substitué par un halogène ou CH₃ ;
R⁴ est H, un groupe alkyle en C₁-C₂ ou OCH₃ ;
R⁵ et R⁶ sont indépendamment un groupe alkyle en C₁-C₂ ;
R⁷ est H, un groupe alkyle en C₁-C₂ ou C(O)R⁸ ;
R⁸ est un groupe alkyle en C₁-C₅ facultativement substitué par alcoxy en C₁-C₂, OH, 1 à 3 atomes d'halogène, OC(O)(alkyle en C₁-C₂) ou alkylthio en C₁-C₂ ; CH₂(cycloalkyle en C₃-C₄) ; cycloalkyle en C₃-C₄ facultativement substitué par 1 à 3 CH₃, 1 à 2 F ou 1 à 2 Cl ; alcényle en C₂-C₄ ; halogénoalcényle en C₂-C₄ ; alcynyle en C₃-C₄ ; C(O) (alkyle en C₁-C₂) ; benzyle ; ou un hétérocycle penta- ou hexagonal contenant un seul hétéroatome choisi parmi O, N et S, chaque cycle étant facultativement substitué par un halogène ou CH₃ ; et
n est 0 ou 1 ;
avec la condition que si R³ est un groupe alcényle en C₂, un groupe halogénoalcényle en C₂ ou un hétérocycle, alors X soit une liaison simple.

2. Le procédé de la revendication 1, dans lequel R est un groupe halogénoalkyle en C₁-C₂, halogénoalcoxy en C₁-C₂, halogénoalkylthio en C₁-C₂, halogéno ou CN.

3. Le procédé de la revendication 2, dans lequel R¹ est un groupe alkyle en C₁-C₂, alcoxy en C₁-C₂, alkylthio en C₁-C₂, alcoxyalkyle en C₂-C₃, alkylthioalkyle en C₂-C₃, Cl, Br, CN, NHR⁵ ou NR⁵R⁶ ; et R⁷ est H.

4. Le procédé de la revendication 3, dans lequel
Q est Q-1 ou Q-2 ; et
R³ est un groupe alkyle en C₁-C₄ facultativement substitué par un groupe alcoxy en C₁-C₂ ; CH₂(cycloalkyle en C₃-C₄) ; cycloalkyle en C₃-C₄ facultativement substitué par 1 à 3 CH₃ ; ou alcényle en C₂-C₄.

5. Le procédé de la revendication 4, dans lequel R est un groupe fluoroalkyle en C₁-C₂, fluoroalcoxy en C₁-C₂, fluoroalkylthio en C₁-C₂, Cl, Br ou CN.

6. Le procédé de la revendication 4, dans lequel la Formule I est le [5-méthyl-3'-(trifluorométhyl)[1,1'-biphényle]-2-yl]carbamate de 1-méthyléthyle.

7. Le procédé de la revendication 4, dans lequel la Formule I est le 3-méthyl-N-[5-méthyl-3'-(trifluorométhyl)[1,1'-biphényle]-2-yl]butanamide.

8. Une composition utilisable en agriculture pour combattre la croissance de végétation indésirable, comprenant une quantité efficace d'un composé de l'une quelconque des revendications 1 à 7, avec la condition supplémentaire que si Q est Q-1, R¹ est H et Y est NHC(O)CH₃, alors R soit autre chose que Br ou F, et au moins l'un des ingrédients suivants : agent tensio-actif, diluant solide ou liquide.

9. Un composé de la Formule I 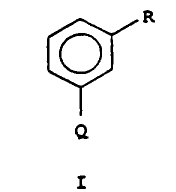 où
Q est 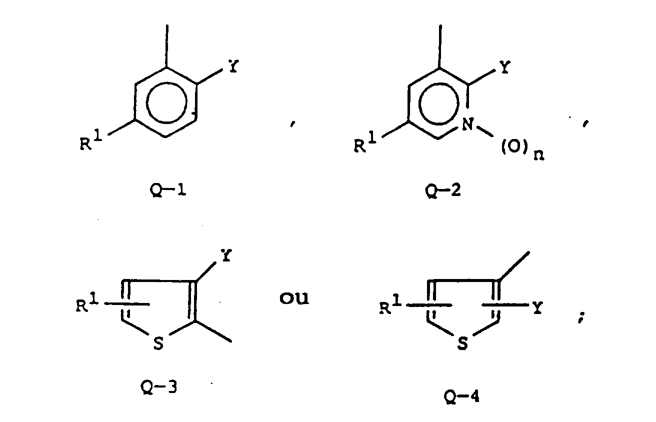
R est un groupe alkyle en C₁-C₂, halogénoalkyle en C₁-C₂, alcoxy en C₁-C₂, halogénoalcoxy en C₁-C₂, halogénoalkylthio en C₁-C₂, halogéno, CN ou NO₂ ;
Y est NR⁷C(O)XR³;
X est une liaison simple, O, S ou NR⁴ ;
R¹ est H, un groupe alkyle en C₁-C₃, alcoxy en C₁-C₃, halogénoalcoxy en C₁-C₃, alkylthio en C₁-C₃, alcynyle en C₂-C₃, alcoxyalkyle en C₂-C₃, alkylthioalkyle en C₂-C₃, halogéno, NO₂, CN, NHR⁵ ou NR⁵R⁶ ;
R³ est un groupe alkyle en C₁-C₅ facultativement substitué par alcoxy en C₁-C₂, OH, 1 à 3 atomes d'halogène, OC(O)(alkyle en C₁-C₂) ou alkylthio en C₁-C₂ ; CH₂(cycloalkyle en C₃-C₄) ; cycloalkyle en C₃-C₄ facultativement substitué par 1 à 3 CH₃, 1 à 2 F ou 1 à 2 Cl ; alcényle en C₂-C₄ ; halogénoalcényle en C₂-C₄ ; alcynyle en C₃-C₄ ; C(O) (alkyle en C₁-C₂) ; benzyle ; ou un hétérocycle penta- ou hexagonal contenant un seul hétéroatome choisi parmi O, N et S, chaque cycle étant facultativement substitué par un halogène ou CH₃ ;
R⁴ est H, un groupe alkyle en C₁-C₂ ou OCH₃ ;
R⁵ et R⁶ sont indépendamment un groupe alkyle en C₁-C₂ ;
R⁷ est H, un groupe alkyle en C₁-C₂ ou C(O)R⁸ ;
R⁸ est un groupe alkyle en C₁-C₅ facultativement substitué par alcoxy en C₁-C₂, OH, 1 à 3 atomes d'halogène, OC(O)(alkyle en C₁-C₂) ou alkylthio en C₁-C₂ ; CH₂(cycloalkyle en C₃-C₄) ; cycloalkyle en C₃-C₄ facultativement substitué par 1 à 3 CH₃, 1 à 2 F ou 1 à 2 Cl ; alcényle en C₂-C₄ ; halogénoalcényle en C₂-C₄ ; alcynyle en C₃-C₄ ; C(O) (alkyle en C₁-C₂) ; benzyle ; ou un hétérocycle penta- ou hexagonal contenant un seul hétéroatome choisi parmi O, N et S, chaque cycle étant facultativement substitué par un halogène ou CH₃ ; et
n est 0 ou 1 ;
avec les conditions que si R³ est un groupe alcényle en C₂, un groupe halogénoalcényle en C₂ ou un hétérocycle, alors X soit une liaison simple, et si Q est Q-1, R¹ est H et Y est NHC(O)CH₃, alors R soit autre chose que Br ou F.
